# EUROPEAN PATENT APPLICATION

(11) **EP 4 421 181 A2**
(43) Date of publication of application: **28.08.2024**
(21) Application number: 24162808.0
(22) Date of filing: 18.09.2015
(51) Int. Cl.: C12P 7/16

(54) **NON-NATURAL MICROBIAL ORGANISMS WITH IMPROVED ENERGETIC EFFICIENCY**

(30) Priority: 18.09.2014 US 201462052341 P
(62) Divisional of application: 20159174.0
(71) Applicant: Genomatica, Inc., San Diego CA 92121 (US)
(72) Inventor: PHARKYA, Priti, San Diego, 92121 (US); BURGARD, Anthony P., San Diego, 92121 (US); NUNEZ VAN NAME, Eric Roland, San Diego, 92121 (US)
(74) Representative: Schiweck Weinzierl Koch Patentanwälte Partnerschaft mbB

(57) **Abstract**

The invention provides non-natural microbial organisms containing enzymatic pathways and/or metabolic modifications for enhancing carbon flux through acetyl-CoA, or oxaloacetate and acetyl-CoA. Embodiments of the invention include microbial organisms having a pathway to acetyl-CoA and oxaloacetate that includes phosphoketolase (a PK pathway). The organisms also have either (i) a genetic modification that enhances the activity of the non-phosphotransferase system (non-PTS) for sugar uptake, and/or (ii) a genetic modification(s) to the organism's electron transport chain (ETC) that enhances efficiency of ATP production, that enhances availability of reducing equivalents or both. The microbial organisms can optionally include (iii) a genetic modification that maintains, attenuates, or eliminates the activity of a phosphotransferase system (PTS) for sugar uptake. The enhanced carbon flux through acetyl-CoA and oxaloacetate can be used for production of a bioderived compound, and the microbial organisms can further include a pathway capable of producing the bioderived compound.

## Description

### PRIORITY CLAIM

This application claims the benefit of U.S. Provisional Patent Application Serial Number 62/052,341 filed September 18, 2014, entitled NON-NATURAL MICROBIAL ORGANISMS WITH IMPROVED ENERGETIC EFFICIENCY, the disclosure of which is incorporated herein by reference.

### SUMMARY OF INVENTION

The invention provides non-natural microbial organisms containing enzymatic pathways for enhancing carbon flux to acetyl-CoA, or oxaloacetate and acetyl-CoA, and methods for their use to produce bio-products, and bio-products made using such microbial organisms.

Generally, microbial organisms are provided that make acetyl-CoA, or oxaloacetate and acetyl-CoA, have a phosphoketolase pathway (PK pathway) and has (i) a genetic modification that enhances the activity of the non-phosphotransferase system (non-PTS) for sugar uptake, and/or (ii) a genetic modification(s) to the organism's electron transport chain (ETC) that enhances efficiency of ATP production, that enhances availability of reducing equivalents or both. The modifications enhance energetic efficiency of the modified microbial organism. Optionally, the organism can include (iii) a genetic modification that maintains, attenuates, or eliminates the activity of a phosphotransferase system (PTS) for sugar uptake.

Through experimental studies associated with the current disclosure, it has been discovered that the PK pathway in combination with (i) and/or (ii), and optionally (iii) enhanced carbon flux through acetyl-CoA, or both oxaloacetate and acctyl-CoA. In turn, this increased the pool of acetyl-CoA and oxaloacetate useful for enhancing the production of a desired product or its intermediate (a bioderived compound) while advantageously minimizing undesired compounds. Therefore, the non-natural microbial organisms containing enzymatic pathways for enhancing carbon flux through acctyl-CoA, or both oxaloacetate and acetyl-CoA, with the modifications as described herein can increase the production of intermediates or products such as alcohols (e.g., propanediol or a butanediol), glycols, organic acids, alkenes, dienes (e.g., butadiene), isoprenoids (e.g. isoprene), organic amines, organic aldehydes, vitamins, nutraceuticals, and pharmaceuticals.

Therefore, in one aspect (e.g., a first aspect) the invention provides a non-natural microbial organism that includes (a) a pathway to acetyl-CoA, or both oxaloacetate and acetyl-CoA, comprising a phosphoketolase pathway, and (b) a genetic modification that increases non-PTS activity for sugar uptake. Optionally, the organism can include (c) a genetic modification that maintains, attenuates, or eliminates a PTS activity for sugar uptake. The genetic modification includes those that change an enzyme or protein of the PTS or non-PTS, its activity, a gene-encoding that enzyme or protein, or the gene's expression. The organism can also have a pathway to a bioderived compound, and a modification to the non-PTS to increase non-PTS activity that improves production of the bioderived compound via improvements in synthesis of acetyl-CoA, or both oxaloacetate and acetyl-CoA, which serve as intermediates. Modification to the non-PTS can balance the fluxes from phosphoenolpyruvate (PEP) into oxaloacetate and pyruvate, which offers an improvement over organisms that rely on an endogenous PTS system for sugar uptake, and which can advantageously lead into the bioderived compound pathway.

The PTS and non-PTS can allow for uptake of primarily C5, C6 or C12 sugars and their oligomers. Non-natural microbial organism having a PTS for sugar (e.g., C6, C12, sugar alcohols, and amino sugars) uptake are able to phosphorylate sugars by conversion of PEP into pyruvate.. The non-PTS allows for uptake of sugars via a facilitator or a permease and subsequent phosphorylation via a kinase. The non-PTS further allows uptake of C5 sugars such as xylose, disaccharides such as lactose, melibiose, and maltose. Other substrates such as ascorbate may be recognized by a specific PTS or non-PTS enzyme or protein. Phosphorylated sugar then goes through the majority of reactions in glycolysis to generate reducing equivalents and ATP that are associated with the organism's electron transport chain (ETC).

Illustrative PK pathways, can include the following enzymes:
both fructose-6-phosphate phosphoketolase (1T) and a phosphotransacetylase (1V);
all three of fructose-6-phosphate phosphoketolase (1T), an acetate kinase (1W), and an acetyl-CoA transferase, an acetyl-CoA synthetase, or an acetyl-CoA ligase (1X);
both xylulose-5-phosphate phosphoketolase (1U) and a phosphotransacctylasc (1V); or
all three of xylulose-5-phosphate phosphoketolase (1U), an acetate kinase (1W), and an acetyl-CoA transferase, an acetyl-CoA synthetase, or an acetyl-CoA ligase (1X).

A non-natural microbial organism of the first aspect with the (a) a pathway to oxaloacetate, acetyl-CoA or both, comprising a phosphoketolase, and (b) a genetic modification to enhance non-PTS activity, can optionally further include one or more modification(s) to the organism's electron transport chain to enhance efficiency of ATP production" to enhance availability of reducing equivalents, or both.

In another aspect (e.g., a second aspect) the invention provides a non-natural microbial organism that includes (a) a pathway to oxaloacetate, acetyl-CoA or both, comprising a phosphoketolase pathway, and (b) one or more modification(s) to the organism's electron transport chain to enhance efficiency of ATP production, to enhance availability of reducing equivalents, or both. For example, modifications as described herein that increase the number of protons translocated per electron pair that reaches cytochrome oxidases or complex IV of the electron transport chain provide increased protons that are used by ATP synthase to produce ATP. Consequently, by increasing the amount of ATP generated per pair of electrons channeled through the electron transport chain, the energetic efficiency (also referred to as P/O ratio) of the cell is increased. Similarly, modifications that attenuate or eliminate NADH dehydrogenases that do not transport or inefficiently transport protons increases the NADH pool available for the more efficient NADH dehydrogenases, e.g. nuo. Again, the energetic efficiency of the cell is increased.

Organisms of the second aspect may include a pathway for assimilation of an alternate carbon source (e.g., methanol, syngas, glycerol, formate, methane), for example, if the PTS and non-PTS are modified, not present in the organism, or otherwise do not provide the desired influx of a hydrocarbon energy source. Accordingly, organisms making oxaloacetate and/or acetyl-CoA and that contain a phosphoketolase pathway can also comprise a pathway for using non-sugar carbon substrates such as glycerol, syngas, formate, methane and methanol.

Modifications that enhance the organism's ETC function include attenuation or elimination of expression or activity of an enzyme or protein that competes with efficient electron transport chain function. Examples are attenuation or elimination of NADH-dehydrogenases that do not translocate protons or attenuation or elimination of cytochrome oxidases that have lower efficiency of proton translocation per pair of electrons. ETC modifications also include enhancing function of an enzyme or protein of the organism's ETC, particularly when such a function is rate-limiting. Examples in bacteria of modifications that enhance an enzymes or protein are increasing activity of an enzyme or protein of Complex I of the ETC and attenuating or eliminating the global negative regulatory factor arcA.

Microbial organisms having a PK pathway can also synthesize succinyl-CoA subsequent to the synthesis of acetyl-CoA and oxaloacetate, and succinyl-CoA can further be used in a product pathway to a bioderived compound. Oxaloacetate is produced anaplerotically from phosphoenolpyruvate or from pyruvate. Succinyl-CoA is produced either by oxidative TCA cycle whereby both acetyl-CoA and oxaloacetate are used as precursors, via the reductive TCA cycle where oxaloacetate is used as the precursor or by a combination of both oxidative and reductive TCA branches. Microbial organisms having a PK pathway can optionally further include increased activity of one or more enzymes that can enable higher flux into oxaloacetate which, when combined with acetyl-CoA, leads to higher flux through oxidative TCA and the products derived therefrom, or increased flux for producing succinyl-CoA via the reductive TCA branch. Examples of enzymes that can have increased activity in the cells include PEP synthetase, pyruvate carboxylase, and phosphoenolpyruvate carboxylase, which can be present in the microbial organisms of the first or second aspect.

Optionally, organisms having a PK pathway can further include attenuation or elimination of one or more endogenous enzymes in order to further enhance carbon flux through acetyl-CoA, or both acetyl-CoA and oxaloacetate, or a gene disruption of one or more endogenous nucleic acids encoding such enzymes. For example, the attenuated or eliminated endogenous enzyme could be one of the isozymes of pyruvate kinase, and its deletion can be used in microbial organisms of the first or second aspect or both.

The enhanced carbon flux through acetyl-CoA, or both oxaloacetate and acetyl-CoA, in the microbial organisms described herein can be used for production of a bioderived compound. Accordingly, in further aspects, the microbial organism can further include a pathway capable of producing a desired bioderived compound. That is, the microbial organism of the first or second aspect can further include one or more pathway enzyme(s) that promote production of the bioderived compound.

Bioderived compounds include alcohols, glycols, organic acids, alkenes, dienes, isoprenoids, olefins, organic amines, organic aldehydes, vitamins, nutraceuticals and pharmaceuticals. In some embodiments, the bioderived compound is 1,3-butanediol, crotyl alcohol, butadiene, 3-buten-2-ol, 1,4-butanediol, adipate, 6-aminocaproate, caprolactam, hexamethylenediamine, propylene, isoprene, methacrylic acid, 2-hydroxyisobutyric acid, or an intermediate thereto. One or more pathway enzyme(s) can utilize enhanced carbon flux through acetyl-CoA, or both oxaloacetate and acetyl-CoA, as precursor promoting the production of the bioderived compound.

### BRIEF DESCRIPTION OF THE FIGURES

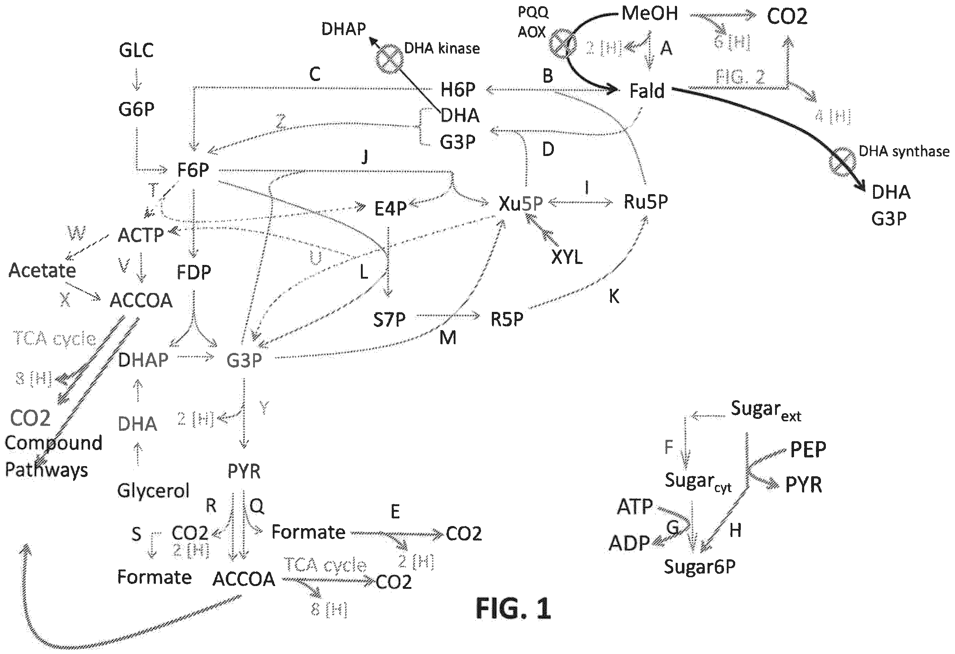

Compounds as described **Figures 1 - 14** are abbreviated as follows. MeOH or MEOH = methanol; Fald = formaldehyde; GLC = glucose; G6P = glucose-6-phosphate; H6P = hexulose-6-phosphate; F6P = fructose-6-phosphate; FDP = fructose diphosphate or fructose-1,6-diphosphate; 13DPG: 1,3-diphosphoglycerate, 3PG= 3-phosphoglycerate, 2PG= 2-phosphoglycerate, DHA = dihydroxyacetone; DHAP = dihydroxyacetone phosphate; G3P = glyceraldehyde-3-phosphate; PEP: phosphoenolpyruvate, PYR = pyruvate; ACTP = acetyl-phosphate; ACCOA = acetyl-CoA; AACOA = acetoacetyl-CoA; MALCOA = malonyl-CoA; E4P = erythrose-4-phosphate: Xu5P = xyulose-5-phosphate; Ru5P = ribulose-5-phosphate; S7P = sedoheptulose-7-phosphate: R5P = ribose-5-phosphate; XYL = xylose; TCA = tricarboxylic acid; PEP = Phosphoenolpyruvate; OAA = Oxaloacetate; MAL = malate; CIT = citrate; ICIT = isocitrate; AKG = alpha-ketoglutarate; FUM = Fumarate; SUCC = Succinate; SUCCOA = Succinyl-CoA; 3HBCOA = 3-hydroxybutyryl-CoA; 3-HB = 3-hydroxybutyrate; 3HBALD = 3-hydroxybutyraldehyde; 13BDO = 1,3-butanediol; CROTCOA = crotonyl-CoA; CROT = crotonate; CROTALD = crotonaldehyde; CROTALC = crotyl alcohol; CROT-Pi = crotyl phosphate; CROT-PPi = crotyl diphosphate or 2-butenyl-4-diphosphate.

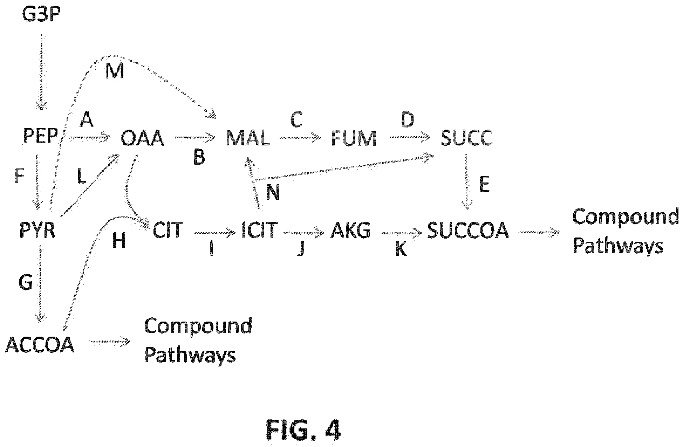

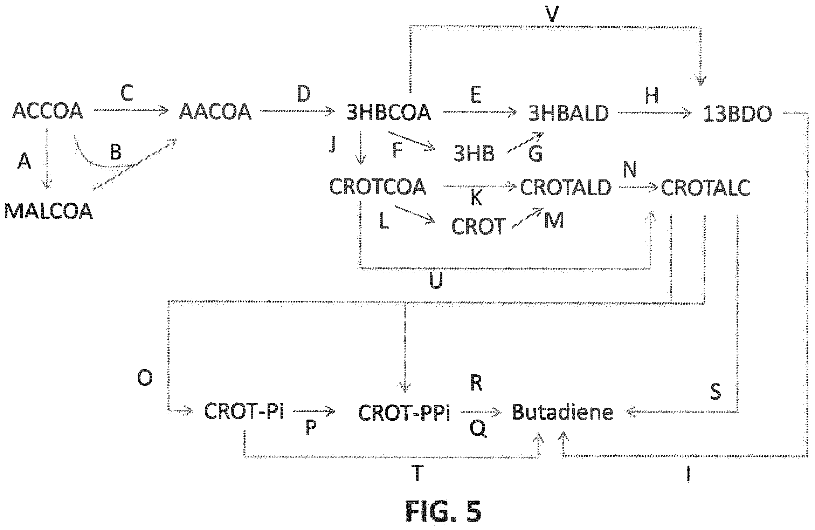

**Figure 1** shows exemplary metabolic pathways enabling the conversion of exemplary PTS and non-PTS sugars such as glucose (GLC) and xylose (XYL) to acetyl-CoA (ACCOA) as well as the pathways for assimilation of other carbon sources such as methanol and glycerol to form acetyl-CoA. Arrows with alphabetical designations represent enzymatic transformations of a precursor compound to an intermediate compound. Enzymatic transformations shown are carried out by the following enzymes: A) methanol dehydrogenase, B) 3-hexulose-6-phosphatc synthase, C) 6-phospho-3-hcxuloisomcrasc, D) dihydroxyacetone synthase, E) formate dehydrogenase (NAD or NADP-dependent), F) sugar permease or facilitator protein (non-PTS), G) sugar kinase (non-pts), H) PTS system of sugar transport, I) ribulose-5-phosphate-3-epimerase, J) transketolase, K) ribulose-5-phosphate isomerase, L) transaldolasc, M) transketolase, Q) pyruvate formate lyase, R) pyruvate dehydrogenase, pyruvate ferredoxin oxidoreductase, or pyruvate:NADP+ oxidoreductase, S) formate dehydrogenase, T) fructose-6-phosphate phosphoketolase, U) xylulose-5-phosphate phosphoketolase, V) phosphotransacetylase, W) acetate kinase, X) acetyl-CoA transferase, synthetase, or ligase, Y) lower glycolysis including glyceraldehyde-3-phosphate dehydrogenase, Z) fructose-6-phosphate aldolase. **Figure 1** also shows exemplary endogenous enzyme targets for optional attenuation or disruption. The endogenous enzyme targets include DHA kinase, methanol oxidase (AOX), PQQ-dcpcndcnt methanol dehydrogenase (PQQ) and/or DHA synthase. **Figure 1** also shows acetyl-CoA can be led into an into another "intermediate pathway" as depicted in **Figure 4****,** or into "compound pathways" (bioderived compound pathways), such as those depicted in **Figures 5-11****.**

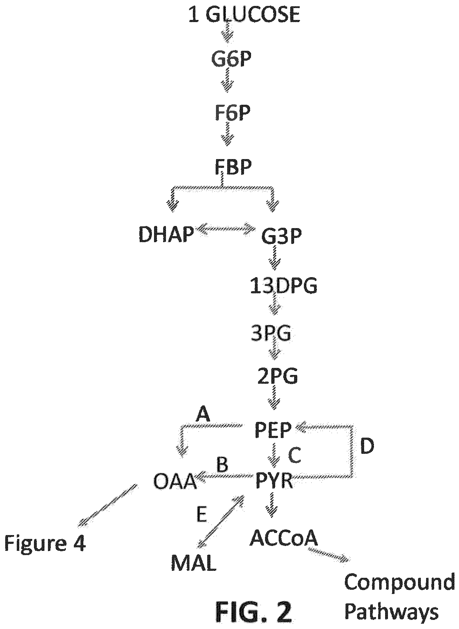

**Figure 2** shows pathways that enable formation of oxaloacetate. The enzymatic transformations are: A) PEP Carboxylase, B) Pyruvate carboxylase, C) Pyruvate kinase and D) PEP synthetase, E) Malic enzyme

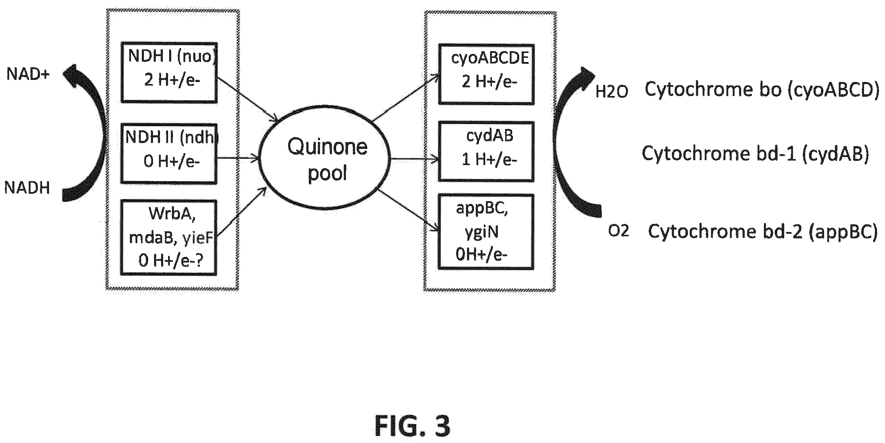

**Figure 3** shows various enzymes and proteins (components) of the electron transport chain (ETC). As an example, the ETC of E. coli is shown. NADH dehydrogenases form the Complex I of the electron transport chain and transfer electrons to the quinone pool. Components of the ETC that do not translocate protons are targets for attenuation or elimination of expression or activity in the non-natural microbial organisms in order to increase efficiency of ATP production . Cytochrome oxidases receive electrons from the quinone pool and reduce oxygen. Cytochrome oxidases that do no translocate protons or reduce lower number of protons per pair of electrons are targets for attenuation or elimination of expression or activity in the non-natural microbial organisms for increasing efficiency of ATP production in the cells.
**Figure 4** shows exemplary metabolic pathways enabling the conversion of the glycolysis intermediate glyccraldchyc-3-phosphatc (G3P) to acetyl-CoA (ACCOA) and/or succinyl-CoA (SUCCOA). The enzymatic transformations shown can be carried out by the following enzymes: A) PEP carboxylase or PEP carboxykinase, B) malate dehydrogenase, C) fumarase, D) fumarate reductase, E) succinyl-CoA synthetase or transferase, F) pyruvate kinase or PTS-dependent substrate import, G) pyruvate dehydrogenase, pyruvate formate lyase, or pyruvate:ferredoxin oxidoreductase, H) citrate synthase, I) aconitase, J) isocitrate dehydrogenase, K) alpha-ketoglutarate dehydrogenase, L) pyruvate carboxylase, M) malic enzyme, N) isocitrate lyase and malate synthase.

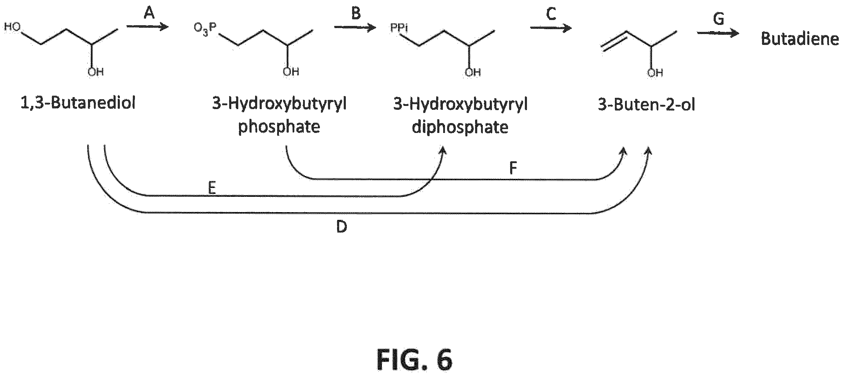

**Figure 5** shows exemplary pathways enabling production of 1,3-butanediol, crotyl alcohol, and butadiene from acetyl-CoA. The 1,3-butanediol, crotyl alcohol, and butadiene production can be carried out by the following enzymes: A) acetyl-CoA carboxylase, B) an acetoacetyl-CoA synthase, C) an acetyl-CoA:acetyl-CoA acyltransferase, D) an acetoacetyl-CoA reductase (ketone reducing), E) a 3-hydroxybutyryl-CoA reductase (aldehyde forming), F) a 3-hydroxybutyryl-CoA hydrolase, transferase or synthetase, G) a 3-hydroxybutyrate reductase, H) a 3-hydroxybutyraldchydc reductase, I) chemical dehydration or Figure 6, J) a 3-hydroxybutyryl-CoA dehydratase, K) a crotonyl-CoA reductase (aldehyde forming), L) a crotonyl-CoA hydrolase, transferase or synthetase, M) a crotonate reductase, N) a crotonaldehyde reductase, O) a crotyl alcohol kinase, P) a 2-butenyl-4-phosphate kinase, Q) a butadiene synthase, R) a crotyl alcohol diphosphokinasc, S) chemical dehydration or a crotyl alcohol dehydratase, T) a butadiene synthase (monophosphate), T) a butadiene synthase (monophosphate), U) a crotonyl-CoA reductase (alcohol forming), and V) a 3-hydroxybutyryl-CoA reductase (alcohol forming).
**Figure 6** shows exemplary pathways for converting 1,3-butanediol to 3-buten-2-ol and/or butadiene. The 3-buten-2-ol and butadiene production can be carried out by the following enzymes: A. 1,3-butanediol kinase, B. 3-hydroxybutyrylphosphate kinase, C. 3-hydroxybutyryldiphosphate lyase, D. 1,3-butanediol diphosphokinase, E. 1,3-butanediol dehydratase, F. 3-hydroxybutyrylphosphate lyase, G. 3-buten-2-ol dehydratase or chemical dehydration.

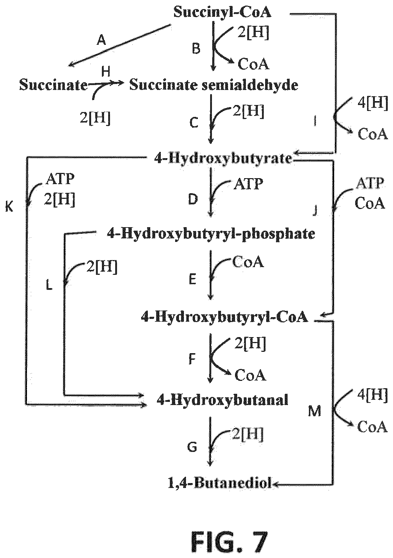

**Figure 7** shows exemplary pathways enabling production of 1,4-butancdiol from succinyl-CoA. The 1,4-butanediol production can be carried out by the following enzymes: A) a succinyl-CoA transferase or a succinyl-CoA synthetase, B) a succinyl-CoA reductase (aldehyde forming), C) a 4-HB dehydrogenase, D) a 4-HB kinase, E) a phosphotrans-4-hydroxybutyrylase, F) a 4-hydroxybutyryl-CoA reductase (aldehyde forming), G) a 1,4-butanediol dehydrogenase, H) a succinate reductase, I) a succinyl-CoA reductase (alcohol forming), J) a 4-hydroxybutyryl-CoA transferase or 4-hydroxybutyryl-CoA synthetase, K) a 4-HB reductase, L) a 4-hydroxybutyryl-phosphatc reductase, and M) a 4-hydroxybutyryl-CoA reductase (alcohol forming).

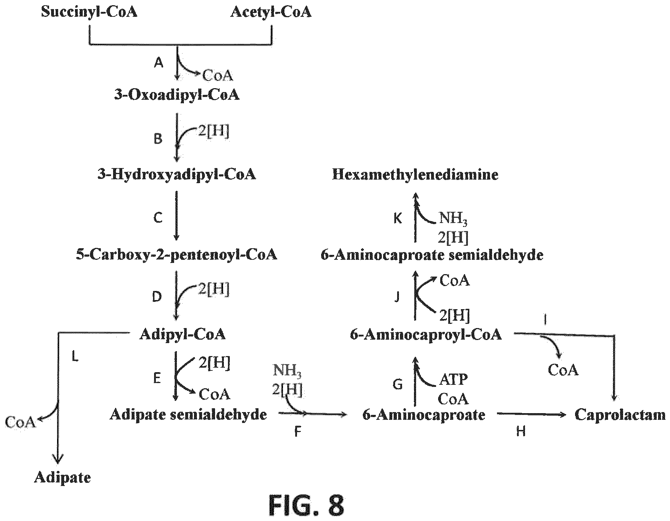

**Figure 8** shows exemplary pathways enabling production of adipate, 6-aminocaproic acid, caprolactam, and hexamethylenediamine from succinyl-CoA and acetyl-CoA. Adipate, 6-aminocaproic acid, caprolactam, and hexamethylenediamine production can be carried out by the following enzymes: A) 3-oxoadipyl-CoA thiolase, B) 3-oxoadipyl-CoA reductase, C) 3-hydroxyadipyl-CoA dehydratase, D) 5-carboxy-2-pentenoyl-CoA reductase, E) adipyl-CoA reductase (aldehyde forming), F) 6-aminocaproatc transaminase or 6-aminocaproatc dehydrogenase, G) 6-aminocaproyl-CoA/acyl-CoA transferase or 6-aminocaproyl-CoA synthase, H) amidohydrolase, T) spontaneous cyclization, J) 6-aminocaproyl-CoA reductase (aldehyde forming), K) HMDA transaminase or HMDA dehydrogenase, L) Adipyl-CoA hydrolase, adipyl-CoA ligase, adipyl-CoA transferase, or phosphotransadipylase/adipate kinase.

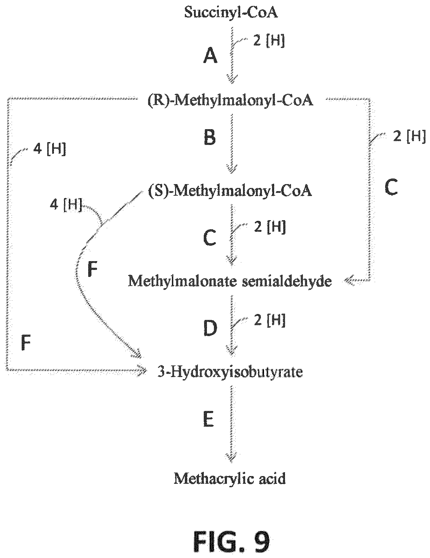

**Figure 9** shows exemplary pathways enabling production of 3-hydroxyisobutyrate and methacrylic acid from succinyl-CoA. 3-Hydroxyisobutyrate and methacrylic acid production arc carried out by the following enzymes: A) Methylmalonyl-CoA mutase, B) Methylmalonyl-CoA epimerase, C) Methylmalonyl-CoA reductase (aldehyde forming), D) Methylmalonate semialdehyde reductase, E) 3-hydroxyisobutyrate dehydratase, F) Methylmalonyl-CoA reductase (alcohol forming).

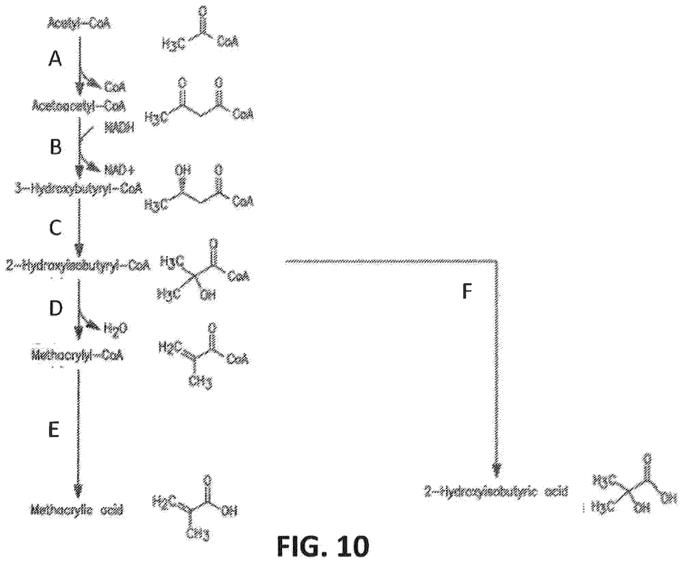

**Figure 10** shows exemplary pathways enabling production of 2-hydroxyisobutyrate and methacrylic acid from acetyl-CoA. 2-Hydroxyisobutyrate and methacrylic acid production can be carried out by the following enzymes: A) acctyl-CoA:acetyl-CoA acyltransferase, B) acetoacetyl-CoA reductase (ketone reducing), C) 3-hydroxybutyrl-CoA mutase, D) 2-hydroxyisobutyryl-CoA dehydratase, E) methacrylyl-CoA synthetase, hydrolase, or transferase, F) 2-hydroxyisobutyryl-CoA synthetase, hydrolase, or transferase.

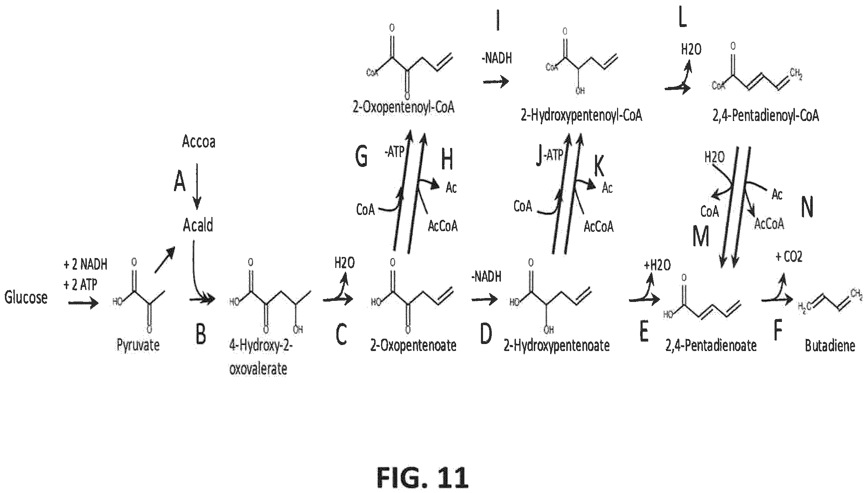

**Figure 11** shows exemplary pathways enabling production of 2,4-pentadieonate (2,4PD)/butadiene from acetyl-coA. The following enzymes can be used for 2,4-PD/butadiene production. Enzyme names: A. Acetaldehyde dehydrogenase, B. 4-hydroxy 2-oxovalerate aldolase, C. 4-hydroxy 2-oxovalerate dehydratase, D. 2-oxopentenoate reductase, E. 2-hydroxypentenoate dehydratase, F. 2,4-pentadienoate decarboxylase, G. 2-oxopentenoate ligase, H. 2-oxopentenoate: acetyl CoA transferase, I. 2-oxopentenoyl-CoA reductase, J. 2-hydroxypentenoate ligase, K. 2-hydroxypentenoate: acetyl-CoA CoA transferase, L. 2-hydroxypentenoyl-CoA dehydratase, M. 2,4-Pcntadicnoyl-CoA hydrolase, N. 2,4-Pentadienoyl-CoA: acetyl CoA transferase

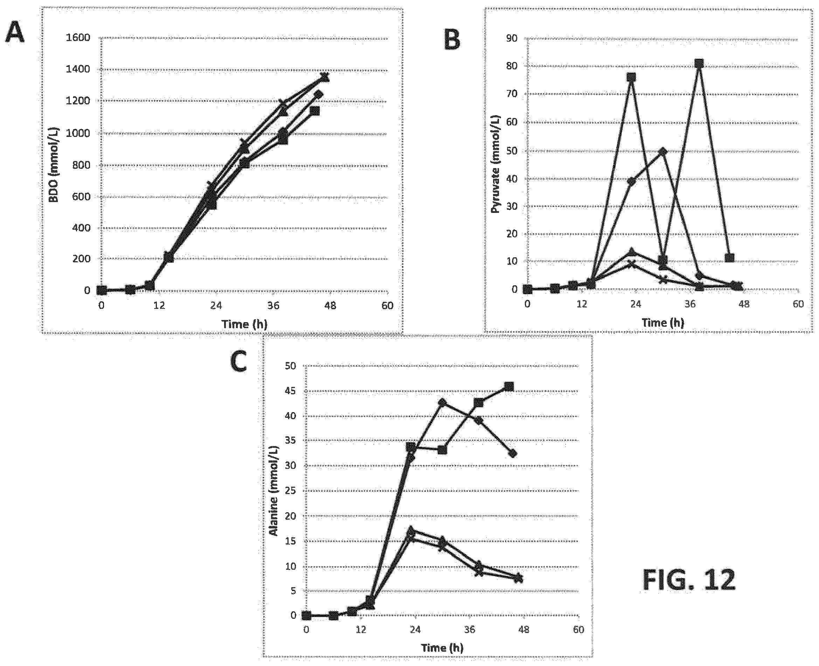

**Figures 12A-C** show the increase in BDO titers and reduction in C3 byproducts such as alanine and pyruvate when PK is expressed in a strain that employs both the PTS and the Non-PTS system of glucose transport. The diamond and the square symbols represent the fermentation runs where PK was not expressed. The crosses and the triangles represent the fermentation runs where PK was expressed with a p115 promoter.

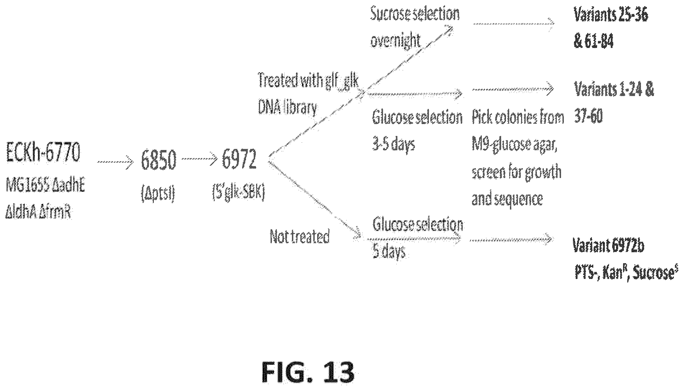

**Figure 13** illustrates steps in the construction expression mutants of native glk and Zymomonas mobilis glf that were inserted into the PTS- cells and selection of those mutants that had an improved growth rate on glucose as described in Example 1.

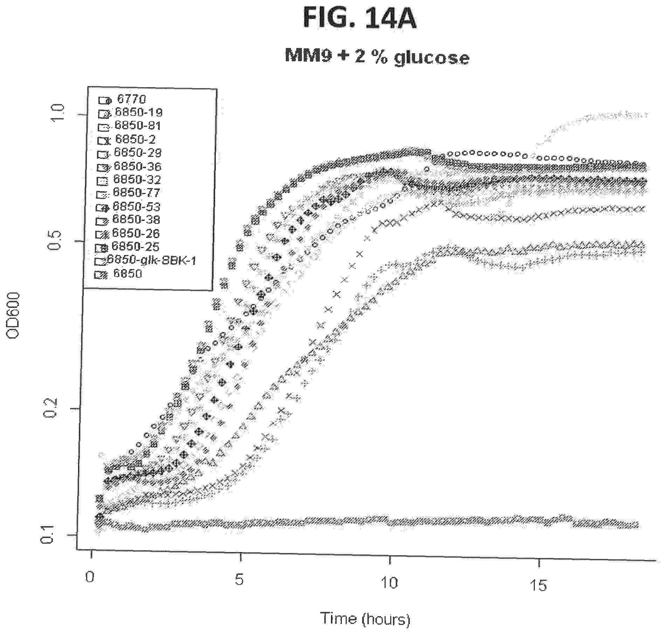

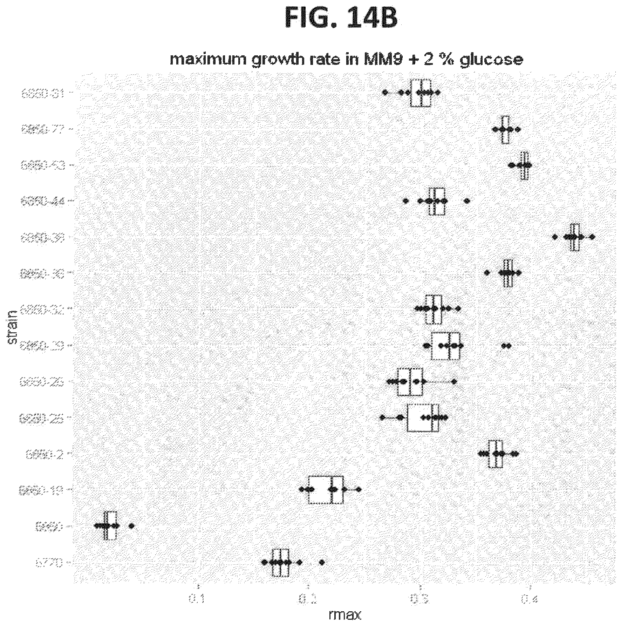

**Figure 14A** shows growth rate curves of expression variants of glk-glf as described in Example 1 and **Figure 14B** shows maximum growth rates of select variants and parent strains.

### DETAILED DESCRIPTION

The present disclosure provides metabolic and biosynthetic processes and non-natural microbial organisms capable of enhancing carbon flux through acetyl-CoA, or both oxaloacetate and acetyl-CoA, synthesized using a pathway comprising phosphoketolase for producing acetyl-CoA (a PK pathway). The non-natural microbial organisms can utilize the enhanced pool of acetyl-CoA, or both oxaloacetate and acetyl-CoA, in a further compound pathway to produce a bio-derived product.

To generate enhanced carbon flux through acetyl-CoA, or both oxaloacetate and acetyl-CoA, a pathway comprising phosphoketolase is used in conjunction with (i) a non-phosphotransferase system (non-PTS) for sugar uptake comprising a genetic modification to a non-PTS component to increase non-PTS activity, and/or (ii) one or more modification(s) to the organism's electron transport chain to enhance efficiency of ATP production, to enhance availability or synthesis of reducing equivalents, or both. Optionally, the non-natural microbial organisms can include (iii) a genetic modification of a phosphotransferase system (PTS) component that attenuates or eliminates a PTS activity.

A first aspect of the disclosure is directed to a non-natural microbial organism having (a) a pathway to acetyl-CoA, or both oxaloacetate and acetyl-CoA, comprising a phosphoketolase, (b) a non-PTS for sugar uptake comprising a genetic modification to a non-PTS component to increase non-PTS activity. Optionally, the organism can also include a genetic modification of a PTS component that attenuates or eliminates a PTS activity.

The pathway comprising phosphoketolase for producing acetyl-CoA (PK pathway), and a sugar uptake system (e.g., non-PTS) are exemplified in Figure 1. This non-natural organism can use acetyl-CoA, or both oxaloacetate and acetyl-CoA (see Figure 4), in a "compound pathway" to produce a bio-derived product (such as an alcohol, a glycol, an organic acid, an alkene, a diene, an organic amine, an organic aldehyde, a vitamin, a nutraceutical or a pharmaceutical). Therefore the non-natural microbial organisms can further include product pathway enzymes to carry out conversion of acetyl-CoA, or both oxaloacetate and acetyl-CoA, to the desired product (e.g., combining the relevant pathways of Figure 1 or 4, with a pathway of Figures 5-11).

Even further, this non-natural microbial organism can optionally include one or more of the following: (e) one or more modification(s) to the organism's electron transport chain, (f) a carbon substrate (e.g., methanol, syngas, etc.) utilization pathway to increase carbon flux towards acetyl-CoA, or both oxaloacetate and acetyl-CoA, (g) a pathway synthesizing succinyl-CoA as precursors further to the synthesis of acetyl-CoA and oxaloacetate, (h) attenuation or elimination of one or more endogenous enzymes, which enhances carbon flux through acetyl-CoA, or both oxaloacetate and acetyl-CoA, (e.g., pyruvate kinase attenuation), and (i) increased activity of one or more endogenous or heterologous enzymes that can enable higher flux to oxaloacetate or succinyl-CoA (e.g., increases in PEP synthetase, pyruvate carboxylase, or phosphoenolpyruvate carboxylase).

A second aspect of the disclosure is directed to a non-natural microbial organism having (a) a pathway to oxaloacetate, acetyl-CoA or both, comprising phosphoketolase, and (b) one or more modification(s) to the organism's electron transport chain to enhance efficiency of ATP production, to enhance synthesis or availability of reducing equivalents, or both. In this non-natural microbial organism, modifications to the PTS and non-PTS for sugar uptake are not necessary, but optionally can be included. As an alternative to, or in addition to the PTS and non-PTS, a (c) carbon substrate (e.g., methanol, syngas, etc.) utilization pathway to provide carbon flux towards oxaloacetate, acetyl-CoA or both, can be present in the non-natural organism. This non-natural microbial organism can use the oxaloacetate, acetyl-CoA or both, in a (d) product pathway to produce a bio-derived product (such as an alcohol, a glycol, etc.) that includes product pathway enzymes. Even further, this non-natural microbial organism can optionally include, (e) a pathway synthesizing oxaloacetate or succinyl-CoA as precursors, (f) attenuation or elimination of one or more endogenous enzymes, which enhances carbon flux through acetyl-CoA (e.g., pyruvate kinase attenuation), or (g) increased activity of one or more enzymes that can enable higher flux to oxaloacetate or succinyl-CoA (e.g., increases in PEP synthetase, pyruvate carboxylase, or phosphoenolpyruvate carboxylase).

The pathway comprising phosphoketolase can include one, two, three, four, or five, or more than five enzymes to promote flux to acetyl-CoA, or both oxaloacetate and acetyl-CoA.

Some exemplary embodiments of the acetyl-CoA pathway comprising phosphoketolase can be understood with reference to Figure 1. For example, in some embodiments, the acetyl-CoA pathway comprises a pathway selected from: (1) 1T and 1V; (2) 1T, 1W, and 1X; (3) 1U and 1V; (4) 1U, 1W, and 1X; wherein 1T is a fructose-6-phosphate phosphoketolase, wherein 1U is a xylulose-5-phosphate phosphoketolase, wherein 1V is a phosphotransacetylase, wherein 1W is an acetate kinase, wherein 1X is an acetyl-CoA transferase, an acetyl-CoA synthetase, or an acetyl-CoA ligase. In some embodiments, the acetyl-CoA pathway comprises (1) 1T and 1V. In some embodiments, the acetyl-CoA pathway comprises (2) 1T, 1W, and 1X. The enzymes sets (1) and (2) can define a pathway from fructose-6-phosphate (F6P) to acetyl-CoA (AcCoA).

In some embodiments, the acetyl-CoA comprises (3) 1U and 1V. In some embodiments, the acetyl-CoA pathway comprises (4) 1U, 1W, and 1X. The enzymes sets (3) and (4) can define a pathway from fructose-6-phosphate (F6P) to acetyl-CoA (AcCoA). In some embodiments, an enzyme of the methanol metabolic pathway or the acetyl-CoA pathway is encoded by at least one exogenous nucleic acid and is expressed in a sufficient amount to enhance carbon flux through acetyl-CoA.

Any of the acetyl-CoA pathways comprising phosphoketolase (1) 1T and 1V; (2) 1T, 1W, and 1X; (3) 1U and 1V; (4) 1U, 1W, and 1X can be present in organisms of the first or second aspect of the disclosure.

The PTS and non-PTS can allow for uptake of primarily C5, C6 or C12 sugars and their oligomers. Organisms having a PTS for sugar (e.g., C6, C12, sugar alcohols, and amino sugars) uptake arc able to phosphorylate sugars by conversion of PEP into pyruvate.

The non-PTS, on the other hand, uses different sugar uptake enzymes and proteins (components) than the PTS, and this affects the balance of intracellular sugar-derived intermediates that are brought into the cell. In addition to glucose, the non-PTS allows for more robust import of C5 sugars such as xylose, disaccharides such as lactose, melibiose, maltose, and glycerol via a facilitator or a permease and subsequent phopshorylation via a kinase. Other substrates such as ascorbate may be recognized by a specific PTS or non-PTS.

With reference to Figure 1, in some embodiments, the non-natural microorganism comprises an acetyl-CoA, or both oxaloacetate and acetyl-CoA pathway (also see Figure 4) comprising phosphoketolase, a non-PTS for sugar uptake, and a PTS for sugar uptake that comprises a permease or a facilitator protein (1F), and a kinase (1G).

The non-PTS can include a non-PTS permease (e.g., facilitator protein), a non-PTS sugar kinase or a facilitator protein, and these can modified for increased expression or activity in a non-natural microbial organism having (a) a pathway to acetyl-CoA, or both oxaloacetate and acetyl-CoA, comprising a phosphoketolase. The non-PTS permease can be a glucose permease, and the non-PTS sugar kinase can be a glucokinase. An exemplary glucose facilitator proteins is encoded by *Zymomonas mobilis* glf. An exemplary glucokinase is encoded by *E. coli* glk and an exemplary permease is encoded by *E. coli* galP.

The genetic modification to a non-PTS component to increase non-PTS activity can be any one or more of a variety of forms. For example, in some embodiments, the non-PTS component is under the expression of a promoter comprising one or more genetic modifications that enhance its expression. The enhanced expression can result in an increase in activity of the rate of sugar uptake to the cells. For example, the rate of uptake can be at least 10%, at least 25%, at least 50%, at least 75%, at least 100%, at least 125% or at least 150% greater than the rate of sugar uptake of an organism that does not include the non-PTS genetic modification. Exemplary rate of uptake increases can be in the range of 10% to 150%, or from 25% to 125%.

An organism with a genetic modification to a non-PTS enzyme or protein to increase non-PTS activity prevents PEP conversion into pyruvate associated with sugar phosphorylation and therefore allows for a better balance of fluxes into oxaloacetate and pyruvate from PEP. Phosphorylated sugar then goes through the majority of reactions in glycolysis to generate reducing equivalents and ATP that are associated with the organism's electron transport chain (ETC).

In some embodiments, the non-natural microbial organism comprises a genetic modification of a PTS component that attenuates or eliminates a PTS activity. In a non-natural microbial organism system that includes non-PTS for sugar uptake, an attenuating or eliminating genetic modification of a PTS component can shift the sugar uptake towards the non-PTS, thereby providing an improved pool of sugar derived intermediates than can be utilized by the pathway comprising phosphoketolase for the production of acetyl-CoA, or both oxaloacetate and acetyl-CoA.

In embodiments wherein the non-natural organism has one or more genetic modifications that attenuates or eliminates expression or activity of a PTS component.

The PTS system comprises Enzyme I (EI), histidine phosphocarrier protein (HPr), Enzyme II (EII), and transmembrane Enzyme II C (EIIC). The system allows specific uptake of sugars into the cell, with the sugars transported up at a concentration gradient along phosphorylation. Phosphoenolpyruvate (PEP) is the phosphate donor, with the phosphate transferred via the (non-sugar specific) enzymes EI and HPr to the enzyme complex EII. The enzyme complex EII includes components A, B and C. These components can be domains of composite proteins, according to sugar specificity and the type of bacteria. Component/domain C is a permease and anchored to the cytoplasmic membrane. In *E. coli,* the glucose PTS ETTA is a soluble protein, and the EIIB/C is membrane bound. In *E. coli* the two nonspecific components are encoded by ptsI (Enzyme 1) and ptsH (HPr). The sugar-dependent components are encoded by err and ptsG. Any one or more of these PTS enzymes or proteins (components) can be targeted for attenuated or eliminated expression or activity. Alternatively, the non-natural organism having attenuated or eliminated non-natural microbial organism that includes (a) a pathway to oxaloacetate, acetyl-CoA or both, comprising a phosphoketolase, and (b) one or more modification(s) to the organism's electron transport chain.

Optionally one or more modification(s) to the organism's electron transport chain can also be used along with the non-PTS for sugar uptake comprising a genetic modification to a non-PTS component to increase non-PTS activity, in a non-natural microbial organism having a PK pathway. For example, in an organism having flux through phosphoketolase, the non-oxidative pentose phosphate pathway does not generate any ATP or reducing equivalents and it is therefore desirable to have the electron transport chain operate efficiently. Such modifications to the ETC are useful when the organism uses the PK pathway irrespective of the carbon source used. For example, such modifications will be useful when methanol, methane, formate, syngas or glycerol are used as the carbon sources.

Modifications that enhance the organism's electron transport chain function include attenuation of enzymes, proteins or co-factors that compete with efficient electron transport chain function. Examples are attenuation of NADH-dehydrogenases that do not translocate protons or an attenuation of cytochrome oxidases that have lower efficiency of proton translocation per pair of electrons. Modifications that enhance the organism's electron transport chain function include enhancing function of enzymes, proteins or co-factors of the organism's electron transport chain particularly when such a function is rate-limiting. Examples in bacteria of modifications that enhance enzymes, proteins or co-factors are increasing activity of NADH dehydrogenases of the electron transport chain or the desired cytochrome oxidase cyo and attenuating the global negative regulatory factor arcA.

In some embodiments, the invention provides a non-naturally occurring microbial organism having attenuation or elimination of endogenous enzyme expression or activity that compete with efficient electron transport chain function, thereby enhancing carbon flux through acetyl-CoA or oxaloacetate into the desired products. Elimination of endogenous enzyme expression can be carried out by gene disruption of one or more endogenous nucleic acids encoding such enzymes. For example, in some aspects the endogenous enzymes targeted for modification include genes such as ndh, wrbA, mdaB, yhdH, yieF, ytfG, qor, ygiN, appBC and cydAB in expression of PTS enzymes or proteins is caused by alteration, such as deletion of, the ptsI gene.

The PTS can include proteins specific for the uptake of certain sugar species. These are generally known as "permeases" or "facilitator proteins." For example, the PTS can comprises one or more proteins selected from the group consisting of glucose permease (EIICBA), glucosamine permease (EIICBA), N-acetyl muramic acid-specific permease (ETIBC component), mannitol permease, galactomannan permease, trehalose permease, maltose permease, fructose permease, mannose permease, N-acetylglucosamine permease, (EIICB component), fructose permease, sucrose permease (high affinity), sucrose permease (low affinity), lichenan permease, and β-glucoside permease. The non-natural microbial organisms of the disclosure can include attenuated or eliminated expression of one or more proteins specific for the uptake of certain sugar species.

Proteins of the PTS can be encoded by genes of the microorganism. For example, glucose permease can be encoded by PtsG, the glucosamine permease can be encoded by GamP, the N-acetyl muramic acid-specific permease can be encoded by MurP, the mannitol permease can be encoded by MtlA or MtlF, the galactomannan permease can be encoded by GmuA, GmuB, or GmuC, the trehalose permease can be encoded by TreP, the maltose permease can be encoded by MalP, the fructose permease can be encoded by FruA, the mannose permease is encoded can be ManP, the N-acetylglucosamine permease can be encoded by NagP, the fructose permease can be encoded by LevD, LevE, LevF, LevG, the sucrose permease (high affinity) can be encoded by SacP, the sucrose permease (low affinity) can be encoded by SacY, the lichenan permease can be encoded by LicA, LicB, or LicC, and the β-glucoside permease can be encoded by BglP. The non-natural microbial organisms of the disclosure can include genetic modification of one or more of these genes for attenuated or eliminated expression of their corresponding proteins.

Non-natural microbial organisms of the disclosure can also include one or more modification(s) to the organism's electron transport chain to enhance efficiency of ATP production, to enhance availability or synthesis of reducing equivalents, or both. For example, the second aspect of the disclosure provides a E. coli. Similar non-cfficicnt components of the electron transport chain can be eliminated or modified from other organisms.

The electron transport chain of *Escherichia coli* has multiple NADH dehydrogenases and cytochrome oxidases, with varying ability to translocate protons. For example, NADH dehydrogenase II in *E. coli* is an NADH consuming system that is not linked with proton translocation (H+/2e- = 0) whereas NADH dehydrogenase I encoded by nuo is reported to translocate 4 protons per pair of electrons. The major role of Ndh-II is to oxidize NADH and to feed electrons into the respiratory chain (Yun *et al.,* 2005). The affinity of NdhII for NADH is relatively low (Hayashi *et al.,* 1989), it has been suggested that NdhII may operate to regulate the NADH pool independently of energy generation and is likely to be important when the capacity of bacteria to generate energy exceeds demand. The ndh gene has been shown to be repressed by the fnr gene product in such a way that the expression is optimal under conditions of high oxygen concentrations. The deletion of ndh would thus help in improving the redox availability and therefore the ATP availability of the cell upon oxidation of this NADH. Similarly, there are several other NADH dehydrogenases that are not known to translocate any protons and thus do not help in ATP production, example, those encoded by wrbA, mdaB, yhdH, yieF, ytfG, qor in E. coli. Homologues of these can be found in other organisms and eliminated to improve the ATP production for every unit of oxygen consumed.

On the electron output side of the electron transport chain, multiple cytochrome oxidases are present that have different energy-conserving efficiencies. The cytochrome bo complex, encoded by the *cyo* operon, actively pumps electrons over the membrane and results in an H+/2c- stoichiometry of 4. The cytochrome bd-I complex does not actively pump protons, but due to the oxidation of the quinol on the periplasmic side of the membrane and subsequent uptake of protons from the cytoplasmic side of the membrane which are used in the formation of water, the net electron transfer results in a H+/2c- stoichiometry of 2. This is encoded by the *cyd* operon. Till recently, the proton translocation stoichiometry of cytochrome bd-II oxidase, encoded by *appBC,* was not known but it has now been established that this oxidase is non-electrogenic [ Bekker M, de VS, Ter BA, Hellingwerf KJ, de Mattos MJ. 2009. Respiration of Escherichia coli can be fully uncoupled via the nonelectrogenic terminal cytochrome bd-II oxidase. J Bacteriol 191:5510-5517.]. These genes are normally induced upon entry into stationary phase or under conditions of carbon and phosphate starvation Atlung et al*.,* 1997 (Atlung T, Knudsen K, Heerfordt L, Brondstcd L. 1997. Effects of sigmaS and the transcriptional activator AppY on induction of the Escherichia coli hya and cbdAB-appA operons in response to carbon and phosphate starvation. J Bacteriol 179:2141-2146.). Deletion of the the cytochrome oxidases appBC and cydAB will therefore improve the ATP formation per NADH via oxidative phosphorylation, thus increasing efficiency of ATP production. The quinol monooxygenase, ygiN, also falls in this category.

In addition to or as an alternative to attenuating such host functions, an enhanced electron transport function can be provided in a non-naturally organism that contains an acetyl-CoA, or acetyl-CoA and oxaloacetate pathway by providing a modification that increases an enzyme, protein or co-factor function of the organism's electron transport chain to enhance efficiency of ATP production , production of reducing equivalents or both, particularly when such functions arc rate-limiting. Examples in bacteria of such target genes include those that comprise Complex I (which can be increased by such methods as increased copy number, overexpression or enhanced activity variants) of the electron transport chain and the global negative regulatory factor arcA (which can be attenuated).

Additionally, given the non-NADH generating nature of the PK pathway, it is important that mechanisms for generating NADH are introduced into recombinant organisms for making reduced products. For example, a 14BDO producing organism (described in Example X1) requires conversion of pyruvate into acetyl-CoA. When a PK is introduced into this organism, it is important that the pyruvate to acetyl-CoA conversion takes place either through pyruvate dehydrogenase or by a combination of pyruvate formate lyase and an NADH-generating formate dehydrogenase. Native formate dchydrogcnasc(s) that do not generate NADH can be optionally deleted. In *E. coli* these formate dehydrogenases are formate dehydrogenase H, N and O.

In some other embodiments, the invention provides a process with the described acetyl-CoA pathway to have a lower aeration process compared to an organism that does not have such a pathway.

Since flux through phosphoketolase does not produce NADH, any organism that has flux through PK should require less oxygen to regenerate NAD. For example, the stoichiometry of making 14BDO via only the oxidative TCA cycle is shown below:

C₆H₁₂O₆ + 0.5 O₂ → C₄H₁₀O₂ + 2 CO₂ + H₂O

In contrast, an organism that can increase its BDO yield by using PK will have the following stoichiometry, still using only the oxidative TCA for routing carbon into the BDO pathway:

C₆H₁₂O₆ + 0.313 O₂ → 1.034 C₄H₁₀O₂ + 1.864 CO₂ + 0.830 H₂O

This organism has 60% of the oxygen demand per glucose metabolized as compared to an organism that does not use PK. This lowers the aeration/oxygen requirements in a fermentation process while increasing the product yields.

A reducing equivalent can also be readily obtained from a glycolysis intermediate by any of several central metabolic reactions including glyceraldehyde-3-phosphate dehydrogenase, pyruvate dehydrogenase, pyruvate formate lyase and NAD(P)-dependent formate dehydrogenase, isocitrate dehydrogenase, alpha-ketoglutarate dehydrogenase, succinate dehydrogenase, and malate dehydrogenase. Additionally, reducing equivalents can be generated from glucose 6-phosphate-1-dehydrogenase and 6-phosphogluconate dehydrogenase of the pentose phosphate pathway. Overall, at most twelve reducing equivalents can be obtained from a C6 glycolysis intermediate (e.g., glucose-6-phosphate, fructose-6-phosphate, fructose-1,6-diphosphatc) and at most six reducing equivalents can be generated from a C3 glycolysis intermediate (e.g., dihydroxyacetone phosphate, glyceraldehyde-3-phosphate).

Optionally, non-natural microbial organisms of the disclosure having a PK pathway can also use both acetyl-CoA and oxaloacetate or succinyl-CoA as precursors to product pathways. Oxaloacetate is produced anaplerotically from phosphoenolpyruvate or from pyruvate. Succinyl-CoA is produced either by oxidative TCA cycle whereby both acetyl-CoA and oxaloacetate are used as precursors, via the reductive TCA cycle where oxaloacetate is used as the precursor or by a combination of both oxidative and reductive TCA branches. Non-natural microbial organisms of the first, or second aspect can further use both acetyl-CoA and oxaloacetate or succinyl-CoA as precursors. Genetic modifications can include increasing the activity of one or more endogenous or heterologous enzymes, or attenuating or eliminating one or more endogenous enzymes to increase flux into oxaloacetate or succinyl-CoA.

In some embodiments, the invention provides a non-natural organism having increased activity of one or more endogenous enzymes, that combined with the acetyl-CoA (PK) pathway, enables higher flux to the product. These enzymes are targeted towards increased flux into oxaloacetate which, when combined with acetyl-CoA leads to higher flux through oxidative TCA and the products derived therefrom. Alternatively, the increased flux into oxaloacetate can be used for producing succinyl-CoA via the reductive TCA branch. This includes PEP synthetase, pyruvate carboxylase, and phosphoenolpyruvate carboxylase. This increased activity can be achieved by increasing the expression of an endogenous or exogenous gene either by overexpressing it under a stronger promoter or by expressing an extra copy of the gene or by adding copies of a gene not expressed endogenously.

Embodiments of the disclosure provide non-naturally organism comprising a phosphoketolase (PK)-containing pathway that makes acetyl-CoA, or acetyl-CoA and oxaloacetate, and one or more of the following: (i) a genetic modification that enhances the activity of the non-PTS system for sugar uptake, and/or (ii) one or more modification(s) to the organism's electron transport chain to enhance efficiency of ATP production, to enhance availability of reducing equivalents, or both, and further, one or more modifications that enhance flux to oxaloacetate or succinyl-CoA. In turn , these modifications can enhance production of bioproducts in combination with the product pathways.

The one or more modifications that enhance flux to oxaloacetate or succinyl-CoA can be any one or more of the following.

In some embodiments, the non-natural microbial organism further includes attenuation of pyrvuate kinase. Pyruvate kinase leads to the formation of pyruvate from PEP, concomitantly converting ADP into ATP.

PEP + ADP → pyruvate + ATP + H⁺

Attenuation or deletion of this enzyme will allow more PEP to be converted into oxaloacetate (and not into pyruvate that subsequently gets converted into acetyl-CoA). This leads to a better balance of carbon flux into oxaloacetate and into acetyl-CoA. In *E. coli,* this reaction is carried out by two isozymes, pykA and pykF. The deletion of even one of them can have the desired effect of reducing PEP flux into pyruvate and increasing it into oxaloacetate.

In some embodiments, the non-natural microbial organism increases phosphoenol-pyruvate availability by enhancing PEP synthetase activity in a strain that requires oxaloacetate as a bioproduct precursor. This enzyme converts pyruvate back into PEP with the cost of two ATP equivalents as shown below. This is a mechanism that the cell can use to balance the flux that goes into acetyl-CoA versus the carbon flux that goes into PEP and then onto oxaloacetate.

pyruvate + ATP⁺ H₂O <=> phosphoenolpyruvate + AMP + phosphate + 2 H⁺

In some embodiments, the non-natural microbial organism increases oxaloacetate availability via enhancing pyruvate carboxylase activity in a strain that requires oxaloacetate as a bioproduct precursor. Pyruvate carboxylase catalyzes the carboxylation of pyruvate into oxaloacetate using biotin and ATP as cofactors as shown below.

pyruvate + hydrogen carbonate + ATP → oxaloacetate + ADP + phosphate + H+

Pyruvate carboxylase is present in several bacteria such as *Corynebacterium glumaticum* and Mycobacteria, but not present in *E. coli.* Pyruvate carboxylase can be expressed heterologously in E. coli via methods well known in the art. Optimal expression of this enzyme would allow for sufficient generation of oxaloacetate and is also expected to reduce the formation of byproducts such as alanine, pyruvate, acetate and ethanol.

In some embodiments, the non-natural microbial organism increases oxaloacetate availability via enhancing phosphoenolpyruvate (PEP) carboxylase activity in a strain that requires oxaloacetate as a bioproduct precursor. The gene ppc encodes for phosphoenolpyruvate (PEP) carboxylase activity. The net reaction involves the conversion of PEP and bicarbonate into oxaloacetate and phosphate. The overexpression of PEP carboxylase leads to conversion of more phosphoenolpyruvate (PEP) into OAA, thus reducing the flux from PEP into pyruvate, and subsequently into acetyl-CoA. This leads to increased flux into the TCA cycle and thus into the pathway. Further, this overexpression also decreases the intracellular acetyl-CoA pools available for the ethanol-forming enzymes to work with, thus reducing the formation of ethanol and acetate. The increased flux towards oxaloacetate will also reduce pyruvate and alanine byproducts.

In some embodiments, the non-natural microbial organism increases phosphoenol-pyruvate availability via enhancing PEP carboxykinase (pck) activity in a strain that requires oxaloacetate as a bioproduct precursor. PEP carboxykinase is an alternative enzyme for converting phosphoenolpyruvate to oxaloacetate, which simultaneously forms an ATP while carboxylating PEP. In most organisms PEP carboxykinase serves a gluconeogenic function and converts oxaloacetate to PEP at the expense of one ATP. *S*. *cerevisiae* is one such organism whose native PEP carboxykinase, *PCK1,* serves a gluconeogenic role (Valdes-Hevia et al., FEBS Lett. 258:313-316 (1989). *E. coli* is another such organism, as the role of PEP carboxykinase in producing oxaloacetate is believed to be minor when compared to PEP carboxylase, which does not form ATP, possibly due to the higher Kₘ for bicarbonate of PEP carboxykinase (Kim et al., Appl. Environ. Microbiol. 70:1238-1241 (2004)). Nevertheless, activity of the native *E. coli* PEP carboxykinase from PEP towards oxaloacetate has been recently demonstrated in *ppc* mutants of *E. coli* K-12 (Kwon ct al., J. Microbiol. Biotechnol. 16:1448-1452 (2006)). These strains exhibited no growth defects and had increased succinate production at high NaHCO₃ concentrations. In some embodiments, the non-natural microbial organism can increase its oxaloacetate availability by increasing expression or activity of malic enzyme. Malic enzyme can be applied to convert CO₂ and pyruvate to malate at the expense of one reducing equivalent. Malate can then be converted into oxaloacetate via native malate dehydrogenases. Malic enzymes for this purpose can include, without limitation, malic enzyme (NAD-dependent) and malic enzyme (NADP-dependent). For example, one of the *E. coli* malic enzymes (Takeo, J. Biochem. 66:379-387 (1969)) or a similar enzyme with higher activity can be expressed to enable the conversion of pyruvate and CO₂ to malate. By fixing carbon to pyruvate as opposed to PEP, malic enzyme allows the high-energy phosphate bond from PEP to be conserved by pyruvate kinase whereby ATP is generated in the formation of pyruvate or by the phosphotransferase system for glucose transport. Although malic enzyme is typically assumed to operate in the direction of pyruvate formation from malate, overexpression of the NAD-dependent enzyme, encoded by maeA, has been demonstrated to increase succinate production in *E. coli* while restoring the lethal delta pfl-delta ldhA phenotype (inactive or deleted pfl and ldhA) under anaerobic conditions by operating in the carbon-fixing direction (Stols and Donnelly, Appl. Environ. Microbial. 63(7) 2695-2701 (1997)). A similar observation was made upon overexpressing the malic enzyme from *Ascaris suum* in *E. coli* (Stols ct al., Appl. Biochem. Biotechnol. 63-65(1), 153-158 (1997)). The second *E*. *coli* malic enzyme, encoded by *maeB,* is NADP-dependent and also decarboxylates oxaloacetate and other alpha-keto acids (Iwakura et al., J. Biochem. 85(5):1355-65 (1979)).

In some embodiments, the non-natural microbial organism increases the non oxidative pentose phosphate pathway activity, ribose-5-phosphate isomerase (encoded by rpiAB in E. coli), ribulose-5-phosphate epimerase (rpe), transaldolase (talAB) and transketolase (tktAB), to convert C5 sugars to glycolytic intermediates glyceraldehyde-3-phosphate and fructose-6-phosphate that are substrates for the phosphoketolase pathway.

The non oxidative pentose phosphate pathway comprises numerous enzymes that have the net effect of converting C5 sugar intermediates into C3 and C6 glycolytic intermediates, namely, glyceraldehyde-3-phosphate and fructose-6-phosphate. The enzymes included in the non-oxidative PP branch are ribose-5-phosphate isomerase (encoded by rpiAB in E. coli), ribulose-5-phosphate epimerase (rpe), transaldolase (talAB), and transketolase (tktAB).

Ribose-5-phopshate epimerase catalyzes the interconversion of ribose-5-phosphate and ribulose-5-phosphate. There are two distinct ribose-5-phosphate isomerases present in *E. coli. RpiA encodes for* the constitutive ribose-5-phosphate isomerase A and typically accounts for more than 99% of the ribose-5-phosphate isomerase activity in the cell. The inducible ribose-5-phosphate isomerase B can substitute for RpiA's function if its expression is induced.

Ribulosc-5-phosphatc-3-cpimcrasc (rpe) catalyzes the interconversion of D-ribulose-5-phosphate and xylulose-5-phosphate. Transketolase catalyzes the reversible transfer of a ketol group between several donor and acceptor substrates. This enzyme is a reversible link between glycolysis and the pentose phosphate pathway. The enzyme is involved in the catabolism of pentose sugars, the formation of D-ribose 5-phosphate, and the provision of D-erythrose 4-phosphate. There are two transketolase enzymes in E. coli, catalyzed by tktA and tktB. Transketolase leads to the reversible conversion of erythrose-4-phosphate and xylulose-5-phosphate to form fructose-6-phosphate and glyccraldchydc-3-phosphatc. Yet another reaction catalyzed by the enzyme is the interconversion of sedoheptulose-7-phosphate and glyceraldehyde-3-phosphate to form ribose-5-phosphate and xylulose-5-phosphate.

Transaldolase is another enzyme that forms a reversible link between the pentose phosphate pathway and glycolysis. It catalyzes the interconversion of glyceraldehyde-3-phosphate and sedoheptulose-7-phosphate to fructose-6-phosphate and erythrose-4-phosphate. There are two closely related transaldolases in *E. coli,* encoded by *talA* and *talB.* Homologues of these genes can be found in other microbes including C. *glutamicum, S. cerevisiae, Pseudomonas putida, Bacillus subtilis.* For sufficient flux to be carried through phosphoketolase, it is important to ensure that the flux capacity of the non-oxidative PP enzymes in not limiting.

When glucose is used as the carbon substrate the carbon flux distribution through the PTS and the Non-PTS system as well as the phosphoketolase can be modified to enhance bioderived product production.. If Non-PTS system is not used, some flux will have to be diverted from pyruvate into oxaloacetate. This can be done by enzymes such as PEP synthetase in combination with phoshoenolpyruvate carboxylase or phoshoenolpyruvate carboxykinase, or by pyruvate carboxylase.

In some embodiments, the disclosure provides a non-naturally occurring microbial organism having an acetyl-CoA pathway, or oxaloacetate and acetyl-CoA pathway comprising phosphoketolase, and one or more of the following: (i) a non-PTS for sugar uptake comprising one or more genetic modification(s) to increase non-PTS activity, and/or (ii) one or more modification(s) to the organism's electron transport chain to enhance efficiency of ATP, to enhance synthesis or availability of reducing equivalents, or both, wherein the microbial organism further includes attenuation of one or more endogenous enzymes, which enhances carbon flux through acetyl-CoA into the product.

In some embodiments, the disclosure provides a non-naturally occurring microbial organism as having an acetyl-CoA pathway, or oxaloacetate and acetyl-CoA pathway comprising phosphoketolase, and/or, (ii) one or more modification(s) to the organism's electron transport chain to enhance efficiency of ATP production, to enhance synthesis or availability of reducing equivalents, or both, wherein the microbial organism further includes attenuation of one or more endogenous enzymes of a competing formaldehyde assimilation or dissimilation pathway. Examples of these endogenous enzymes are disclosed in FIG. 1 and described in Example XIII.

It is understood that a person skilled in the art would be able to readily identify enzymes of such competing pathways. Competing pathways can be dependent upon the host microbial organism and/or the exogenous nucleic acid introduced into the microbial organism as described herein. Accordingly, in some aspects of the disclosure, the microbial organism includes attenuation of one, two, three, four, five, six, seven, eight, nine, ten or more endogenous enzymes of a competing formaldehyde assimilation or dissimilation pathway.

For example, in some aspects, the endogenous enzyme can be selected from DHA kinase, methanol oxidase, PQQ-dependent methanol dehydrogenase, DHA synthase or any combination thereof. Accordingly, in some aspects, the attenuation is of the endogenous enzyme DHA kinase. In some aspects, the attenuation is of the endogenous enzyme methanol oxidase. In some aspects, the attenuation is of the endogenous enzyme PQQ-dependent methanol dehydrogenase. In some aspects, the attenuation is of the endogenous enzyme DHA synthase. The invention also provides a microbial organism wherein attenuation is of any combination of two or three endogenous enzymes described herein. For example, a microbial organism of the invention can include attenuation of DHA kinase and DHA synthase, or alternatively methanol oxidase and PQQ-dependent methanol dehydrogenase, or alternatively DHA kinase, methanol oxidase, and PQQ-dependent methanol dehydrogenase, or alternatively DHA kinase, methanol oxidase, and DHA synthase. The invention also provides a microbial organism wherein attenuation is of all endogenous enzymes described herein. For example, in some aspects, a microbial organism described herein includes attenuation of DHA kinase, methanol oxidase, PQQ-dependent methanol dehydrogenase and DHA synthase. Competing pathways can be dependent upon the host microbial organism and/or the exogenous nucleic acid introduced into the microbial organism as described herein. Accordingly, in some aspects of the invention, the microbial organism includes a gene disruption of one, two, three, four, five, six, seven, eight, nine, ten or more endogenous nucleic acids encoding enzymes of a competing formaldehyde assimilation or dissimilation pathway.

In the case of gene disruptions, a particularly useful stable genetic alteration is a gene deletion. The use of a gene deletion to introduce a stable genetic alteration is particularly useful to reduce the likelihood of a reversion to a phenotype prior to the genetic alteration. For example, stable growth-coupled production of a biochemical can be achieved, for example, by deletion of a gene encoding an enzyme catalyzing one or more reactions within a set of metabolic modifications. The stability of growth-coupled production of a biochemical can be further enhanced through multiple deletions, significantly reducing the likelihood of multiple compensatory reversions occurring for each disrupted activity.

Also provided is a method of producing a non-naturally occurring microbial organisms having stable growth-coupled production of acetyl-CoA or both oxaloacetate and acetyl-CoA or a bioderived compound. The method can include identifying *in silico* a set of metabolic modifications that increase production of acetyl-CoA or both oxaloacetate and acetyl-CoA or a bioderived compound, for example, increase production during exponential growth; genetically modifying an organism to contain the set of metabolic modifications that increase production of acetyl-CoA or both oxaloacetate and acetyl-CoA or a bioderived compound, and culturing the genetically modified organism. If desired, culturing can include adaptively evolving the genetically modified organism under conditions requiring production of acetyl-CoA or both oxaloacetate and acetyl-CoA or a bioderived compound. The methods of the invention are applicable to bacterium, yeast and fungus as well as a variety of other cells and microorganism, as disclosed herein.

Thus, the invention provides a non-naturally occurring microbial organism comprising one or more gene disruptions that confer increased synthesis or production of acetyl-CoA or both oxaloacetate and acetyl-CoA or a bioderived compound. In one embodiment, the one or more gene disruptions confer growth-coupled production of acetyl-CoA or both oxaloacetate and acetyl-CoA or a bioderived compound, and can, for example, confer stable growth-coupled production of acetyl-CoA or both oxaloacetate and acetyl-CoA or a bioderived compound. In another embodiment, the one or more gene disruptions can confer obligatory coupling of acetyl-CoA or both oxaloacetate and acetyl-CoA or a bioderived compound production to growth of the microbial organism. Such one or more gene disruptions reduce the activity of the respective one or more encoded enzymes.

The non-naturally occurring microbial organism can have one or more gene disruptions included in a gene encoding an enzyme or protein disclosed herein. As disclosed herein, the one or more gene disruptions can be a deletion. Such non-naturally occurring microbial organisms of the invention include bacteria, yeast, fungus, or any of a variety of other microorganisms applicable to fermentation processes, as disclosed herein.

Thus, the invention provides a non-naturally occurring microbial organism, comprising one or more gene disruptions, where the one or more gene disruptions occur in genes encoding proteins or enzymes where the one or more gene disruptions confer increased production of acetyl-CoA or both oxaloacetate and acetyl-CoA or a bioderived compound. The production of acetyl-CoA or both oxaloacetate and acetyl-CoA or a bioderived compound can be growth-coupled or not growth-coupled. In a particular embodiment, the production of acetyl-CoA or both oxaloacetate and acetyl-CoA or a bioderived compound can be obligatorily coupled to growth of the organism, as disclosed herein.

The invention provides non naturally occurring microbial organisms having genetic alterations such as gene disruptions that increase production of acetyl-CoA or both oxaloacetate and acetyl-CoA or a bioderived compound, for example, growth-coupled production of acetyl-CoA or both oxaloacetate and acetyl-CoA or a bioderived compound. Product production can be, for example, obligatorily linked to the exponential growth phase of the microorganism by genetically altering the metabolic pathways of the cell, as disclosed herein. The genetic alterations can increase the production of the desired product or even make the desired product an obligatory product during the growth phase. Metabolic alterations or transformations that result in increased production and elevated levels of acetyl-CoA or both oxaloacetate and acetyl-CoA or a bioderived compound biosynthesis are exemplified herein. Each alteration corresponds to the requisite metabolic reaction that should be functionally disrupted. Functional disruption of all reactions within one or more of the pathways can result in the increased production of acetyl-CoA or both oxaloacetate and acetyl-CoA or a bioderived compound by the engineered strain during the growth phase.

Each of these non-naturally occurring alterations result in increased production and an enhanced level of acetyl-CoA or both oxaloacetate and acetyl-CoA or a bioderived compound, for example, during the exponential growth phase of the microbial organism, compared to a strain that does not contain such metabolic alterations, under appropriate culture conditions. Appropriate conditions include, for example, those disclosed herein, including conditions such as particular carbon sources or reactant availabilities and/or adaptive evolution.

Given the teachings and guidance provided herein, those skilled in the art will understand that to introduce a metabolic alteration such as attenuation of an enzyme, it can be necessary to disrupt the catalytic activity of the one or more enzymes involved in the reaction. Alternatively, a metabolic alteration can include disrupting expression of a regulatory protein necessary for enzyme activity or maximal activity. Disruption can occur by a variety of methods including, for example, deletion of an encoding gene or incorporation of a genetic alteration in one or more of the encoding gene sequences. The encoding genes targeted for disruption can be one, some, or all of the genes encoding enzymes involved in the catalytic activity. For example, where a single enzyme is involved in a targeted catalytic activity, disruption can occur by a genetic alteration that reduces or eliminates the catalytic activity of the encoded gene product. Similarly, where the single enzyme is multimeric, including heteromeric, disruption can occur by a genetic alteration that reduces or destroys the function of one or all subunits of the encoded gene products. Destruction of activity can be accomplished by loss of the binding activity of one or more subunits required to form an active complex, by destruction of the catalytic subunit of the multimeric complex or by both. Other functions of multimeric protein association and activity also can be targeted in order to disrupt a metabolic reaction of the invention. Such other functions are well known to those skilled in the art. Similarly, a target enzyme activity can be reduced or eliminated by disrupting expression of a protein or enzyme that modifies and/or activates the target enzyme, for example, a molecule required to convert an apoenzyme to a holoenzyme. Further, some or all of the functions of a single polypeptide or multimeric complex can be disrupted according to the invention in order to reduce or abolish the catalytic activity of one or more enzymes involved in a reaction or metabolic modification of the invention. Similarly, some or all of enzymes involved in a reaction or metabolic modification of the invention can be disrupted so long as the targeted reaction is reduced or eliminated.

Given the teachings and guidance provided herein, those skilled in the art also will understand that an enzymatic reaction can be disrupted by reducing or eliminating reactions encoded by a common gene and/or by one or more orthologs of that gene exhibiting similar or substantially the same activity. Reduction of both the common gene and all orthologs can lead to complete abolishment of any catalytic activity of a targeted reaction. However, disruption of either the common gene or one or more orthologs can lead to a reduction in the catalytic activity of the targeted reaction sufficient to promote coupling of growth to product biosynthesis. Exemplified herein arc both the common genes encoding catalytic activities for a variety of metabolic modifications as well as their orthologs. Those skilled in the art will understand that disruption of some or all of the genes encoding an enzyme(s) of a targeted metabolic reaction can be practiced in the methods of the invention and incorporated into the non-naturally occurring microbial organisms of the invention in order to achieve the increased production of acetyl-CoA or both oxaloacetate and acetyl-CoA or a bioderived compound or growth-coupled product production.

Given the teachings and guidance provided herein, those skilled in the art also will understand that enzymatic activity or expression can be attenuated using well known methods. Reduction of the activity or amount of an enzyme can mimic complete disruption of a gene if the reduction causes activity of the enzyme to fall below a critical level that is normally required for a pathway to function. Reduction of enzymatic activity by various techniques rather than use of a gene disruption can be important for an organism's viability. Methods of reducing enzymatic activity that result in similar or identical effects of a gene disruption include, but are not limited to: reducing gene transcription or translation; destabilizing mRNA, protein or catalytic RNA; and mutating a gene that affects enzyme activity or kinetics (*See,* Sambrook et al., Molecular Cloning: A Laboratory Manual, Third Ed., Cold Spring Harbor Laboratory, New York (2001); and Ausubel et al., Current Protocols in Molecular Biology, John Wiley and Sons, Baltimore, MD (1999). Natural or imposed regulatory controls can also accomplish enzyme attenuation including: promoter replacement (*See,* Wang et al., Mol. Biotechnol. 52(2):300-308 (2012)); loss or alteration of transcription factors (Dietrick et al., Annu. Rev. Biochem. 79:563-590 (2010); and Simicevic et al., Mol. Biosyst. 6(3):462-468 (2010)); introduction of inhibitory RNAs or peptides such as siRNA, antisense RNA, RNA or peptide/small-molecule binding aptamers, ribozymes, aptazymes and riboswitches (Wieland et al., Methods 56(3):351-357 (2012); O'Sullivan, Anal. Bioanal. Chem. 372(1):44-48 (2002); and Lee et al., Curr. Opin. Biotechnol. 14(5):505-511 (2003)); and addition of drugs or other chemicals that reduce or disrupt enzymatic activity such as an enzyme inhibitor, an antibiotic or a target-specific drug.

One skilled in the art will also understand and recognize that attenuation of an enzyme can be done at various levels. For example, at the gene level, a mutation causing a partial or complete null phenotype, such as a gene disruption, or a mutation causing epistatic genetic effects that mask the activity of a gene product (Miko, Nature Education 1(1) (2008)), can be used to attenuate an enzyme. At the gene expression level, methods for attenuation include: coupling transcription to an endogenous or exogenous inducer, such as isopropylthio-β-galactoside (IPTG), then adding low amounts of inducer or no inducer during the production phase (Donovan et al., J. Ind. Microbiol. 16(3): 145-154 (1996); and Hansen et al., Curr. Microbiol. 36(6):341-347 (1998)); introducing or modifying a positive or a negative regulator of a gene; modify histone acetylation/deacetylation in a eukaryotic chromosomal region where a gene is integrated (Yang et al., Curr. Opin. Genet. Dev. 13(2):143-153 (2003) and Kurdistani et al., Nat. Rev. Mol. Cell Biol. 4(4):276-284 (2003)); introducing a transposition to disrupt a promoter or a regulatory gene (Bleykasten-Brosshans et al., C. R. Biol. 33(8-9):679-686 (2011); and McCue et al., PLoS Genet. 8(2):e1002474 (2012)); flipping the orientation of a transposable element or promoter region so as to modulate gene expression of an adjacent gene (Wang et al., Genetics 120(4):875-885 (1988); Hayes, Annu. Rev. Genet. 37:3-29 (2003); in a diploid organism, deleting one allele resulting in loss of heterozygosity (Daigaku et al., Mutation Research/Fundamental and Molecular Mechanisms of Mutagenesis 600(1-2)177-183 (2006)); introducing nucleic acids that increase RNA degradation (Houseley et al., Cell, 136(4):763-776 (2009); or in bacteria, for example, introduction of a transfer-messenger RNA (tmRNA) tag, which can lead to RNA degradation and ribosomal stalling (Sunohara et al., RNA 10(3):378-386 (2004); and Sunohara et al., J. Biol. Chem. 279:15368-15375 (2004)). At the translational level, attenuation can include: introducing rare codons to limit translation (Angov, Biotechnol. J. 6(6):650-659 (2011)); introducing RNA interference molecules that block translation (Castel et al., Nat. Rev. Genet. 14(2):100-112 (2013); and Kawasaki et al., Curr. Opin. Mol. Ther. 7(2):125-131 (2005); modifying regions outside the coding sequence, such as introducing secondary structure into an untranslated region (UTR) to block translation or reduce efficiency of translation (Ringnér et al., PLoS Comput. Biol. 1(7):e72 (2005)); adding RNAase sites for rapid transcript degradation (Pasquinelli, Nat. Rev. Genet. 13(4):271-282 (2012); and Arraiano et al., FEMS Microbiol. Rev. 34(5):883-932 (2010); introducing antisense RNA oligomers or antisense transcripts (Nashizawa et al., Front. Biosci. 17:938-958 (2012)); introducing RNA or peptide aptamers, ribozymes, aptazymes, riboswitches (Wieland et al., Methods 56(3):351-357 (2012); O'Sullivan, Anal. Bioanal. Chem. 372(1):44-48 (2002); and Lee et al., Curr. Opin. Biotechnol. 14(5):505-511 (2003)); or introducing translational regulatory elements involving RNA structure that can prevent or reduce translation that can be controlled by the presence or absence of small molecules (Araujo et al., Comparative and Functional Genomics, Article ID 475731, 8 pages (2012)). At the level of enzyme localization and/or longevity, enzyme attenuation can include: adding a degradation tag for faster protein turnover (Hochstrasser, Annual Rev. Genet. 30:405-439 (1996); and Yuan et al., PLoS One 8(4):e62529 (2013)); or adding a localization tag that results in the enzyme being secreted or localized to a subcellular compartment in a eukaryotic cell, where the enzyme would not be able to react with its normal substrate (Nakai et al. Genomics 14(4):897-911 (1992); and Russell et al., J. Bact. 189(21)7581-7585 (2007)). At the level of post-translational regulation, enzyme attenuation can include: increasing intracellular concentration of known inhibitors; or modifying post-translational modified sites (Mann et al., Nature Biotech. 21:255-261 (2003)). At the level of enzyme activity, enzyme attenuation can include: adding an endogenous or an exogenous inhibitor, such as an enzyme inhibitor, an antibiotic or a target-specific drug, to reduce enzyme activity; chelating a metal ion that is required for enzyme activity; or introducing a dominant negative mutation. The applicability of a technique for attenuation described above can depend upon whether a given host microbial organism is prokaryotic or eukaryotic, and it is understand that a determination of what is the appropriate technique for a given host can be readily made by one skilled in the art.

In some embodiments, microaerobic designs can be used based on the growth-coupled formation of the desired product. To examine this, production cones can be constructed for each strategy by first maximizing and, subsequently minimizing the product yields at different rates of biomass formation feasible in the network. If the rightmost boundary of all possible phenotypes of the mutant network is a single point, it implies that there is a unique optimum yield of the product at the maximum biomass formation rate possible in the network. In other cases, the rightmost boundary of the feasible phenotypes is a vertical line, indicating that at the point of maximum biomass the network can make any amount of the product in the calculated range, including the lowest amount at the bottommost point of the vertical line. Such designs are given a low priority.

The acetyl-CoA or both oxaloacetate and acetyl-CoA or a bioderived compound production strategies identified herein can be disrupted to increase production of acetyl-CoA or both oxaloacetate and acetyl-CoA or a bioderived compound. Accordingly, the invention also provides a non-naturally occurring microbial organism having metabolic modifications coupling acetyl-CoA or both oxaloacetate and acetyl-CoA or a bioderived compound production to growth of the organism, where the metabolic modifications includes disruption of one or more genes selected from the genes encoding proteins and/or enzymes shown in the various tables disclosed herein.

Each of the strains can be supplemented with additional deletions if it is determined that the strain designs do not sufficiently increase the production of acetyl-CoA or both oxaloacetate and acetyl-CoA or a bioderived compound and/or couple the formation of the product with biomass formation. Alternatively, some other enzymes not known to possess significant activity under the growth conditions can become active due to adaptive evolution or random mutagenesis. Such activities can also be knocked out. However, the genes disclosed herein allows the construction of strains exhibiting high-yield syncthesis or production of acetyl-CoA or both oxaloacetate and acetyl-CoA or a bioderived compound, including growth-coupled production of acetyl-CoA or both oxaloacetate and acetyl-CoA or a bioderived compound.

The invention is described herein with general reference to the metabolic reaction, reactant or product thereof, or with specific reference to one or more nucleic acids or genes encoding an enzyme associated with or catalyzing, or a protein associated with, the referenced metabolic reaction, reactant or product. Unless otherwise expressly stated herein, those skilled in the art will understand that reference to a reaction also constitutes reference to the reactants and products of the reaction. Similarly, unless otherwise expressly stated herein, reference to a reactant or product also references the reaction, and reference to any of these metabolic constituents also references the gene or genes encoding the enzymes that catalyze or proteins involved in the referenced reaction, reactant or product. Likewise, given the well-known fields of metabolic biochemistry, enzymology and genomics, reference herein to a gene or encoding nucleic acid also constitutes a reference to the corresponding encoded enzyme and the reaction it catalyzes or a protein associated with the reaction as well as the reactants and products of the reaction

Depending on the non-natural microorganism capable of producing acetyl-CoA and oxaloacetate having a pathway(s) comprising phosphoketolase and a non-phosphotransferase system (non-PTS) for sugar uptake comprising a genetic modification to a non-PTS component to increase non-PTS activity, and/or (ii) one or more modification(s) to the organism's electron transport chain to enhance efficiency of ATP production, to enhance availability of reducing equivalents, or both. the non-naturally occurring microbial organisms of the invention can include at least one exogenous modification of a nucleic acid(s) from the pathway comprising phosphoketolase, the non-PTS system, or the ETC system. The non-natural microorganism can also include one or more exogenously expressed nucleic acid(s) from a bioderived compound pathway

For example, acetyl-CoA and oxaloacetate biosynthesis can be established in a host deficient in a pathway enzyme or protein through exogenous expression of the corresponding encoding nucleic acid. In a host deficient in all enzymes or proteins of an acetyl-CoA and oxaloacetate pathway, exogenous expression of all enzyme or proteins in the pathway can be included, although it is understood that all enzymes or proteins of a pathway can be expressed even if the host contains at least one of the pathway enzymes or proteins. For example, exogenous expression of all enzymes or proteins in a pathway for production of acetyl-CoA or a bioderived compound or for methanol utilization can be included, such as methanol dehydrogenase (see, e.g.., Figure 1)a fructose-6-phosphate phosphoketolase and a phosphotransacetylase (*see, e.g.* Figure 1), or a xylulose-5-phosphate phosphoketolase and a phosphotransacetylase (*see, e.g.* Figure 1), or a methanol dehydrogenase, a 3-hexulose-6-phosphate synthase, a 6-phospho-3-hexuloisomerase, a fructose-6-phosphate phosphoketolase and a phosphotransacetylase (*see, e.g.* Figure 1), or an acetyl-CoA:acetyl-CoA acyltransferase, an acctoacctyl-CoA reductase (ketone reducing), a 3-hydroxybutyryl-CoA reductase (aldehyde forming), and a 3-hydroxybutyraldehyde reductase (*see, e.g.* Figure 5), or a succinyl-CoA reductase (aldehyde forming) , a 4-HB dehydrogenase, a 4-HB kinase, a phosphotrans-4-hydroxybutyrylase, a 4-hydroxybutyryl-CoA reductase (aldehyde forming), and a 1,4-butanediol dehydrogenase (*see, e.g.* Figure 7), or a 3-oxoadipyl-CoA thiolase, a 3-oxoadipyl-CoA reductase, a 3-hydroxyadipyl-CoA dehydratase, a 5-carboxy-2-pentenoyl-CoA reductase, an adipyl-CoA reductase (aldehyde forming), and 6-aminocaproate transaminase (*see, e.g.* Figure 8), or an acetyl-CoA:acetyl-CoA acyltransferase, an acetoacetyl-CoA reductase (ketone reducing), a 3-hydroxybutyrl-CoA mutase, a 2-hydroxyisobutyryl-CoA dehydratase, and a methacrylyl-CoA synthetase (*see, e.g.* Figure 10).

Given the teachings and guidance provided herein, those skilled in the art will understand that the number of encoding nucleic acids to introduce in an expressible form will, at least, parallel the pathway comprising phosphoketolase for acetyl-CoA and oxaloacetate production, the non-PTS system, and/or the ETC systempathway deficiencies of the selected host microbial organism. Therefore, a non-naturally occurring microbial organism of the invention can have one, two, three, four, five, six, seven, eight, nine, ten, eleven, or twelve up to all nucleic acids encoding the enzymes or proteins constituting a pathway(s) comprising phosphoketolase to acetyl-CoA and oxaloacetate, a non-PTS, or ETC component, pathway disclosed herein. In some embodiments, the non-naturally occurring microbial organisms also can include other genetic modifications that facilitate or optimize acetyl-CoA biosynthesis, sugar uptake through the PTS or non-PTS, or ETC function, or that confer other useful functions onto the host microbial organism. One such other functionality can include, for example, augmentation of the synthesis of one or more of the acetyl-CoA pathway precursors. Optionally, the organism can include augmentation of the synthesis of one or more of the bioderived compound pathway precursors such as Fald, H6P, DHA, G3P, malonyl-CoA, acetoacetyl-CoA, PEP, PYR and Succinyl-CoA.

In some embodiments, a host microbial organism is selected such that it produces the precursor of an acetyl-CoA and oxaloacetate, and optionally further, a bioderived compound pathway, either as a naturally produced molecule or as an engineered product that either provides *de novo* production of a desired precursor or increased production of a precursor naturally produced by the host microbial organism. For example, malonyl-CoA, acetoacetyl-CoA and pyruvate are produced naturally in a host organism such as *E. coli.* A host organism can be engineered to increase production of a precursor, as disclosed herein. In addition, a microbial organism that has been engineered to produce a desired precursor can be used as a host organism and further engineered to express enzymes or proteins of an acetyl-CoA and oxaloacetate, and optionally a bioderived compound pathway.

In some embodiments, a non-naturally occurring microbial organism of the invention is generated from a host that contains the enzymatic capability to synthesize acetyl-CoA and oxaloactetate. In this specific embodiment it can be useful to increase the synthesis or accumulation of acetyl-CoA and oxaloactetate pathway product to, for example, to enhance bioderived compound pathway reactions. In turn, an increase in acetyl-CoA and oxaloactetate can be useful for enhancing a desired bioderived compound production. Increased synthesis or accumulation can be accomplished by, for example, overexpression of nucleic acids encoding one or more of the pathway(s) comprising phosphoketolase to acetyl-CoA and oxaloactetate, the non-PTS, and/or the ETC system, enzymes or proteins. Overexpression of the enzyme or enzymes and/or protein or proteins of the pathway(s) comprising phoshokctolasc, the non-PTS, and/or ETC system can occur, for example, through exogenous expression of the endogenous gene or genes, or through exogenous expression of the heterologous gene or genes. Therefore, naturally occurring organisms can be readily generated to be non-naturally occurring microbial organisms of the invention, for example, producing acetyl-CoA, through overexpression of one, two, three, four, five, six, seven, eight, nine, ten, eleven, twelve, that is, up to all nucleic acids encoding acetyl-CoA pathway enzymes or proteins. In addition, a non-naturally occurring organism can be generated by mutagenesis of an endogenous gene that results in an increase in activity of an enzyme in one or more of the pathway(s) comprising phosphoketolase to acetyl-CoA and oxaloactetate, the non-PTS system, and/or ETC system..

In particularly useful embodiments, exogenous expression of the encoding nucleic acids is employed. Exogenous expression confers the ability to custom tailor the expression and/or regulatory elements to the host and application to achieve a desired expression level that is controlled by the user. However, endogenous expression also can be utilized in other embodiments such as by removing a negative regulatory effector or induction of the gene's promoter when linked to an inducible promoter or other regulatory element. Thus, an endogenous gene having a naturally occurring inducible promoter can be up-regulated by providing the appropriate inducing agent, or the regulatory region of an endogenous gene can be engineered to incorporate an inducible regulatory element, thereby allowing the regulation of increased expression of an endogenous gene at a desired time. Similarly, an inducible promoter can be included as a regulatory element for an exogenous gene introduced into a non-naturally occurring microbial organism.

It is understood that, in methods of the invention, any of the one or more exogenous nucleic acids can be introduced into a microbial organism to produce a non-naturally occurring microbial organism of the disclosure. The nucleic acids can be introduced so as to confer, for example, an acetyl-CoA pathway onto the microbial organism. Alternatively, encoding nucleic acids can be introduced to produce an intermediate microbial organism having the biosynthetic capability to catalyze some of the required reactions to confer acetyl-CoA biosynthetic capability. For example, a non-naturally occurring microbial organism having an acetyl-CoA pathway can comprise at least two exogenous nucleic acids encoding desired enzymes or proteins, such as the combination of a 3-hexulose-6-phosphate synthase and a fructose-6-phosphate phosphoketolase, or alternatively a xylulose-5-phosphate phosphoketolase and an acetyl-CoA transferase, or alternatively a fructose-6-phosphate phosphoketolase and a formate reductase, or alternatively a xylulose-5-phosphate phosphoketolase and a methanol dehydrogenase, and the like. Thus, it is understood that any combination of two or more enzymes or proteins of a biosynthetic pathway can be included in a non-naturally occurring microbial organism of the invention.

The organism can include an exogenous nucleic acid encoding an enzyme or protein of the pathway(s) to acetyl-CoA and oxaloactetate comprising phosphoketolase, and can include modification of one or more natural or exogenous nucleic acids encoding an enzyme or protein of the non-PTS, and/or of an ETC system. For example, in some embodiments one or more exogenous nucleic acid(s) encoding an enzyme or protein of the pathway(s) to acetyl-CoA and oxaloactetate comprising phosphoketolase is introduced into an organism along with one or more modifications to an organism that provide or modify a non-phosphotransferase system (non-PTS) for sugar uptake, wherein the modification increases non-PTS activity. For example, the non-PTS modification can be one where the non-PTS for sugar uptake is introduced into an organism that does not have a non-PTS, or an organism having an endogenous (naturally-occurring or native ) non-PTS can be modified to increase the activity or expression of one or more natural enzymes or proteins of the non-PTS. Optionally, this organism can also include one or more genetic modification(s) that attenuates or eliminates a PTS activity.

In other embodiments, for example, one or more exogenous nucleic acid(s) encoding an enzyme or protein of the pathway(s) to acetyl-CoA and oxaloactetate comprising phosphoketolase is introduced into an organism along with one or more genetic modifications of an ETC component(s). For example, the genetic modification that can enhance efficiency of ATP production can be (i) attenuation or elimination of an NADH-dependent dehydrogenase (e.g., Ndh, WrbA or YhdH, YieF, YtfG, Qor , MdaB) that does not translocate protons, or (ii) attenuation or elimination of a first cytochrome oxidase (e.g., CydAB or AppBC or YgiN) that has a lower efficiency of proton translocation per pair of electrons as compared to a second cytochrome oxidase. Energetic efficiency of the cell is thus increased. In another approach, the genetic modification can also increase expression or activity of a native or heterologous Complex I enzyme or protein and of cytochrome oxidases, such as by attenuating arcA. In another approach, the genetic modification that enhances the availability or synthesis of a reducing equivalent, can be done by using a genetic modification to increase the expression or activity of a pyruvate dehydrogenase, a pyruvate formate lyase together with an NAD(P)H-generating formate dehydrogenase.

Similarly, it is understood that any combination of three or more enzymes or proteins of a biosynthetic pathway can be included in a non-naturally occurring microbial organism of the disclosure, for example, a methanol dehydrogenase, a fructose-6-phosphate aldolase, and a fructose-6-phosphate phosphoketolase, or alternatively a fructose-6-phosphate phosphoketolase and a 3-hydroxybutyraldehyde reductase, or alternatively a xylulose-5-phosphate phosphoketolase, a pyruvate formate lyase and a 4-hydroxybutyryl-CoA reductase (alcohol forming), or alternatively a fructose-6-phosphate aldolase, a phosphotransacetylase, and a 3-hydroxyisobutyrate dehydratase, and so forth, as desired, so long as the combination of enzymes and/or proteins of the desired biosynthetic pathway results in production of the corresponding desired product. Similarly, any combination of four, five, six, seven, eight, nine, ten, eleven, twelve or more enzymes or proteins of a biosynthetic pathway as disclosed herein can be included in a non-naturally occurring microbial organism of the invention, as desired, so long as the combination of enzymes and/or proteins of the desired biosynthetic pathway results in production of the corresponding desired product.

In addition to the biosynthesis of acetyl-CoA and oxaloacetate as described herein, the non-naturally occurring microbial organisms and methods of the invention also can be utilized in various combinations with each other and/or with other microbial organisms and methods well known in the art to achieve product biosynthesis by other routes. For example, a non-natural organism having pathway(s) to acetyl-CoA and oxaloacetate comprising phosphoketolase, and that includes a non-PTS system modification, or an ETC system modification can be used to produce acetyl-CoA and oxaloacetate, which in turn can be utilized by a second organism capable of utilizing acetyl-CoA and/or oxaloacetate as a precursor in a bioderived compound pathway for the production of a desired product.

For example, the acetyl-CoA and/or oxaloacetate can be added directly to another culture of the second organism or the original culture of the acetyl-CoA and/or oxaloacetate can be depleted of these microbial organisms by, for example, cell separation, and then subsequent addition of the second organism to the fermentation broth can be utilized to produce the final product without intermediate purification steps.

In other embodiments, the non-naturally occurring microbial organisms and methods of the invention can be assembled in a wide variety of subpathways to achieve biosynthesis of, for example, acetyl-CoA and oxaloacctatc, or optionally any a bioderived compound that uses acetyl-CoA and oxaloacetate in a pathway. In these embodiments, biosynthetic pathways for a desired product of the disclosure can be segregated into different microbial organisms, and the different microbial organisms can be co-cultured to produce the final product. In such a biosynthetic scheme, the product of one microbial organism is the substrate for a second microbial organism until the final product is synthesized. For example, the biosynthesis of acetyl-CoA and oxaloacetate, and optionally any bioderived compound, can be accomplished by constructing a microbial organism that contains biosynthetic pathways for conversion of one pathway intermediate to another pathway intermediate or the product. Alternatively, acetyl-CoA and oxaloacetate, and optionally any bioderived compound also can be biosynthetically produced from microbial organisms through co-culture or co-fermentation using two organisms in the same vessel, where the first microbial organism produces an acetyl-CoA and oxaloacetate or a bioderived compound intermediate and the second microbial organism converts the intermediate , acetyl-CoA, or oxaloacetate to a bioderived compound.

Given the teachings and guidance provided herein, those skilled in the art will understand that a wide variety of combinations and permutations exist for the non-naturally occurring microbial organisms and methods of the invention together with other microbial organisms, with the co-culture of other non-naturally occurring microbial organisms having subpathways and with combinations of other chemical and/or biochemical procedures well known in the art to produce acetyl-CoA and oxaloacetate, and optionally, further, a bioderived compound.

Similarly, it is understood by those skilled in the art that a host organism can be selected based on desired characteristics for introduction of one or more gene disruptions to increase synthesis or production of acetyl-CoA and oxaloacetate, and optionally, further, a bioderived compound. Thus, it is understood that, if a genetic modification is to be introduced into a host organism to disrupt a gene, any homologs, orthologs or paralogs that catalyze similar, yet non-identical metabolic reactions can similarly be disrupted to ensure that a desired metabolic reaction is sufficiently disrupted. Because certain differences exist among metabolic networks between different organisms, those skilled in the art will understand that the actual genes disrupted in a given organism may differ between organisms. However, given the teachings and guidance provided herein, those skilled in the art also will understand that the methods of the invention can be applied to any suitable host microorganism to identify the cognate metabolic alterations needed to construct an organism in a species of interest that will increase acetyl-CoA and optionally, further, a bioderived compound biosynthesis. In a particular embodiment, the increased production couples biosynthesis of acetyl-CoA and oxaloacetate and a bioderived compound to growth of the organism, and can obligatorily couple production of acetyl-CoA and a bioderived compound to growth of the organism if desired and as disclosed herein.

The non-naturally occurring microbial organisms of the invention can be produced by introducing expressible nucleic acids encoding one or more of the enzymes or proteins participating in one or more of the acetyl-CoA, or acetyl-CoA and oxaloacetate pathway comprising a phosphoketolase, (ii) a modification to enhance the non-PTS for sugar uptake, and/or (iii) one or more modification(s) to the organism's electron transport chain (ETC) to enhance efficiency of ATP production, to enhance availability of reducing equivalents, or both, or a bioderived compound biosynthetic pathways. Depending on the host microbial organism chosen for biosynthesis, nucleic acids for some or all of these pathways or systems can be expressed. For example, if a chosen host is deficient in one or more enzymes or proteins for a desired biosynthetic pathway, then expressible nucleic acids for the deficient enzyme(s) or protein(s) are introduced into the host for subsequent exogenous expression. Alternatively, if the chosen host exhibits endogenous expression of some pathway genes, but is deficient in others, then an encoding nucleic acid is needed for the deficient enzyme(s) or protein(s) to achieve acetyl-CoA and oxaloacetate production, the PTS, the non-PTS, the ETC modification, or a bioderived compound biosynthesis. Thus, a non-naturally occurring microbial organism of the invention can be produced by introducing exogenous enzyme or protein activities to obtain a desired biosynthetic pathway or a desired biosynthetic pathway can be obtained by introducing one or more exogenous enzyme or protein activities that, together with one or more endogenous enzymes or proteins, produces a desired product such as acetyl-CoA or a bioderived compound.

Host microbial organisms can be selected from, and the non-naturally occurring microbial organisms generated in, for example, bacteria, yeast, fungus or any of a variety of other microorganisms applicable or suitable to fermentation processes. Exemplary bacteria include any species selected from the order *Enterobacteriales,* family *Enterobacteriaceae,* including the genera *Escherichia* and *Klebsiella;* the order *Aeromonadales,* family *Succinivibrionaceae,* including the genus *Anaerobiospirillum;* the order *Pasteurellules,* family *Pasteurellaceae,* including the genera *Actinobacillus* and *Mannheimia;* the order *Rhizobiales,* family *Bradyrhizobiaceae,* including the genus *Rhizobium;* the order *Bacillales,* family *Bacillaceae,* including the genus *Bacillus;* the order *Actinomycetales,* families *Corynebacteriaceae* and *Streptomycetaceae,* including the genus *Corynebacterium* and the genus *Streptomyces,* respectively; order *Rhodospirillales,* family *Acetobacteraceae,* including the genus *Gluconobacter,* the order *Sphingomonadales,* family *Sphingomonadaceae,* including the genus *Zymomonas;* the order *Lactobacillales,* families *Lactobacillaceae* and *Streptococcaceae,* including the genus *Lactobacillus* and the genus *Lactococcus,* respectively; the order *Clostridiales,* family *Clostridiaceae,* genus *Clostridium;* and the order *Pseudomonadales,* family *Pseudomonadaceae,* including the genus *Pseudomonas.* Non-limiting species of host bacteria include *Escherichia coli, Klebsiella oxytoca, Anaerobiospirillum succiniciproducens, Actinobacillus succinogenes, Mannheimia succiniciproducens, Rhizobium etli, Bacillus subtilis, Corynebacterium glutamicum, Gluconobacter oxydans, Zymomonas mobilis, Lactococcus lactis, Lactobacillus plantarum, Streptomyces coelicolor, Clostridium acetobutylicum, Pseudomonas fluorescens,* and *Pseudomonas putida.* Exemplarly bacterial methylotrophs include, for example, *Bacillus, Methylobacterium, Methyloversatilis, Methylococcus, Methylocystis* and *Hyphomicrobium.*

Similarly, exemplary species of yeast or fungi species include any species selected from the order *Scacchcaromycetales,* family *Saccaromycetaceae,* including the genera *Saccharomyces, Kluyveromyces* and *Pichia;* the order *Saccharomycetales,* family *Dipodascaceae,* including the genus *Yarrowia;* the order *Schizosaccharomycetales,* family *Schizosaccaromycetaceae,* including the genus *Schizosaccharomyces*; the order *Eurotiales,* family *Trichocomaceae,* including the genus *Aspergillus*; and the order *Mucorales,* family *Mucoraceae,* including the genus *Rhizopus.* Non-limiting species of host yeast or fungi include *Saccharomyces cerevisiae, Schizosaccharomyces pombe, Kluyveromyces lactis, Kluyveromyces marxianus, Aspergillus terreus, Aspergillus niger, Pichia pastoris, Rhizopus arrhizus, Rhizobus oryzae, Yarrowia lipolytica,* and the like. *E. coli* and C. glutamicum are particularly useful host organisms since they are well characterized microbial organism suitable for genetic engineering. Other particularly useful host organisms include yeast such as *Saccharomyces cerevisiae* and yeasts or fungi selected from the genera *Saccharomyces, Schizosaccharomyces, Schizochytrium, Rhodotorula, Thraustochytrium, Aspergillus, Kluyveromyces, Issatchenkia, Yarrowia, Candida, Pichia, Ogataea, Kuraishict, Hansenula and Komagataella. Useful host organisms include Saccharomyces cerevisiae, Schizosaccharomyces pombe, Hansenula polymorpha, Pichia methanolica, Candida boidinii, Kluyveromyces lactis, Kluyveromyces marxianus, Aspergillus terreus, Aspergillus niger, Pichia pastoris, Rhizopus arrhizus, Rhizobus oryzae, Yarrowia lipolytica, Issatchenkia orientalis* and the like. It is understood that any suitable microbial host organism can be used to introduce metabolic and/or genetic modifications to produce a desired product.

As used herein, the terms "non-natural" and "non-naturally occurring" when used in reference to a microbial organism or microorganism of the invention are intended to mean that the microbial organism has at least one genetic alteration not normally found in a naturally occurring strain of the referenced species, including wild-type strains of the referenced species. Genetic alterations include, for example, modifications introducing expressible nucleic acids encoding metabolic polypeptides, other nucleic acid additions, nucleic acid deletions and/or other functional disruption of the microbial organism's genetic material. Such modifications include, for example, coding regions and functional fragments thereof, for heterologous, homologous or both heterologous and homologous polypeptides for the referenced species. Additional modifications include, for example, noncoding regulatory regions in which the modifications alter expression of a gene or opcron. Exemplary metabolic polypeptides include enzymes or proteins within a pathway to acetyl-CoA, or both oxaloacetate and acetyl-CoA , or bioderived compound biosynthetic pathway.

A metabolic modification refers to a biochemical reaction that is altered from its naturally occurring state. Therefore, non-naturally occurring microorganisms can have genetic modifications to nucleic acids encoding metabolic polypeptides, or functional fragments thereof. Exemplary metabolic modifications are disclosed herein.

As used herein, the term "isolated" when used in reference to a microbial organism is intended to mean an organism that is substantially free of at least one component as the referenced microbial organism is found in nature. The term includes a microbial organism that is removed from some or all components as it is found in its natural environment. The term also includes a microbial organism that is removed from some or all components as the microbial organism is found in non-naturally occurring environments. Therefore, an isolated microbial organism is partly or completely separated from other substances as it is found in nature or as it is grown, stored or subsisted in non-naturally occurring environments. Specific examples of isolated microbial organisms include partially pure microbes, substantially pure microbes and microbes cultured in a medium that is non-naturally occurring.

As used herein, the terms "microbial," "microbial organism" or "microorganism" are intended to mean any organism that exists as a microscopic cell that is included within the domains of archaea, bacteria or eukarya. Therefore, the term is intended to encompass prokaryotic or eukaryotic cells or organisms having a microscopic size and includes bacteria, archaca and cubactcria of all species as well as eukaryotic microorganisms such as yeast and fungi. The term also includes cell cultures of any species that can be cultured for the production of a biochemical. The terms "bacterial," and "bacterial organism" microbial organism are intended to mean any organism that exists as a microscopic cell within the domain of bacteria.

As used herein, the term "CoA" or "coenzyme A" is intended to mean an organic cofactor or prosthetic group (nonprotein portion of an enzyme) whose presence is required for the activity of many enzymes (the apoenzyme) to form an active enzyme system. Coenzyme A functions in certain condensing enzymes, acts in acetyl or other acyl group transfer and in fatty acid synthesis and oxidation, pyruvate oxidation and in other acetylation.

As used herein, the term "substantially anaerobic" when used in reference to a culture or growth condition is intended to mean that the amount of oxygen is less than about 10% of saturation for dissolved oxygen in liquid media. The term also is intended to include sealed chambers of liquid or solid medium maintained with an atmosphere of less than about 1% oxygen.

"Exogenous" as it is used herein is intended to mean that the referenced molecule or the referenced activity is introduced into the host microbial organism. The molecule can be introduced, for example, by introduction of an encoding nucleic acid into the host genetic material such as by integration into a host chromosome or as non-chromosomal genetic material such as a plasmid. Therefore, the term as it is used in reference to expression of an encoding nucleic acid refers to introduction of the encoding nucleic acid in an expressible form into the microbial organism. Included is any nucleic acid encoding a polypeptide in which its regulatory region, e.g. promoter, terminator, enhancer, has been changed from it native sequence. For example, modifying a native gene by replacing its promoter with a weaker or stronger results in an exogenous nucleic acid (or gene) encoding the referenced polypeptide. When used in reference to a biosynthetic activity, the term refers to an activity that is introduced into the host reference organism. The biosynthetic activity can be achieved by modifying a regulatory region, e.g. promoter, terminator, enhancer, to produce the biosynthetic activity from a native gene. For example, modifying a native gene by replacing its promoter with a constitutive or inducible promoter results in an exogenous biosynthetic activity. The source can be, for example, a homologous or heterologous encoding nucleic acid that expresses the referenced activity following introduction into the host microbial organism. Therefore, the term "endogenous" refers to a referenced molecule or activity that is present in the host. Similarly, the term when used in reference to expression of an encoding nucleic acid refers to expression of an encoding nucleic acid contained within the microbial organism. The term "heterologous" refers to a molecule or activity derived from a source other than the referenced species whereas "homologous" refers to a molecule or activity derived from the host microbial organism. Accordingly, exogenous expression of an encoding nucleic acid of the invention can utilize either or both a heterologous or homologous encoding nucleic acid.

It is understood that when more than one exogenous nucleic acid is included in a microbial organism that the more than one exogenous nucleic acids refers to the referenced encoding nucleic acid or biosynthetic activity, as discussed above. It is further understood, as disclosed herein, that such more than one exogenous nucleic acids can be introduced into the host microbial organism on separate nucleic acid molecules, on polycistronic nucleic acid molecules, or a combination thereof, and still be considered as more than one exogenous nucleic acid. For example, as disclosed herein a microbial organism can be engineered to express two or more exogenous nucleic acids encoding a desired pathway enzyme or protein. In the case where two exogenous nucleic acids encoding a desired activity are introduced into a host microbial organism, it is understood that the two exogenous nucleic acids can be introduced as a single nucleic acid, for example, on a single plasmid, on separate plasmids, can be integrated into the host chromosome at a single site or multiple sites, and still be considered as two exogenous nucleic acids. Similarly, it is understood that more than two exogenous nucleic acids can be introduced into a host organism in any desired combination, for example, on a single plasmid, on separate plasmids, can be integrated into the host chromosome at a single site or multiple sites, and still be considered as two or more exogenous nucleic acids, for example three exogenous nucleic acids. Thus, the number of referenced exogenous nucleic acids or biosynthetic activities refers to the number of encoding nucleic acids or the number of biosynthetic activities, not the number of separate nucleic acids introduced into the host organism.

As used herein, the phrase "enhance carbon flux" is intended to mean to intensify, increase, or further improve the extent or flow of metabolic carbon through or to a desired pathway, pathway product, intermediate, or bioderived compound. The intensity, increase or improvement can be relative to a predetermined baseline of a pathway product, intermediate or bioderived compound. For example, an increased yield of acetyl-CoA can be achieved per mole of methanol with a phosphoketolase enzyme described herein (*see, e.g.,* Figure 1) than in the absence of a phosphoketolase enzyme. Since an increased yield of acetyl-CoA can be achieved, a higher yield of acetyl-CoA derived compounds, such as 1,3-butanediol, crotyl alcohol, butadiene, 3-buten-2-ol, 2,4-pentadienoate, 1,4-butanediol, adipate, 6-aminocaproate, caprolactam, hexamethylenediamine, fatty alcohols such hexanol, octanol and dodecanol, propylene, isoprene, isopropanol, butanol, methacrylic acid and 2-hydroxyisobutyric acid the invention, can also be achieved.

As used herein, the term "gene disruption," or grammatical equivalents thereof, is intended to mean a genetic alteration that renders the encoded gene product inactive or attenuated. The genetic alteration can be, for example, deletion of the entire gene, deletion of a regulatory sequence required for transcription or translation, deletion of a portion of the gene which results in a truncated gene product, or by any of various mutation strategies that inactivate or attenuate the encoded gene product, for example, replacement of a gene's promoter with a weaker promoter, replacement or insertion of one or more amino acid of the encoded protein to reduce its activity, stability or concentration, or inactivation of a gene's transactivating factor such as a regulatory protein. One particularly useful method of gene disruption is complete gene deletion because it reduces or eliminates the occurrence of genetic reversions in the non-naturally occurring microorganisms of the invention. A gene disruption also includes a null mutation, which refers to a mutation within a gene or a region containing a gene that results in the gene not being transcribed into RNA and/or translated into a functional gene product. Such a null mutation can arise from many types of mutations including, for example, inactivating point mutations, deletion of a portion of a gene, entire gene deletions, or deletion of chromosomal segments.

As used herein, the term "growth-coupled" when used in reference to the production of a biochemical product is intended to mean that the biosynthesis of the referenced biochemical product is produced during the growth phase of a microorganism. In a particular embodiment, the growth-coupled production can be obligatory, meaning that the biosynthesis of the referenced biochemical is an obligatory product produced during the growth phase of a microorganism.

As used herein, the term "attenuate," or grammatical equivalents thereof, is intended to mean to weaken, reduce or diminish the activity or amount of an enzyme or protein compared to the activity of the naturally occurring enzyme which may be zero because it is not present. Attenuation of the activity or amount of an enzyme or protein can mimic complete disruption if the attenuation causes the activity or amount to fall below a critical level required for a given pathway to function. However, the attenuation of the activity or amount of an enzyme or protein that mimics complete disruption for one pathway, can still be sufficient for a separate pathway to continue to function. For example, attenuation of an endogenous enzyme or protein can be sufficient to mimic the complete disruption of the same enzyme or protein for production of acetyl-CoA or a biodcrivcd compound of the invention, but the remaining activity or amount of enzyme or protein can still be sufficient to maintain other pathways, such as a pathway that is critical for the host microbial organism to survive, reproduce or grow. Attenuation of an enzyme or protein can also be weakening, reducing or diminishing the activity or amount of the enzyme or protein in an amount that is sufficient to increase yield of acetyl-CoA or a bioderived compound of the invention, but does not necessarily mimic complete disruption of the enzyme or protein.. As used herein, the term "eliminated," when referring to an enzyme or protein (or other molecule) or its activity, means the enzyme or protein or its activity is not present in the cell. Expression of an enzyme or protein can be eliminated when the nucleic acid that normally encodes the enzyme or protein, is not expressed.

Comparatively, if an enzyme or protein (or other molecule), such as one in a modified form, exhibits activity greater than the activity of its wild-type form, its activity is referred to as "enhanced" or "increased." This includes a modification where there was an absence in the host organism of the enzyme, protein, other molecule or activity to be enhanced or increased. For example, the inclusion and expression of an exogenous or heterologous nucleic acid in a host that otherwise in a wild-type form does not have the nucleic acid can be referred to as "enhanced" or "increased." Likewise, if an enzyme is expressed in a non-natural cell in an amount greater than the amount expressed in the natural (unmodified) cell (including where the enzyme is absent in the starting cell), its expression is referred to as "enhanced" or "upregulated."

As used herein, the term "bioderived" means derived from or synthesized by a biological organism and can be considered a renewable resource since it can be generated by a biological organism. Such a biological organism, in particular the microbial organisms of the invention, can utilize a variety of carbon sources described herein including feedstock or biomass, such as, sugars and carbohydrates obtained from an agricultural, plant, bacterial, or animal source. Alternatively, the biological organism can utilize, for example, atmospheric carbon and/or methanol as a carbon source.

As used herein, the term "biobased" means a product as described herein that is composed, in whole or in part, of a bioderived compound of the invention. A biobased product is in contrast to a petroleum based product, wherein such a product is derived from or synthesized from petroleum or a petrochemical feedstock.

A "bioderived compound," as used herein, refers to a target molecule or chemical that is derived from or synthesized by a biological organism. In the context of the present invention, engineered microbial organisms are used to produce a bioderived compound or intermediate thereof via acetyl-CoA, including optionally further through acetoacetyl-CoA, malonyl-CoA and/or succinyl-CoA. Biodcrivcd compounds of the invention include, but are not limited to, alcohols, glycols, organic acids, alkenes, dienes, organic amines, organic aldehydes, vitamins, nutraceuticals and pharmaceuticals.

The non-naturally occurring microbial organisms of the invention can contain stable genetic alterations, which refers to microorganisms that can be cultured for greater than five generations without loss of the alteration. Generally, stable genetic alterations include modifications that persist greater than 10 generations, particularly stable modifications will persist more than about 25 generations, and more particularly, stable genetic modifications will be greater than 50 generations, including indefinitely.

In the case of gene disruptions, a particularly useful stable genetic alteration is a gene deletion. The use of a gene deletion to introduce a stable genetic alteration is particularly useful to reduce the likelihood of a reversion to a phenotype prior to the genetic alteration. For example, stable growth-coupled production of a biochemical can be achieved, for example, by deletion of a gene encoding an enzyme catalyzing one or more reactions within a set of metabolic modifications. For example, a non-natural organism of the current disclosure can include a gene deletion of pyruvate kinase, a gene deletion of an enzyme or protein of the PTS, such as deletion of ptsI, a deletion of a cytochrome oxidase that has a lower efficiency of proton translocation per pair of electrons, such as deletion of CydAB or AppBC or YgiN, or deletion of a NADH-dependent dehydrogenase that does not translocate protons, such as deletion of Ndh, WrbA, YhdH, YieF, YtfG, Qor, MdaB, or combinations thereof. The stability of growth-coupled production of a biochemical can be further enhanced through multiple deletions, significantly reducing the likelihood of multiple compensatory reversions occurring for each disrupted activity.

Those skilled in the art will understand that the genetic alterations, including metabolic modifications exemplified herein, are described with reference to a suitable host organism such as *E. coli* and their corresponding metabolic reactions or a suitable source organism for desired genetic material such as genes for a desired metabolic pathway. However, given the complete genome sequencing of a wide variety of organisms and the high level of skill in the area of genomics, those skilled in the art will readily be able to apply the teachings and guidance provided herein to essentially all other organisms. For example, the *E. coli* metabolic alterations exemplified herein can readily be applied to other species by incorporating the same or analogous encoding nucleic acid from species other than the referenced species. Such genetic alterations include, for example, genetic alterations of species homologs, in general, and in particular, orthologs, paralogs or nonorthologous gene displacements.

An ortholog is a gene or genes that are related by vertical descent and are responsible for substantially the same or identical functions in different organisms. For example, mouse epoxide hydrolase and human epoxide hydrolase can be considered orthologs for the biological function of hydrolysis of epoxides. Genes are related by vertical descent when, for example, they share sequence similarity of sufficient amount to indicate they are homologous, or related by evolution from a common ancestor. Genes can also be considered orthologs if they share three-dimensional structure but not necessarily sequence similarity, of a sufficient amount to indicate that they have evolved from a common ancestor to the extent that the primary sequence similarity is not identifiable. Genes that are orthologous can encode proteins with sequence similarity of about 25% to 100% amino acid sequence identity. Genes encoding proteins sharing an amino acid similarity less than 25% can also be considered to have arisen by vertical descent if their three-dimensional structure also shows similarities. Members of the serine protease family of enzymes, including tissue plasminogen activator and elastase, are considered to have arisen by vertical descent from a common ancestor.

Orthologs include genes or their encoded gene products that through, for example, evolution, have diverged in structure or overall activity. For example, where one species encodes a gene product exhibiting two functions and where such functions have been separated into distinct genes in a second species, the three genes and their corresponding products arc considered to be orthologs. For the production of a biochemical product, those skilled in the art will understand that the orthologous gene harboring the metabolic activity to be introduced or disrupted is to be chosen for construction of the non-naturally occurring microorganism. An example of orthologs exhibiting separable activities is where distinct activities have been separated into distinct gene products between two or more species or within a single species. A specific example is the separation of elastase proteolysis and plasminogen proteolysis, two types of serine protease activity, into distinct molecules as plasminogen activator and elastase. A second example is the separation of mycoplasma 5'-3' exonuclease and *Drosophila* DNA polymerase III activity. The DNA polymerase from the first species can be considered an ortholog to either or both of the exonuclease or the polymerase from the second species and vice versa.

In contrast, paralogs are homologs related by, for example, duplication followed by evolutionary divergence and have similar or common, but not identical functions. Paralogs can originate or derive from, for example, the same species or from a different species. For example, microsomal epoxide hydrolase (epoxide hydrolase I) and soluble epoxide hydrolase (epoxide hydrolase IT) can be considered paralogs because they represent two distinct enzymes, co-evolved from a common ancestor, that catalyze distinct reactions and have distinct functions in the same species. Paralogs are proteins from the same species with significant sequence similarity to each other suggesting that they are homologous, or related through co-evolution from a common ancestor. Groups of paralogous protein families include HipA homologs, luciferase genes, peptidases, and others.

A nonorthologous gene displacement is a nonorthologous gene from one species that can substitute for a referenced gene function in a different species. Substitution includes, for example, being able to perform substantially the same or a similar function in the species of origin compared to the referenced function in the different species. Although generally, a nonorthologous gene displacement will be identifiable as structurally related to a known gene encoding the referenced function, less structurally related but functionally similar genes and their corresponding gene products nevertheless will still fall within the meaning of the term as it is used herein. Functional similarity requires, for example, at least some structural similarity in the active site or binding region of a nonorthologous gene product compared to a gene encoding the function sought to be substituted. Therefore, a nonorthologous gene includes, for example, a paralog or an unrelated gene.

Therefore, in identifying and constructing the non-naturally occurring microbial organisms of the invention having acetyl-CoA or a bioderived compound biosynthetic capability, those skilled in the art will understand with applying the teaching and guidance provided herein to a particular species that the identification of metabolic modifications can include identification and inclusion or inactivation of orthologs. To the extent that paralogs and/or nonorthologous gene displacements are present in the referenced microorganism that encode an enzyme catalyzing a similar or substantially similar metabolic reaction, those skilled in the art also can utilize these evolutionally related genes. Similarly for a gene disruption, evolutionally related genes can also be disrupted or deleted in a host microbial organism to reduce or eliminate functional redundancy of enzymatic activities targeted for disruption.

Orthologs, paralogs and nonorthologous gene displacements can be determined by methods well known to those skilled in the art. For example, inspection of nucleic acid or amino acid sequences for two polypeptides will reveal sequence identity and similarities between the compared sequences. Based on such similarities, one skilled in the art can determine if the similarity is sufficiently high to indicate the proteins are related through evolution from a common ancestor. Algorithms well known to those skilled in the art, such as Align, BLAST, Clustal W and others compare and determine a raw sequence similarity or identity, and also determine the presence or significance of gaps in the sequence which can be assigned a weight or score. Such algorithms also are known in the art and are similarly applicable for determining nucleotide sequence similarity or identity. Parameters for sufficient similarity to determine relatedness are computed based on well-known methods for calculating statistical similarity, or the chance of finding a similar match in a random polypeptide, and the significance of the match determined. A computer comparison of two or more sequences can, if desired, also be optimized visually by those skilled in the art. Related gene products or proteins can be expected to have a high similarity, for example, 25% to 100% sequence identity. Proteins that are unrelated can have an identity which is essentially the same as would be expected to occur by chance, if a database of sufficient size is scanned (about 5%). Sequences between 5% and 24% may or may not represent sufficient homology to conclude that the compared sequences are related. Additional statistical analysis to determine the significance of such matches given the size of the data set can be carried out to determine the relevance of these sequences.

Exemplary parameters for determining relatedness of two or more sequences using the BLAST algorithm, for example, can be as set forth below. Briefly, amino acid sequence alignments can be performed using BLASTP version 2.0.8 (Jan-05-1999) and the following parameters: Matrix: 0 BLOSUM62; gap open: 11; gap extension: 1; x_dropoff: 50; expect: 10.0; wordsize: 3; filter: on. Nucleic acid sequence alignments can be performed using BLASTN version 2.0.6 (Sept-16-1998) and the following parameters: Match: 1; mismatch: -2; gap open: 5; gap extension: 2; x_dropoff: 50; expect: 10.0; wordsize: 11; filter: off. Those skilled in the art will know what modifications can be made to the above parameters to either increase or decrease the stringency of the comparison, for example, and determine the relatedness of two or more sequences.

The non-naturally occurring microbial organisms of the invention can contain stable genetic alterations, which refers to microorganisms that can be cultured for greater than five generations without loss of the alteration. Generally, stable genetic alterations include modifications that persist greater than 10 generations, particularly stable modifications will persist more than about 25 generations, and more particularly, stable genetic modifications will be greater than 50 generations, including indefinitely.

In the case of gene disruptions, a particularly useful stable genetic alteration is a gene deletion. The use of a gene deletion to introduce a stable genetic alteration is particularly useful to reduce the likelihood of a reversion to a phenotype prior to the genetic alteration. For example, stable growth-coupled production of a biochemical can be achieved, for example, by deletion of a gene encoding an enzyme catalyzing one or more reactions within a set of metabolic modifications. The stability of growth-coupled production of a biochemical can be further enhanced through multiple deletions, significantly reducing the likelihood of multiple compensatory reversions occurring for each disrupted activity.

Those skilled in the art will understand that the genetic alterations, including metabolic modifications exemplified herein, are described with reference to a suitable host organism such as *E. coli* and their corresponding metabolic reactions or a suitable source organism for desired genetic material such as genes for a desired metabolic pathway. However, given the complete genome sequencing of a wide variety of organisms and the high level of skill in the area of genomics, those skilled in the art will readily be able to apply the teachings and guidance provided herein to essentially all other organisms. For example, the *E. coli* metabolic alterations exemplified herein can readily be applied to other species by incorporating the same or analogous encoding nucleic acid from species other than the referenced species. Such genetic alterations include, for example, genetic alterations of species homologs, in general, and in particular, orthologs, paralogs or nonorthologous gene displacements.

Alcohols of the invention, including biofuel alcohols, include primary alcohols, secondary alcohols, diols and triols, preferably having C3 to C10 carbon atoms. Alcohols include n-propanol and isopropanol. Biofuel alcohols are preferably C3-C10 and include 1-Propanol, Isopropanol, 1-Butanol, Isobutanol, 1-Pentanol, Isopentenol, 2-Methyl-1-butanol, 3-Methyl-1-butanol, 1-Hexanol, 3-Methyl-1-pentanol, 1-Heptanol, 4-Methyl-1-hexanol, and 5-Methyl-1-hexanol. Diols include propanediols and butanediols, including 1,4 butanediol, 1,3-butanediol and 2,3-butanediol. Fatty alcohols include C4-C27 fatty alcohols, including C12-C18, especially C12-C14, including saturate or unsaturated linear fatty alcohols.

Further exemplary bioderived compounds of the invention include: (a) 1,4-butanediol and intermediates thereto, such as 4-hydroxybutanoic acid (4-hydroxybutanoate, 4-hydroxybutyratc (4-HB); (b) butadiene (1,3-butadiene) and intermediates thereto, such as 1,4-butanediol, 1,3-butanediol, 2,3-butanediol, crotyl alcohol, 3-buten-2-ol (methyl vinyl carbinol), 2,4-pentadienoate and 3-buten-1-ol; (c) 1,3-butanediol and intermediates thereto, such as 3-hydroxybutyrate (3-HB), 2,4-pentadienoate, crotyl alcohol or 3-butcn-1-ol; (d) adipate, 6-aminocaproic acid (6-ACA), caprolactam, hexamethylenediamine (HMDA) and levulinic acid and their intermediates, e.g. adipyl-CoA, 4-aminobutyryl-CoA; (e) methacrylic acid (2-methyl-2-propenoic acid) and its esters, such as methyl methacrylate and methyl methacrylate (known collectively as methacrylates), 3-hydroxyisobutyratc and/or 2-hydroxyisobutyrate and their intermediates; (f) glycols, including 1,2-propanediol (propylene glycol), 1,3-propanediol, glycerol, ethylene glycol, diethylene glycol, triethylene glycol, dipropylene glycol, tripropylene glycol, neopentyl glycol and bisphenol A and their intermediates; (g) other olefins including propylene and isoprenoids, (h) succinic acid and intermediates thereto; and (i) fatty alcohols, which are aliphatic compounds containing one or more hydroxyl groups and a chain of 4 or more carbon atoms, or fatty acids and fatty aldehydes thereof, which are preferably C4-C27 carbon atoms. Fatty alcohols include saturated fatty alcohols, unsaturated fatty alcohols and linear saturated fatty alcohols. Examples fatty alcohols include butyl, pentyl, hexyl, heptyl, octyl, nonyl, decyl, undecyl and dodecyl alcohols, and their corresponding oxidized derivatives, *i.e.* fatty aldehydes or fatty acids having the same number of carbon atoms. Preferred fatty alcohols, fatty aldehydes and fatty acids have C8 to C18 carbon atoms, especially C12-C18, C12-C14, and C16-C18, including C12, C13, C14, C15, C16, C17, and C18 carbon atoms. Preferred fatty alcohols include linear unsaturated fatty alcohols, such as dodecanol (C12; lauryl alcohol), tridecyl alcohol (C13; 1-tridecanol, tridecanol, isotridecanol), myristyl alcohol (C14; 1-tetradecanol), pentadecyl alcohol (C15; 1-pentadecanol, pentadecanol), cetyl alcohol (C16; 1-hexadecanol), heptadecyl alcohol (C17; 1-n-heptadecanol, heptadecanol) and stearyl alcohol (C18; 1-octadecanol) and unsaturated counterparts including palmitoleyl alcohol (C16 unsaturated; cis-9-hexadecen-1-ol), or their corresponding fatty aldehydes or fatty acids.

1,4-Butanediol and intermediates thereto, such as 4-hydroxybutanoic acid (4-hydroxybutanoate, 4-hydroxybutyrate, 4-HB), are bioderived compounds that can be made via enzymatic pathways described herein and in the following publications. Suitable bioderived compound pathways and enzymes, methods for screening and methods for isolating are found in: WO2008115840A2 published 25 September 2008 entitled Compositions and Methods for the Biosynthesis of 1,4-Butanediol and Its Precursors; WO2010141780A1 published 9 December 2010 entitled Process of Separating Components of A Fermentation Broth ; WO2010141920A2 published 9 December 2010 entitled Microorganisms for the Production of 1,4-Butanediol and Related Methods; WO2010030711A2 published 18 March 2010 entitled Microorganisms for the Production of 1,4-Butanediol; WO2010071697A1 published 24 June 2010 Microorganisms and Methods for Conversion of Syngas and Other Carbon Sources to Useful Products; WO2009094485A1 published 30 July 2009 Methods and Organisms for Utilizing Synthesis Gas or Other Gaseous Carbon Sources and Methanol; WO2009023493A1 published 19 February 2009 entitled Methods and Organisms for the Growth-Coupled Production of 1,4-Butanediol; and WO2008115840A2 published 25 September 2008 entitled Compositions and Methods for the Biosynthesis of 1,4-Butanediol and Its Precursors, which are all incorporated herein by reference.

Butadiene and intermediates thereto, such as 1,4-butanediol, 2,3-butanediol, 1,3-butanediol, crotyl alcohol, 3-buten-2-ol (methyl vinyl carbinol) and 3-buten-1-ol, are bioderived compounds that can be made via enzymatic pathways described herein and in the following publications. In addition to direct fermentation to produce butadiene, 1,3-butanediol, 1,4-butanediol, crotyl alcohol, 3-buten-2-ol (methyl vinyl carbinol) or 3-buten-1-ol can be separated, purified (for any use), and then chemically dehydrated to butadiene by metal-based catalysis. Suitable bioderived compound pathways and enzymes, methods for screening and methods for isolating are found in: WO2011140171A2 published 10 November 2011 entitled Microorganisms and Methods for the Biosynthesis of Butadiene; WO2012018624A2 published 9 February 2012 entitled Microorganisms and Methods for the Biosynthesis of Aromatics, 2,4-Pentadienoate and 1,3-Butadiene; WO2011140171A2 published 10 November 2011 entitled Microorganisms and Methods for the Biosynthesis of Butadiene; WO2013040383A1 published 21 March 2013 entitled Microorganisms and Methods for Producing Alkenes; WO2012177710A1 published 27 December 2012 entitled Microorganisms for Producing Butadiene and Methods Related thereto; WO2012106516A1 published 9 August 2012 entitled Microorganisms and Methods for the Biosynthesis of Butadiene; and WO2013028519A1 published 28 February 2013 entitled Microorganisms and Methods for Producing 2,4-Pentadienoate, Butadiene, Propylene, 1,3-Butanediol and Related Alcohols, which are all incorporated herein by reference.

1,3-Butanediol and intermediates thereto, such as 2,4-pcntadicnoatc, crotyl alcohol or 3-buten-1-ol, are bioderived compounds that can be made via enzymatic pathways described herein and in the following publications. Suitable bioderived compound pathways and enzymes, methods for screening and methods for isolating are found in: WO2011071682A1 published 16 June 2011 entitled Methods and Organisms for Converting Synthesis Gas or Other Gaseous Carbon Sources and Methanol to 1,3-Butanediol; WO2011031897A published 17 March 2011 entitled Microorganisms and Methods for the Co-Production of Isopropanol with Primary Alcohols, Diols and Acids; WO2010127319A2 published 4 November 2010 entitled Organisms for the Production of 1,3-Butanediol; WO2013071226A1 published 16 May 2013 entitled Eukaryotic Organisms and Methods for Increasing the Availability of Cytosolic Acetyl-CoA, and for Producing 1,3-Butanediol; WO2013028519A1 published 28 February 2013 entitled Microorganisms and Methods for Producing 2,4-Pentadienoate, Butadiene, Propylene, 1,3-Butanediol and Related Alcohols; WO2013036764A1 published 14 March 2013 entitled Eukaryotic Organisms and Methods for Producing 1,3-Butanediol; WO2013012975A1 published 24 January 2013 entitled Methods for Increasing Product Yields; and WO2012177619A2 published 27 December 2012 entitled Microorganisms for Producing 1,3-Butanediol and Methods Related Thereto, which are all incorporated herein by reference.

Adipate, 6-aminocaproic acid, caprolactam, hexamethylenediamine and levulinic acid, and their intermediates, e.g. 4-aminobutyryl-CoA, are bioderived compounds that can be made via enzymatic pathways described herein and in the following publications. Suitable bioderived compound pathways and enzymes, methods for screening and methods for isolating are found in: WO2010129936A1 published 11 November 2010 entitled Microorganisms and Methods for the Biosynthesis of Adipate, Hexamethylenediamine and 6-Aminocaproic Acid; WO2013012975A1 published 24 January 2013 entitled Methods for Increasing Product Yields; WO2012177721A1 published 27 December 2012 entitled Microorganisms for Producing 6-Aminocaproic Acid; WO2012099621A1 published 26 July 2012 entitled Methods for Increasing Product Yields; and WO2009151728 published 17 Dec. 2009 entitled Microorganisms for the production of adipic acid and other compounds, which arc all incorporated herein by reference.

Methacrylic acid (2-methyl-2-propenoic acid) is used in the preparation of its esters, known collectively as methacrylates (e.g. methyl methacrylate, which is used most notably in the manufacture of polymers). Methacrylate esters such as methyl methacrylate, 3-hydroxyisobutyrate and/or 2-hydroxyisobutyrate and their intermediates are bioderived compounds that can be made via enzymatic pathways described herein and in the following publications. Suitable bioderived compound pathways and enzymes, methods for screening and methods for isolating are found in: WO2012135789A2 published 4 October 2012 entitled Microorganisms for Producing Methacrylic Acid and Methacrylate Esters and Methods Related Thereto; and WO2009135074A2 published 5 November 2009 entitled Microorganisms for the Production of Methacrylic Acid, which are all incorporated herein by reference.

1,2-Propanediol (propylene glycol), n-propanol, 1,3-propanediol and glycerol, and their intermediates are bioderived compounds that can be made via enzymatic pathways described herein and in the following publications. Suitable bioderived compound pathways and enzymes, methods for screening and methods for isolating are found in: WO2009111672A1 published 9 November 2009 entitled Primary Alcohol Producing Organisms; WO2011031897A1 17 March 2011 entitled Microorganisms and Methods for the Co-Production of Isopropanol with Primary Alcohols, Diols and Acids; WO2012177599A2 published 27 December 2012 entitled Microorganisms for Producing N-Propanol 1,3-Propancdiol, 1,2-Propanediol or Glycerol and Methods Related Thereto, which are all incorporated herein by referenced.

Succinic acid and intermediates thereto, which are useful to produce products including polymers (e.g. PBS), 1,4-butanediol, tetrahydrofuran, pyrrolidone, solvents, paints, deicers, plastics, fuel additives, fabrics, carpets, pigments, and detergents, are bioderived compounds that can be made via enzymatic pathways described herein and in the following publication. Suitable bioderived compound pathways and enzymes, methods for screening and methods for isolating are found in: EP1937821A2 published 2 July 2008 entitled Methods and Organisms for the Growth-Coupled Production of Succinate, which is incorporated herein by reference.
Primary alcohols and fatty alcohols (also known as long chain alcohols), including fatty acids and fatty aldehydes thereof, and intermediates thereto, are bioderived compounds that can be made via enzymatic pathways in the following publications. Suitable bioderived compound pathways and enzymes, methods for screening and methods for isolating arc found in: WO2009111672 published 11 September 2009 entitled Primary Alcohol Producing Organisms; WO2012177726 published 27 December 2012 entitled Microorganism for Producing Primary Alcohols and Related Compounds and Methods Related Thereto, which are all incorporated herein by reference.

Olefins includes an isoprenoid, which can be a bioderived product. Pathways and enzymes for producing an isoprenoid in a microbial organism and microbial organisms having those pathways and enzymes include those described in WO2013071172 entitled "Production of acetyl-coenzyme A derived isoprenoids", WO2012154854 entitled "Production of acetyl-coenzyme A derived compounds", WO2012016172 entitled "Genetically modified microbes producing increased levels of acetyl-CoA derived compounds", WO2012016177 entitled "Genetically modified microbes producing increased levels of acetyl-CoA derived compounds", WO2008128159 entitled "Production of isoprenoids" or US

Patent 8415136 entitled "Production of acetyl-coenzyme A derived isoprenoids", which are incorporated herein by reference. The isoprenoid can a hemiterpene, monoterpene, diterpene, triterpene, tetraterpene, sesquiterpene, and polyterpene. The isoprenoid is preferably a C5-C20 isoprenoid. The isoprenoid can be abietadiene, amorphadiene, carene, a-farnesene, β-farnesene, farnesol, geraniol, geranylgeraniol, isoprene, linalool, limonene, myrcene, ncrolidol, ocimene, patchoulol, β-pinene, sabinene, γ-terpinene, terpinolene, and valencene. A particularly preferred isoprenoid is isoprene.

Further suitable bioderived compounds that the microbial organisms of the invention can be used to produce via acetyl-CoA, including optionally further through acetoacetyl-CoA and/or succinyl-CoA, are included in the invention. Exemplary well known bioderived compounds, their pathways and enzymes for production, methods for screening and methods for isolating are found in the following patents and publications: succinate (U.S. publication 2007/0111294, WO 2007/030830, WO 2013/003432), 3-hydroxypropionic acid (3-hydroxypropionate) (U.S. publication 2008/0199926, WO 2008/091627, U.S. publication 2010/0021978), 1,4-butanediol (U.S. patent 8067214, WO 2008/115840, U.S. patent 7947483, WO 2009/023493, U.S. patent 7858350, WO 2010/030711, U.S. publication 2011/0003355, WO 2010/141780, U.S. patent 8129169, WO 2010/141920, U.S. publication 201 1/0201068, WO 2011/031897, U.S. patent 8377666, WO 2011/047101, U.S. publication 2011/0217742, WO 2011/066076, U.S. publication 2013/0034884, WO 2012/177943), 4-hydroxybutanoic acid (4-hydroxybutanoate, 4-hydroxybutyrate, 4-hydroxybutryate) (U.S. patent 8067214, WO 2008/115840, U.S. patent 7947483, WO 2009/023493, U.S. patent 7858350, WO 2010/030711, U.S. publication 2011/0003355, WO 2010/141780, U.S. patent 8129155, WO 2010/071697), γ-butyrolactone (U.S. patent 8067214, WO 2008/115840, U.S. patent 7947483, WO 2009/023493, U.S. patent 7858350, WO 2010/030711, U.S. publication 2011/0003355, WO 2010/141780, U.S. publication 2011/0217742, WO 2011/066076), 4-hydroxybutyryl-CoA (U.S. publication 2011/0003355, WO 2010/141780, U.S. publication 2013/0034884, WO 2012/177943), 4-hydroxybutanal (U.S. publication 2011/0003355, WO 2010/141780, U.S. publication 2013/0034884, WO 2012/177943), putrescine (U.S. publication 2011/0003355, WO 2010/141780, U.S. publication 2013/0034884, WO 2012/177943), Olefins (such as acrylic acid and acrylate ester) (U.S. patent 8026386, WO 2009/045637), acetyl-CoA (U.S. patent 8323950, WO 2009/094485), methyl tetrahydrofolate (U.S. patent 8323950, WO 2009/094485), ethanol (U.S. patent 8129155, WO 2010/071697), isopropanol (U.S. patent 8129155, WO 2010/071697, U.S. publication 2010/0323418, WO 2010/127303, U.S. publication 2011/0201068, WO 2011/031897), n-butanol (U.S. patent 8129155, WO 2010/071697), isobutanol (U.S. patent 8129155, WO 2010/071697), n-propanol (U.S. publication 2011/0201068, WO 2011/031897), methylacrylic acid (methylacrylate) (U.S. publication 2011/0201068, WO 2011/031897), primary alcohol (U.S. patent 7977084, WO 2009/111672, WO 2012/177726), long chain alcohol (U.S. patent 7977084, WO 2009/111672, WO 2012/177726), adipate (adipic acid) (U.S. patent 8062871, WO 2009/151728, U.S. patent 8377680, WO 2010/129936, WO 2012/177721), 6-aminocaproatc (6-aminocaproic acid) (U.S. patent 8062871, WO 2009/151728, U.S. patent 8377680, WO 2010/129936, WO 2012/177721), caprolactam (U.S. patent 8062871, WO 2009/151728, U.S. patent 8377680, WO 2010/129936, WO 2012/177721), hexamethylenediamine (U.S. patent 8377680, WO 2010/129936, WO 2012/177721), levulinic acid (U.S. patent 8377680, WO 2010/129936), 2-hydroxyisobutyric acid (2-hydroxyisobutyrate) (U.S. patent 8241877, WO 2009/135074, U.S. publication 2013/0065279, WO 2012/135789), 3-hydroxyisobutyric acid (3-hydroxyisobutyrate) (U.S. patent 8241877, WO 2009/135074, U.S. publication 2013/0065279, WO 2012/135789), methacrylic acid (methacrylate) (U.S. patent 8241877, WO 2009/135074, U.S. publication 2013/0065279, WO 2012/135789), methacrylate ester (U.S. publication 2013/0065279, WO 2012/135789), fumarate (fumaric acid) (U.S. patent 8129154, WO 2009/155382), malate (malic acid) (U.S. patent 8129154, WO 2009/155382), acrylate (carboxylic acid) (U.S. patent 8129154, WO 2009/155382), methyl ethyl ketone (U.S. publication 2010/0184173, WO 2010/057022, U.S. patent 8420375, WO 2010/144746), 2-butanol (U.S. publication 2010/0184173, WO 2010/057022, U.S. patent 8420375, WO 2010/144746), 1,3-butanediol (U.S. publication 2010/0330635, WO 2010/127319, U.S. publication 2011/0201068, WO 2011/031897, U.S. patent 8268607, WO 2011/071682, U.S. publication 2013/0109064, WO 2013/028519, U.S. publication 2013/0066035, WO 2013/036764), cyclohexanone (U.S. publication 2011/0014668, WO 2010/132845), terephthalate (terephthalic acid) (U.S. publication 2011/0124911, WO 2011/017560, U.S. publication 2011/0207185, WO 2011/094131, U.S. publication 2012/0021478, WO 2012/018624), muconate (muconic acid) (U.S. publication 2011/0124911, WO 2011/017560), aniline (U.S. publication 2011/0097767, WO 2011/050326), p-toluate (p-toluic acid) (U.S. publication 2011/0207185, WO 2011/094131, U.S. publication 2012/0021478, WO 2012/018624), (2-hydroxy-3-methyl-4-oxobutoxy)phosphonate (U.S. publication 2011/0207185, WO 2011/094131, U.S. publication 2012/0021478, WO 2012/018624), ethylene glycol (U.S. publication 2011/0312049, WO 2011/130378, WO 2012/177983), propylene (U.S. publication 2011/0269204, WO 2011/137198, U.S. publication 2012/0329119, U.S. publication 2013/0109064, WO 2013/028519), butadiene (1,3-butadiene) (U.S. publication 2011/0300597, WO 2011/140171, U.S. publication 2012/0021478, WO 2012/018624, U.S. publication 2012/0225466, WO 2012/106516, U.S. publication 2013/0011891, WO 2012/177710, U.S. publication 2013/0109064, WO 2013/028519), toluene (U.S. publication 2012/0021478, WO 2012/018624), benzene (U.S. publication 2012/0021478, WO 2012/018624), (2-hydroxy-4-oxobutoxy)phosphonate (U.S. publication 2012/0021478, WO 2012/018624), benzoate (benzoic acid) (U.S. publication 2012/0021478, WO 2012/018624), styrene (U.S. publication 2012/0021478, WO 2012/018624), 2,4-pentadienoate (U.S. publication 2012/0021478, WO 2012/018624, U.S. publication 2013/0109064, WO 2013/028519), 3-butene-l-ol (U.S. publication 2012/0021478, WO 2012/018624, U.S. publication 2013/0109064, WO 2013/028519), 3-buten-2-ol (U.S. publication 2013/0109064, WO 2013/028519), 1,4-cyclohexanedimethanol (U.S. publication 2012/0156740, WO 2012/082978), crotyl alcohol (U.S. publication 2013/0011891, WO 2012/177710, U.S. publication 2013/0109064, WO 2013/028519), alkene (U.S. publication 2013/0122563, WO 2013/040383, US 2011/0196180), hydroxyacid (WO 2012/109176), ketoacid (WO 2012/109176), wax esters (WO 2007/136762) or caprolactone (U.S. publication 2013/0144029, WO 2013/067432) pathway. The patents and patent application publications listed above that disclose bioderived compound pathways are herein incorporated herein by reference.

Acetyl-CoA is the immediate precursor for the synthesis of bioderived compounds as shown in Figures 5-10. Phosphoketolase pathways make possible synthesis of acetyl-CoA without requiring decarboxylation of pyruvate (Bogorad et al, Nature, 2013, published online 29 September 2013; United States Publication 2006-0040365), which thereby provides higher yields of bioderived compounds of the invention from carbohydrates and methanol than the yields attainable without phosphoketolase enzymes.

For example, synthesis of an exemplary fatty alcohol, dodecanol, from methanol-using methanol dehydrogenase (step A of Figure 1), a formaldehyde assimilation pathway (steps B, C, D of Figure 1) the pentose phosphate pathway, and glycolysis can provide a maximum theoretical yield of 0.0556 mole dodecanol/mole methanol.

18 CH₄O + 9 O₂ → C₁₂H₂₆O + 23 H₂O + 6 CO₂

However, if these pathways are combined with an acetyl CoA pathway comprising Phosphoketolase (e.g, steps T, U, V, W, X of Figure 1), a maximum theoretical yield of 0.0833 mole dodecanol/mole methanol can be obtained (pathway calculations not removing any ATP for cell growth and maintenance requirements).

12 CH₄O → C₁₂H₂₆O + 11 H₂O

ATP for energetic requirements can be synthesized, at the expense of lowering the maximum theoretical product yield, by oxidizing methanol to CO₂ using several combinations of enzymes, glycolysis, the TCA cycle, the pentose phosphate pathway, and oxidative phosphorylation.

Similarly, synthesis of isopropanol from methanol using methanol dehydrogenase (step A of Figure 1), a formaldehyde assimilation pathway (e.g., steps B, C, D of Figure 1), the pentose phosphate pathway and glycolysis can provide a maximum theoretical yield of 0.1667 mole isopropanol/mole methanol.

6 CH₄O + 4.5 O₂ → C₃H₈O + 8 H₂O + 3 CO₂

However, if these pathways are applied in combination with an acetyl CoA pathway comprising Phosphoketolase (e.g., steps T, U, V, W, X of Figure 1), a maximum theoretical yield of 0.250 mole isopropanol/mole methanol can be obtained.

4 CH₄O + 1.5 O₂ → C₃H₈O + 4 H₂O + CO₂

The overall pathway is ATP and redox positive enabling synthesis of both ATP and NAD(P)H from conversion of MeOH to isopropanol. Additional ATP can be synthesized, at the expense of lowering the maximum theoretical product yield, by oxidizing methanol to CO₂ using several combinations of enzymes, glycolysis, the TCA cycle, the pentose phosphate pathway, and oxidative phosphorylation.

Employing one or more methanol metabolic enzymes as described herein, for example as shown in Figures 1 and 2, methanol can enter central metabolism in most production hosts by employing methanol dehydrogenase (Figure 1, step A) along with a pathway for formaldehyde assimilation. One exemplary formaldehyde assimilation pathway that can utilize formaldehyde produced from the oxidation of methanol is shown in Figure 1, which involves condensation of formaldehyde and D-ribulose-5-phosphate to form hcxulosc-6-phosphatc (H6P) by hcxulosc-6-phosphate synthase (Figure 1, step B). The enzyme can use Mg²⁺ or Mn²⁺ for maximal activity, although other metal ions are useful, and even non-metal-ion-dependent mechanisms are contemplated. H6P is converted into fructose-6-phosphate by 6-phospho-3-hcxuloisomcrasc (Figure 1, step C). Another exemplary pathway that involves the detoxification and assimilation of formaldehyde produced from the oxidation of methanol proceeds through dihydroxyacetone. Dihydroxyacetone synthase (Figure 1, step D) is a transketolase that first transfers a glycoaldehyde group from xylulose-5-phosphate to formaldehyde, resulting in the formation of dihydroxyacetone (DHA) and glyceraldehyde-3-phosphate (G3P), which is an intermediate in glycolysis. The DHA obtained from DHA synthase can be then further phosphorylated to form DHA phosphate by a DHA kinase. DHAP can be assimilated into glycolysis, e.g. via isomerization to G3P, and several other pathways. Alternatively, DHA and G3P can be converted by fructose-6-phosphate aldolase to form fructose-6-phosphate (F6P) (Figure 1, step Z).

Synthesis of several other products from methanol using methanol dehydrogenase (step A of Figure 1), a formaldehyde assimilation pathway (e.g., steps B, C, D of Figure 1), the pentose phosphate pathway and glycolysis can provide the following maximum theoretical yield stoichiometries:

| **Product** | **CH4O** | **O2** | **NH3** | | **Product** | **H2O** | **CO2** |
|---|---|---|---|---|---|---|---|
| 1,3-Butanediol | 6.000 | 3.500 | 0.000 | --> | 1.000 | 7.000 | 2.000 |
| Crotyl Alcohol | 6.000 | 3.500 | 0.000 | --> | 1.000 | 8.000 | 2.000 |
| 3-Buten-2-ol | 6.000 | 3.500 | 0.000 | --> | 1.000 | 8.000 | 2.000 |
| Butadiene | 6.000 | 3.500 | 0.000 | --> | 1.000 | 9.000 | 2.000 |
| 2-Hydroxyisobutyrate | 6.000 | 4.500 | 0.000 | --> | 1.000 | 8.000 | 2.000 |
| Methacrylate (via 2-hydroxyisobutyrate) | 6.000 | 4.500 | 0.000 | --> | 1.000 | 9.000 | 2.000 |
| 3-Hydroxyisobutyrate (oxidative TCA cycle) | 6.000 | 4.500 | 0.000 | --> | 1.000 | 8.000 | 2.000 |
| Methacrylate (via 3-hydroxyisobutyrate) | 6.000 | 4.500 | 0.000 | --> | 1.000 | 9.000 | 2.000 |
| 1,4-Butanediol (oxidative TCA cycle) | 6.000 | 3.500 | 0.000 | --> | 1.000 | 9.000 | 2.000 |
| Adipate (oxidative TCA cycle) | 9.000 | 7.000 | 0.000 | --> | 1.000 | 13.000 | 3.000 |
| 6-Aminocaproate (oxidative TCA cycle) | 9.000 | 6.000 | 1.000 | --> | 1.000 | 13.000 | 3.000 |
| Caprolactam (via 6-aminocaproate) | 9.000 | 6.000 | 1.000 | --> | 1.000 | 14.000 | 3.000 |
| Hexamethylenediamine (oxidative TCA cycle) | 9.000 | 5.000 | 2.000 | --> | 1.000 | 13.000 | 3.000 |

In the products marked "oxidative TCA cycle", the maximum yield stoichiometries assume that the reductive TCA cycle enzymes (e.g., malate dehydrogenase, fumarase, fumarate reductase, and succinyl-CoA ligase) are not utilized for product formation. Exclusive use of the oxidative TCA cycle for product formation can be advantageous for succinyl-CoA derived products such as 3-hydroxyisobutyrate, 1,4-butanediol, adipate, 6-aminocaproate, and hexamethylenediamine because it enables all of the product pathway flux to originate from alpha-ketoglutarate dehydrogenase - an irreversible enzyme *in vivo.* Production of succinyl-CoA via the oxidative TCA branch uses both acetyl-CoA and oxaoloacctatc as precursors.

However, if these pathways are applied in combination with an acetyl CoA pathway comprising Phosphoketolase (e.g., steps T, U, V, W, X of Figure 1), an increased maximum theoretical yield on methanol can be obtained as shown below:

| **Product** | **CH4O** | **O2** | **NH3** | | **Produc t** | **H2O** | **CO2** |
|---|---|---|---|---|---|---|---|
| 1,3-Butanediol | 4.000 | 0.500 | 0.000 | --> | 1.000 | 3.000 | 0.000 |
| Crotyl Alcohol | 4.000 | 0.500 | 0.000 | --> | 1.000 | 4.000 | 0.000 |
| 3-Buten-2-ol | 4.000 | 0.500 | 0.000 | --> | 1.000 | 4.000 | 0.000 |
| Butadiene | 4.000 | 0.500 | 0.000 | --> | 1.000 | 5.000 | 0.000 |
| 2-Hydroxyisobutyrate | 4.000 | 1.500 | 0.000 | --> | 1.000 | 4.000 | 0.000 |
| Methacrylate (via 2-hydroxyisobutyrate) | 4.000 | 1.500 | 0.000 | --> | 1.000 | 5.000 | 0.000 |
| 3-Hydroxyisobutyrate (oxidative TCA cycle) | 5.000 | 3.000 | 0.000 | --> | 1.000 | 6.000 | 1.000 |
| Methacrylate (via 3-hydroxyisobutyrate) | 5.000 | 3.000 | 0.000 | --> | 1.000 | 7.000 | 1.000 |
| 1,4-Butanediol (oxidative TCA cycle) | 5.000 | 2.000 | 0.000 | --> | 1.000 | 5.000 | 1.000 |
| Adipate (oxidative TCA cycle) | 7.000 | 4.000 | 0.000 | --> | 1.000 | 9.000 | 1.000 |
| 6-Aminocaproate (oxidative TCA cycle) | 7.000 | 3.000 | 1.000 | --> | 1.000 | 9.000 | 1.000 |
| Caprolactam (via 6-aminocaproate) | 7.000 | 3.000 | 1.000 | --> | 1.000 | 10.000 | 1.000 |
| Hexamethylenediamine (oxidative TCA cycle) | 7.000 | 2.000 | 2.000 | --> | 1.000 | 9.000 | 1.000 |

The theoretical yield of bioderived compounds of the invention from carbohydrates including but not limited to glucose, glycerol, sucrose, fructose, xylose, arabinose, and galactose, can also be enhanced by phosphoketolase enzymes. This is because phosphoketolase enzymes provide acetyl-CoA synthesis with 100% carbon conversion efficiency *(e.g.,* 3 acetyl-CoA's per glucose, 2.5 acetyl-CoA's per xylose, 1.5 acetyl-CoA's per glycerol).

For example, synthesis of isopropanol from glucose in the absence of phosphoketolase enzymes can achieve a maximum theoretical isopropanol yield of 1.000 mole isopropanol/molc glucose.

C₆H₁₂O₆ + 1.5 O₂ → C₃H₈O + 2 H₂O + 3 CO₂

However, if enzyme steps T, U, V, W, X of Figure 1 are applied in combination with glycolysis and the pentose phosphate pathway, the maximum theoretical yield can be increased to 1.333 mole isopropanol/mole glucose.

C₆H₁₂O₆ → 1.333 C₃H₈O + 0.667 H₂O + 2 CO₂

In the absence of phosphoketolase activity, synthesis of several other products from a carbohydrate source (e.g., glucose) can provide the following maximum theoretical yield stoichiometries using glycolysis, pentose phosphate pathway, and TCA cycle reactions to build the pathway precursors.

| **Product** | **C6H12O6** | **O2** | **NH3** | | **Product** | **H2O** | **CO2** |
|---|---|---|---|---|---|---|---|
| 1,3-Butanediol | 1.000 | 0.500 | 0.000 | → | 1.000 | 1.000 | 2.000 |
| Crotyl Alcohol | 1.000 | 0.500 | 0.000 | → | 1.000 | 2.000 | 2.000 |
| 3-Buten-2-ol | 1.000 | 0.500 | 0.000 | → | 1.000 | 2.000 | 2.000 |
| Butadiene | 1.000 | 0.500 | 0.000 | → | 1.000 | 3.000 | 2.000 |
| 2-Hydroxyisobutyrate | 1.000 | 1.500 | 0.000 | → | 1.000 | 2.000 | 2.000 |
| Methacrylate (via 2-hydroxyisobutyrate) | 1.000 | 1.500 | 0.000 | → | 1.000 | 3.000 | 2.000 |
| 3-Hydroxyisobutyrate (oxidative TCA cycle) | 1.000 | 1.500 | 0.000 | → | 1.000 | 2.000 | 2.000 |
| Methacrylate (via 3-hydroxyisobutyrate) | 1.000 | 1.500 | 0.000 | → | 1.000 | 3.000 | 2.000 |
| 1,4-Butanediol (oxidative TCA cycle) | 1.000 | 0.500 | 0.000 | → | 1.000 | 1.000 | 2.000 |
| Adipate (oxidative TCA cycle) | 1.000 | 1.667 | 0.000 | → | 0.667 | 2.667 | 2.000 |
| 6-Aminocaproate (oxidative TCA cycle) | 1.000 | 1.000 | 0.667 | → | 0.667 | 2.667 | 2.000 |
| Caprolactam (via 6-aminocaproate) | 1.000 | 1.000 | 0.667 | → | 0.667 | 3.333 | 2.000 |
| Hexamethylenediamine (oxidative TCA cycle) | 1.000 | 0.333 | 1.333 | → | 0.667 | 2.667 | 2.000 |

In the products marked "oxidative TCA cycle", the maximum yield stoichiometries assume that the TCA cycle enzymes (e.g., malate dehydrogenase, fumarase, fumarate reductase, and succinyl-CoA ligase) are not utilized for product formation in the reductive direction. Exclusive use of the oxidative TCA cycle for product formation can be advantageous for succinyl-CoA derived products such as 3-hydroxyisobutyrate, 1,4-butanediol, adipate, 6-aminocaproate, and hexamethylenediamine because it enables all of the product pathway flux to originate from alpha-ketoglutarate dehydrogenase - an irreversible enzyme *in vivo.*

Notably, when these product pathways are applied in combination with an acetyl CoA pathway comprising Phosphoketolase (e.g., steps T, U, V, W, X of Figure 1), an increased maximum theoretical yield can be obtained as shown below:

| **Product** | **C6H12O6** | **O2** | **NH3** | | **Product** | **H2O** | **CO2** |
|---|---|---|---|---|---|---|---|
| 1,3-Butanediol | 1.000 | 0.000 | 0.000 | → | 1.091 | 0.545 | 1.636 |
| Crotyl Alcohol | 1.000 | 0.000 | 0.000 | → | 1.091 | 1.636 | 1.636 |
| 3-Buten-2-ol | 1.000 | 0.000 | 0.000 | → | 1.091 | 1.636 | 1.636 |
| Butadiene | 1.000 | 0.107 | 0.000 | → | 1.071 | 2.786 | 1.714 |
| 2-Hydroxyisobutyrate | 1.000 | 0.014 | 0.000 | → | 1.330 | 0.679 | 0.679 |
| Methacrylate (via 2-hydroxyisobutyrate) | 1.000 | 0.014 | 0.000 | → | 1.330 | 2.009 | 0.679 |
| 3-Hydroxyisobutyrate (oxidative TCA cycle) | 1.000 | 0.600 | 0.000 | → | 1.200 | 1.200 | 1.200 |
| Methacrylate (via 3-hydroxyisobutyrate) | 1.000 | 0.600 | 0.000 | → | 1.200 | 2.400 | 1.200 |
| 1,4-Butanediol (oxidative TCA cycle) | 1.000 | 0.124 | 0.000 | → | 1.068 | 0.658 | 1.727 |
| Adipate (oxidative TCA cycle) | 1.000 | 0.429 | 0.000 | → | 0.857 | 1.714 | 0.857 |
| 6-Aminocaproate (oxidative TCA cycle) | 1.000 | 0.000 | 0.800 | → | 0.800 | 2.000 | 1.200 |
| Caprolactam (via 6-aminocaproate) | 1.000 | 0.000 | 0.800 | → | 0.800 | 2.800 | 1.200 |
| Hexamethylenediamine (oxidative TCA cycle) | 1.000 | 0.064 | 1.397 | → | 0.698 | 2.508 | 1.810 |
| Butadiene via 2,4-pentadienoate | 1 | 0.000 | 0.000 | → | 1.091 | 2.727 | 1.636 |

As with glucose, a similar yield increase can occur with use of a phosphoketolase enzyme on other carbohydrates such as glycerol, sucrose, fructose, xylose, arabinose and galactose.

Pathways identified herein, and particularly pathways exemplified in specific combinations presented herein, are superior over other pathways based in part on the applicant's ranking of pathways based on attributes Additional benefits and superior aspects include one or more of the following: maximum theoretical compound yield, maximal carbon flux, better efficiency of ATP production and reducing equivalents availability, reduced requirement for aeration, minimal production of CO₂, pathway length, number of non-native steps, thermodynamic feasibility, number of enzymes active on pathway substrates or structurally similar substrates, and having steps with currently characterized enzymes, and furthermore, the latter pathways are even more favored by having in addition at least the fewest number of non-native steps required, the most enzymes known active on pathway substrates or structurally similar substrates, and the fewest total number of steps from central metabolism.

In some embodiments, the invention also provides a method for producing a bioderived compound described herein. Such a method can comprise culturing the non-naturally occurring microbial organism as described herein under conditions and for a sufficient period of time to produce the bioderived compound. In another embodiment, method further includes separating the bioderived compound from other components in the culture. In this aspect, separating can include extraction, continuous liquid-liquid extraction, pervaporation, membrane filtration, membrane separation, reverse osmosis, electrodialysis, distillation, crystallization, centrifugation, extractive filtration, ion exchange chromatography, absorption chromatography, or ultrafiltration.

In some embodiments, depending on the bioderived compound, the method of the invention may further include chemically converting a bioderived compound to the directed final compound. For example, in some embodiments, wherein the bioderived compound is butadiene, the method of the invention can further include chemically dehydrating 1,3-butanediol, crotyl alcohol, or 3-buten-2-ol to produce the butadiene.

Suitable purification and/or assays to test for the production of acetyl-CoA, oxaloacetate, or a bioderived compound can be performed using well known methods. Suitable replicates such as triplicate cultures can be grown for each engineered strain to be tested. For example, product and byproduct formation in the engineered production host can be monitored. The final product and intermediates, and other organic compounds, can be analyzed by methods such as HPLC (High Performance Liquid Chromatography), GC-MS (Gas Chromatography-Mass Spectroscopy) and LC-MS (Liquid Chromatography-Mass Spectroscopy) or other suitable analytical methods using routine procedures well known in the art. The release of product in the fermentation broth can also be tested with the culture supernatant. Byproducts and residual glucose can be quantified by HPLC using, for example, a refractive index detector for glucose and alcohols, and a UV detector for organic acids (Lin et al., Biotechnol. Bioeng. 90:775-779 (2005)), or other suitable assay and detection methods well known in the art. The individual enzyme or protein activities from the exogenous DNA sequences can also be assayed using methods well known in the art.

The bioderived compound can be separated from other components in the culture using a variety of methods well known in the art. Such separation methods include, for example, extraction procedures as well as methods that include continuous liquid-liquid extraction, pervaporation, membrane filtration, membrane separation, reverse osmosis, electrodialysis, distillation, crystallization, centrifugation, extractive filtration, ion exchange chromatography, size exclusion chromatography, adsorption chromatography, and ultrafiltration. All of the above methods are well known in the art.

Any of the non-naturally occurring microbial organisms described herein can be cultured to produce and/or secrete the biosynthetic products of the invention. For example, the bioderived compound producers can be cultured for the biosynthetic production of a bioderived compound disclosed herein. Accordingly, in some embodiments, the invention provides culture medium having the bioderived compound or bioderived compound pathway intermediate described herein. In some aspects, the culture medium can also be separated from the non-naturally occurring microbial organisms of the invention that produced the bioderived compound or bioderived compound pathway intermediate. Methods for separating a microbial organism from culture medium are well known in the art. Exemplary methods include filtration, flocculation, precipitation, centrifugation, sedimentation, and the like.

For the production of acetyl-CoA, oxaloacetate, or a bioderived compound, the recombinant strains are cultured in a medium with carbon source and other essential nutrients. It is sometimes desirable and can be highly desirable to maintain anaerobic conditions in the fermenter to reduce the cost of the overall process. Such conditions can be obtained, for example, by first sparging the medium with nitrogen and then scaling the flasks with a septum and crimp-cap. For strains where growth is not observed anaerobically, microaerobic or substantially anaerobic conditions can be applied by perforating the septum with a small hole for limited aeration. Exemplary anaerobic conditions have been described previously and are well-known in the art. Exemplary aerobic and anaerobic conditions are described, for example, in United State publication 2009/0047719, filed August 10, 2007. Fermentations can be performed in a batch, fed-batch or continuous manner, as disclosed herein. Fermentations can also be conducted in two phases, if desired. The first phase can be aerobic to allow for high growth and therefore high productivity, followed by an anaerobic phase of high acetyl-CoA , oxaloacetate, or a bioderived compound yields.

If desired, the pH of the medium can be maintained at a desired pH, in particular neutral pH, such as a pH of around 7 by addition of a base, such as NaOH or other bases, or acid, as needed to maintain the culture medium at a desirable pH. The growth rate can be determined by measuring optical density using a spectrophotometer (600 nm), and the glucose uptake rate by monitoring carbon source depletion over time.

The growth medium, can include, for example, any carbohydrate source which can supply a source of carbon to the non-naturally occurring microbial organism of the invention. Such sources include, for example, sugars such as glucose, xylose, arabinose, galactose, mannose, fructose, sucrose and starch; or glycerol, alone as the sole source of carbon or in combination with other carbon sources described herein or known in the art. In one embodiment, the carbon source is a sugar. In one embodiment, the carbon source is a sugar-containing biomass. In some embodiments, the sugar is glucose. In one embodiment, the sugar is xylose. In another embodiment, the sugar is arabinose. In one embodiment, the sugar is galactose. In another embodiment, the sugar is fructose. In other embodiments, the sugar is sucrose. In one embodiment, the sugar is starch. In certain embodiments, the carbon source is glycerol. In some embodiments, the carbon source is crude glycerol. In one embodiment, the carbon source is crude glycerol without treatment. In other embodiments, the carbon source is glycerol and glucose. In another embodiment, the carbon source is methanol and glycerol. In one embodiment, the carbon source is carbon dioxide. In one embodiment, the carbon source is formate. In one embodiment, the carbon source is methane. In one embodiment, the carbon source is methanol. In certain embodiments, methanol is used alone as the sole source of carbon or in combination with other carbon sources described herein or known in the art. In a specific embodiment, the methanol is the only (sole) carbon source. In one embodiment, the carbon source is chemoelectro-generated carbon (see, e.g., Liao et al. (2012) Science 335:1596). In one embodiment, the chemoelectro-generated carbon is methanol. In one embodiment, the chemoelectro-generated carbon is formate. In one embodiment, the chemoelectro-generated carbon is formate and methanol. In one embodiment, the carbon source is a carbohydrate and methanol. In one embodiment, the carbon source is a sugar and methanol. In another embodiment, the carbon source is a sugar and glycerol. In other embodiments, the carbon source is a sugar and crude glycerol. In yet other embodiments, the carbon source is a sugar and crude glycerol without treatment. In one embodiment, the carbon source is a sugar-containing biomass and methanol. In another embodiment, the carbon source is a sugar-containing biomass and glycerol. In other embodiments, the carbon source is a sugar-containing biomass and crude glycerol. In yet other embodiments, the carbon source is a sugar-containing biomass and crude glycerol without treatment. In some embodiments, the carbon source is a sugar-containing biomass, methanol and a carbohydrate. Other sources of carbohydrate include, for example, renewable feedstocks and biomass. Exemplary types of biomasses that can be used as feedstocks in the methods provided herein include cellulosic biomass, hemicellulosic biomass and lignin feedstocks or portions of feedstocks. Such biomass feedstocks contain, for example, carbohydrate substrates useful as carbon sources such as glucose, xylose, arabinose, galactose, mannose, fructose and starch. Given the teachings and guidance provided herein, those skilled in the art will understand that renewable feedstocks and biomass other than those exemplified above also can be used for culturing the microbial organisms provided herein for the production of succinate and other pathway intermediates.

In one embodiment, the carbon source is glycerol. In certain embodiments, the glycerol carbon source is crude glycerol or crude glycerol without further treatment. In a further embodiment, the carbon source comprises glycerol or crude glycerol, and also sugar or a sugar-containing biomass, such as glucose. In a specific embodiment, the concentration of glycerol in the fermentation broth is maintained by feeding crude glycerol, or a mixture of crude glycerol and sugar (*e.g.,* glucose). In certain embodiments, sugar is provided for sufficient strain growth. In some embodiments, the sugar (e.g., glucose) is provided at a molar concentration ratio of glycerol to sugar of from 200:1 to 1:200.

In one embodiment, the carbon source is methanol or formate. In certain embodiments, methanol is used as a carbon source in a formaldehyde fixation pathway provided herein. In one embodiment, the carbon source is methanol or formate. In other embodiments, formate is used as a carbon source in a formaldehyde fixation pathway provided herein. In specific embodiments, methanol is used as a carbon source in a methanol oxidation pathway provided herein, either alone or in combination with the fatty alcohol, fatty aldehyde, fatty acid or isopropanol pathways provided herein. In one embodiment, the carbon source is methanol. In another embodiment, the carbon source is formate.

In one embodiment, the carbon source comprises methanol, and sugar (e.g., glucose) or a sugar-containing biomass. In another embodiment, the carbon source comprises formate, and sugar (e.g., glucose) or a sugar-containing biomass. In one embodiment, the carbon source comprises methanol, formate, and sugar (e.g., glucose) or a sugar-containing biomass. In specific embodiments, the methanol or formate, or both, in the fermentation feed is provided as a mixture with sugar (*e.g*., glucose) or sugar-comprising biomass. In certain embodiments, sugar is provided for sufficient strain growth.

In certain embodiments, the carbon source comprises formate and a sugar *(e.g.,* glucose). In some embodiments, the sugar *(e.g.,* glucose) is provided at a molar concentration ratio of formate to sugar of from 200:1 to 1:200. Accordingly, given the teachings and guidance provided herein, those skilled in the art will understand that a non-naturally occurring microbial organism can be produced that secretes the biosynthesized compounds of the invention when grown on a carbon source such as a carbohydrate. Such compounds include, for example, acetyl-CoA or a bioderived compound and any of the intermediate metabolites in the acetyl-CoA or the bioderived compound pathway. All that is required is to engineer in one or more of the required enzyme or protein activities to achieve biosynthesis of the desired compound or intermediate including, for example, inclusion of some or all of the acetyl-CoA or the bioderived compound biosynthetic pathways. Accordingly, the invention provides a non-naturally occurring microbial organism that produces and/or secretes acetyl-CoA or a bioderived compound when grown on a carbohydrate or other carbon source and produces and/or secretes any of the intermediate metabolites shown in the acetyl-CoA or the bioderived compound pathway when grown on a carbohydrate or other carbon source. The acetyl-CoA or the bioderived compound producing microbial organisms of the invention can initiate synthesis from an intermediate, for example, F6P, E4P, formate, formyl-CoA, G3P, PYR, DHA, H6P, 3HBCOA, 3HB, 3-hydroxybutyryl phosphate, 4-hydroxybutyrate, 4-hydroxybutyryl-CoA, adipyl-CoA, adipate semialdehyde, 3-hydroxyisobutyrate, or 2-hydroxyisobutyryl-CoA.

The non-naturally occurring microbial organisms of the invention are constructed using methods well known in the art as exemplified herein to exogenously express at least one nucleic acid encoding an acetyl-CoA or a bioderived compound pathway enzyme or protein in sufficient amounts to produce acetyl-CoA or a bioderived compound. It is understood that the microbial organisms of the invention are cultured under conditions sufficient to produce acetyl-CoA or a bioderived compound. Following the teachings and guidance provided herein, the non-naturally occurring microbial organisms of the invention can achieve biosynthesis of a bioderived compound resulting in intracellular concentrations between about 0.1-200 mM or more. Generally, the intracellular concentration of a bioderived compound is between about 3-150 mM, particularly between about 5-125 mM and more particularly between about 8-100 mM, including about 10 mM, 20 mM, 50 mM, 80 mM, or more. Intracellular concentrations between and above each of these exemplary ranges also can be achieved from the non-naturally occurring microbial organisms of the invention.

In some embodiments, culture conditions include anaerobic or substantially anaerobic growth or maintenance conditions. Exemplary anaerobic conditions have been described previously and are well known in the art. Exemplary anaerobic conditions for fermentation processes are described herein and are described, for example, in U.S. publication 2009/0047719, filed August 10, 2007. Any of these conditions can be employed with the non-naturally occurring microbial organisms as well as other anaerobic conditions well known in the art. Under such anaerobic or substantially anaerobic conditions, the acetyl-CoA or the bioderived compound producers can synthesize a bioderived compound at intracellular concentrations of 5-10 mM or more as well as all other concentrations exemplified herein. It is understood that, even though the above description refers to intracellular concentrations, acetyl-CoA or a bioderived compound producing microbial organisms can produce a bioderived compound intracellularly and/or secrete the product into the culture medium.

Exemplary fermentation processes include, but are not limited to, fed-batch fermentation and batch separation; fed-batch fermentation and continuous separation; and continuous fermentation and continuous separation. In an exemplary batch fermentation protocol, the production organism is grown in a suitably sized bioreactor sparged with an appropriate gas. Under anaerobic conditions, the culture is sparged with an inert gas or combination of gases, for example, nitrogen, N₂/CO₂ mixture, argon, helium, and the like. As the cells grow and utilize the carbon source, additional carbon source(s) and/or other nutrients are fed into the bioreactor at a rate approximately balancing consumption of the carbon source and/or nutrients. The temperature of the bioreactor is maintained at a desired temperature, generally in the range of 22-37 degrees C, but the temperature can be maintained at a higher or lower temperature depending on the growth characteristics of the production organism and/or desired conditions for the fermentation process. Growth continues for a desired period of time to achieve desired characteristics of the culture in the fermenter, for example, cell density, product concentration, and the like. In a batch fermentation process, the time period for the fermentation is generally in the range of several hours to several days, for example, 8 to 24 hours, or 1, 2, 3, 4 or 5 days, or up to a week, depending on the desired culture conditions. The pH can be controlled or not, as desired, in which case a culture in which pH is not controlled will typically decrease to pH 3-6 by the end of the run. Upon completion of the cultivation period, the fermenter contents can be passed through a cell separation unit, for example, a centrifuge, filtration unit, and the like, to remove cells and cell debris. In the case where the desired product is expressed intracellularly, the cells can be lysed or disrupted enzymatically or chemically prior to or after separation of cells from the fermentation broth, as desired, in order to release additional product. The fermentation broth can be transferred to a product separations unit. Isolation of product occurs by standard separations procedures employed in the art to separate a desired product from dilute aqueous solutions. Such methods include, but are not limited to, liquid-liquid extraction using a water immiscible organic solvent (e.g., toluene or other suitable solvents, including but not limited to diethyl ether, ethyl acetate, tetrahydrofuran (THF), methylene chloride, chloroform, benzene, pentane, hexane, heptane, petroleum ether, methyl tertiary butyl ether (MTBE), dioxane, dimethylformamide (DMF), dimethyl sulfoxide (DMSO), and the like) to provide an organic solution of the product, if appropriate, standard distillation methods, and the like, depending on the chemical characteristics of the product of the fermentation process.

In an exemplary fully continuous fermentation protocol, the production organism is generally first grown up in batch mode in order to achieve a desired cell density. When the carbon source and/or other nutrients are exhausted, feed medium of the same composition is supplied continuously at a desired rate, and fermentation liquid is withdrawn at the same rate. Under such conditions, the product concentration in the bioreactor generally remains constant, as well as the cell density. The temperature of the fermenter is maintained at a desired temperature, as discussed above. During the continuous fermentation phase, it is generally desirable to maintain a suitable pH range for optimized production. The pH can be monitored and maintained using routine methods, including the addition of suitable acids or bases to maintain a desired pH range. The bioreactor is operated continuously for extended periods of time, generally at least one week to several weeks and up to one month, or longer, as appropriate and desired. The fermentation liquid and/or culture is monitored periodically, including sampling up to every day, as desired, to assure consistency of product concentration and/or cell density. In continuous mode, fermenter contents are constantly removed as new feed medium is supplied. The exit stream, containing cells, medium, and product, are generally subjected to a continuous product separations procedure, with or without removing cells and cell debris, as desired. Continuous separations methods employed in the art can be used to separate the product from dilute aqueous solutions, including but not limited to continuous liquid-liquid extraction using a water immiscible organic solvent (e.g., toluene or other suitable solvents, including but not limited to diethyl ether, ethyl acetate, tetrahydrofuran (THF), methylene chloride, chloroform, benzene, pentane, hexane, heptane, petroleum ether, methyl tertiary butyl ether (MTBE), dioxane, dimethylformamide (DMF), dimethyl sulfoxide (DMSO), and the like), standard continuous distillation methods, and the like, or other methods well known in the art.

In addition to the culturing and fermentation conditions disclosed herein, growth condition for achieving biosynthesis of acetyl-CoA or a bioderived compound can include the addition of an osmoprotectant to the culturing conditions. In certain embodiments, the non-naturally occurring microbial organisms of the invention can be sustained, cultured or fermented as described herein in the presence of an osmoprotectant. Briefly, an osmoprotectant refers to a compound that acts as an osmolyte and helps a microbial organism as described herein survive osmotic stress. Osmoprotectants include, but are not limited to, betaines, amino acids, and the sugar trehalose. Non-limiting examples of such are glycine betaine, praline betaine, dimethylthetin, dimethylslfonioproprionate, 3-dimethylsulfonio-2-methylproprionate, pipecolic acid, dimethylsulfonioacetate, choline, L-carnitine and ectoine. In one aspect, the osmoprotectant is glycine betaine. It is understood to one of ordinary skill in the art that the amount and type of osmoprotectant suitable for protecting a microbial organism described herein from osmotic stress will depend on the microbial organism used. The amount of osmoprotectant in the culturing conditions can be, for example, no more than about 0.1 mM, no more than about 0.5 mM, no more than about 1.0 mM, no more than about 1.5 mM, no more than about 2.0 mM, no more than about 2.5 mM, no more than about 3.0 mM, no more than about 5.0 mM, no more than about 7.0 mM, no more than about 10mM, no more than about 50mM, no more than about 100mM or no more than about 500mM.

In some embodiments, the carbon feedstock and other cellular uptake sources such as phosphate, ammonia, sulfate, chloride and other halogens can be chosen to alter the isotopic distribution of the atoms present in acetyl-CoA or a bioderived compound or any acetyl-CoA or a bioderived compound pathway intermediate. The various carbon feedstock and other uptake sources enumerated above will be referred to herein, collectively, as "uptake sources." Uptake sources can provide isotopic enrichment for any atom present in the acetyl-CoA, bioderived compound or pathway intermediate, or for side products generated in reactions diverging away from an acetyl-CoA or a bioderived compound pathway. Isotopic enrichment can be achieved for any target atom including, for example, carbon, hydrogen, oxygen, nitrogen, sulfur, phosphorus, chloride or other halogens.

The invention further provides a composition comprising bioderived compound described herein and a compound other than the bioderived bioderived. The compound other than the bioderived product can be a cellular portion, for example, a trace amount of a cellular portion of, or can be fermentation broth or culture medium, or a purified or partially purified fraction thereof produced in the presence of, a non-naturally occurring microbial organism of the invention. The composition can comprise, for example, a reduced level of a byproduct when produced by an organism having reduced byproduct formation, as disclosed herein. The composition can comprise, for example, bioderived compound, or a cell lysate or culture supernatant of a microbial organism of the invention.

In certain embodiments, provided herein is a composition comprising a bioderived compound provided herein produced by culturing a non-naturally occurring microbial organism described herein. In some embodiments, the composition further comprises a compound other than said bioderived compound. In certain embodiments, the compound other than said bioderived compound is a trace amount of a cellular portion of a non-naturally occurring microbial organism descirbe herein.

The culture conditions can include, for example, liquid culture procedures as well as fermentation and other large scale culture procedures. As described herein, particularly useful yields of the biosynthetic products of the invention can be obtained under anaerobic or substantially anaerobic culture conditions.

As described herein, one exemplary growth condition for achieving biosynthesis of acetyl-CoA or a bioderived compound includes anaerobic culture or fermentation conditions. In certain embodiments, the non-naturally occurring microbial organisms of the invention can be sustained, cultured or fermented under anaerobic or substantially anaerobic conditions. Briefly, an anaerobic condition refers to an environment devoid of oxygen. Substantially anaerobic conditions include, for example, a culture, batch fermentation or continuous fermentation such that the dissolved oxygen concentration in the medium remains between 0 and 10% of saturation. Substantially anaerobic conditions also includes growing or resting cells in liquid medium or on solid agar inside a sealed chamber maintained with an atmosphere of less than 1% oxygen. The percent of oxygen can be maintained by, for example, sparging the culture with an N₂/CO₂ mixture or other suitable non-oxygen gas or gases.

The culture conditions described herein can be scaled up and grown continuously for manufacturing of acetyl-CoA or a bioderived compound. Exemplary growth procedures include, for example, fed-batch fermentation and batch separation; fed-batch fermentation and continuous separation, or continuous fermentation and continuous separation. All of these processes are well known in the art. Fermentation procedures are particularly useful for the biosynthetic production of commercial quantities of acetyl-CoA or a bioderived compound. Generally, and as with non-continuous culture procedures, the continuous and/or near-continuous production of acetyl-CoA or a bioderived compound will include culturing a non-naturally occurring acetyl-CoA or a bioderived compound producing organism of the invention in sufficient nutrients and medium to sustain and/or nearly sustain growth in an exponential phase. Continuous culture under such conditions can include, for example, growth or culturing for 1 day, 2, 3, 4, 5, 6 or 7 days or more. Additionally, continuous culture can include longer time periods of 1 week, 2, 3, 4 or 5 or more weeks and up to several months. Alternatively, organisms of the invention can be cultured for hours, if suitable for a particular application. It is to be understood that the continuous and/or near-continuous culture conditions also can include all time intervals in between these exemplary periods. It is further understood that the time of culturing the microbial organism of the invention is for a sufficient period of time to produce a sufficient amount of product for a desired purpose.

Fermentation procedures are well known in the art. Briefly, fermentation for the biosynthetic production of acetyl-CoA or a bioderived compound can be utilized in, for example, fed-batch fermentation and batch separation; fed-batch fermentation and continuous separation, or continuous fermentation and continuous separation. Examples of batch and continuous fermentation procedures are well known in the art.

In addition to the above fermentation procedures using the acetyl-CoA or the bioderived compound producers of the invention for continuous production of substantial quantities of acetyl-CoA or a bioderived compound, the acetyl-CoA or the bioderived compound producers also can be, for example, simultaneously subjected to chemical synthesis and/or enzymatic procedures to convert the product to other compounds or the product can be separated from the fermentation culture and sequentially subjected to chemical and/or enzymatic conversion to convert the product to other compounds, if desired.

It is understood that modifications which do not substantially affect the activity of the various embodiments of this invention are also provided within the definition of the invention provided herein. Accordingly, the following examples are intended to illustrate but not limit the present invention.

### EXAMPLE I

### Example 1: Construction of glk-glf libraries

The PTS system of sugar transport was inactivated by removing *ptsI* from E. coli K12 MG1655 strain that had deletions to remove some competing byproducts. Expectedly, the strain had a very poor growth. As mentioned, similar effects will be observed for E. coli strains with other genetic changes as well. This strain did not form colonies on M9 agar + 2% glucose (after 2 days at 37°C), and shows little/no growth in MM9 + 2% glucose media.

Therefore, expression mutants of native glk and Zymomonas mobilis glf (GI no: 155589) were made and inserted into the PTS- cells to increase glucose consumption and by selecting for those mutants that had an improved growth rate on glucose. The mutant library is constructed by inserting the glf gene in proximity and divergent to the glk gene. Accompanying the glf gene are divergent and degenerate promoters that tune expression of the glf and glk genes, respectively.

*Promoter-glf cassette construction*: The promoter-glf cassette (termed "PX2-gIf") was constructed by two rounds of PCR. The first round PCR amplified the P115 promoter-glf-terminator sequence from PZS*13S-P115-glf using a partially-degenerate sequence for the P115 promoter region. A partially-degenerate ultramer (IDT) was used in the second round of PCR to construct the full length cassette. The resulting library of DNA has 6 degenerate sites in the promoter controlling glk expression, and 3 degenerate sites in the promoter controlling glf expression.

*Insertion of gif library into the chromosome in a ptsI- strain:* gblocks were designed to insert the sacB-kan cassette (5'glk-SBK) or the PX2-glf cassette into the chromosome of the ptsI- strain described above, exactly 20 bp upstream of the glk gene. After selection for recombinants on sucrose, sequencing showed nucleotide degeneracy at all 9 promoter sites.

*Selection for improved growth on glucose.:* Following creation of the mutant library a portion of the cells were selected for growth on sucrose to remove non-recombinant cells that do not have the PX2-glf cassette. As a negative control, cells that were not treated with the PX2-glf cassette were propagated in parallel. After sucrose selection, an aliquot of cells from each population were plated on M9 agar + 2% glucose. Isolates were sequenced and tested for growth in MM9 +2% glucose in the Bioscreen instrument (Variants 25-36 & 61-84).

Figure 13 illustrates steps in the construction of the glk-glf libraries.

*Direct selection for improved glucose consumption:* Following electroporation of the PX2-glf cassette into 6972, an aliquot of these cells was used to inoculate a 1L capped bottle of MM9 media containing 2% glucose and 100 mM sodium bicarbonate. As a negative control, cells that were not treated with the PX2-glf cassette were propagated in parallel. Growth selection on glucose was carried out for 5 days. Aliquots were taken after 3 days and 5 days and plated on the M9 glucose agar. Diverse colony sizes were observed on the M9 glucose agar plates, and generally, colonies arose faster from the population treated with the PX2-glf DNA. Isolates were sequenced and tested for growth in MM9 +2% glucose in the Bioscreen instrument (Variants 1-24, 37-60, 85-130).*Bioscreen Growth results:* Following growth selections in glucose, variants were isolated as colonies on M9 agar plates containing glucose. 130 variants were tested for growth on MM9 + 2% glucose in the Bioscreen, in duplicate. The fastest growing variants from each of the selection conditions were retested for growth in the Bioscrccn in replicates of 10. Included in the growth experiment are the PTS+ grandparent (6770) and the PTS- parent (6850). The PTS+ grandparent 6770 is MG1655 with deletions of adhE, ldhA and frmR.

The growth curves are shown in Figure 14A, average of 10 replicates. Maximum growth rates (rmax in 1/hr on the x axis below) of select variants and parent strains are shown in Figure 14B based on 10 replicates.

### EXAMPLE II:

### Coexpression of the PTS and Non-PTS system of glucose transport with PK in E. coli cells making 1,4-butanediol

An F6P phosphoketolase (EC 4.1.2.22, Genbank ID number 118765289), was cloned from *Bifidobacterium adolescentis* into a plasmid suitable for expression in E. coli., plasmid pZS*13S obtained from R. Lutz (Expressys, Germany). These plasmids are based on the pZ Expression System (Lutz, R. & Bujard, H., Independent and tight regulation of transcriptional units in Escherichia coli via the LacR/O, the TetR/O and AraC/I1-I2 regulatory elements. Nucleic Acids Res. 25, 1203-1210 (1997)).

*E. coli* strain MG1655 variant having a pathway to produce 1,4-butanediol via alpha-ketoglutarate (designated 7542 herein) was transformed with the expression plasmid and selected and maintained using antibiotic selection with carbenicillin. This strain had a glk-glf cassette described in Example I inserted into the chromosome. The insertion site was upstream of glk.

Fermentation runs comparing the performance of the host strain with and without phosphoketolase showed an increase in BDO titer by approximately 10 g/L (Figure 12A). Quite interestingly, it led to a reduction in pyruvate and alanine formation (Figures 12B and C), typical of BDO-producing strains coexpressing the PTS and the Non-PTS sytems of glucose transport. This was a completely unexpected result. Additionally, the pyruvate production in the parent strain indicated a very odd profile consistent with the production, consumption and reproduction of pyruvate.

### EXAMPLE III

### Attenuation of pykF in a strain expressing theNon-PTS

### system of sugar transport and PK

Pyruvate kinase isozyme pykF was deleted from the E. coli K12 variant of the previous example that had the PTS system of sugar transport deleted (by ptsI deletion) and that expressed a Non-PTS system by insertion of a glk-glf cassette #25, described in Example I above. Fructose-6-phosphate phosphoketolase, E.C. 4.1.2.22, Genbank ID number 118765289, was cloned from *Bifidobacterium adolescentis* into a plasmid suitable for expression in E. coli plasmid pZS* 13S obtained from R. Lutz (Expressys, Germany) and which is based on the pZ Expression System (Lutz, R. & Bujard, H., Independent and tight regulation of transcriptional units in Escherichia coli via the LacR/O, the TetR/O and AraC/I1-I2 regulatory elements. Nucleic Acids Res. 25, 1203-1210 (1997)). The parent strain had the PTS system deleted and the non-PTS system enhance as described previously. Additionally, PK was expressed at different levels with promoters of various strengths. EV represents the empty vector and the promoter strength in increasing order is: p115<p105<p108<p100. The results showed an optimal level of PK expression for each strain and this was the levels provided by either the p108 or the p105 promoter. As shown in the table below, the deletion of pykF in conjunction with the optimal levels of PK led to an increase in BDO production. Quite interestingly and unexpectedly, it also led to a decrease in pyruvate production, an overflow metabolism byproduct and a key challenge with the BDO-producing strains that express the Non-PTS system of glucose transport either solely or in combination with the PTS-system of glucose transport. Experiments done earlier (not shown here) with overexpression (OE) of PK with a strain that had the PTS deleted and non-PTS enhanced did not show such reduction in pyruvate.

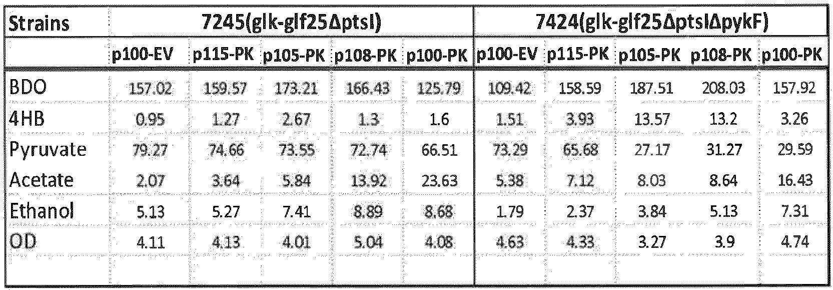

### EXAMPLE IV

### Additional Pathways and Enzymes

This example describes enzymatic pathways for converting pyruvate to formaldehyde, and optionally in combination with producing acetyl-CoA and/or reproducing pyruvate as described in the text above.

### Step Y, Figure 1: Glyceraldehydes-3-phosphate Dehydrogenase and Enzymes of Lower Glycolysis

Enzymes comprising Step Y, G3P to PYR include: Glyceraldehyde-3-phosphate dehydrogenase; Phosphoglycerate kinase; Phosphoglyceromutase; Enolase; Pyruvate kinase or PTS-dependent substrate import.

Glyceraldehyde-3-phosphate dehydrogenase enzymes include: NADP-dependent glyceraldehyde-3-phosphate dehydrogenase, exemplary enzymes are:

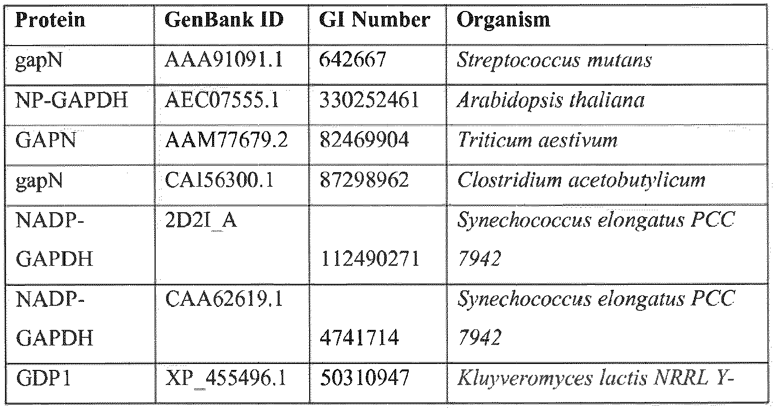

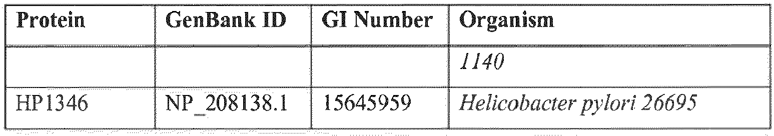

and NAD-dependent glyceraldehyde-3-phosphate dehydrogenase, exemplary enzymes are:

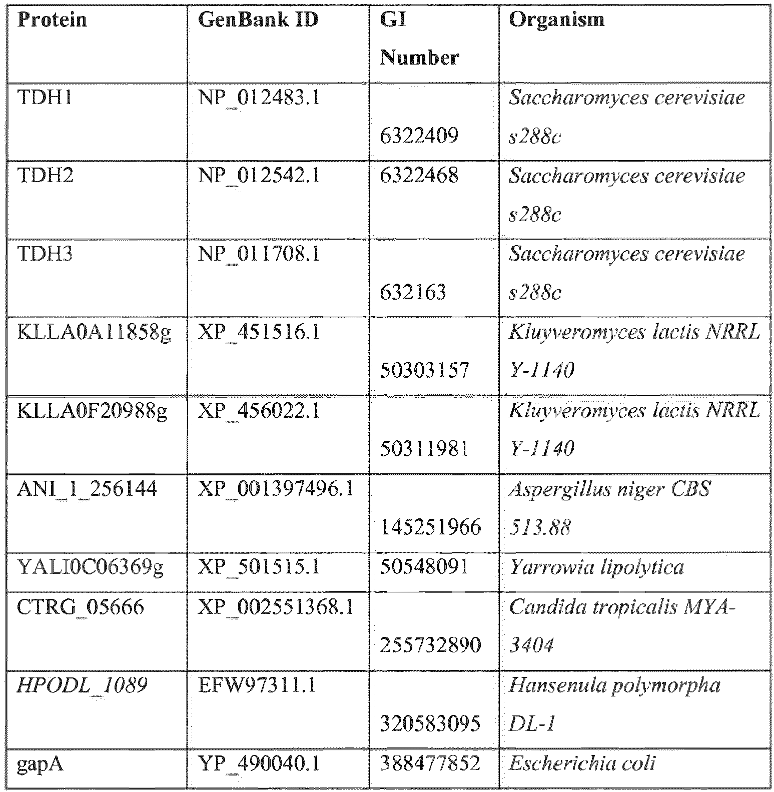

Phosphoglycerate kinase enzymes include:

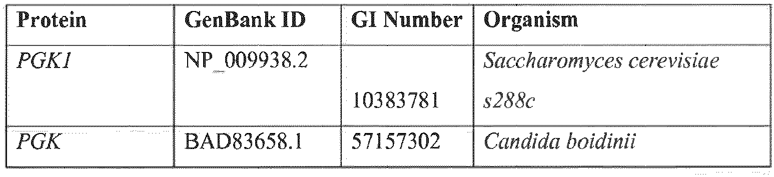

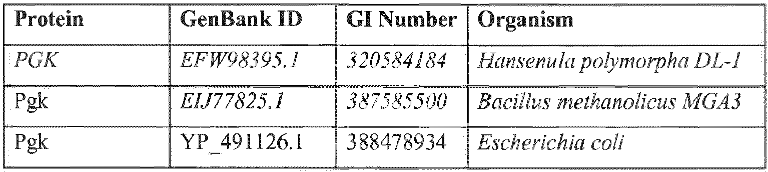

**Phosphoglyceromutase** (aka phosphoglycerate mutase) enzymes include;

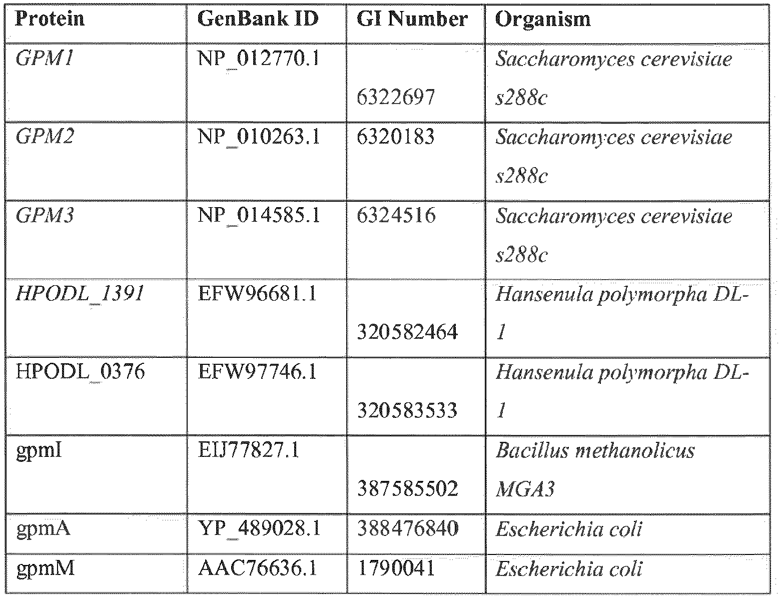

**Enolase** (also known as phosphopyruvate hydratase and 2-phosphoglycerate dehydratase) enzymes include:

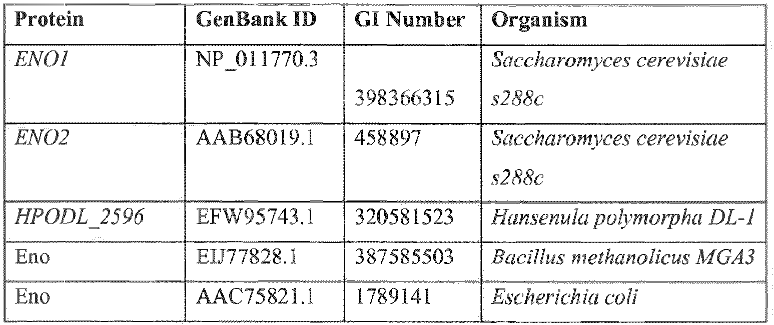

**Pyruvate kinase** (also known as phosphoenolpyruvate kinase and phosphoenolpyruvate kinase) or **PTS-dependent substrate import enzymes** include those below. Pyruvate kinase, also known as phosphoenolpyruvate synthase (EC 2.7.9.2), converts pyruvate and ATP to PEP and AMP. This enzyme is encoded by the PYK1 (Burke et al., J. Biol. Chem. 258:2193-2201 (1983)) and PYK2 (Boles et al., J. Bacteriol. 179:2987-2993 (1997)) genes in S. cerevisiae. In *E. coli,* this activity is catalyzed by the gene products of pykF and pykA. Note that pykA and pykF are genes encoding separate enzymes potentially capable of carrying out the PYK reaction. Selected homologs of the S. cerevisiae enzymes are also shown in the table below.

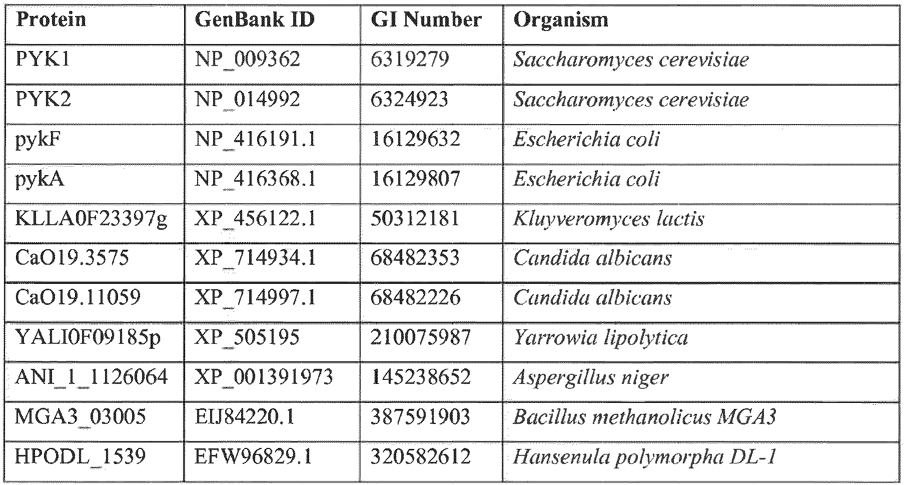

PTS-dependent substrate uptake systems catalyze a phosphotransfer cascade that couples conversion of PEP to pyruvate with the transport and phosphorylation of carbon substrates. For example, the glucose PTS system transports glucose, releasing glucose-6-phosphate into the cytoplasm and concomitantly converting phosphoenolpyruvate to pyruvate. PTS systems are comprised of substrate-specific and non-substrate-specific enzymes or proteins (components). In *E. coli* the two non-specific components are encoded by ptsI (Enzyme I) and ptsH (HPr). The sugar-dependent components are encoded by crr and ptsG. Pts systems have been extensively studied and are reviewed, for example in Postma et al, Microbiol Rev 57: 543-94 (1993).

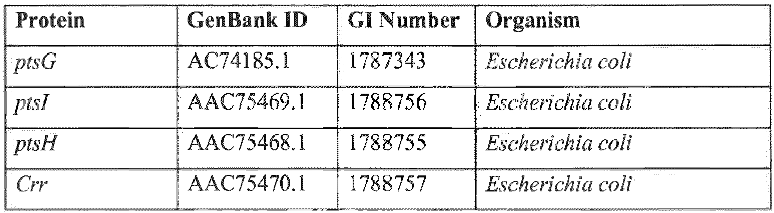

The IIA[Glc] component mediates the transfer of the phosphoryl group from histidine protein Hpr (*ptsH*) to the IIB[Glc] (*ptsG*) component. A truncated variant of the err gene was introduced into 1,4-butancdiol producing strains.

Alternatively, **Phosphoenolpyruvate phosphatase** (EC 3.1.3.60) catalyzes the hydrolysis of PEP to pyruvate and phosphate. Numerous phosphatase enzymes catalyze this activity, including alkaline phosphatase (EC 3.1.3.1), acid phosphatase (EC 3.1.3.2), phosphoglycerate phosphatase (EC 3.1.3.20) and PEP phosphatase (EC 3.1.3.60). PEP phosphatase enzymes have been characterized in plants such as Vignia radiate, Bruguiera sexangula and Brassica nigra. The phytase from Aspergillus fumigates, the acid phosphatase from Homo sapiens and the alkaline phosphatase of E. coli also catalyze the hydrolysis of PEP to pyruvate (Bruggcr ct al, Appl Microbiol Biotech 63:383-9 (2004); Hayman et al, Biochem J 261:601-9 (1989); et al, The Enzymes 3rd Ed. 4:373-415 (1971))). Similar enzymes have been characterized in Campylobacter jejuni (van Mourik et al., Microbiol. 154:584-92 (2008)), Saccharomyces cerevisiae (Oshima et al., Gene 179:171-7 (1996)) and Staphylococcus aureus (Shah and Blobel, J. Bacteriol. 94:780-1 (1967)). Enzyme engineering and/or removal of targeting sequences may be required for alkaline phosphatase enzymes to function in the cytoplasm.

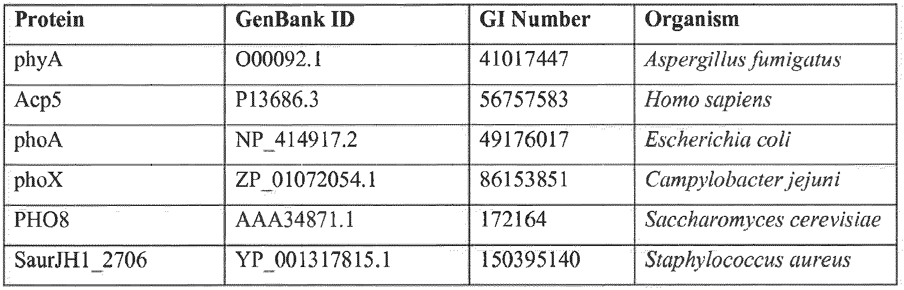

### Step Q, Figure 1: Pyruvate Formate Lyase

Pyruvate formate-lyase (PFL, EC 2.3.1.54), encoded by *pflB* in *E*. *coli,* can convert pyruvate into acetyl-CoA and formate. The activity of PFL can be enhanced by an activating enzyme encoded by *pflA* (Knappe et al., Proc.Natl.Acad.Sci U.S.A 81:1332-1335 (1984); Wong et al., Biochemistry 32:14102-14110 (1993)). Ketoacid formate-lyase (EC 2.3.1.-), also known as 2-ketobutyrate formate-lyase (KFL) and pyruvate formate-lyase 4, is the gene product of *tdcE* in *E.* coli. This enzyme catalyzes the conversion of 2-ketobutyrate to propionyl-CoA and formate during anaerobic threonine degradation, and can also substitute for pyruvate formate-lyase in anaerobic catabolism (Simanshu et al., J Biosci. 32:1195-1206 (2007)). The enzyme is oxygen-sensitive and, like PflB, can require post-translational modification by PFL-AE to activate a glycyl radical in the active site (Hesslinger et al., Mol.Microbiol 27:477-492 (1998)). A pyruvate formate-lyase from *Archaeglubus fulgidus* encoded by *pflD* has been cloned, expressed in *E. coli* and characterized (Lehtio et al., Protein Eng Des Sel 17:545-552 (2004)). The crystal structures of the *A. fulgidus* and *E. coli* enzymes have been resolved (Lehtio et al., J Mol.Biol. 357:221-235 (2006); Leppanen et al., Structure. 7:733-744 (1999)). Additional PFL and PFL-AE candidates are found in *Lactococcus lactis* (Melchiorsen et al., Appl Microbiol Biotechnol 58:338-344 (2002)), and *Streptococcus mutans* (Takahashi-Abbe et al., Oral.Microbial Immunol. 18:293-297

(2003)), *Chlamydomonas reinhardtii* (Hcmschemcier et al., Eukaryot.Cell 7:518-526 (2008b); Atteia et al., J.Biol.Chem. 281:9909-9918 (2006)) and *Clostridium pasteurianum* (Weidner et al., J Bacteriol. 178:2440-2444 (1996)).

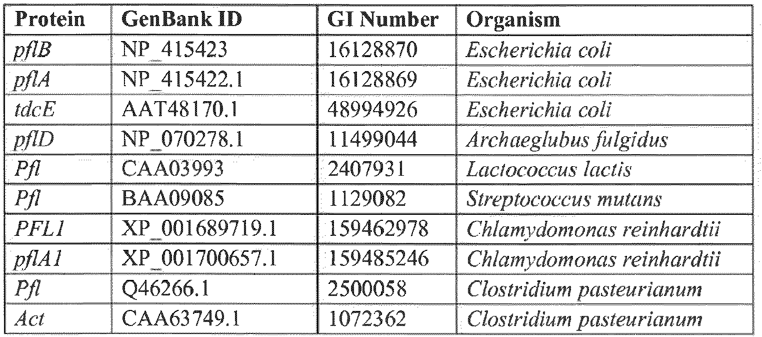

### Step R, Figure 1: Pyruvate dehydrogenase, Pyruvate ferredoxin oxidoreductase, Pyruvate:NADP+ oxidoreductase

The pyruvate dehydrogenase (PDH) complex catalyzes the conversion of pyruvate to acetyl-CoA (e.g., Figure 1 Step R). The *E. coli* PDH complex is encoded by the genes *aceEF* and *lpdA.* Enzyme engineering efforts have improved the *E. coli* PDH enzyme activity under anaerobic conditions (Kim et al., J.Bacteriol. 190:3851-3858 (2008); Kim et al., Appl.Environ.Microbiol. 73:1766-1771 (2007); Zhou et al., Biotechnol.Lett. 30:335-342 (2008)). In contrast to the *E. coli* PDH, the *B. subtilis* complex is active and required for growth under anaerobic conditions (Nakano et al., 179:6749-6755 (1997)). The *Klebsiella pneumoniae* PDH, characterized during growth on glycerol, is also active under anaerobic conditions (Menzel et al., 56:135-142 (1997)). Crystal structures of the enzyme complex from bovine kidney (Zhou et al., 98:14802-14807 (2001)) and the E2 catalytic domain *from Azotobacter vinelandii* are available (Mattevi et al., Science. 255:1544-1550 (1992)). Some mammalian PDH enzymes complexes can react on alternate substrates such as 2-oxobutanoate. Comparative kinetics of *Rattus norvegicus* PDH and BCKAD indicate that BCKAD has higher activity on 2-oxobutanoate as a substrate (Paxton et al., Biochem.J. 234:295-303 (1986)). The S. *cerevisiae* PDH complex can consist of an E2 (*LAT1*) core that binds E1 (*PDA1, PDB1*)*,* E3 (*LPD1*)*,* and Protein X (*PDX1*) components (Pronk et al., Yeast 12:1607-1633 (1996)). The PDH complex of *S*. *cerevisiae* is regulated by phosphorylation of E1 involving PKP1 (PDH kinase I), PTC5 (PDH phosphatase 1), PKP2 and PTC6. Modification of these regulators may also enhance PDH activity. Coexpression of lipoyl ligase (*LplA* of E. coli and A1M22 in *S. cerevisiae*) with PDH in the cytosol may be necessary for activating the PDH enzyme complex. Increasing the supply of cytosolic lipoate, either by modifying a metabolic pathway or media supplementation with lipoate, may also improve PDH activity.

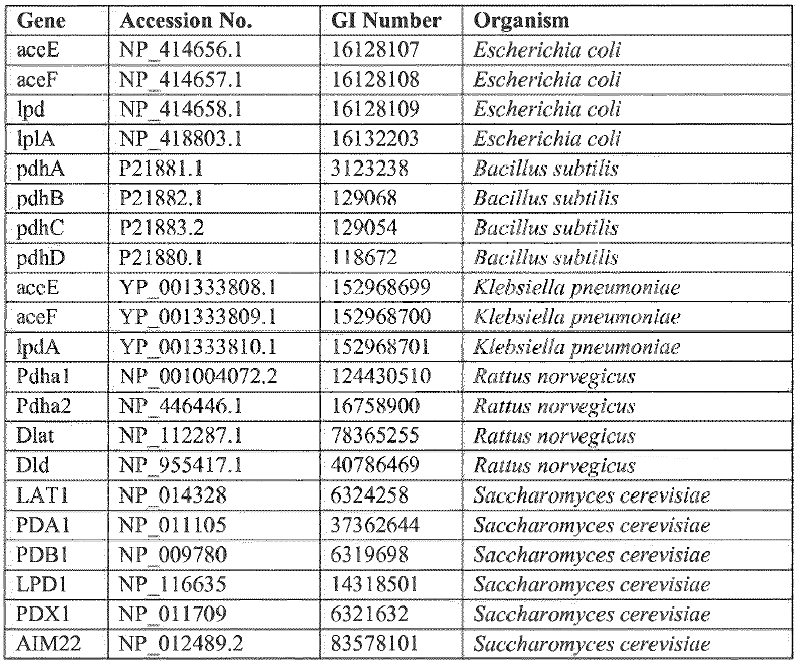

As an alternative to the large multienzyme PDH complexes described above, some organisms utilize enzymes in the 2-ketoacid oxidoreductase family (OFOR) to catalyze acylating oxidative decarboxylation of 2-kcto-acids. Unlike the PDH complexes, PFOR enzymes contain iron-sulfur clusters, utilize different cofactors and use ferredoxin or flavodixin as electron acceptors in lieu of NAD(P)H. Pyruvate ferredoxin oxidoreductase (PFOR) can catalyze the oxidation of pyruvate to form acetyl-CoA (e.g., Figure 1 Step R). The PFOR from *Desulfovibrio africanus* has been cloned and expressed in *E. coli* resulting in an active recombinant enzyme that was stable for several days in the presence of oxygen (Pieulle et al., J Bacteriol. 179:5684-5692 (1997)). Oxygen stability is relatively uncommon in PFORs and is believed to be conferred by a 60 residue extension in the polypeptide chain of the *D. cifricanus* enzyme. The *M. thermoacetica* PFOR is also well characterized (Menon et al., Biochemistry 36:8484-8494 (1997)) and was even shown to have high activity in the direction of pyruvate synthesis during autotrophic growth (Furdui et al., J Biol Chem. 275:28494-28499 (2000)). Further, *E. coli* possesses an uncharacterized open reading frame, *ydbK,* that encodes a protein that is 51% identical to the *M. thermoacetica* PFOR. Evidence for pyruvate oxidoreductase activity in *E. coli* has been described (Blaschkowski et al., Eur.J Biochem. 123:563-569 (1982)). Several additional PFOR enzymes are described in Ragsdale, Chem.Rev. 103:2333-2346 (2003). Finally, flavodoxin reductases (e.g., fqrB from *Helicobacter pylori* or *Carvpylobacter jejuni* (St Maurice et al., J.Bacteriol. 189:4764-4773 (2007))) or Rnf-type proteins (Seedorf et al., Proc. Natl.Acad.Sci. U S.A. 105:2128-2133 (2008); Herrmann et al., J.Bacteriol. 190:784-791 (2008)) provide a means to generate NADH or NADPH from the reduced ferredoxin generated by PFOR. These proteins are identified below.

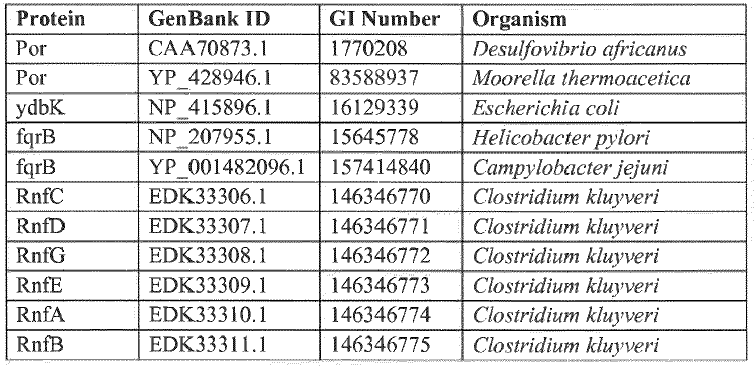

Pyruvate:NADP oxidoreductase (PNO) catalyzes the conversion of pyruvate to acetyl-CoA. This enzyme is encoded by a single gene and the active enzyme is a homodimer, in contrast to the multi-subunit PDH enzyme complexes described above. The enzyme from *Euglena gracilis* is stabilized by its cofactor, thiamin pyrophosphate (Nakazawa et al, Arch Biochem Biophys 411:183-8 (2003)). The mitochondrial targeting sequence of this enzyme should be removed for expression in the cytosol. The PNO protein of *E*. *gracilis* and other NADP-dependent pyruvate:NADP+ oxidoreductase enzymes are listed in the table below.

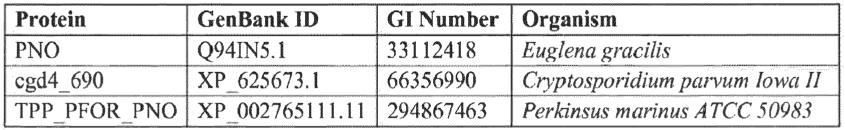

### EXAMPLE V

### Production of Reducing Equivalents

This example describes additional enzymes including those useful for generating reducing equivalents.

### Formate Hydrogen Lyase (e.g. Figure 1, Step Q)

A formate hydrogen lyase enzyme can be employed to convert formate to carbon dioxide and hydrogen. An exemplary formate hydrogen lyase enzyme can be found in *Escherichia coli.* The *E. coli* formate hydrogen lyase consists of hydrogenase 3 and formate dehydrogenase-H (Maeda et al., Appl Microbiol Biotechnol 77:879-890 (2007)). It is activated by the gene product of *fhlA.* (Maeda et al., Appl Microbiol Biotechnol 77:879-890 (2007)). The addition of the trace elements, selenium, nickel and molybdenum, to a fermentation broth has been shown to enhance formate hydrogen lyase activity (Soini et al., Microb. Cell Fact. 7:26 (2008)). Various hydrogenase 3, formate dehydrogenase and transcriptional activator genes are shown below.

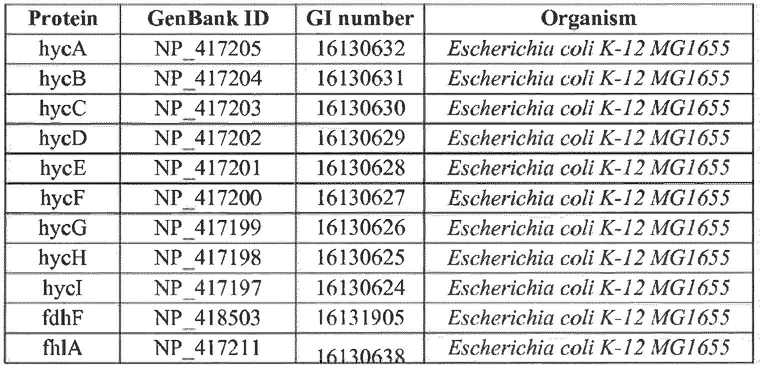

A formate hydrogen lyase enzyme also exists in the hyperthermophilic archaeon, *Thermococcus litoralis* (Takacs et al., BMC.Microbiol 8:88 (2008)).

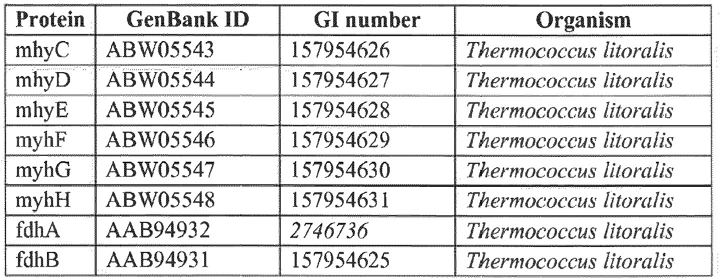

Additional formate hydrogen lyase systems have been found in *Salmonella typhimurium, Klebsiella pneumoniae, Rhodospirillum rubrum, Methanobacterium formicicum* (Vardar-Schara et al., Microbial Biotechnology 1:107-125 (2008)).

### Hydrogenase

Hydrogenase enzymes can convert hydrogen gas to protons and transfer electrons to acceptors such as ferredoxins, NAD+, or NADP+. *Ralstonia eutropha* H16 uses hydrogen as an energy source with oxygen as a terminal electron acceptor. Its membrane-bound uptake [NiFe]-hydrogenase is an "O2-tolerant" hydrogenase (Cracknell, et al. Proc Nat Acad Sci, 106(49) 20681-20686 (2009)) that is periplasmically-oriented and connected to the respiratory chain via a b-type cytochrome (Schink and Schlegel, Biochim. Biophys. Acta, 567, 315-324 (1979); Bernhard et al., Eur. J. Biochem. 248, 179-186 (1997)). *R. eutropha* also contains an O₂-tolerant soluble hydrogenase encoded by the *Hox* operon which is cytoplasmic and directly reduces NAD+ at the expense of hydrogen (Schneider and Schlegel, Biochim. Biophys. Acta 452, 66-80 (1976); Burgdorf, J. Bact. 187(9) 3122-3132(2005)). Soluble hydrogenase enzymes are additionally present in several other organisms including *Geobacter sulfurreducens* (Coppi, Nlicrobiology 151, 1239-1254 (2005)), *Synechocystis* str. PCC 6803 (Germer, J. Biol. Chem., 284(52), 36462-36472 (2009)), and *Thiocapsa roseopersicina* (Rakhely, Appl. Environ. Microbiol. 70(2) 722-728 (2004)). The *Synechocystis* enzyme is capable of generating NADPH from hydrogen. Overexpression of both the *Hox* operon from *Synechocystis* str. PCC 6803 and the accessory genes encoded by the *Hyp* operon from *Nostoc* sp. PCC 7120 led to increased hydrogenase activity compared to expression of the *Hox* genes alone (Germer, J. Biol. Chem. 284(52), 36462-36472 (2009)).

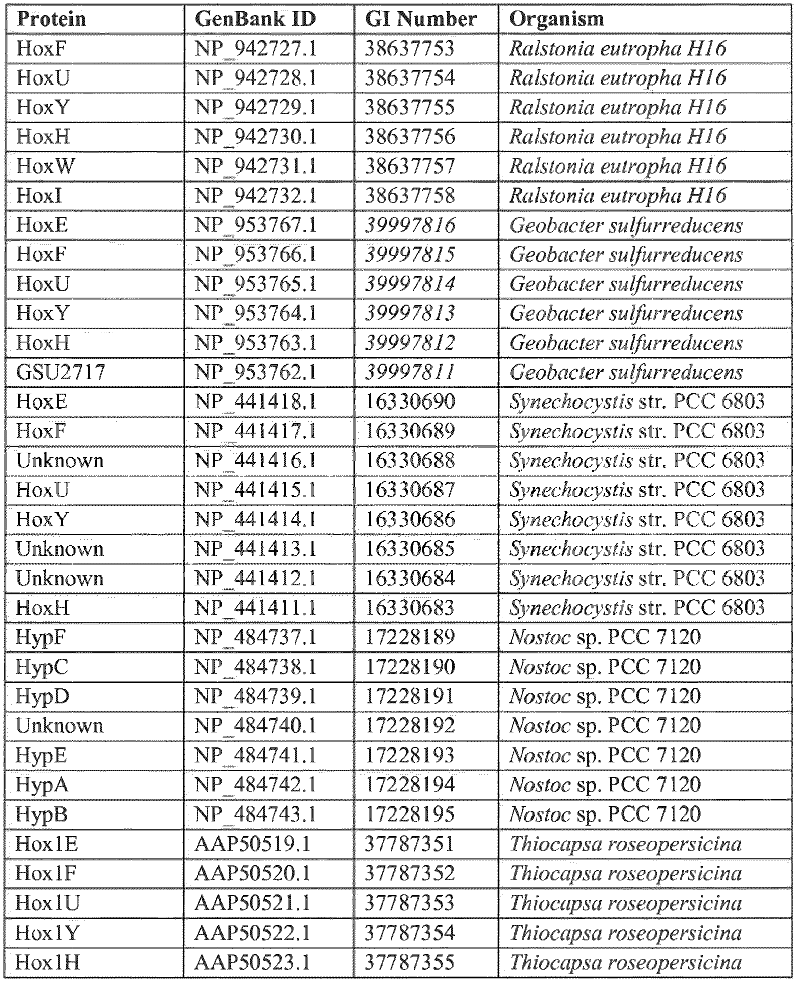

The genomes of *E*. *coli* and other enteric bacteria encode up to four hydrogenase enzymes (Sawers, G., Antonie Van Leeuwenhoek 66:57-88 (1994); Sawers et al., J Bacteriol. 164:1324-1331 (1985); Sawers and Boxer, Eur.J Biochem. 156:265-275 (1986); Sawers et al., J Bacteriol. 168:398-404 (1986)). Given the multiplicity of enzyme activities *E. coli* or another host organism can provide sufficient hydrogenase activity to split incoming molecular hydrogen and reduce the corresponding acceptor. Endogenous hydrogen-lyase enzymes of *E*. *coli* include hydrogenase 3, a membrane-bound enzyme complex using ferredoxin as an acceptor, and hydrogenase 4 that also uses a ferredoxin acceptor. Hydrogenase 3 and 4 are encoded by the *hyc* and *hyf* gene clusters, respectively. Hydrogenase activity in *E. coli* is also dependent upon the expression of the *hyp* genes whose corresponding proteins are involved in the assembly of the hydrogenase complexes (Jacobi et al., Arch.Microbiol 158:444-451 (1992); Rangarajan et al., J Bacteriol. 190:1447-1458 (2008)). The *M. thermoacetica* and *Clostridium ljungdahli* hydrogenases are suitable for a host that lacks sufficient endogenous hydrogenase activity. *M. thermoacetica* and C. *ljungdahli* can grow with CO₂ as the exclusive carbon source indicating that reducing equivalents are extracted from H₂ to enable acetyl-CoA synthesis via the Wood-Ljungdahl pathway (Drake, H. L., J Bacteriol. 150:702-709 (1982); Drake and Daniel, Res Microbiol 155:869-883 (2004); Kellum and Drake, J Bacteriol. 160:466-469 (1984)). *M. thermoacetica* has homologs to several *hyp, hyc,* and *hyf* genes from *E. coli.* These protein sequences encoded for by these genes are identified by the following GenBank accession numbers. In addition, several gene clusters encoding hydrogenase functionality are present in *M*. *thermoacetica* and C. *ljungdahli* (see for example US 2012/0003652).

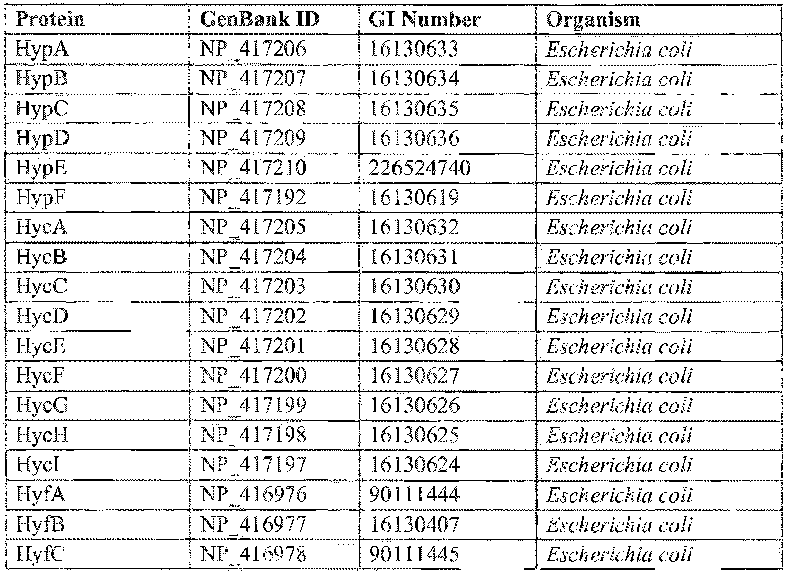

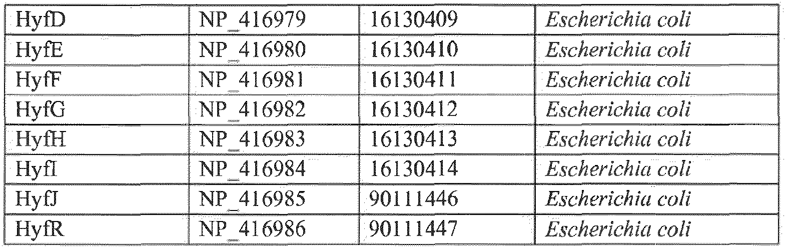

Proteins in *M. thermoacetica* whose genes are homologous to the *E. coli* hydrogenase genes are shown below.

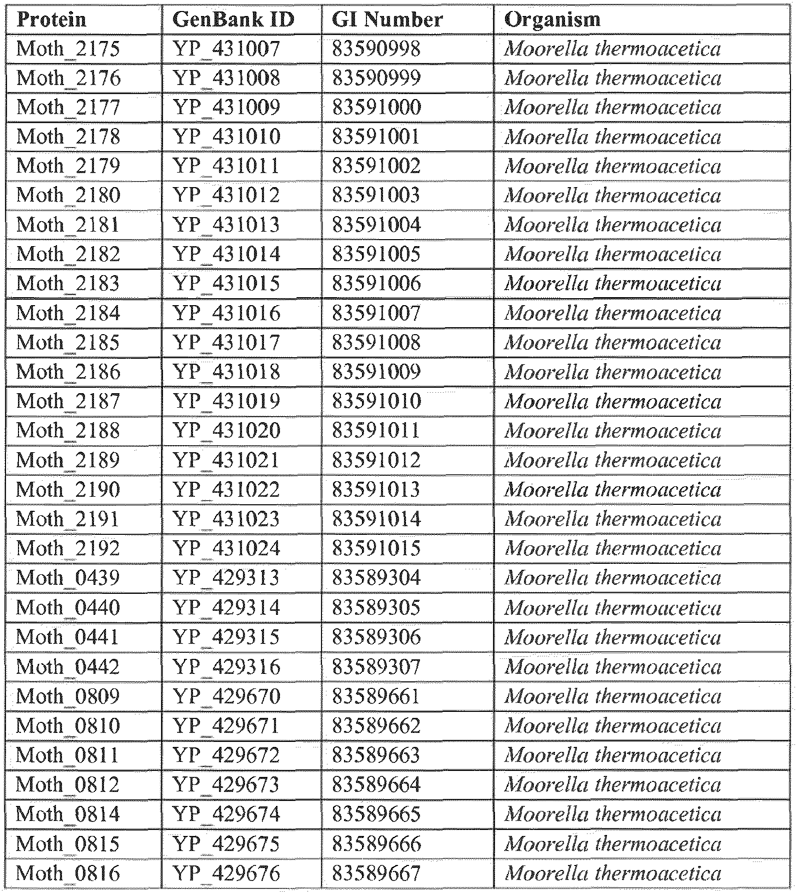

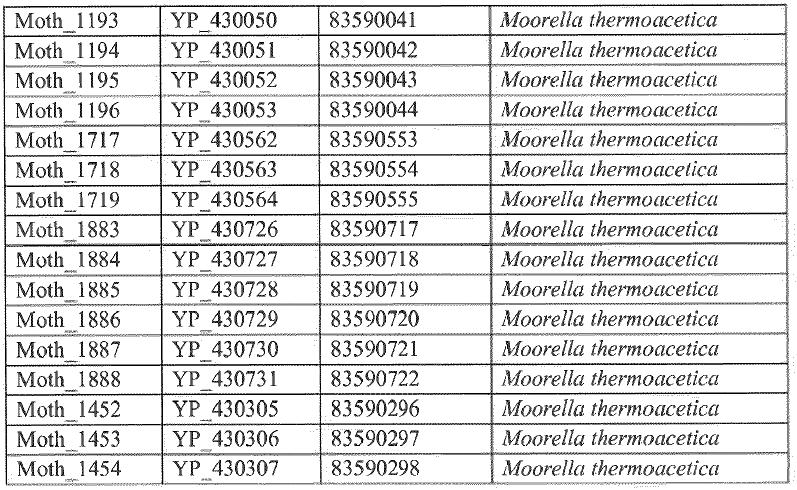

Genes encoding hydrogenase enzymes from C. *ljungdahli* are shown below.

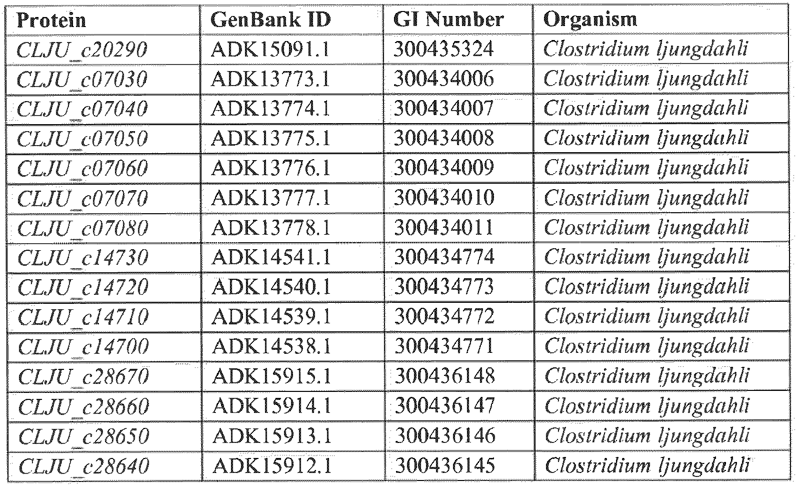

In some cases, hydrogenase encoding genes are located adjacent to a CODH. In *Rhodospirillum rubrum,* the encoded CODH/hydrogenase proteins form a membrane-bound enzyme complex that has been indicated to be a site where energy, in the form of a proton gradient, is generated from the conversion of CO and H₂O to CO₂ and H₂ (Fox et al., J Bacteriol. 178:6200-6208 (1996)). The CODH-I of C. *hydrogenoformans* and its adjacent genes have been proposed to catalyze a similar functional role based on their similarity to the *R. rubrum* CODH/hydrogenase gene cluster (Wu et al., PLoS Genet. 1:e65 (2005)). The C. *hydrogenoformans* CODH-I was also shown to exhibit intense CO oxidation and CO₂ reduction activities when linked to an electrode (Parkin et al., J Am.Chem.Soc. 129:10328-10329 (2007)).

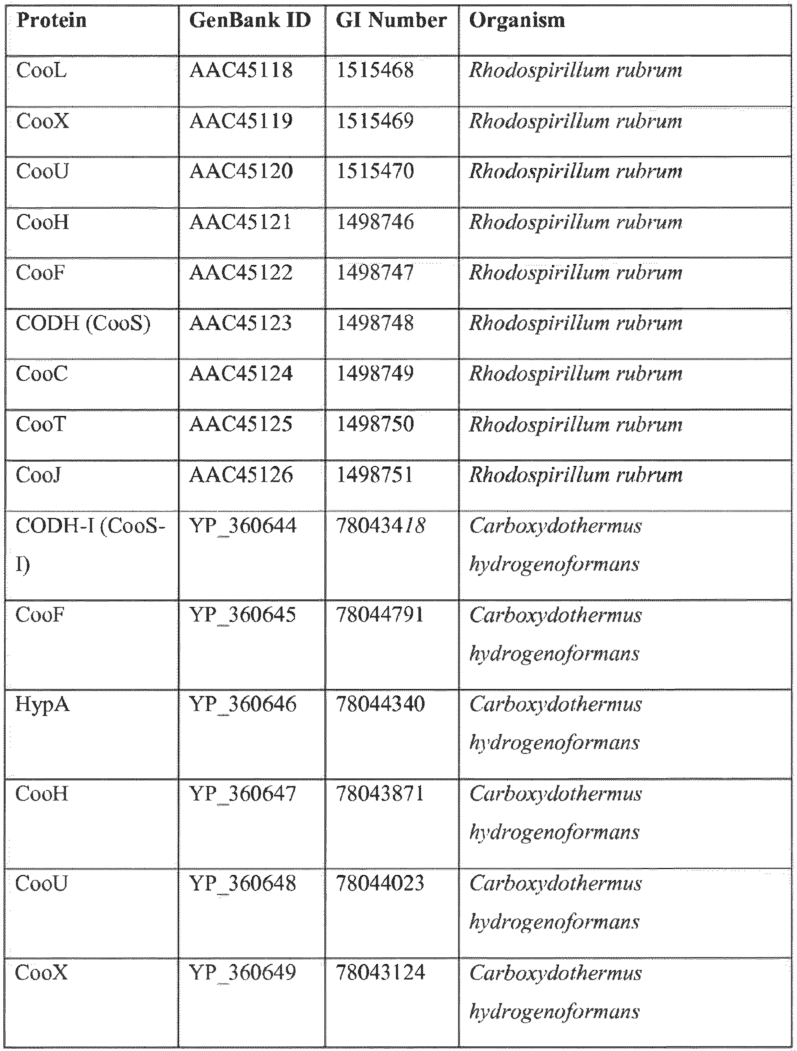

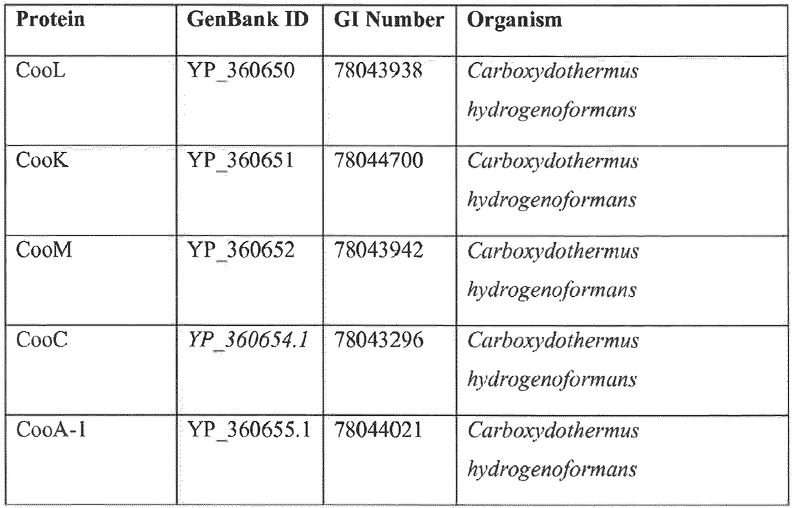

Some hydrogenase and CODH enzymes transfer electrons to ferredoxins. Ferredoxins are small acidic proteins containing one or more iron-sulfur clusters that function as intracellular electron carriers with a low reduction potential. Reduced ferredoxins donate electrons to Fe-dependent enzymes such as ferredoxin-NADP⁺ oxidoreductase, pyruvate:ferredoxin oxidoreductase (PFOR) and 2-oxoglutarate:ferredoxin oxidoreductase (OFOR). The *H. thermophilus* gene*fdx1* encodes a [4Fe-4S]-type ferredoxin that is required for the reversible carboxylation of 2-oxoglutarate and pyruvate by OFOR and PFOR, respectively (Yamamoto et al., Extremophiles 14:79-85 (2010)). The ferredoxin associated with the *Sulfolobus solfataricus* 2-oxoacid:ferredoxin reductase is a monomeric dicluster [3Fe-4S][4Fe-4S] type ferredoxin (Park et al. 2006). While the gene associated with this protein has not been fully sequenced, the N-terminal domain shares 93% homology with the *zfx* ferredoxin from S. *acidocaldarius.* The *E. coli* genome encodes a soluble ferredoxin of unknown physiological function, *fdx.* Some evidence indicates that this protein can function in iron-sulfur cluster assembly (Takahashi and Nakamura, 1999). Additional ferredoxin proteins have been characterized in Helicobacter pylori (Mukhopadhyay et al. 2003) and *Campylobacter jejuni* (van Vliet et al. 2001). A 2Fe-2S ferredoxin from *Clostridium prasteuricanum* has been cloned and expressed in *E. coli* (Fujinaga and Meyer, Biochemical and Biophysical Research Communications, 192(3): (1993)). Acctogcnic bacteria such as *Moorella thermoacetica, Clostridium carboxidivorans P7, Clostridium ljungdahli* and *Rhodospirillum rubrum* are predicted to encode several ferredoxins, listed below.

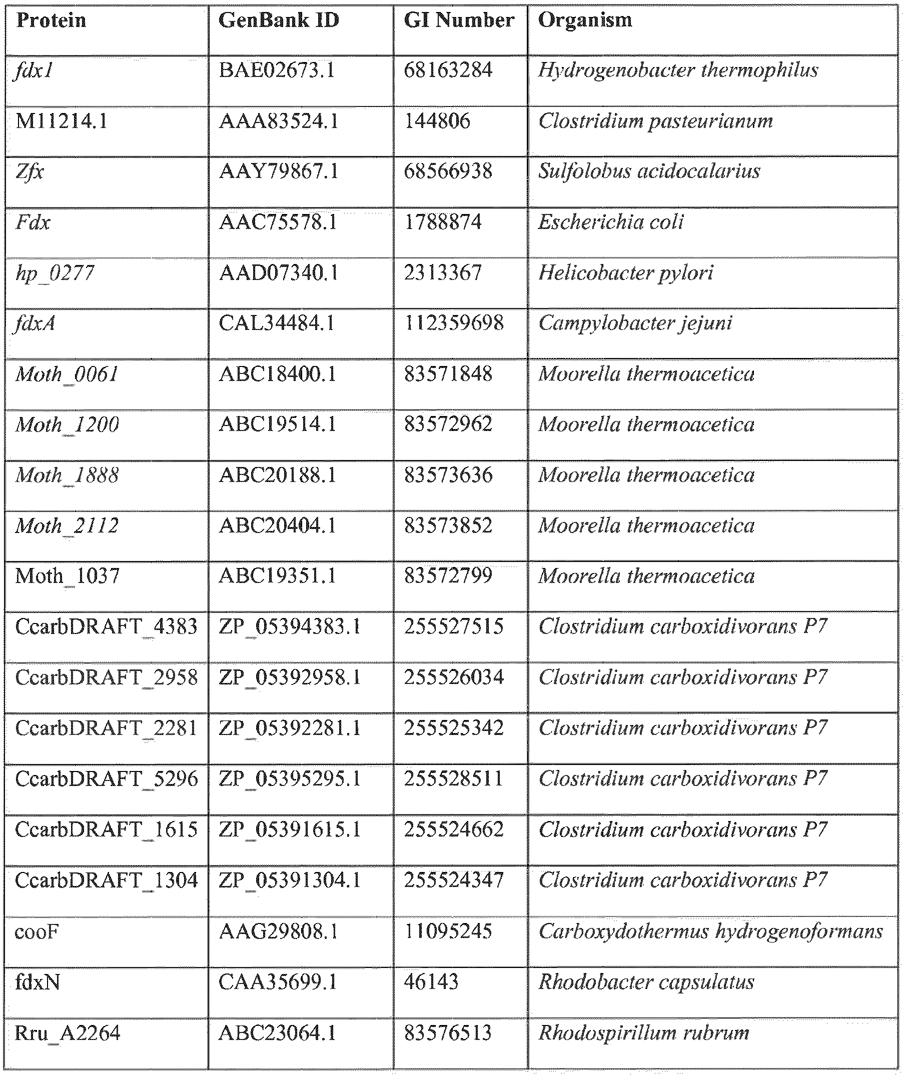

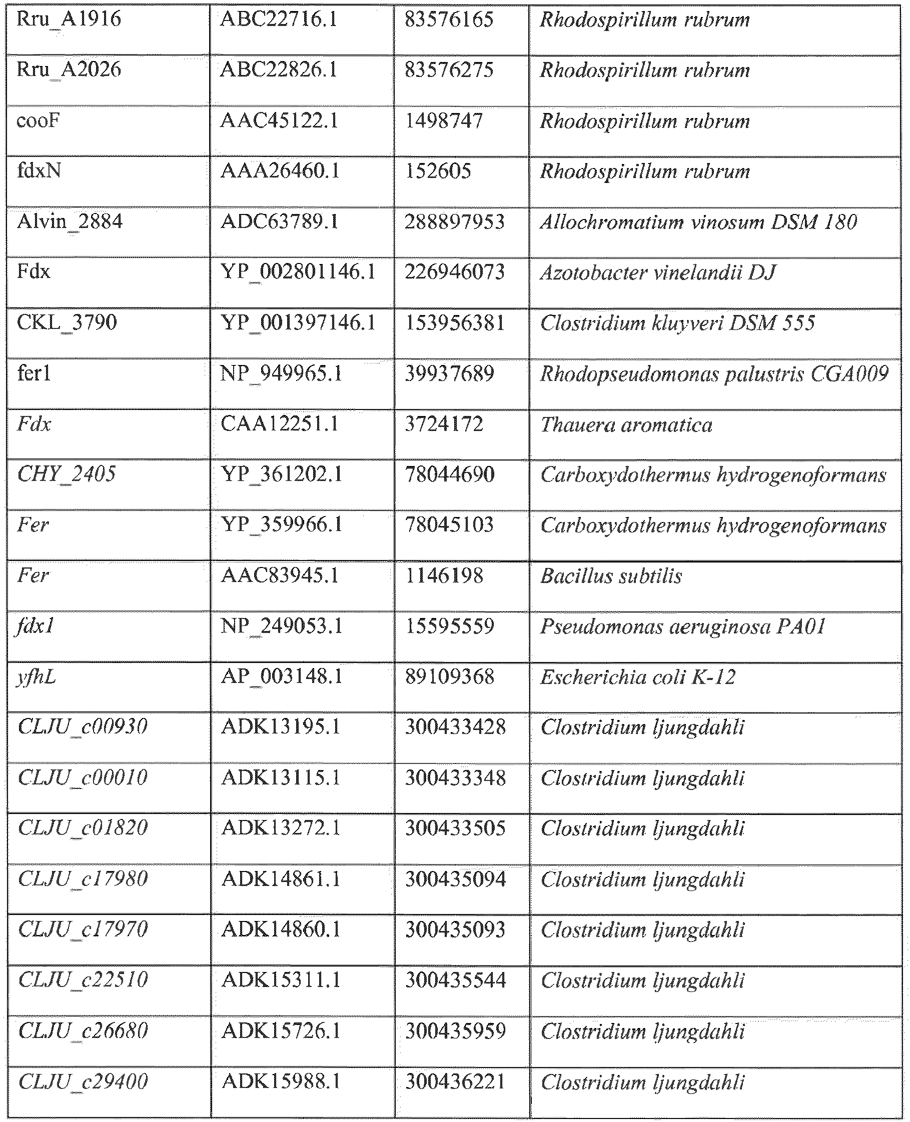

Ferredoxin oxidoreductase enzymes transfer electrons from ferredoxins or flavodoxins to NAD(P)H. Two enzymes catalyzing the reversible transfer of electrons from reduced ferredoxins to NAD(P)+ are ferredoxin:NAD+ oxidoreductase (EC 1.18.1.3) and ferredoxin:NADP+ oxidoreductase (FNR, EC 1.18.1.2). Ferredoxin:NADP+ oxidoreductase (FNR, EC 1.18.1.2) has a noncovalently bound FAD cofactor that facilitates the reversible transfer of electrons from NADPH to low-potential acceptors such as ferredoxins or flavodoxins (Blaschkowski et al., Eur. J. Biochem. 123:563-569 (1982); Fujii et al., 1977). The *Helicobacter pylori* FNR, encoded by HP1164 (fqrB), is coupled to the activity of pyruvate:ferredoxin oxidoreductase (PFOR) resulting in the pyruvate-dependent production of NADPH (St et al. 2007). An analogous enzyme is found in *Campylobacter jejuni* (St Maurice et al., J. Bacteriol. 189:4764-4773 (2007)). A ferredoxin:NADP+ oxidoreductase enzyme is encoded in the *E*. *coli* genome by *fpr* (Bianchi et al. 1993). Ferredoxin:NAD+ oxidoreductase utilizes reduced ferredoxin to generate NADH from NAD+. In several organisms, including *E. coli,* this enzyme is a component of multifunctional dioxygenase enzyme complexes. The ferredoxin:NAD+ oxidoreductase of *E. coli,* encoded by *hcccD,* is a component of the 3-phenylproppionate dioxygenase system involved in involved in aromatic acid utilization (Diaz et al. 1998). NADH:ferredoxin reductase activity was detected in cell extracts of *Hydrogenobacter thermophilus,* although a gene with this activity has not yet been indicated (Yoon et al. 2006). Additional ferredoxin:NAD(P)+ oxidoreductases have been annotated in *Clostridium carboxydivorans* P7. The NADH-dependent reduced ferredoxin: NADP oxidoreductase of *C. kluyveri,* encoded by *nfnAB,* catalyzes the concomitant reduction of ferredoxin and NAD+ with two equivalents of NADPH (Wang et al, J Bacteriol 192: 5115-5123 (2010)). Finally, the energy-conserving membrane-associated Rnf-type proteins (Seedorf et al, PNAS 105:2128-2133 (2008); and Herrmann, J. Bacteriol 190:784-791 (2008)) provide a means to generate NADH or NADPH from reduced ferredoxin.

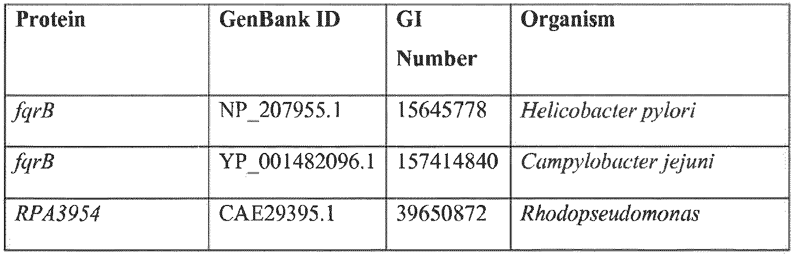

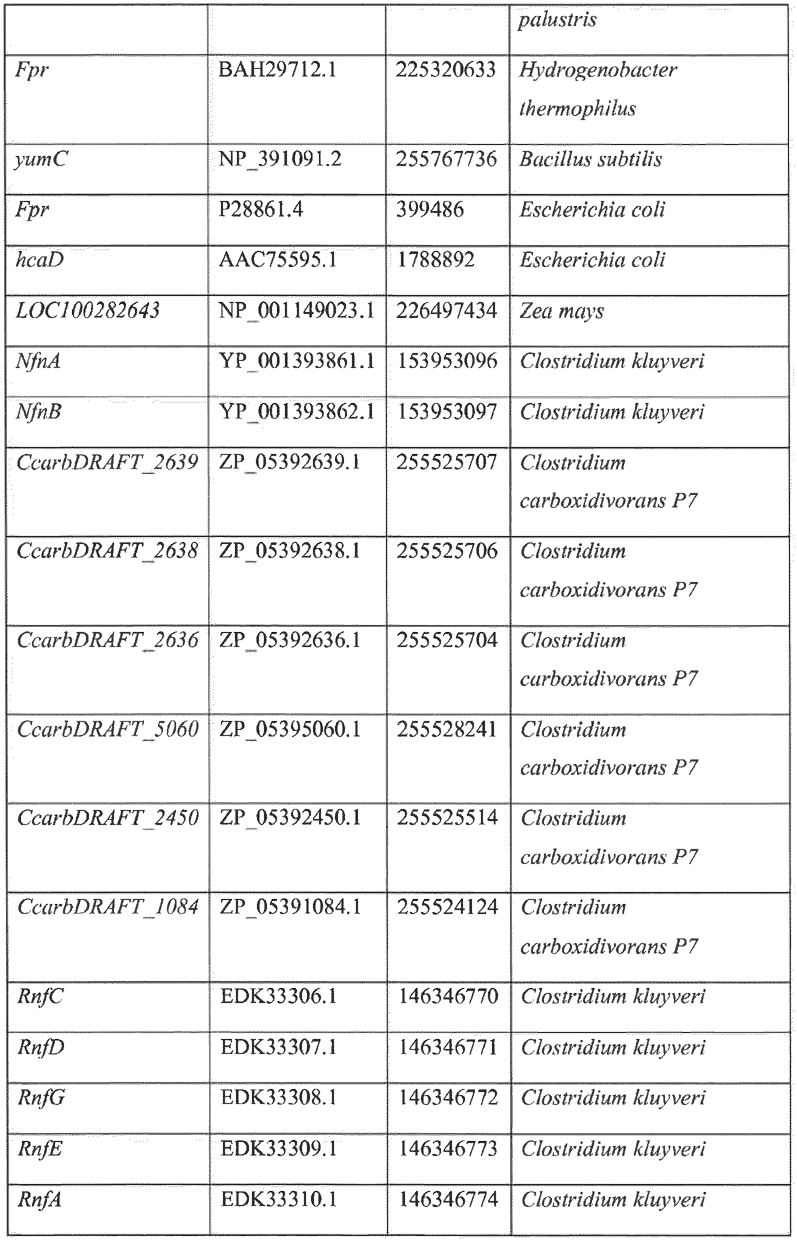

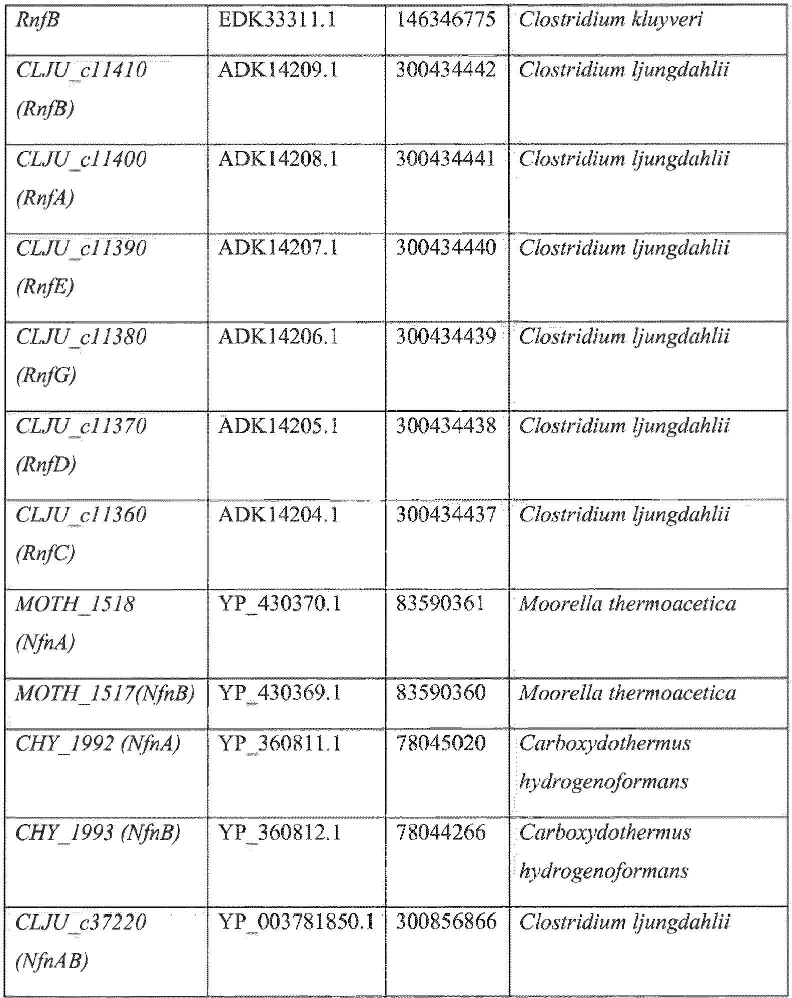

### Formate Dehydrogenase

Formate dehydrogenase (FDH) catalyzes the reversible transfer of electrons from formate to an acceptor. See also Figure 1 Step S. Enzymes with FDH activity utilize various electron carriers such as, for example, NADH (EC 1.2.1.2), NADPH (EC 1.2.1.43), quinols (EC 1.1.5.6), cytochromes (EC 1.2.2.3) and hydrogenases (EC 1.1.99.33). FDH enzymes have been characterized from *Moorella thermoacetica* (Andreesen and Ljungdahl, J Bacteriol 116:867-873 (1973); Li et al., J Bacterial 92:405-412 (1966); Yamamoto et al., J Biol Chem. 258:1826-1832 (1983). The loci, Moth_2312 is responsible for encoding the alpha subunit of formate dehydrogenase while the beta subunit is encoded by Moth_2314 (Pierce et al., Environ Microbiol (2008)). Another set of genes encoding formate dehydrogenase activity with a propensity for CO₂ reduction is encoded by Sfum_2703 through Sfum_2706 in *Syntrophobacter fumaroxidans* (de Bok et al., Eur J Biochem. 270:2476-2485 (2003)); Reda et al., PNAS 105:10654-10658 (2008)). A similar set of genes presumed to carry out the same function are encoded by CHY_0731, CHY_0732, and CHY_0733 in C. *hydrogenoformans* (Wu et al., PLoS Genet 1:e65 (2005)). Formate dehydrogenases are also found many additional organisms including C. *carboxidivorans P7, Bacillus methanolicus, Burkholderia stabilis, Moorella thermoacetica* ATCC 39073, *Candida boidinii, Candida methylica,* and *Saccharomyces cerevisiae* S288c. The soluble formate dehydrogenase from *Ralstonia eutropha* reduces NAD⁺ (*fdsG, -B,* -*A*, *C, -D)* (Oh and Bowien, 1998).

Several formate dehydrogenase enzymes have been identified that have higher specificity for NADP as the cofactor as compared to NAD. This enzyme has been deemed as the NADP-dependent formate dehydrogenase and has been reported from 5 species of the *Burkholderia cepacia* complex. It was tested and verified in multiple strains of *Burkholderia multivorans, Burkholderia stabilis, Burkholderia pyrrocinia,* and *Burkholderia cenocepacia* (Hatrongjit et al., Enzyme and Microbial Tech., 46: 557-561 (2010)). The enzyme from *Burkholderia stabilis* has been characterized and the apparent Kₘ of the enzyme were reported to be 55.5 mM, 0.16 mM and 1.43 mM for formate, NADP, and NAD respectively. More gene candidates can be identified using sequence homology of proteins deposited in Public databases such as NCBI, JGI and the metagenomic databases.

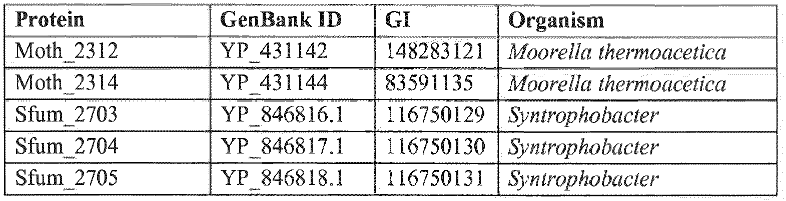

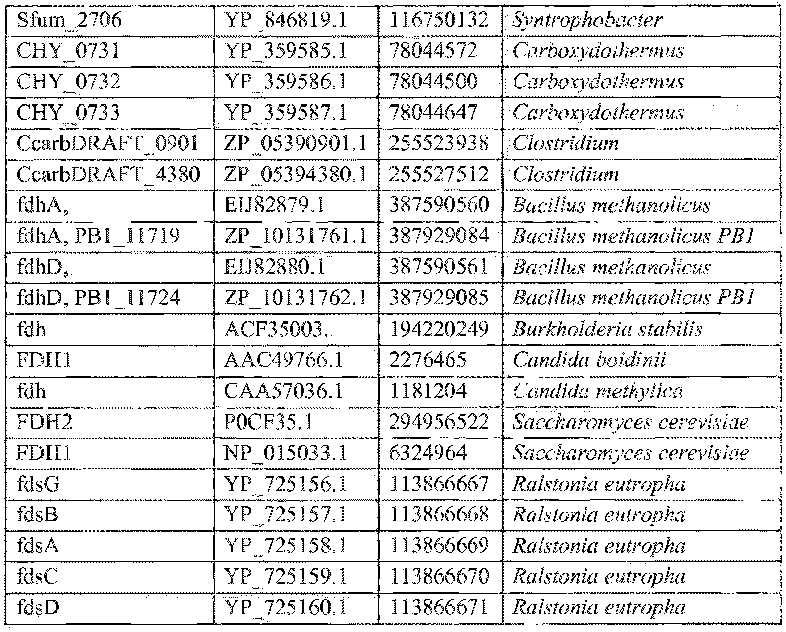

### Figure 1 Step A-Methanol Dehydrogenase

NAD+ dependent methanol dehydrogenase enzymes (EC 1.1.1.244) catalyze the conversion of methanol and NAD+ to formaldehyde and NADH, which is a first step in a methanol oxidation pathway. An enzyme with this activity was first characterized in *Bacillus methanolicus* (Heggeset, et al., Applied and Environmental Microbiology, 78(15):5170-5181 (2012)). This enzyme is zinc and magnesium dependent, and activity of the enzyme is enhanced by the activating enzyme encoded by act (Kloosterman et al, JBiol Chem 277:34785-92 (2002)). The act is a Nudix hydrolase. Several of these candidates have been identified and shown to have activity on methanol. Additional NAD(P)+ dependent enzymes can be identified by sequence homology. Methanol dehydrogenase enzymes utilizing different electron acceptors are also known in the art. Examples include cytochrome dependent enzymes such as *mxaIF* of the methylotroph *Methylobacterium extorquens* (Nunn et al, Nucl Acid Res 16:7722 (1988)). Methanol dehydrogenase enzymes of methanotrophs such as *Methylococcus capsulatis* function in a complex with methane monooxygenase (MMO) (Myronova et al, Biochem 45:11905-14 (2006)). Methanol can also be oxidized to formaldehyde by alcohol oxidase enzymes such as methanol oxidase (EC 1.1.3.13) of *Candida boidinii* (Sakai et al, Gene 114: 67-73 (1992)).

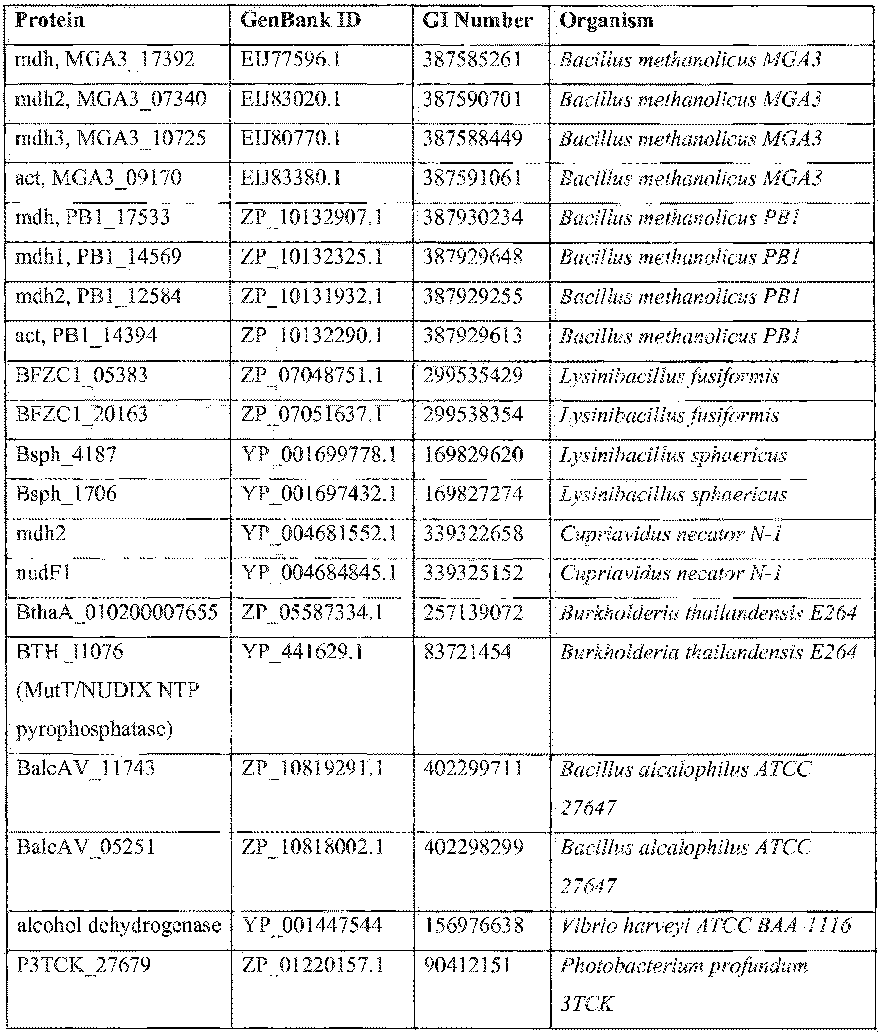

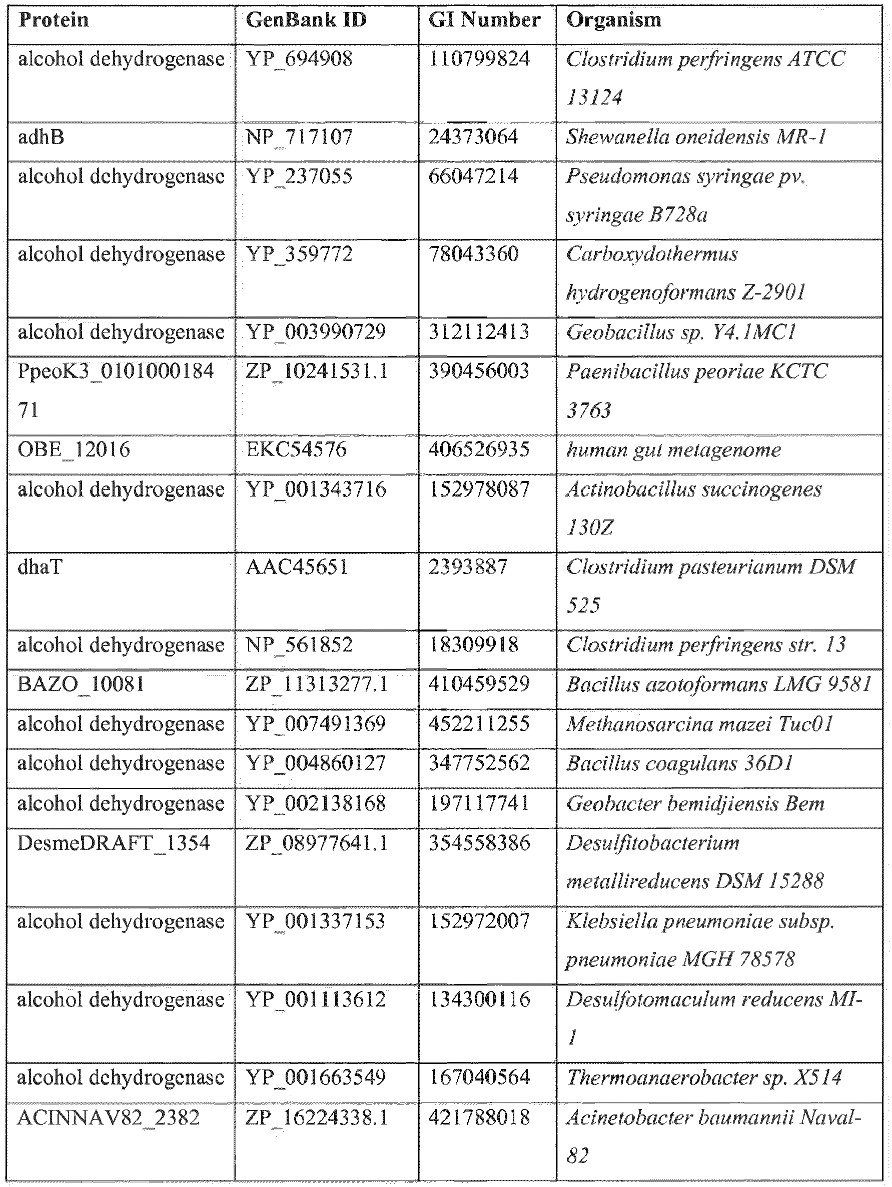

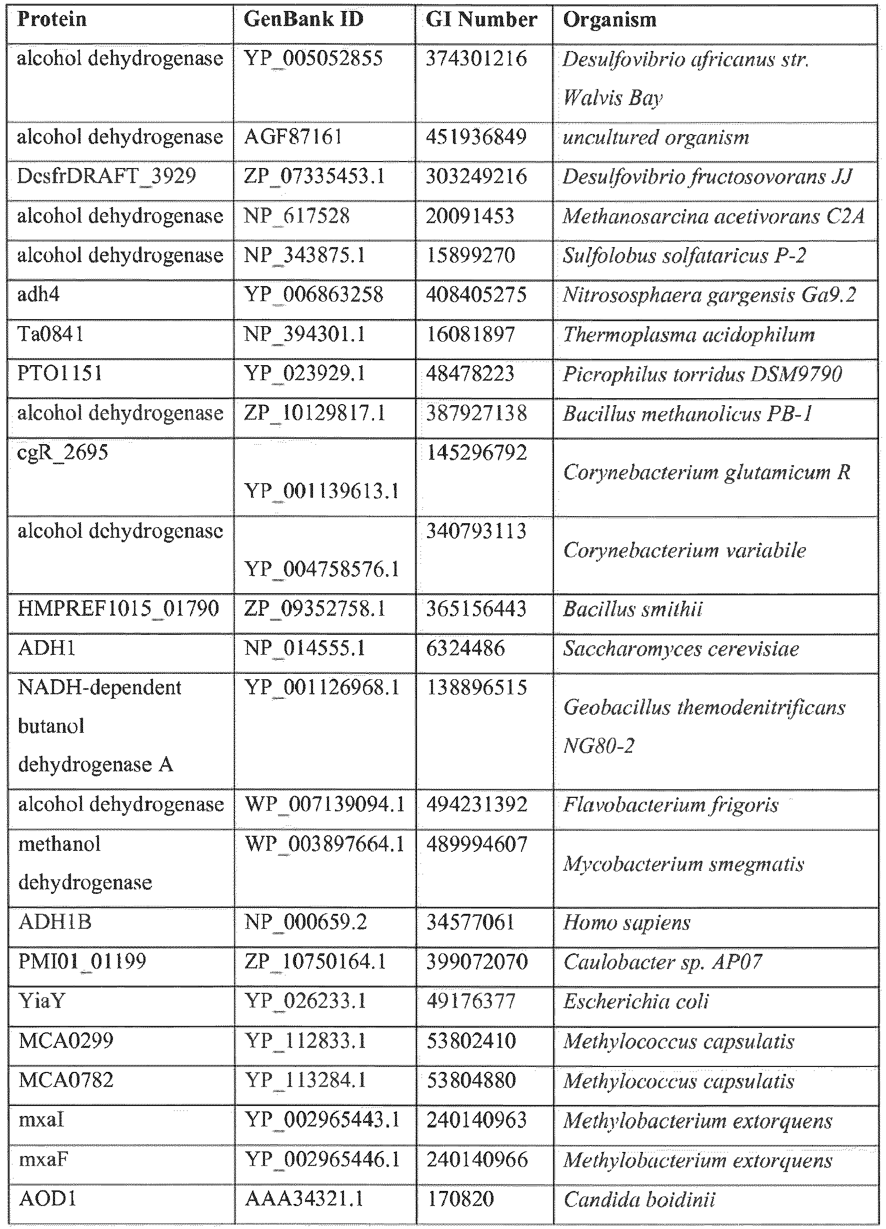

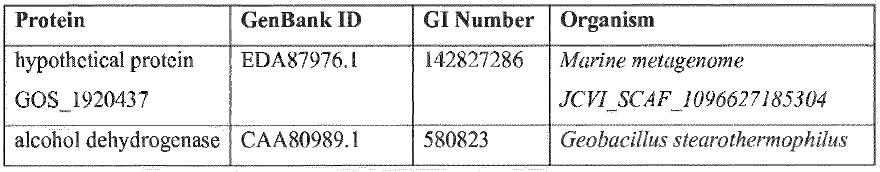

An *in vivo* assay was developed to determine the activity of methanol dehydrogenases. This assay relies on the detection of formaldehyde (HCHO), thus measuring the forward activity of the enzyme (oxidation of methanol). To this end, a strain comprising a BDOP and lacking *frmA, frmB, frmR* was created using Lambda Red recombinase technology (Datsenko and Wanner, Proc. Natl. Acad. Sci. USA, 6 97(12): 6640-5 (2000). Plasmids expressing methanol dehydrogenases were transformed into the strain, then grown to saturation in LB medium + antibiotic at 37° C with shaking. Transformation of the strain with an empty vector served as a negative control. Cultures were adjusted by O.D. and then diluted 1:10 into M9 medium + 0.5% glucose + antibiotic and cultured at 37° C with shaking for 6-8 hours until late log phase. Methanol was added to 2% v/v and the cultures were further incubated for 30 min. with shaking at 37° C. Cultures were spun down and the supernatant was assayed for formaldehyde produced using DETECTX Formaldehyde Detection kit (Arbor Assays; Ann Arbor, MI) according to manufacturer's instructions. The *frmA, frmB, frmR* deletions resulted in the native formaldehyde utilization pathway to be deleted, which enables the formation of formaldehyde that can be used to detect methanol dehydrogenase activity in the non-naturally occurring microbial organism.

The activity of several enzymes was measured using the assay described above. The results of four independent experiments are provided in Table 5 below.

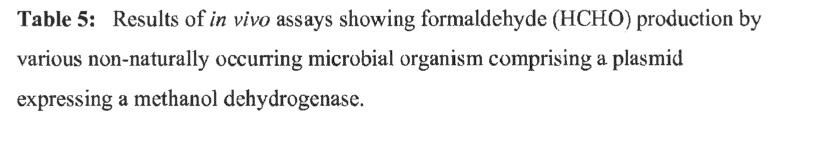

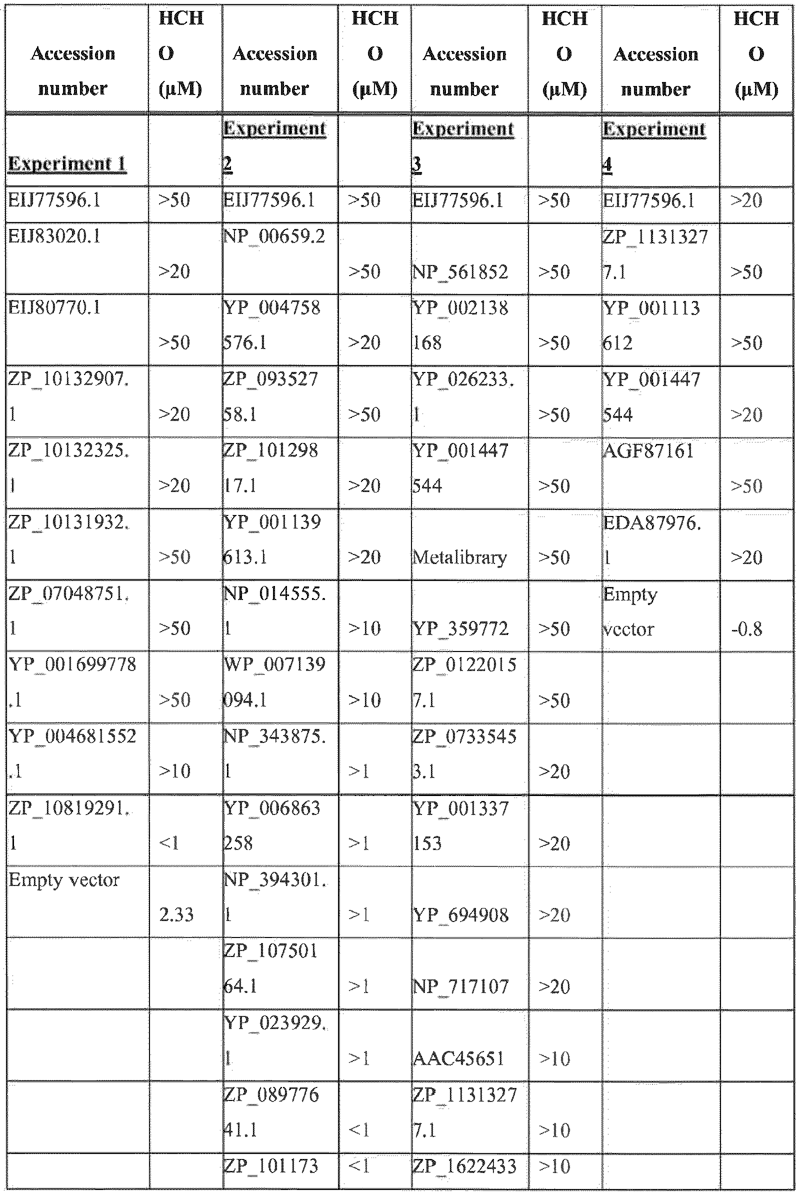

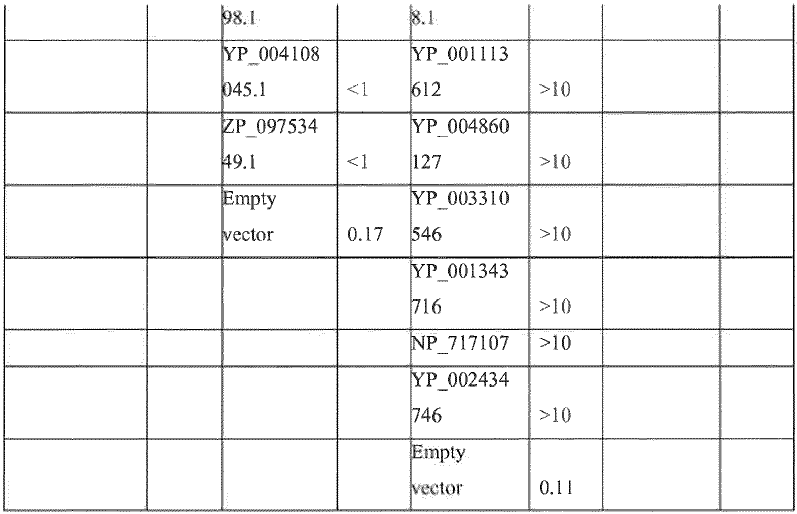

### Formaldehyde Dehydrogenase

Oxidation of formaldehyde to formate is catalyzed by formaldehyde dehydrogenase. Where methanol is used as a carbon source, a host's native formaldehyde dehydrogenase can be a target for elimination or attenuation, particularly when it competes with and reduces formaldehyde assimilation that is shown in Figure 1. An NAD+ dependent formaldehyde dehydrogenase enzyme is encoded by *fdhA* of *Pseudomonas putida* (Ito et al, J Bacteriol 176: 2483-2491 (1994)). Additional formaldehyde dehydrogenase enzymes include the NAD+ and glutathione independent formaldehyde dehydrogenase from *Hyphomicrobium zavarzinii* (Jerome et al, Appl Microbiol Biotechnol 77:779-88 (2007)), the glutathione dependent formaldehyde dehydrogenase *of Pichia pastoris* (Sunga et al, Gene 330:39-47 (2004)) and the NAD(P)+ dependent formaldehyde dehydrogenase of *Methylobacter marinus* (Speer et al, FEMS Microbiol Lett, 121(3):349-55 (1994)).

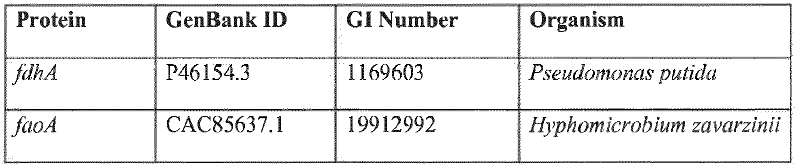

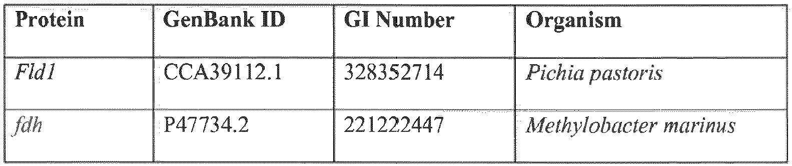

In addition to the formaldehyde dehydrogenase enzymes listed above, alternate enzymes and pathways for converting formaldehyde to formate are known in the art. For example, many organisms employ glutathione-dependent formaldehyde oxidation pathways, in which formaldehyde is converted to formate in three steps via the intermediates S-hydroxymethylglutathione and S-formylglutathione (Vorholt et al, J Bacterial 182:6645-50 (2000)). The enzymes of this pathway are S-(hydroxymethyl)glutathione synthase (EC 4.4.1.22), glutathione-dependent formaldehyde dehydrogenase (EC 1.1.1.284) and S-formylglutathione hydrolase (EC 3.1.2.12).

### Carbon Monoxide Dehydrogenase (CODH)

CODH is a reversible enzyme that interconverts CO and CO₂ at the expense or gain of electrons. The natural physiological role of the CODH in ACS/CODH complexes is to convert CO₂ to CO for incorporation into acetyl-CoA by acetyl-CoA synthase. Nevertheless, such CODH enzymes are suitable for the extraction of reducing equivalents from CO due to the reversible nature of such enzymes. Expressing such CODH enzymes in the absence of ACS allows them to operate in the direction opposite to their natural physiological role (i.e., CO oxidation). In *M. thermoacetica, C. hydrogenoformans, C. carboxidivorans P7,* and several other organisms, additional CODH encoding genes are located outside of the ACS/CODH operons. These enzymes provide a means for extracting electrons (or reducing equivalents) from the conversion of carbon monoxide to carbon dioxide. The *M*. *thermoacetica* gene (Genbank Accession Number: YP_430813) is expressed by itself in an operon and is believed to transfer electrons from CO to an external mediator like ferredoxin in a "Ping-pong" reaction. The reduced mediator then couples to other reduced nicolinamide adenine dinucleotide phosphate (NAD(P)H) carriers or ferredoxin-dependent cellular processes (Ragsdale, Annals of the New York Academy of Sciences 1125: 129-136 (2008)). The genes encoding the C. *hydrogenoformans* CODH-II and CooF, a neighboring protein, were cloned and sequenced (Gonzalez and Robb, FEMS Microbiol Lett. 191:243-247 (2000)). The resulting complex was membrane-bound, although cytoplasmic fractions of CODH-II were shown to catalyze the formation of NADPH suggesting an anabolic role (Svctlitchnyi et al., J Bacteriol. 183:5134-5144 (2001)). The crystal structure of the CODH-II is also available (Dobbek et al., Science 293:1281-1285 (2001)). Similar ACS-free CODH enzymes can be found in a diverse array of organisms including *Geobacter metallireducens* GS-15, *Chlorobium phaeobacteroides* DSM 266, *Clostridium cellulolyticum* H10, *Desulfovibrio desulfuricans* subsp. *desulfuricans* str. ATCC 27774, *Pelobacter carbinolicus* DSM 2380, C. *ljungdahli* and *Campylobacter curvus* 525.92.

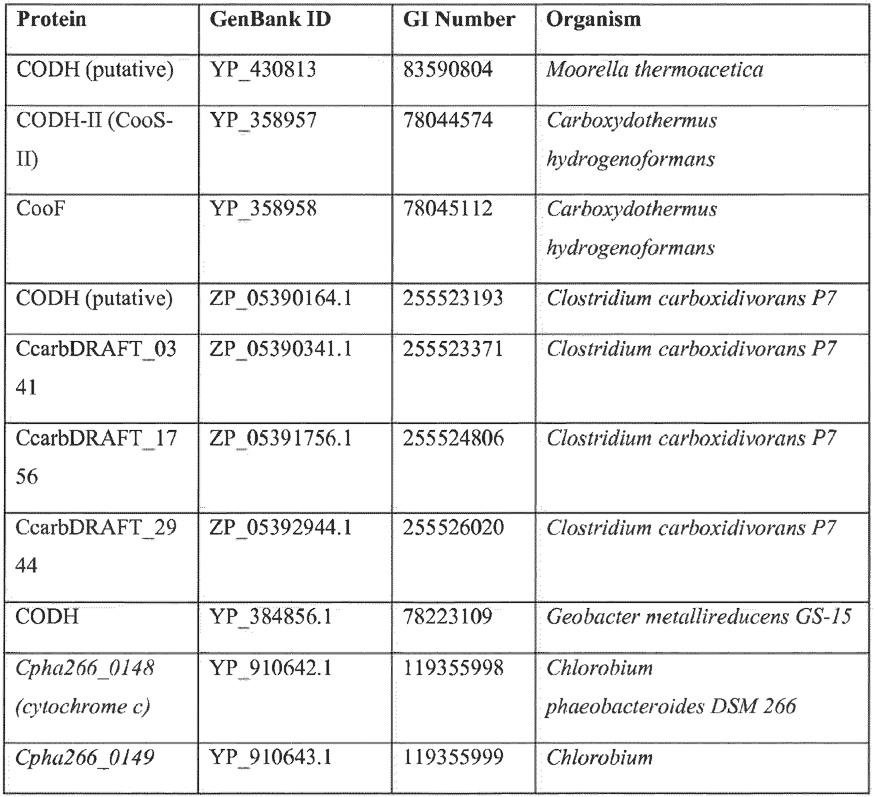

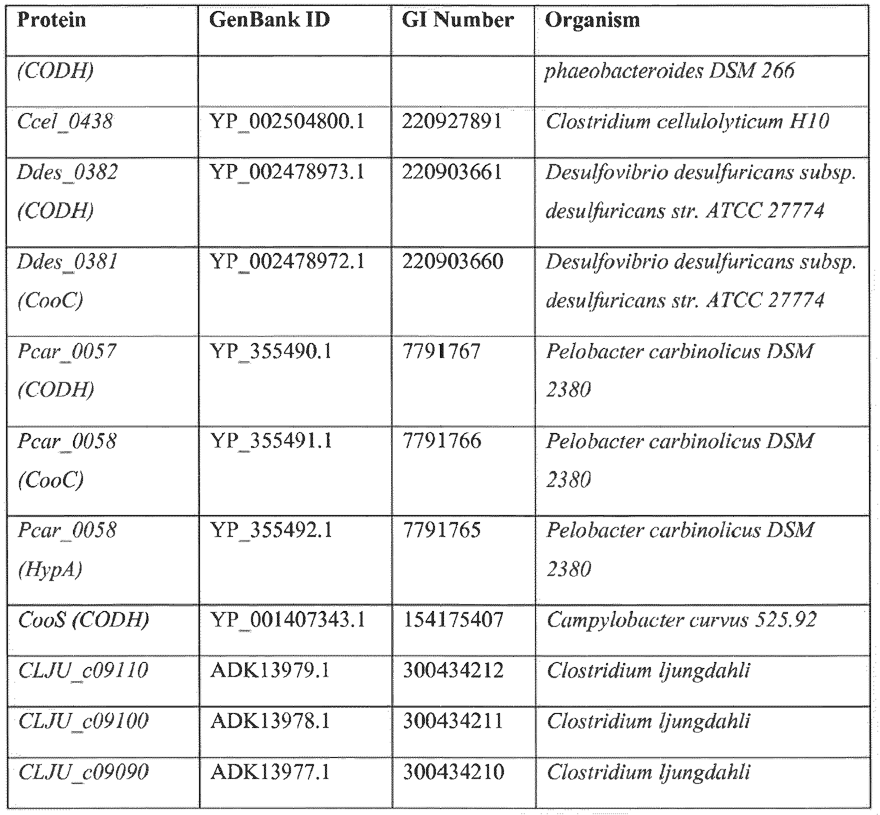

### EXAMPLE VI

### Methods for Formaldehyde Fixation (or Assimilation)

Provided herein are exemplary pathways, which utilize formaldehyde, for example produced from the oxidation of methanol (see, *e.g*., Figure 1, step A) or from formate assimilation, in the formation of intermediates of certain central metabolic pathways that can be used for the production of compounds disclosed herein.

One exemplary pathway that can utilize formaldehyde produced from the oxidation of methanol is shown in Figure 1, which involves condensation of formaldehyde and D-ribulosc-5-phosphatc to form hcxulosc-6-phosphatc (h6p) by hexulose-6-phosphate synthase (Figure 1, step B). The enzyme can use Mg²⁺ or Mn²⁺ for maximal activity, although other metal ions are useful, and even non-metal-ion-dependent mechanisms are contemplated. H6p is converted into fructose-6-phosphate by 6-phospho-3-hexuloisomerase (Figure 1, step C).

Another exemplary pathway that involves the detoxification and assimilation of formaldehyde produced from the oxidation of methanol is shown in Figure 1 and proceeds through dihydroxyacetone. Dihydroxyacetone synthase is a special transketolase that first transfers a glycoaldehyde group from xylulose-5-phosphate to formaldehyde, resulting in the formation of dihydroxyacetone (DHA) and glyccraldchydc-3-phosphate (G3P), which is an intermediate in glycolysis (Figure 1). The DHA obtained from DHA synthase can be further phosphorylated to form DHA phosphate and assimilated into glycolysis and several other pathways (Figure 1). Alternatively, or in addition, a fructose-6-phosphate aldolase can be used to catalyze the conversion of DHA and G3P to fructose-6-phosphate (Figure 1, step Z).

Figure 1, Steps B and C - Hexulose-6-phosphate synthase (Step B) and 6-phospho-3-hexuloisomerase (Step C)

Both of the hexulose-6-phosphate synthase and 6-phospho-3-hcxuloisomcrasc enzymes are found in several organisms, including methanotrophs and methylotrophs where they have been purified (Kato et al., 2006, BioSci Biotechnol Biochem. 70(1): 10-21. In addition, these enzymes have been reported in heterotrophs such as *Bacillus subtilis* also where they are reported to be involved in formaldehyde detoxification (Mitsui et al., 2003, AEM 69(10):6128-32, Yasucda ct al., 1999. J Bac 181(23):7154-60. Genes for these two enzymes from the methylotrophic bacterium *Mycobacterium gastri* MB19 have been fused and *E. coli* strains harboring the hps-phi construct showed more efficient utilization of formaldehyde (Orita et al., 2007, Appl Microbiol Biotechnol. 76:439-445). In some organisms, these two enzymes naturally exist as a fused version that is bifunctional.

Exemplary candidate genes for hexulose-6-phopshate synthase are:

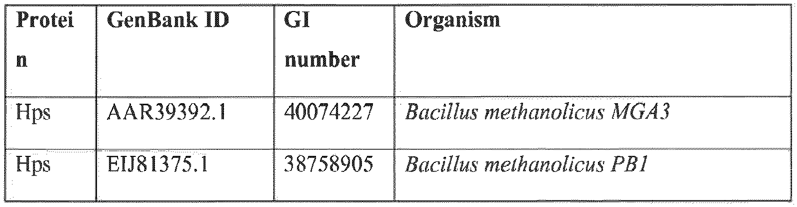

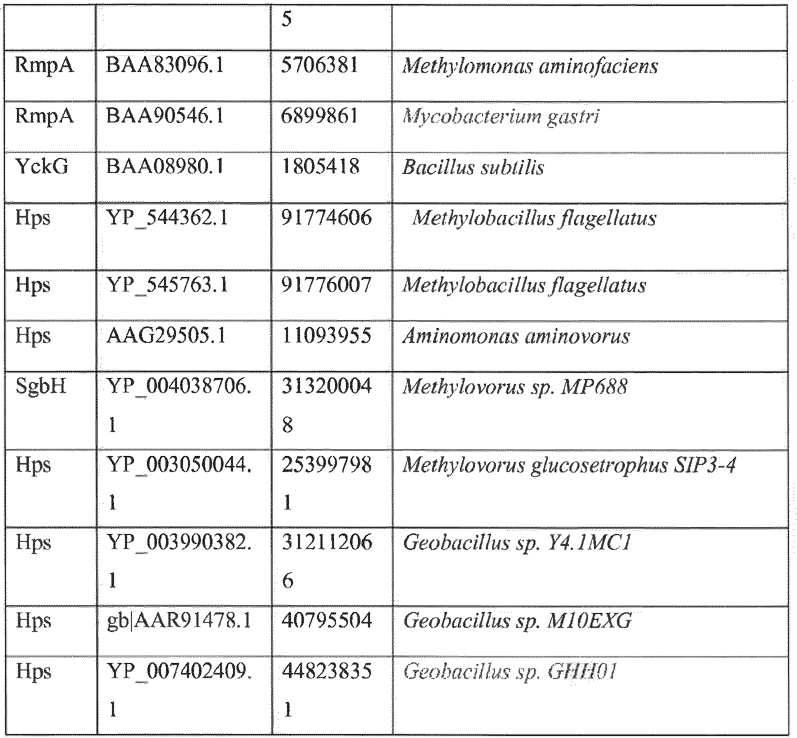

Exemplary gene candidates for 6-phospho-3-hexuloisomerase are:

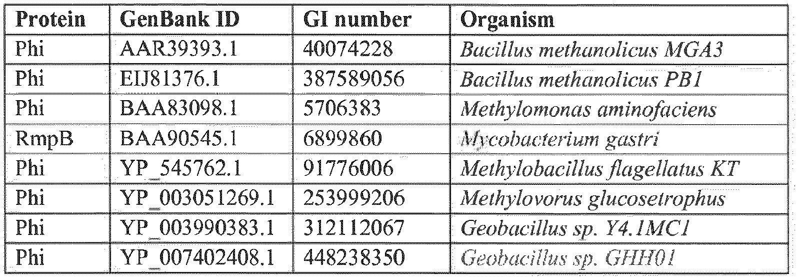

Candidates for enzymes where both of these functions have been fused into a single open reading frame include the following.

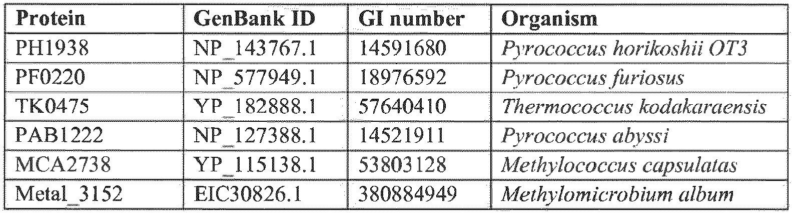

### Figure 1, Step D - Dihydroxyacetone synthase

The dihydroxyacetone synthase enzyme in *Candida boidinii* uses thiamine pyrophosphate and Mg²⁺ as cofactors and is localized in the peroxisome. The enzyme from the methanol-growing carboxydobacterium, *Mycobacter* sp. strain JC1 DSM 3803, was also found to have DHA synthase and kinase activities (Ro et al., 1997, JBac 179(19):6041-7). DHA synthase from this organism also has similar cofactor requirements as the enzyme from C. *boidinii.* The Kₘs for formaldehyde and xylulose 5-phosphate were reported to be 1.86 mM and 33.3 microM, respectively. Several other mycobacteria, excluding only *Mycobacterium tuberculosis,* can use methanol as the sole source of carbon and energy and are reported to use dihydroxyacetone synthase (Part et al., 2003, JBac 185(1):142-7.

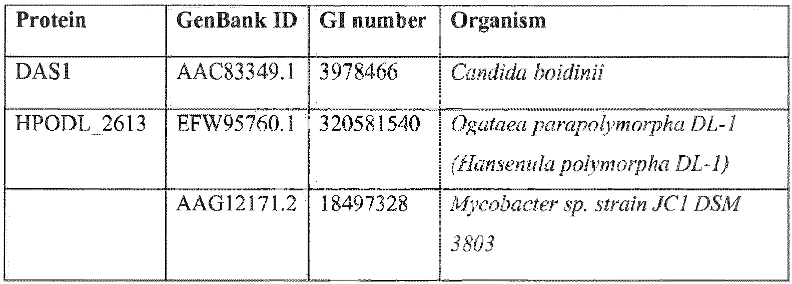

### Figure 1, Step Z - Fructose-6-phosphate aldolase

Fructose-6-phosphate aldolase (F6P aldolase) can catalyze the combination of dihydroxyacetone (DHA) and glyceraldehyde-3-phosphate (G3P) to form fructose-6-phosphate. This activity was recently discovered in *E. coli* and the corresponding gene candidate has been termed *fsa* (Schurmann and Sprenger, J. Biol. Chem., 2001, 276(14), 11055-11061). The enzyme has narrow substrate specificity and cannot utilize fructose, fructose 1-phosphate, fructose 1,6-bisphosphate, or dihydroxyacetone phosphate. It can however use hydroxybutanonc and acetol instead of DHA. The purified enzyme displayed a Vₘₐₓ of 7 units/mg of protein for fructose 6-phosphate cleavage (at 30 degrees C, pH 8.5 in 50 mm glycylglycine buffer). For the aldolization reaction a Vₘₐₓ of 45 units/mg of protein was found; Kₘ values for the substrates were 9 mM for fructose 6-phosphate, 35 mM for dihydroxyacetone, and 0.8 mM for glyceraldehyde 3-phosphatc. The enzyme prefers the aldol formation over the cleavage reaction.

The selectivity of the E. coli enzyme towards DHA can be improved by introducing point mutations. For example, the mutation A129S improved reactivity towards DHA by over 17 fold in terms of Kcat/Km (Gutierrez et al., Chem Commun (Camb), 2011, 47(20), 5762-5764). The same mutation reduced the catalytic efficiency on hydroxyacetone by more than 3 fold and reduced the affinity for glycoaldehyde by more than 3 fold compared to that of the wild type enzyme (Castillo et al., Advanced Synthesis & Catalysis, 352(6), 1039-1046). Genes similar to fsa have been found in other genomes by sequence homology. Some exemplary gene candidates have been listed below.

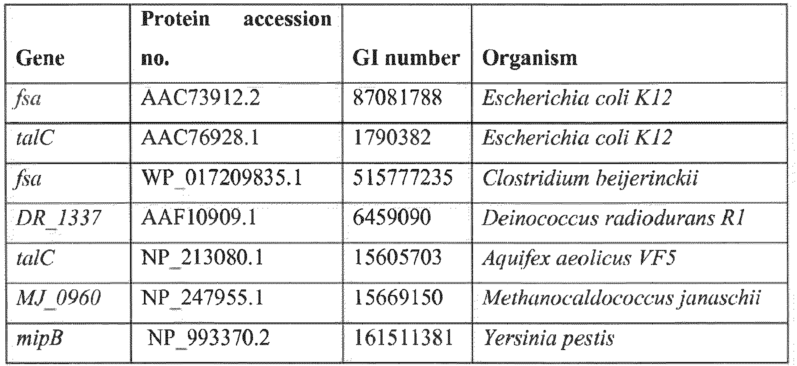

As described below, there is an energetic advantage to using F6P aldolase in the DHA pathway. The assimilation of formaldehyde formed by the oxidation of methanol can proceed either via the dihydroxyacetone (DHA) pathway (step D, Figure 1) or the Ribulose monophosphate (RuMP) pathway (steps B and C, Figure 1). In the RuMP pathway, formaldehyde combines with ribulose-5-phosphate to form F6P. F6P is then either metabolized via glycolysis or used for regeneration of ribulose-5-phosphate to enable further formaldehyde assimilation. Notably, ATP hydrolysis is not required to form F6P from formaldehyde and ribulosc-5-phosphatc via the RuMP pathway.

In contrast, in the DHA pathway, formaldehyde combines with xylulose-5-phosphate (X5P) to form dihydroxyacetone (DHA) and glyceraldehyde-3-phosphate (G3P). Some of the DHA and G3P must be metabolized to F6P to enable regeneration of xylulose-5-phosphate. In the standard DHA pathway, DHA and G3P are converted to F6P by three enzymes: DHA kinase, fructose bisphosphate aldolase, and fructose bisphosphatase. The net conversion of DHA and G3P to F6P requires ATP hydrolysis as described below. First, DHA is phosphorylated to form DHA phosphate (DHAP) by DHA kinase at the expense of an ATP. DHAP and G3P are then combined by fructose bisphosphate aldolase to form fructose-1,6-diphosphate (FDP). FDP is converted to F6P by fructose bisphosphatase, thus wasting a high energy phosphate bond.

A more ATP efficient sequence of reactions is enabled if DHA synthase functions in combination with F6P aldolase as opposed to in combination with DHA kinase, fructose bisphosphate aldolase, and fructose bisphosphatase. F6P aldolase enables direct conversion of DHA and G3P to F6P, bypassing the need for ATP hydrolysis. Overall, DHA synthase when combined with F6P aldolase is identical in energy demand to the RuMP pathway. Both of these formaldehyde assimilation options (i.e., RuMP pathway, DHA synthase + F6P aldolase) are superior to DHA synthase combined with DHA kinase, fructose bisphosphate aldolase, and fructose bisphosphatase in terms of ATP demand.

### EXAMPLE VII

### In Vivo Labeling Assay for Conversion of Methanol to CO₂

This example describes a functional methanol pathway in a microbial organism.

Strains with functional reductive TCA branch and pyruvate formate lyase deletion were grown aerobically in LB medium overnight, followed by inoculation of M9 high-seed media containing IPTG and aerobic growth for 4 hrs. These strains had methanol dehydrogenase/ACT pairs in the presence and absence of formaldehyde dehydrogenase or formate dehydrogenase. ACT is an activator protein (a Nudix hydrolase). At this time, strains were pelleted, resuspended in fresh M9 medium high-seed media containing 2% ¹³CH₃OH, and sealed in anaerobic vials. Head space was replaced with nitrogen and strains grown for 40 hours at 37°C. Following growth, headspace was analyzed for ¹³CO₂. Media was examined for residual methanol as well as 1,4-butanediol and byproducts. All constructs expressing methanol dehydrogenase (McDH) mutants and McDH/ACT pairs grew to slightly lower ODs than strains containing empty vector controls. This is likely due to the high expression of these constructs (Data not shown). One construct (2315/2317) displayed significant accumulation of labeled CO₂ relative to controls in the presence of FalDH, FDH or no coexpressed protein. This shows a functional MeOH pathway in *E. coli* and that the endogenous glutathione-dependent formaldehyde detoxification genes (frmAB) are sufficient to carry flux generated by the current MeDH/ACT constructs.

2315 is internal laboratory designation for the McDH from *Bacillus methanolicus* MGA3 (GenBank Accession number: EIJ77596.1; GI number: 387585261), and 2317 is internal laboratory designation for the activator protein from the same organism (locus tag: MGA3_09170; GenBank Accession number:EIJ83380; GI number: 387591061).

Sequence analysis of the NADH-dependent MeDH from *Bacillus methanolicus* places the enzyme in the alcohol dehydrogenase family III. It does not contain any tryptophan residues, resulting in a low extinction coefficient (18,500 M^{- 1}, cm⁻¹) and should be detected on SDS gels by Coomassie staining.

The enzyme has been characterized as a multisubunit complex built from 43 kDa subunits containing one Zn and 1-2 Mg atoms per subunit. Electron microscopy and sedimentation studies determined it to be a decamer, in which two rings with five-fold symmetry are stacked on top of each other (Vonck et al., J. Biol. Chem. 266:3949-3954, 1991). It is described to contain a tightly but not covalently bound cofactor and requires exogenous NAD⁺ as e⁻-acceptor to measure activity *in vitro.* A strong increase (10-40-fold) *of in vitro* activity was observed in the presence of an activator protein (ACT), which is a homodimer (21 kDa subunits) and contains one Zn and one Mg atom per subunit.

The mechanism of the activation was investigated by Kloosterman et al., J. Biol. Chem. 277:34785-34792, 2002, showing that ACT is a Nudix hydrolase and Hektor et al., J. Biol. Chem. 277:46966-46973, 2002, demonstrating that mutation of residue S97 to G or T in MeDH changes activation characteristics along with the affinity for the cofactor. While mutation of residues G15 and D88 had no significant impact, a role of residue G13 for stability as well as of residues G95, D100, and K103 for the activity is suggested. Both papers together propose a hypothesis in which ACT cleaves MeDH-bound NAD⁺. MeDH retains AMP bound and enters an activated cycle with increased turnover.

The stoichiometric ratio between ACT and MeDH is not well defined in the literature. Kloosterman *et al., supra* determine the ratio of dimeric Act to dccamcric MeDH for full *in vitro* activation to be 10:1. In contrast, Arfman et al. J. Biol. Chem. 266:3955-3960, 1991 determined a ratio of 3:1 *in vitro* for maximum and a 1:6 ratio for significant activation, but observe a high sensitivity to dilution. Based on expression of both proteins in *Bacillus,* the authors estimate the ratio *in vivo* to be around 1:17.5.

However, our *in vitro* experiments with purified activator protein (2317A) and methanol dehydrogenase (2315A) showed the ratio of ACT to MeDH to be 10:1. This *in vitro* test was done with 5 M methanol, 2 mM NAD and 10 µM methanol dehydrogenase 2315A at pH 7.4.

### EXAMPLE VIII

### Phosphoketolase-dependent Acetyl-CoA Synthesis Enzymes

This Example provides genes that can be used for enhancing carbon flux through acetyl-CoA using phosphoketolase enzymes.

### Figure 1, Step T - Fructose-6-phosphate phosphoketolase

Conversion of fructose-6-phosphate and phosphate to acetyl-phosphate and erythrose-5-phosphate can be carried out by fructose-6-phosphate phosphoketolase (EC 4.1.2.22). Conversion of fructose-6-phosphate and phosphate to acetyl-phosphate and erythrose-5-phosphate is one of the key reactions in the *Bifidobacterium* shunt. There is evidence for the existence of two distinct phosphoketolase enzymes in *bifidobacteria* (Sgorbati et al, 1976, Antonie Van Leeuwenhoek, 42(1-2) 49-57; Grill et al, 1995, Curr Microbiol, 31(1);49-54). The enzyme from *Bifidobacterium dentium* appeared to be specific solely for fructose-6-phosphate (EC: 4.1.2.22) while the enzyme from *Bifidobacterium pseudolongum* subsp. *globosum* is able to utilize both fructose-6-phosphate and D-xylulose 5-phosphate (EC: 4.1.2.9) (Sgorbati et al, 1976, Antonie Van Leeuwenhoek, 42(1-2) 49-57). The enzyme encoded by the xfp gene, originally discovered in *Bifidobacterium animalis lactis,* is the dual-specificity enzyme (Mcilc et al., 2001, J Bacteriol, 183, 2929-2936; Yin et al, 2005, FEMS Microbiol Lett, 246(2); 251-257). Additional phosphoketolase enzymes can be found in *Leuconostoc mesenteroides* (Lee et al, Biotechnol Lett. 2005 Jun;27(12):853-8), *Clostridium acetobutylicum* ATCC 824 (Servinsky et al, Journal of Industrial Microbiology & Biotechnology, 2012, 39, 1859-1867), *Aspergillus nidulans* (Kocharin et al, 2013, Biotechnol Bioeng, 110(8), 2216-2224; Papini, 2012, Appl Microbiol Biotechnol, 95 (4), 1001-1010), *Bifidobacterium breve* (Suziki et al, 2010, Acta Crystallogr Sect F Struct Biol Cryst Commun., 66(Pt 8):941-3), *Lactobacillus paraplantarum* (Jeong et al, 2007, J Microbiol Biotechnol, 17(5), 822-9).

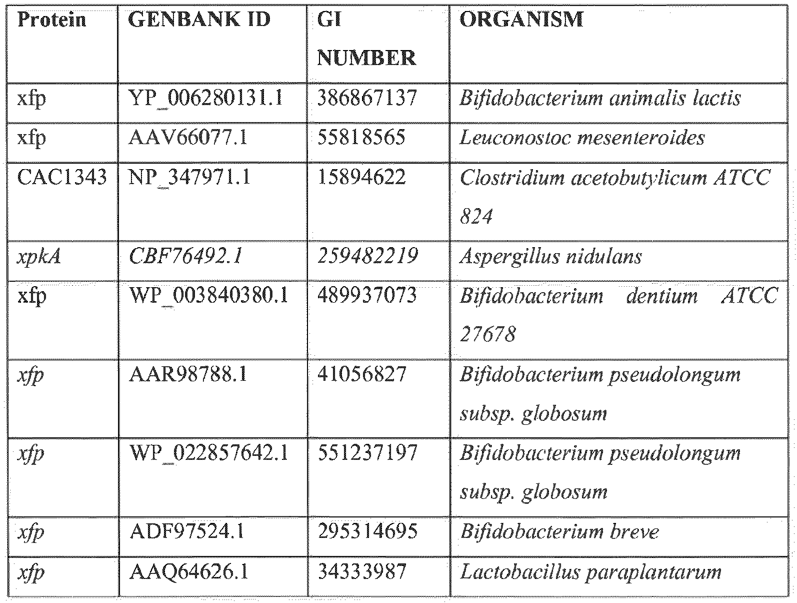

### Figure 1, Step U - Xylulose-5-phospate phosphoketolase

Conversion of xylulose-5-phosphate and phosphate to acetyl-phosphate and glyceraldehyde-3-phosphate can be carried out by xylulose-5-phosphate phosphoketolase (EC 4.1.2.9). There is evidence for the existence of two distinct phosphoketolase enzymes in *bifidobacteria* (Sgorbati et al, 1976, Antonie Van Leeuwenhoek, 42(1-2) 49-57; Grill et al, 1995, Curr Microbiol, 31(1);49-54). The enzyme from *Bifidobacterium dentium* appeared to be specific solely for fructose-6-phosphate (EC: 4.1.2.22) while the enzyme from *Bifidobacterium pseudolongum* subsp. *globosum* is able to utilize both fructose-6-phosphate and D-xylulose 5-phosphate (EC: 4.1.2.9) (Sgorbati et al, 1976, Antonie Van Leeuwenhoek, 42(1-2) 49-57). Many characterized enzymes have dual-specificity for xylulose-5-phosphate and fructose-6-phosphate. The enzyme encoded by the xfp gene, originally discovered in *Bifidobacterium animalis lactis,* is the dual-specificity enzyme (Meile et al., 2001, J Bacteriol, 183, 2929-2936; Yin et al, 2005, FEMS Microbiol Lett, 246(2); 251-257). Additional phosphoketolase enzymes can be found in *Leuconostoc mesenteroides* (Lee et al, Biotechnol Lett. 2005 Jun;27(12):853-8), *Clostridium acetobutylicum* ATCC 824 (Servinsky et al, Journal of Industrial Microbiology & Biotechnology, 2012, 39, 1859-1867), *Aspergillus nidulans* (Kocharin et al, 2013, Biotechnol Bioeng, 110(8), 2216-2224; Papini, 2012, Appl Microbiol Biotechnol, 95 (4), 1001-1010), *Bifidobacterium breve* (Suziki et al, 2010, Acta Crystallogr Sect F Struct Biol Cryst Commun., 66(Pt 8):941-3), and *Lactobacillus paraplantarum* (Jeong et al, 2007, J Microbiol Biotechnol, 17(5), 822-9).

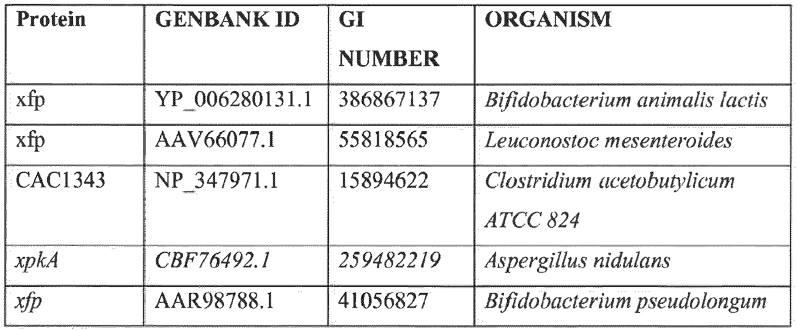

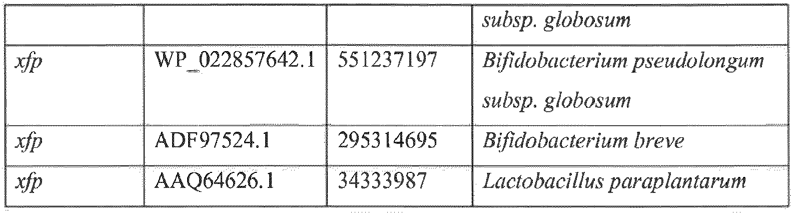

### Figure 1, Step 1' - Phosphotransacetylase

The formation of acetyl-CoA from acetyl-phosphate can be catalyzed by phosphotransacetylase (EC 2.3.1.8) . The pta gene from E. coli encodes an enzyme that reversibly converts acetyl-CoA into acetyl-phosphate (Suzuki, T., Biochim. Biophys. Acta 191:559-569 (969)). Additional acetyltransferase enzymes have been characterized in Bacillus subtilis (Rado and Hoch, Biochim. Biophys. Acta 321:114-125 (1973), Clostridium kluyveri (Stadtman, E., Methods Enzymol. 1:5896-599 (1955), and Thermotoga maritima (Bock et al., J. Bacteriol. 181:1861-1867 (1999)). This reaction can also be catalyzed by some phosphotransbutyrylase enzymes (EC 2.3.1.19), including the ptb gene products from Clostridium acetobutylicum (Wiesenborn et al., App. Environ. Microbiol. 55:317-322 (1989); Walter et al., Gene 134:107-111 (1993)). Additional ptb genes are found in butyrate-producing bacterium L2-50 (Louis et al., J. Bacteriol. 186:2099-2106 (2004) and Bacillus megaterium (Vazquez et al., Curr. Microbiol. 42:345-349 (2001). Homologs to the E. coli pta gene exist in several other organisms including Salmonella enterica and Chlamydomonas reinhardtii.

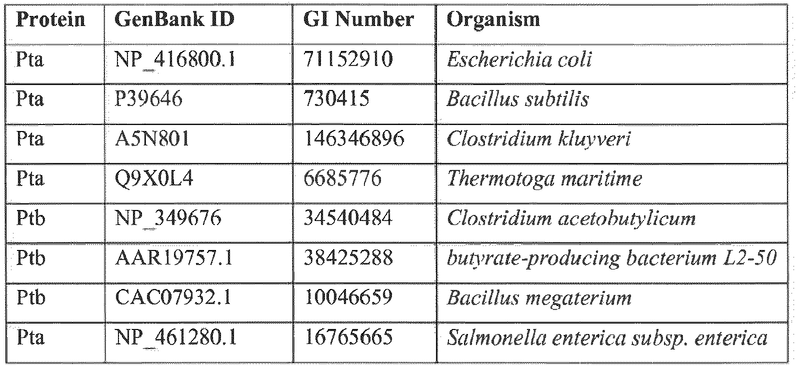

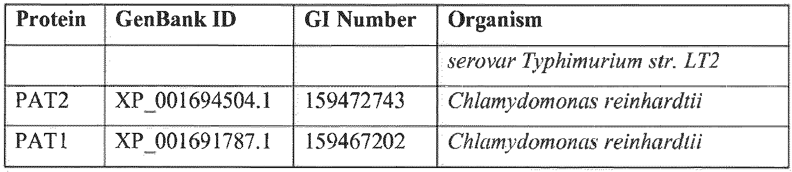

### Figure 1, Sten W - Acetate kinase

Acetate kinase (EC 2.7.2.1) can catalyze the reversible ATP-dependent phosphorylation of acetate to acetylphosphate. Exemplary acetate kinase enzymes have been characterized in many organisms including E. coli, Clostridium acetobutylicum and Methanosarcina thermophila (Ingram-Smith et al., J. Bacteriol. 187:2386-2394 (2005); Fox and Roseman, J. Biol. Chem. 261:13487-13497 (1986); Winzer et al., Microbioloy 143 (Pt 10):3279-3286 (1997)). Acetate kinase activity has also been demonstrated in the gene product of E. coli purT (Marolewski et al., Biochemistry 33:2531-2537 (1994). Some butyrate kinase enzymes (EC 2.7.2.7), for example buk1 and buk2 from Clostridium acetobutylicum, also accept acetate as a substrate (Hartmanis, M.G., J. Biol. Chem. 262:617-621 (1987)). Homologs exist in several other organisms including Salmonella enterica and Chlamydomonas reinhardtii.

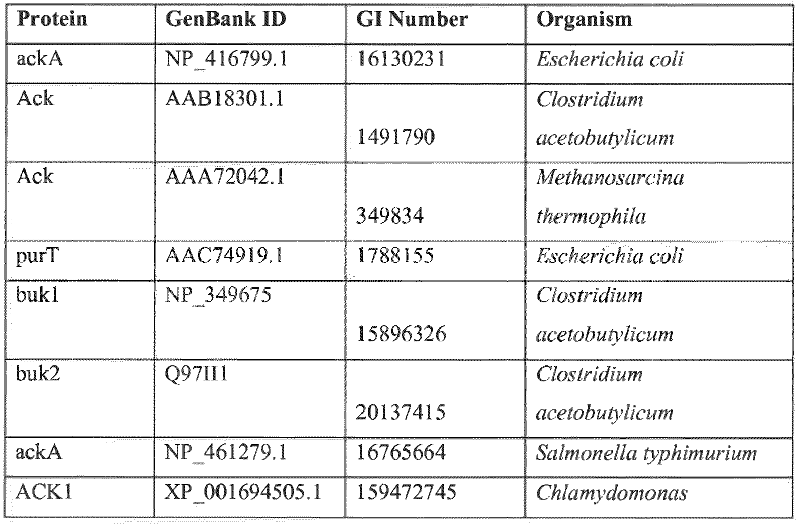

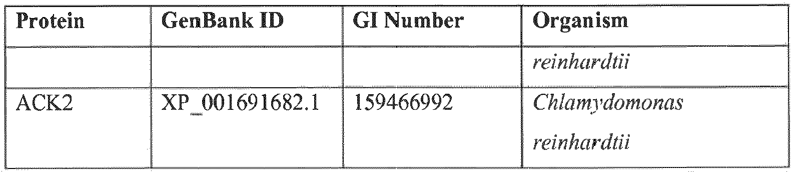

### Figure 1, Step X - Acetyl-CoA transferase, synthetase, or ligase

The acylation of acetate to acetyl-CoA can be catalyzed by enzymes with acetyl-CoA synthetase, ligase or transferase activity. Two enzymes that can catalyze this reaction are AMP-forming acetyl-CoA synthetase or ligase (EC 6.2.1.1) and ADP-forming acetyl-CoA synthetase (EC 6.2.1.13). AMP-forming acetyl-CoA synthetase (ACS) is the predominant enzyme for activation of acetate to acetyl-CoA. Exemplary ACS enzymes are found in E. coli (Brown et al., J. Gen. Microbiol. 102:327-336 (1977)), Ralstonia eutropha (Priefert and Steinbuchel, J. Bacteriol. 174:6590-6599 (1992)), Methanothermobacter thermautotrophicus (Ingram-Smith and Smith, Archaea 2:95-107 (2007)), Salmonella enterica (Gulick et al., Biochemistry 42:2866-2873 (2003)) and Saccharomyces cerevisiae (Jogl and Tong, Biochemistry 43:1425-1431 (2004)). ADP-forming acetyl-CoA synthetases are reversible enzymes with a generally broad substrate range (Musfeldt and Schonheit, J. Bacteriol. 184:636-644 (2002)). Two isozymes of ADP-forming acetyl-CoA synthetases are encoded in the Archaeoglobus fulgidus genome by are encoded by AF1211 and AF1983 (Musfeldt and Schonheit, supra (2002)). The enzyme from Haloarcula marismortui (annotated as a succinyl-CoA synthetase) also accepts acetate as a substrate and reversibility of the enzyme was demonstrated (Brasen and Schonheit, Arch. Microbiol. 182:277-287 (2004)). The ACD encoded by PAE3250 from hyperthermophilic crenarchaeon Pyrobaculum aerophilum showed the broadest substrate range of all characterized ACDs, reacting with acetate, isobutyryl-CoA (preferred substrate) and phcnylacctyl-CoA (Brasen and Schonheit, supra (2004)). Directed evolution or engineering can be used to modify this enzyme to operate at the physiological temperature of the host organism. The enzymes from A. fulgidus, H. marismortui and P. aerophilum have all been cloned, functionally expressed, and characterized in E. coli (Brasen and Schonheit, supra (2004); Musfeldt and Schonheit, supra (2002)). Additional candidates include the succinyl-CoA synthetase encoded by sucCD in E. coli (Buck et al., Biochemistry 24:6245-6252 (1985)) and the acyl-CoA ligase from Pseudomonas putida (Fernandez-Valverde et al., Appl. Environ. Microbiol. 59:1149-1154 (1993)). The aforementioned proteins are shown below.

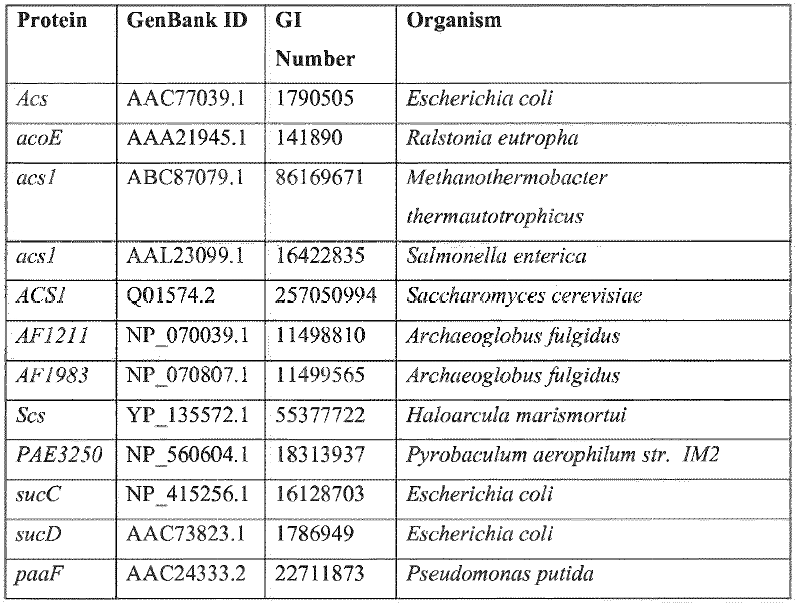

An acetyl-CoA transferase that can utilize acetate as the CoA acceptor is acetoacetyl-CoA transferase, encoded by the *E. coli atoA* (alpha subunit) and *atoD* (beta subunit) genes (Vanderwinkel et al., Biochem.Biophys.Res Commun. 33:902-908 (1968); Korolev et al., Acta Crystallogr.D Biol Crystallogr. 58:2116-2121 (2002)). This enzyme has also been shown to transfer the CoA moiety to acetate from a variety of branched and linear acyl-CoA substrates, including isobutyrate (Matthies et al., Appl Environ Microbiol 58:1435-1439 (1992)), valerate (Vanderwinkel et al., *supra*) and butanoate (Vandcrwinkcl et al., *supra).* Similar enzymes exist in *Corynebacterium glutamicum* ATCC 13032 (Duncan et al., Appl Environ Microbiol 68:5186-5190 (2002)), *Clostridium acetobutylicum* (Cary et al., Appl Environ Microbiol 56:1576-1583 (1990)), and *Clostridium saccharoperbutylacetonicum* (Kosaka et al., Biosci.Biotechnol Biochem. 71:58-68 (2007)). These proteins are identified below.

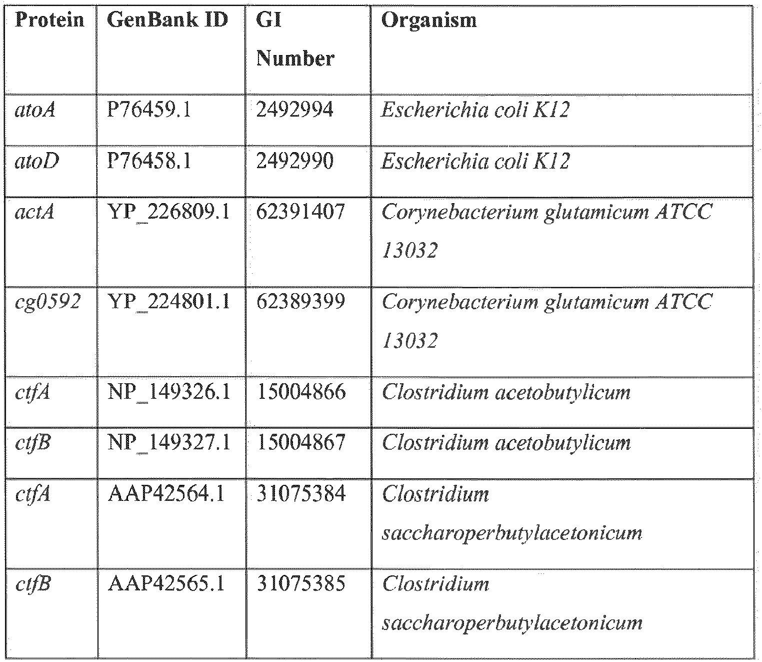

Additional exemplary acetyl-CoA transferase candidates are catalyzed by the gene products of *cat1, cat2,* and *cat3* of *Clostridium kluyveri* which have been shown to exhibit succinyl-CoA, 4-hydroxybutyryl-CoA, and butyryl-CoA transferase activity, respectively (Seedorf et al., *supra;* Sohling et al., Eur.J Biochem. 212:121-127 (1993); Sohling et al., J Bacteriol. 178:871-880 (1996)). Similar CoA transferase activities arc also present in *Trichomonas vaginalis* (van Grinsven et al., J.Biol.Chem. 283:1411-1418 (2008)) and *Trypanosoma brucei* (Riviere et al., J.Biol.Chem. 279:45337-45346 (2004)). These proteins are identified below.

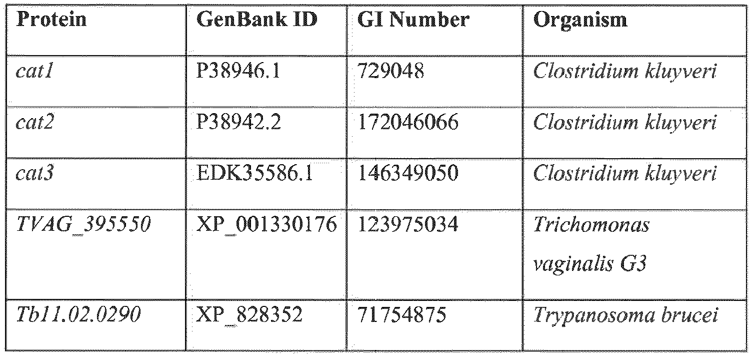

### EXAMPLE IX

### Acetyl-CoA, oxaloacetate and Succinyl-CoA Synthesis Enzymes

This Example provides genes that can be used for conversion of glycolysis intermediate glyceraldehyde-3-phosphate (G3P) to acetyl-CoA and/or succinyl-CoA as depicted in the pathways of Figure 4.

### A. PEP Carboxylase or PEP Carboxykinase.

Carboxylation of phosphoenolpyruvate to oxaloacetate is catalyzed by phosphoenolpyruvate carboxylase. Exemplary PEP carboxylase enzymes are encoded by *ppc* in *E. coli* (Kai et al., Arch. Biochem. Biophys. 414:170-179 (2003), *ppcA* in *Methylobacterium extorquens AM1* (Arps et al., J. Bacteriol. 175:3776-3783 (1993), and *ppc* in *Corynebacterium glutamicum* (Eikmanns et al., Mol. Gen. Genet. 218:330-339 (1989).

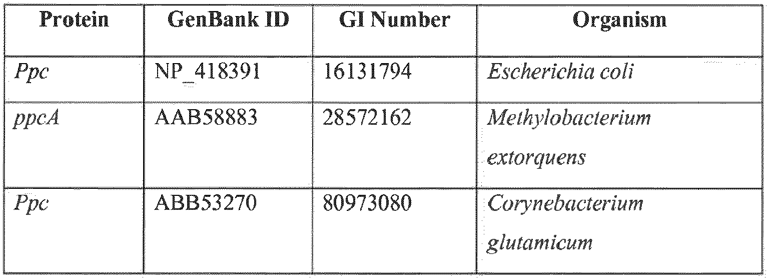

An alternative enzyme for converting phosphoenolpyruvate to oxaloacetate is PEP carboxykinase, which simultaneously forms an ATP while carboxylating PEP. In most organisms PEP carboxykinase serves a gluconeogenic function and converts oxaloacetate to PEP at the expense of one ATP. S. *cerevisiae* is one such organism whose native PEP carboxykinase, *PCK1,* serves a gluconeogenic role (Valdes-Hevia et al., FEBS Lett. 258:313-316 (1989). *E. coli* is another such organism, as the role of PEP carboxykinase in producing oxaloacetate is believed to be minor when compared to PEP carboxylase, which does not form ATP, possibly due to the higher Kₘ for bicarbonate of PEP carboxykinase (Kim et al., Appl. Environ. Microbiol. 70:1238-1241 (2004)). Nevertheless, activity of the native E. *coli* PEP carboxykinase from PEP towards oxaloacetate has been recently demonstrated in *ppc* mutants of *E. coli* K-12 (Kwon et al., J. Microbiol. Biotechnol. 16:1448-1452 (2006)). These strains exhibited no growth defects and had increased succinate production at high NaHCO₃ concentrations. Mutant strains of *E. coli* can adopt Pck as the dominant CO2-fixing enzyme following adaptive evolution (Zhang et al. 2009). In some organisms, particularly rumen bacteria, PEP carboxykinase is quite efficient in producing oxaloacetate from PEP and generating ATP. Examples of PEP carboxykinase genes that have been cloned into *E. co*li include those from *Mannheimia succiniciproducens* (Lee et al., Biotechnol. Bioprocess Eng. 7:95-99 (2002)), *Anaerobiospirillum succiniciproducens* (Laivcnicks et al., Appl. Environ. Microbiol. 63:2273-2280 (1997), and *Actinobacillus succinogenes* (Kim et al. *supra).* The PEP carboxykinase enzyme encoded by *Haemophilus influenza* is effective at forming oxaloacetate from PEP.

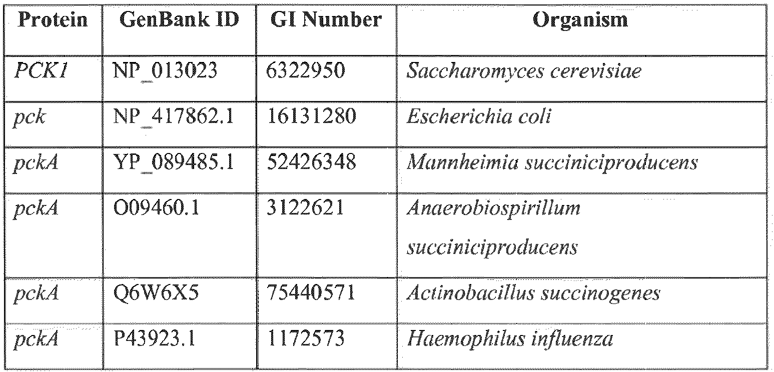

### B. Malate Dehydrogenase.

Oxaloacetate is converted into malate by malate dehydrogenase (EC 1.1.1.37), an enzyme which functions in both the forward and reverse direction. *S. cerevisiae* possesses three copies of malate dehydrogenase, *MDH1* (McAlister-Henn and Thompson, J. Bacteriol. 169:5157-5166 (1987), *MDH2* (Minard and McAlister-Henn, Mol. Cell. Biol. 11:370-380 (1991); Gibson and McAlister-Henn, J. Biol. Chem. 278:25628-25636 (2003)), and *MDH3* (Steffan and McAlister-Henn, J. Biol. Chem. 267:24708-24715 (1992)), which localize to the mitochondrion, cytosol, and peroxisome, respectively. *E. coli* is known to have an active malate dehydrogenase encoded by *mdh.*

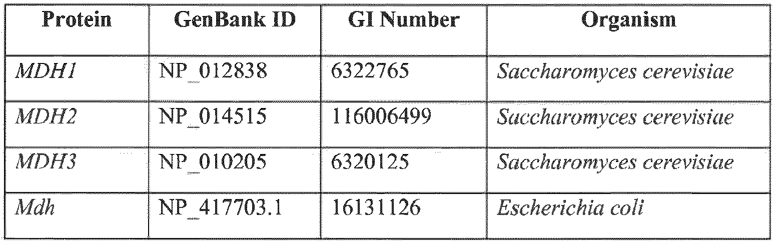

### C. Fumarase.

Fumarate hydratase (EC 4.2.1.2) catalyzes the reversible hydration of fumarate to malate. The three fumarases of *E. coli,* encoded by *fumA, fumB* and *fumC,* are regulated under different conditions of oxygen availability. FumB is oxygen sensitive and is active under anaerobic conditions. FumA is active under microanaerobic conditions, and FumC is active under aerobic growth conditions (Tseng ct al., J. Bacteriol. 183:461-467 (2001);Woods et al., Biochim. Biophys. Acta 954:14-26 (1988); Guest et al., J. Gen. Microbiol. 131:2971-2984 (1985)). S. *cerevisiae* contains one copy of a fumarase-encoding gene, *FUM1,* whose product localizes to both the cytosol and mitochondrion (Sass et al., J. Biol. Chem. 278:45109-45116 (2003)). Additional fumarase enzymes arc found in *Campylobacter jejuni* (Smith et al., Int. J. Biochem. Cell. Biol. 31:961-975 (1999)), *Thermus thermophilus* (Mizobata et al., Arch. Biochem. Biophys. 355:49-55 (1998)) and *Rattus norvegicus* (Kobayashi et al., J. Biochem. 89:1923-1931 (1981)). Similar enzymes with high sequence homology include *fum1* from *Arabidopsis thaliana* and *fumC* from *Corynebacterium glutamicum.* The *MmcBC* fumarase from *Pelotomaculum thermopropionicum* is another class of fumarase with two subunits (Shimoyama et al., FEMS Microbiol. Lett. 270:207-213 (2007)).

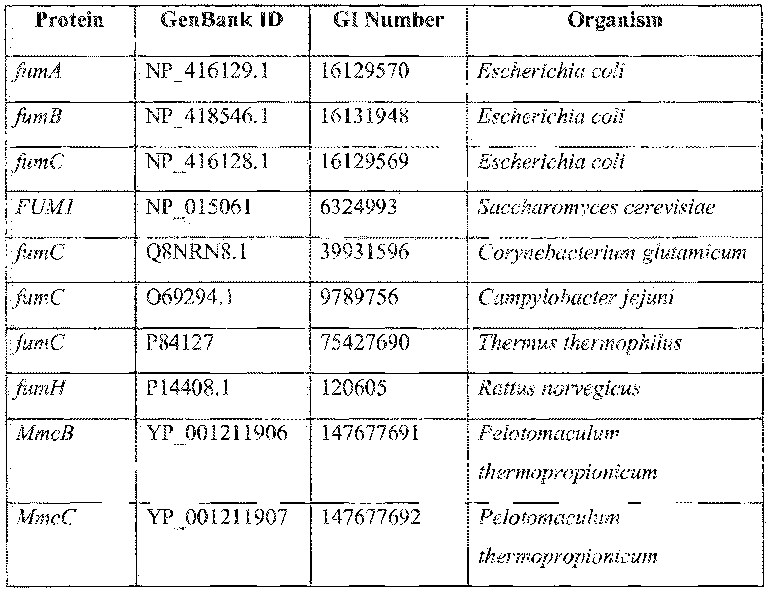

### D. Fumarate Reductase.

Fumarate reductase catalyzes the reduction of fumarate to succinate. The fumarate reductase of *E. coli,* composed of four subunits encoded by *frdABCD,* is membrane-bound and active under anaerobic conditions. The electron donor for this reaction is menaquinone and the two protons produced in this reaction do not contribute to the proton gradient (Iverson et al., Science 284:1961-1966 (1999)). The yeast genome encodes two soluble fumarate reductase isozymes encoded by FRDS1 (Enomoto et al., DNA Res. 3:263-267 (1996)) and FRDS2 (Muratsubaki et al., Arch. Biochem. Biophys. 352:175-181 (1998)), which localize to the cytosol and promitochondrion, respectively, and are used during anaerobic growth on glucose (Arikawa et al., FEMS Microbiol. Lett. 165:111-116 (1998)).

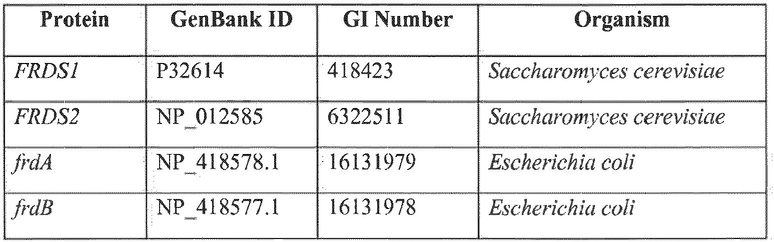

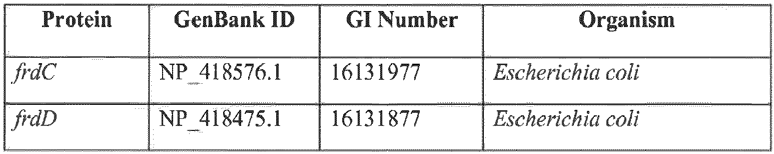

### E. Succinyl-CoA Synthetase or Transferase.

The ATP-dependent acylation of succinate to succinyl-CoA is catalyzed by succinyl-CoA synthetase (EC 6.2.1.5). The product of the *LSC1* and *LSC2* genes of S. *cerevisiae* and the *sucC* and *sucD* genes of *E. coli* naturally form a succinyl-CoA synthetase complex that catalyzes the formation of succinyl-CoA from succinate with the concomitant consumption of one ATP, a reaction which is reversible *in vivo* (Buck et al., Biochemistry 24:6245-6252 (1985)). These proteins are identified below:

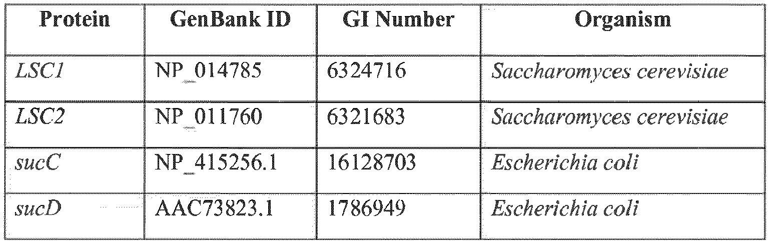

Succinyl-CoA transferase converts succinate and an acyl-CoA donor to succinyl-CoA and an acid. Succinyl-CoA transferase enzymes include *ygfH* of E. *coli* and *cat1* of *Clostridium kluyveri* (Seedorf et al., Proc.Natl.Acad.Sci U.S.A 105:2128-2133 (2008); Sohling et al., J Bacteriol. 178:871-880 (1996); Haller et al., Biochemistry, 39(16) 4622-4629). Homologs can be found in, for example, *Citrobacter youngae* ATCC 29220, *Salmonella enterica* subsp. *arizonae serovar,* and *Yersinia intermedia* ATCC 29909. Succinyl-CoA transferase enzymes are encoded by *peal* and *pcaJ* in *Pseudomonas putida* (Kaschabek et al., J Bacteriol. 184:207-215 (2002)). Similar enzymes are found in *Acinetobacter* sp. ADP1 (Kowalchuk et al., Gene 146:23-30 (1994)), *Streptomyces coelicolor* and *Pseudomonas knackmussii* (formerly *sp. B13*) (Gobcl et al., J Bacteriol. 184:216-223 (2002); Kaschabek et al., J Bacteriol. 184:207-215 (2002)). Additional exemplary succinyl-CoA transferases have been characterized in in *Helicobacter pylori* (Corthesy-Theulaz et al., J Biol.Chem. 272:25659-25667 (1997)), *Bacillus subtilis* (Stols et al., Protein Expr.Purif. 53:396-403 (2007)) and *Homo sapiens* (Fukao, T., et al., Genomics 68:144-151 (2000); Tanaka, H., ct al., Mol Hum Reprod 8:16-23 (2002)). Additional CoA transferases, described herein, are also suitable candidates.

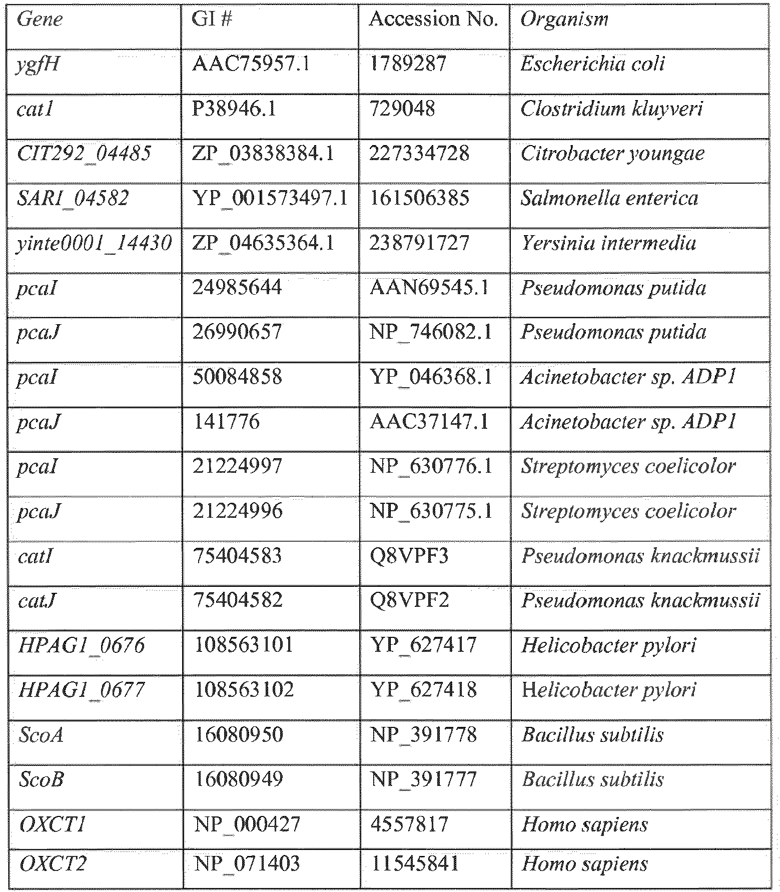

### F. Pyruvate Kinase or PTS-dependent substrate import. See elsewhere herein.

### G. Pyruvate Dehydrogenase, Pyruvate Formate Lyase or Pyruvate:ferredoxin oxidoreductase.

Pyruvate:ferredoxin oxidoreductase (PFOR) catalyzes the reversible oxidation of pyruvate to form acetyl-CoA. Exemplary PFOR enzymes are found in *Desulfovibrio africanus* (Pieulle et al., J. Bacteriol. 179:5684-5692 (1997)) and other *Desulfovibrio* species (Vita et al., Biochemistry, 47: 957-64 (2008)). The *M. thermoacetica* PFOR is also well characterized (Menon and Ragsdale, Biochemistry 36:8484-8494 (1997)) and was shown to have high activity in the direction of pyruvate synthesis during autotrophic growth (Furdui and Ragsdale, J. Biol. Chem. 275:28494-28499 (2000)). Further, *E. coli* possesses an uncharactcrized open reading frame, *ydbK,* encoding a protein that is 51% identical to the *M. thermoacetica* PFOR. Evidence for pyruvate oxidoreductase activity in *E. coli* has been described (Blaschkowski et al., Eur. J. Biochem. 123:563-569 (1982)). Finally, flavodoxin reductases *(e.g., fqrB* from *Helicobacter pylori* or *Campylobacter jejuni)* (St Maurice et al., J. Bacteriol. 189:4764-4773 (2007)) or Rnf-type proteins (Seedorf et al., Proc. Natl. Acad. Sci. U.S.A. 105:2128-2133 (2008); and Herrmann, J. Bacteriol 190:784-791 (2008)) provide a means to generate NADH or NADPH from the reduced ferredoxin generated by PFOR.

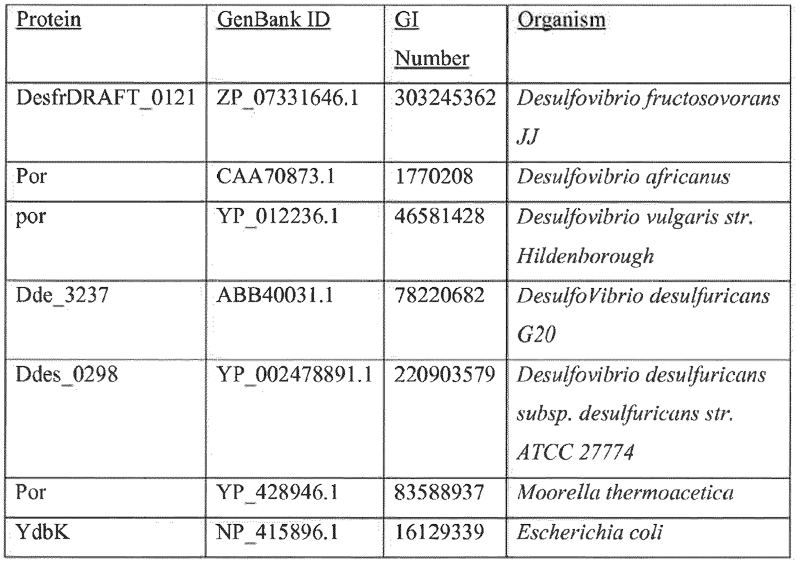

The conversion of pyruvate into acetyl-CoA can be catalyzed by several other enzymes or their combinations thereof. For example, pyruvate dehydrogenase can transform pyruvate into acetyl-CoA with the concomitant reduction of a molecule of NAD into NADH. It is a multi-enzyme complex that catalyzes a series of partial reactions which results in acylating oxidative decarboxylation of pyruvate. The enzyme comprises of three subunits: pyruvate decarboxylase (El), dihydrolipoamide acyltransferase (E2) and dihydrolipoamide dehydrogenase (E3). This enzyme is naturally present in several organisms, including *E. coli* and S. *cerevisiae.* In the *E. coli* enzyme, specific residues in the E1 component are responsible for substrate specificity (Bisswanger, H., J. Biol. Chem. 256:815-82 (1981); Bremer, J., Eur. J. Biochem. 8:535-540 (1969); Gong et al., J. Biol. Chem. 275:13645-13653 (2000)). Enzyme engineering efforts have improved the *E. coli* PDH enzyme activity under anaerobic conditions (Kim et al., J. Bacteriol. 190:3851-3858 (2008); Kim et al., Appl. Environ. Microbiol. 73:1766-1771 (2007); Zhou et al., Biotechnol. Lett. 30:335-342 (2008)). In contrast to the *E. coli* PDH, the *B. subtilis* complex is active and required for growth under anaerobic conditions (Nakano et al., J. Bacteriol. 179:6749-6755 (1997)). The *Klebsiella pneumoniae* PDH, characterized during growth on glycerol, is also active under anaerobic conditions (5).

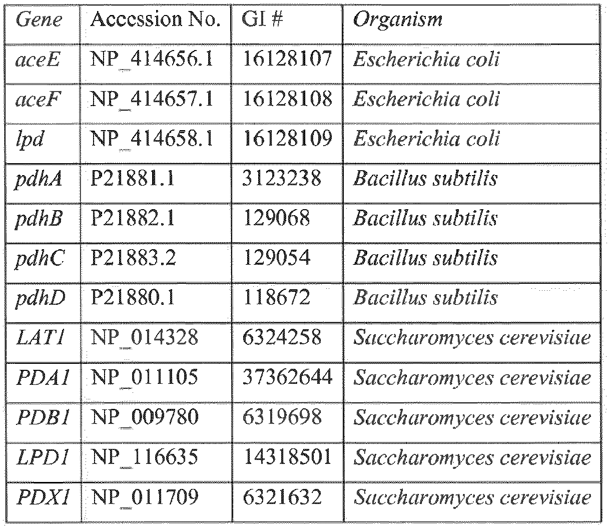

Yet another enzyme that can catalyze this conversion is pyruvate formate lyase. This enzyme catalyzes the conversion of pyruvate and CoA into acetyl-CoA and formate. Pyruvate formate lyase is a common enzyme in prokaryotic organisms that is used to help modulate anaerobic redox balance. Exemplary enzymes can be found in *Escherichia coli* encoded by *pflB* (Knappe and Sawers, FEMS. Microbiol Rev. 6:383-398 (1990)), *Lactococcus lactis* (Melchiorsen et al., Appl Microbiol Biotechnol 58:338-344 (2002)), and *Streptococcus mutans* (Takahashi-Abbe et al., Oral.Microbiol Immunol. 18:293-297 (2003)). *E. coli* possesses an additional pyruvate formate lyase, encoded by *tdcE,* that catalyzes the conversion of pyruvate or 2-oxobutanoate to acetyl-CoA or propionyl-CoA, respectively (Hesslinger et al., Mol. Microbiol 27:477-492 (1998)). *Both pflB* and *tdcE* from *E. coli* require the presence of pyruvate formate lyase activating enzyme, encoded by *pflA.* Further, a short protein encoded by *yfiD* in *E. coli* can associate with and restore activity to oxygen-cleaved pyruvate formate lyase (Vey et al., Proc.Natl. Acad. Sci. U.S.A. 105:16137-16141 (2008). Note that *pflA andpflB* from *E. coli* were expressed in S. *cerevisiae* as a means to increase cytosolic acetyl-CoA for butanol production as described in WO/2008/080124]. Additional pyruvate formate lyase and activating enzyme candidates, encoded by *pfl* and *act,* respectively, are found in *Clostridium pasteurianum* (Weidner et al., J Bacteriol. 178:2440-2444 (1996)).

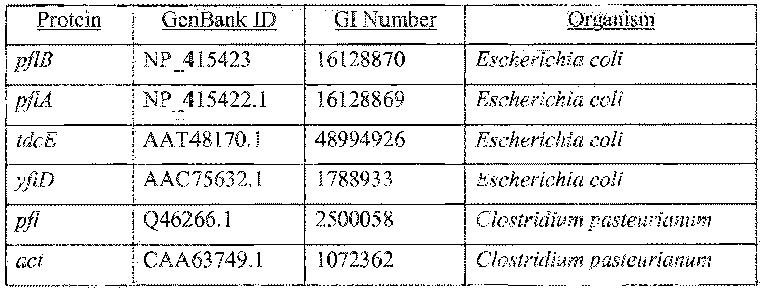

Further, different enzymes can be used in combination to convert pyruvate into acetyl-CoA in multiple steps. For example, in S. *cerevisiae,* acetyl-CoA is obtained in the cytosol by first decarboxylating pyruvate to form acetaldehyde; the latter is oxidized to acetate by acetaldehyde dehydrogenase and subsequently activated to form acetyl-CoA by acetyl-CoA synthetase. Acetyl-CoA synthetase is a native enzyme in several other organisms including *E. coli* (Kumari et al., J. Bacteriol. 177:2878-2886 (1995)), *Salmonella enterica* (Starai et al., Microbiology 151:3793-3801 (2005); Starai et al., J. Biol. Chem. 280:26200-26205 (2005)), and *Moorella thermoacetica* (described already). Alternatively, acetate can be activated to form acetyl-CoA by acetate kinase and phosphotransacetylase. Acetate kinase first converts acetate into acetyl-phosphate with the accompanying use of an ATP molecule. Acetyl-phosphate and CoA are next converted into acetyl-CoA with the release of one phosphate by phosphotransacetylase. Exemplary enzymes encoding acetate kinase, acetyl-CoA synthetase and phosphotransacetlyase are described above.
Yet another way of converting pyruvate to acetyl-CoA is via pyruvate oxidase. Pyruvate oxidase converts pyruvate into acetate, using ubiquinone as the electron acceptor. In *E. coli,* this activity is encoded by *poxB. PoxB* has similarity to pyruvate decarboxylase of S. *cerevisiae* and *Zymomonas mobilis.* The enzyme has a thiamin pyrophosphate cofactor (Koland and Gennis, Biochemistry 21:4438-4442 (1982)); O'Brien et al., Biochemistry 16:3105-3109 (1977); O'Brien and Gennis, J. Biol. Chem. 255:3302-3307 (1980)) and a flavin adenine dinuclcotidc (FAD) cofactor. Acetate can then be converted into acetyl-CoA by either acetyl-CoA synthetase or by acetate kinase and phosphotransacetylase, as described earlier. Some of these enzymes can also catalyze the reverse reaction from acetyl-CoA to pyruvate.

### H. Citrate Synthase.

Citrate synthases are well known in the art. For exampe, the gltA gene of *E.* coli encodes for a citrate synthase. It was previously shown that this gene is inhibited allosterically by NADH, and the amino acids involved in this inhibition have been identified (Pereira et al., J. Biol. Chem. 269(1):412-417 (1994); Stokcll et al., J. Biol. Chem. 278(37):35435-35443 (2003)). An NADH insensitive citrate synthase can be encoded by *gltA,* such as an R163L mutant of *gltA.* Other citrate synthase enzymes are less sensitive to NADH, including the aarA enzyme of *Acetobacter aceti* (Francois et al, Biochem 45:13487-99 (2006)).

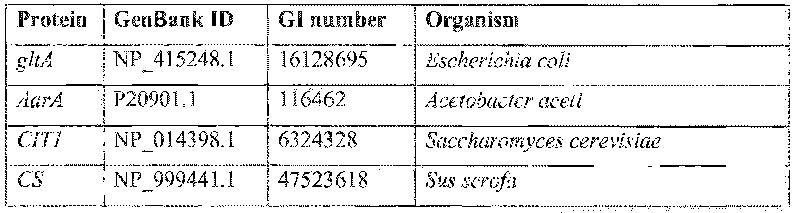

### I. Aconitase.

Aconitase (EC 4.2.1.3) is an iron-sulfur-containing protein catalyzing the reversible isomerization of citrate and iso-citrate via the intermediate *cis-*aconitate. Two aconitase enzymes of *E. coli* are encoded by *acnA* and *acnB.* AcnB is the main catabolic enzyme, while AcnA is more stable and appears to be active under conditions of oxidative or acid stress (Cunningham et al., Microbiology 143 (Pt 12):3795-3805 (1997)). Two isozymes of aconitase in *Salmonella typhimurium* are encoded by *acnA* and *acnB* (Horswill and Escalante-Semerena, Biochemistry 40:4703-4713 (2001)). The S. *cerevisiae* aconitase, encoded by *ACO1,* is localized to the mitochondria where it participates in the TCA cycle (Gangloff et al., Mol. Cell. Biol. 10:3551-3561 (1990)) and the cytosol where it participates in the glyoxylate shunt (Regev-Rudzki et al., Mol. Biol. Cell. 16:4163-4171 (2005)).

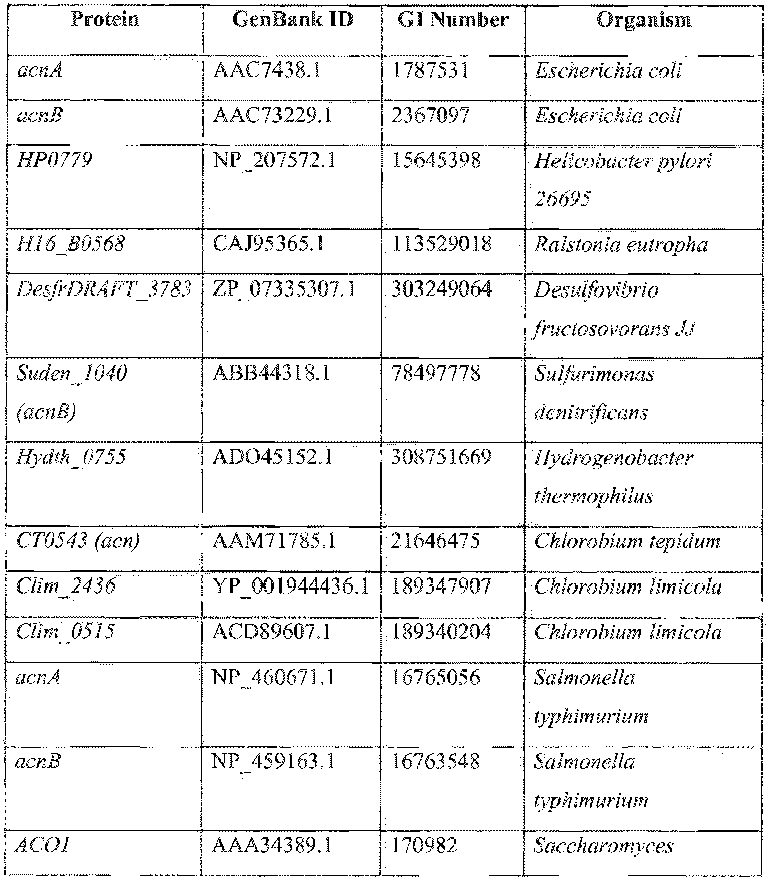

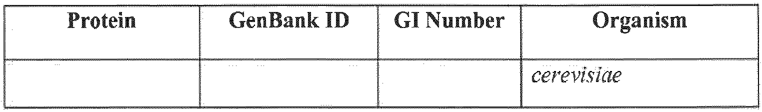

### J. Isocitrate Dehydrogenase.

Isocitrate dehydrogenase catalyzes the decarboxylation of isocitrate to 2-oxoglutarate coupled to the reduction of NAD(P)⁺. IDH enzymes in *Saccharomyces cerevisiae* and *Escherichia coli* are encoded by *IDP1* and *icd,* respectively (Haselbeck and McAlister-Henn, J. Biol. Chem. 266:2339-2345 (1991); Nimmo, H.G., Biochem. J. 234:317-2332 (1986)). The reverse reaction in the reductive TCA cycle, the reductive carboxylation of 2-oxoglutarate to isocitrate, is favored by the NADPH-dependent CO₂-fixing IDH from *Chlorobium limicola* (Kanao et al., Eur. J. Biochem. 269:1926-1931 (2002)). A similar enzyme with 95% sequence identity is found in the C. *tepidum* genome in addition to some other candidates listed below.

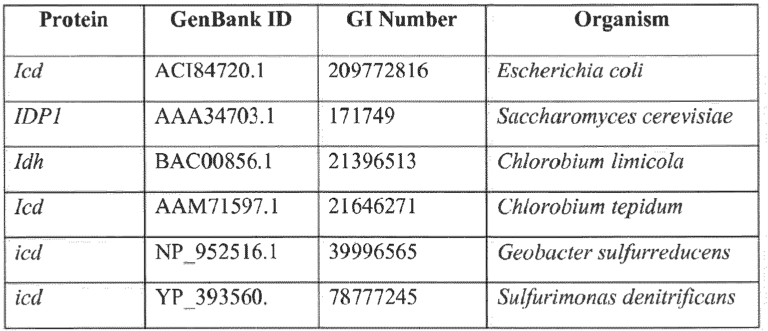

### K. AKG Dehydrogenase.

Alpha-ketoglutarate dehydrogenase (AKGD) converts alpha-ketoglutarate to succinyl-CoA and is the primary site of control of metabolic flux through the TCA cycle (Hansford, Curr.Top.Bioenerg. 10:217-278 (1980)). Encoded by genes *sucA, sucB* and *lpd* in *E. coli,* AKGD gene expression is downregulated under anaerobic conditions and during growth on glucose (Park et al., Mol Micro 15:473-482 (1995)). Other exemplary AKGDH enzymes are found in organisms such as *Bacillus subtilis* and S. *cerevisiae* (Resnekov et al., Mol.Gen.Genet. 234:285-296 (1992); Repetto et al., Mol.Cell Biol. 9:2695-2705 (1989)).

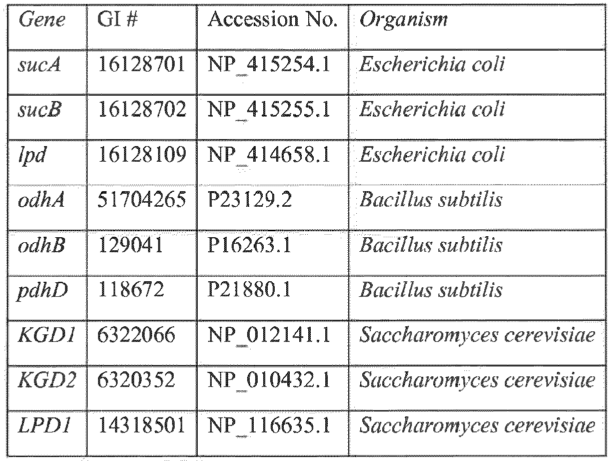

The conversion of alpha-ketoglutarate to succinyl-CoA can also be catalyzed by alpha-ketoglutarate:ferredoxin oxidoreductase (EC 1.2.7.3), also known as 2-oxoglutarate synthase or 2-oxoglutarate:ferredoxin oxidoreductase (OFOR). OFOR and pyruvate:ferredoxin oxidoreductase (PFOR) are members of a diverse family of 2-oxoacid:ferredoxin (flavodoxin) oxidoreductases which utilize thiamine pyrophosphate, CoA and iron-sulfur clusters as cofactors and ferredoxin, flavodoxin and FAD as electron carriers (Adams et al., Archaea. Adv. Protein Chem. 48:101-180 (1996)). Exemplary OFOR enzymes are found in organisms such as *Hydrogenobacter thermophilus, Desulfobacter hydrogenophilus* and *Chlorobium species* (Shiba et al. 1985; Evans et al., Proc. Natl. Acad. ScI. U.S.A. 55:92934 (1966); Buchanan, 1971). The two-subunit enzyme from *H. thermophilus,* encoded by *korAB,* has been cloned and expressed in *E. coli* (Yun et al., Biochem. Biophys. Res. Commun. 282:589-594 (2001)). A five subunit OFOR from the same organism with strict substrate specificity for succinyl-CoA, encoded by *forDABGE,* was recently identified and expressed in *E. coli* (Yun et al. Biochem. Biophys. Res. Commun. 292:280-286 (2002)). Another exemplary OFOR is encoded by *uorDABC* in *Helicobacter pylori* (Hughes et al., J. Bacteriol. 180:1119-1128 (1998)). An enzyme specific to alpha-ketoglutarate has been reported in *Thauera aromatica* (Dorner and Boll, J. Bacteriol. 184 (14), 3975-83 (2002).

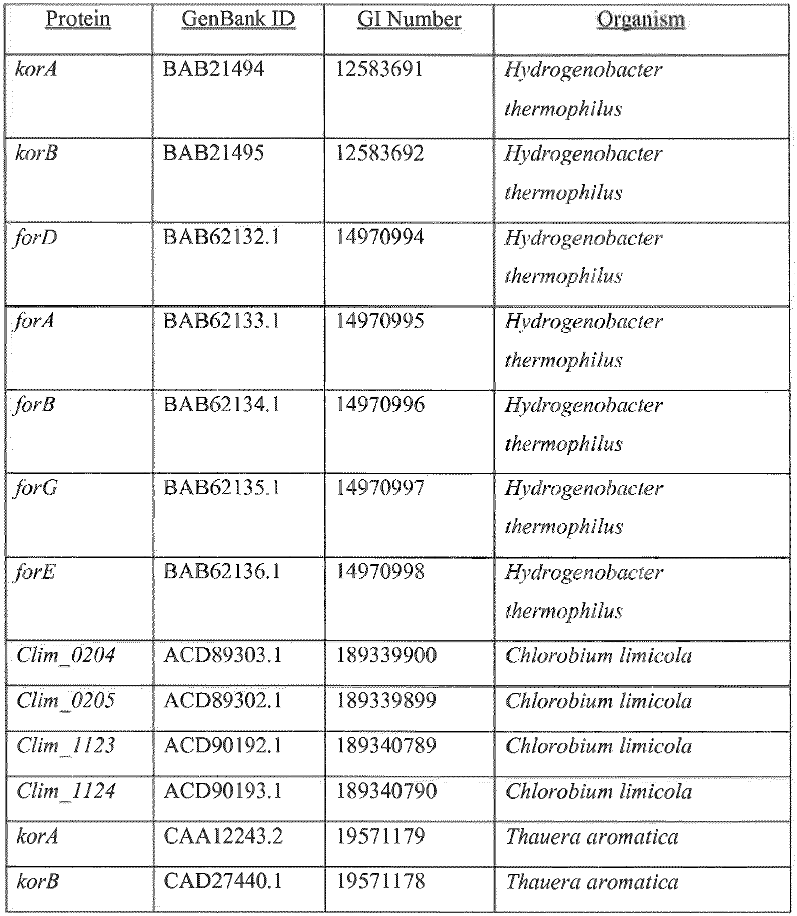

### L. Pyruvate Carboxylase.

Pyruvate carboxylase (EC 6.4.1.1) directly converts pyruvate to oxaloacetate at the cost of one ATP. Pyruvate carboxylase enzymes are encoded by *PYC1* (Walker et al., Biochem. Biophys. Res. Commun. 176:1210-1217 (1991) and *PYC2* (Walker et al., *supra)* in *Saccharomyces cerevisiae,* and *pyc* in *Mycobacterium smegmatis* (Mukhopadhyay and Purwantini, Biochim. Biophys. Acta 1475:191-206 (2000)).

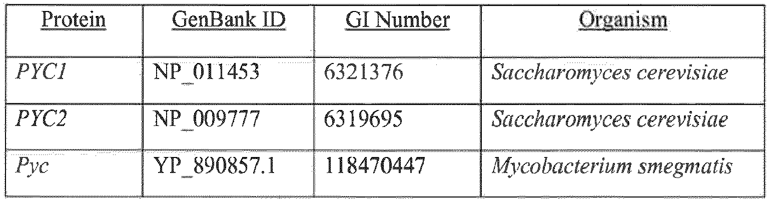

### M. Malic Enzyme.

Malic enzyme can be applied to convert CO₂ and pyruvate to malate at the expense of one reducing equivalent. Malic enzymes for this purpose can include, without limitation, malic enzyme (NAD-dependent) and malic enzyme (NADP-dependent). For example, one of the *E. coli* malic enzymes (Takeo, J. Biochem. 66:379-387 (1969)) or a similar enzyme with higher activity can be expressed to enable the conversion of pyruvate and CO₂ to malate. By fixing carbon to pyruvate as opposed to PEP, malic enzyme allows the high-energy phosphate bond from PEP to be conserved by pyruvate kinase whereby ATP is generated in the formation of pyruvate or by the phosphotransferase system for glucose transport. Although malic enzyme is typically assumed to operate in the direction of pyruvate formation from malate, overexpression of the NAD-dcpcndcnt enzyme, encoded by maeA, has been demonstrated to increase succinate production in *E. coli* while restoring the lethal delta pfl-delta ldhA phenotype (inactive or deleted pfl and ldhA) under anaerobic conditions by operating in the carbon-fixing direction (Stols and Donnelly, Appl. Environ. Microbiol. 63(7) 2695-2701 (1997)). A similar observation was made upon overexpressing the malic enzyme from *Ascaris suum* in *E. coli* (Stols et al., Appl. Biochem. Biotechnol. 63-65(1), 153-158 (1997)). The second *E. coli* malic enzyme, encoded by *maeB,* is NADP-dependent and also decarboxylates oxaloacetate and other alpha-keto acids (Iwakura et al., J. Biochem. 85(5):1355-65 (1979)).

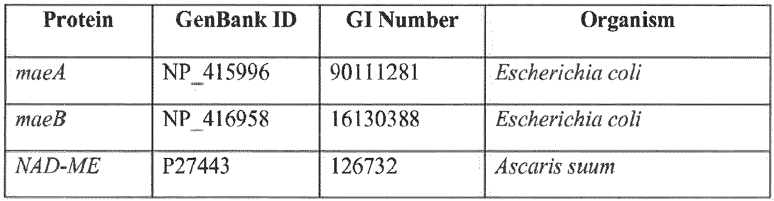

PEP synthetase: Also, known as pyruvate water dikinase, this enzyme converts pyruvate back into PEP at the expense of two ATP equivalents. It converts ATP into AMP. In E coli, this enzyme is encoded by ppsA. It is functional mainly during gluconeogenesis and provides the biomass precursors (Cooper and Kornberg, Biochim Biophys Acta, 104(2);618-20, (1965)). Its activity is regulated by a regulatory protein encoded by ppsR that catalyzes both the Pᵢ-dependent activation and ADP/ATP-dependent inactivation of PEP synthetase. PEP synthetase is protected from inactivation by the presence of pyruvate (Brunell, BMC Biochem. Jan 3;11:1, (2010)). The overexpression of this enzyme has been shown to increase the production of aromatic amino acids by increasing availability of PEP, which is a precursor for aromatic amino acid biosynthesis pathways (Yi et al., Biotechnol Prog., 18(6):1141-8., (2002); Patnaik and Liao. Appl Environ Microbiol. 1994 Nov;60(11):3903-8 (2001))). This enzyme has been studied in other organisms, such as Pyrococcus furiosus (Hutchins et al., J Bacteriol.,183(2):709-15 (2001)) and Psuedomonas fluorescens (J Biotechnol. 2013 Sep 10;167(3):309-15 (2013)).

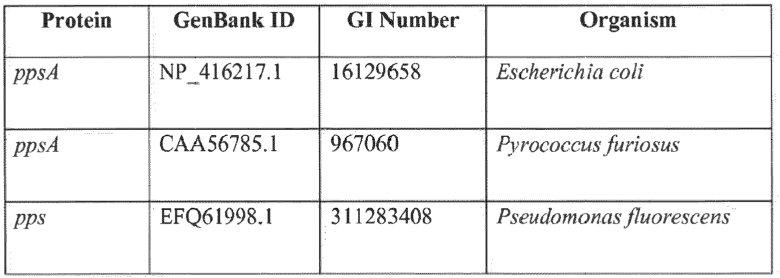

### EXAMPLE X

### 1,3-Butanediol, Crotyl Alcohol, 3-Buten-2-ol, and Butadiene Synthesis Enzymes

This Example provides genes that can be used for conversion of acetyl-CoA to 1,3-butanediol, crotyl alcohol, 3-butcn-2-ol, butadiene as depicted in the pathways of Figures 5 and 6.

Fig. 5. Pathways for converting 1,3-butanediol to 3-buten-2-ol and/or butadiene. A) acetyl-CoA carboxylase, B) an acetoacetyl-CoA synthase, C) an acetyl-CoA:acetyl-CoA acyltransferase, D) an acetoacetyl-CoA reductase (ketone reducing), E) a 3-hydroxybutyryl-CoA reductase (aldehyde forming), F) a 3-hydroxybutyryl-CoA hydrolase, transferase or synthetase, G) a 3-hydroxybutyratc reductase, H) a 3-hydroxybutyraldehyde reductase, I) chemical dehydration or corresponding step in **Figure 6**, J) a 3-hydroxybutyryl-CoA dehydratase, K) a crotonyl-CoA reductase (aldehyde forming), L) a crotonyl-CoA hydrolase, transferase or synthetase, M) a crotonatc reductase, N) a crotonaldehyde reductase, O) a crotyl alcohol kinase, P) a 2-butenyl-4-phosphate kinase, Q) a butadiene synthase, R) a crotyl alcohol diphosphokinase, S) chemical dehydration or a crotyl alcohol dehydratase, T) a butadiene synthase (monophosphate), T) a butadiene synthase (monophosphate), U) a crotonyl-CoA reductase (alcohol forming), and V) a 3-hydroxybutyryl-CoA reductase (alcohol forming).

### A. Acetyl-CoA Carboxylase.

_Acetyl-CoA carboxylase (EC 6.4.1.2) catalyzes the ATP-dependent carboxylation of acetyl-CoA to malonyl-CoA. This enzyme is biotin dependent and is the first reaction of fatty acid biosynthesis initiation in several organisms. Exemplary enzymes are encoded by accABCD of *E.* coli (Davis et al, J Biol Chem 275:28593-8 (2000)), ACC1 of *Saccharomyces cerevisiae* and homologs (Sumper et al, Methods Enzym 71:34-7 (1981)).

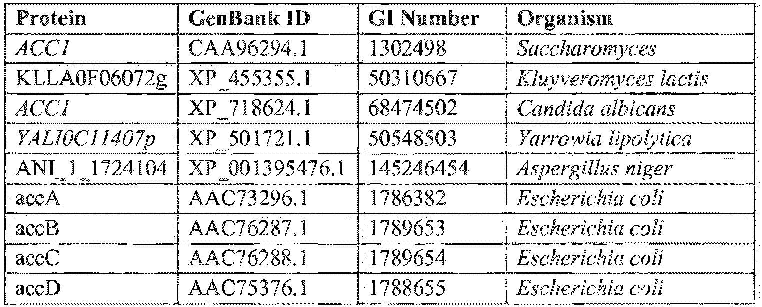

### B. Acetoacetyl-CoA Synthase.

The conversion of malonyl-CoA and acetyl-CoA substrates to acetoacetyl-CoA can be catalyzed by a CoA synthetase in the 2.3.1 family of enzymes. Several enzymes catalyzing the CoA synthetase activities have been described in the literature and represent suitable candidates. 3-Oxoacyl-CoA products such as acetoacetyl-CoA, 3-oxopentanoyl-CoA, 3-oxo-5-hydroxypentanoyl-CoA can be synthesized from acyl-CoA and malonyl-CoA substrates by 3-oxoacyl-CoA synthases. As enzymes in this class catalyze an essentially irreversible reaction, they are particularly useful for metabolic engineering applications for overproducing metabolites, fuels or chemicals derived from 3-oxoacyl-CoA intermediates such as acetoacetyl-CoA. Acetoacetyl-CoA synthase, for example, has been heterologously expressed in organisms that biosynthesize butanol (Lan et al, PNAS USA (2012)) and poly-(3-hydroxybutyrate) (Matsumoto ct al, Biosci Biotech Biochem, 75:364-366 (2011). An acetoacetyl-CoA synthase (EC 2.3.1.194) enzyme (FhsA) has been characterized in the soil bacterium *Streptomyces* sp. CL 190 where it participates in mevalonate biosynthesis (Okamura et al, PNAS USA 107:11265-70 (2010)). Other acetoacetyl-CoA synthase genes can be identified by sequence homology to *fhsA.*

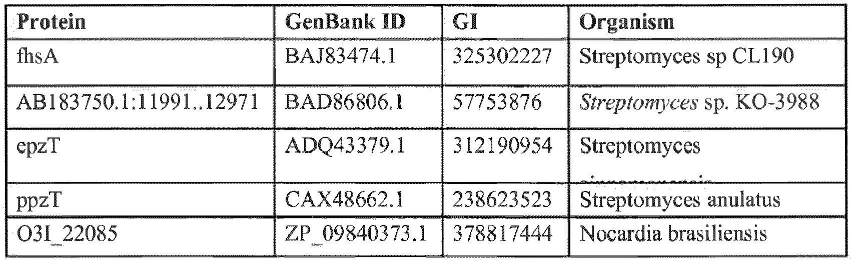

### C. Acetyl-CoA:acetyl-CoA Acyltransferase (Acetoacetyl-CoA thiolase).

Acetoacetyl-CoA thiolase (also known as acetyl-CoA acetyltransferase) converts two molecules of acetyl-CoA into one molecule each of acetoacetyl-CoA and CoA. Exemplary acetoacetyl-CoA thiolase enzymes include the gene products of *atoB* from *E. coli* (Martin et al., Nat.Biotechnol 21:796-802 (2003)), *thlA and thlB* from *C. acetobutylicum* (Hanai et al., Appl Environ Microbiol 73:7814-7818 (2007); Winzer et al., J.Mol.Microbiol Biotechnol 2:531-541 (2000), and *ERG10* from S. *cerevisiae* Hiser et al., J.Biol.Chem. 269:31383-31389 (1994)). The acetoacetyl-CoA thiolase from Zoogloea ramigera is irreversible in the biosynthetic direction and a crystal structure is available (Merilainen et al, Biochem 48: 11011-25 (2009)).These genes/proteins arc identified in the Table below.

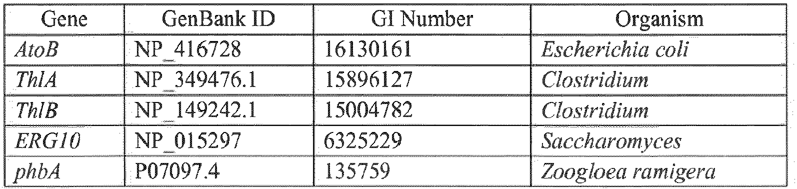

### D. Acetoacetyl-CoA reductase.

A suitable enzyme activity is 1.1.1.a Oxidoreductase (oxo to alcohol). See herein. In addition, Acetoacetyl-CoA reductase (EC 1.1.1.36) catalyzes the reduction of acetoacetyl-CoA to 3-hydroxybutyryl-CoA. This enzyme participates in the acetyl-CoA fermentation pathway to butyrate in several species of *Clostridia* and has been studied in detail (Jones et al., Microbiol Rev. 50:484-524 (1986)). Acetoacetyl-CoA reductase also participates in polyhydroxybutyrate biosynthesis in many organisms, and has also been used in metabolic engineering applications for overproducing PHB and 3-hydroxyisobutyrate (Liu et al., Appl. Microbiol. Biotechnol. 76:811-818 (2007); Qui et al., Appl. Microbiol. Biotechnol. 69:537-542 (2006)). The enzyme from *Clostridium acetobutylicum,* encoded by *hbd,* has been cloned and functionally expressed in *E. coli* (Youngleson et al., J Bacteriol. 171:6800-6807 (1989)). Additional gene candidates include *phbB* from *Zoogloea ramigera* (Ploux et al., Eur.J Biochem. 174:177-182 (1988)) and *phaB* from *Rhodobacter sphaeroides* (Alber et al., Mol.Microbiol 61:297-309 (2006)). The *Z*. *ramigera* gene is NADPH-dependent and the gene has been expressed in *E. coli* (Peoples et al., Mol.Microbiol 3:349-357 (1989)). Substrate specificity studies on the gene led to the conclusion that it could accept 3-oxopropionyl-CoA as a substrate besides acetoacetyl-CoA (Ploux et al., Eur.JBiochem. 174:177-182 (1988)). Additional genes include*phaB* in *Paracoccus denitrificans, Hbdl* (C-terminal domain) and *Hbd2* (N-terminal domain) in *Clostridium kluyveri* (Hillmer and Gottschalk, Biochim. Biophys. Acta 3334:12-23 (1974)) and *HSD17B10* in *Bos taurus* (Wakil et al., J Biol.Chem. 207:631-638 (1954)). The enzyme from *Paracoccus denitrificans* has been functionally expressed and characterized in *E. coli* (Yabutani et al., FEMS Microbiol Lett. 133:85-90 (1995)). A number of similar enzymes have been found in other species of *Clostridia* and in *Metallosphaera sedula* (Berg et al., Science. 318:1782-1786 (2007)). The enzyme from *Candida tropicalis* is a component of the peroxisomal fatty acid beta-oxidation multifunctional enzyme type 2 (MFE-2). The dehydrogenase B domain of this protein is catalytically active on acetoacetyl-CoA. The domain has been functionally expressed in *E. coli,* a crystal structure is available, and the catalytic mechanism is well-understood (Ylianttila et al., Biochem Biophys Res Commun 324:25-30 (2004); Ylianttila et al., J Mol Biol 358:1286-1295 (2006)).

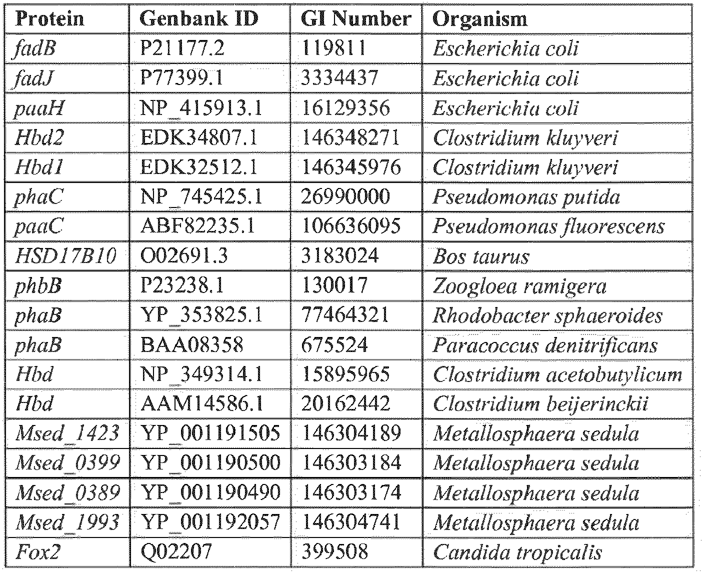

### E) 3-Hydroxybutyryl-CoA Reductase (aldehyde forming).

An EC 1.2.1.b Oxidoreductase (acyl-CoA to aldehyde) provides suitable enzyme activity. Acyl-CoA reductases or acylating aldehyde dehydrogenases reduce an acyl-CoA to its corresponding aldehyde. Exemplary enzymes include fatty acyl-CoA reductase, succinyl-CoA reductase (EC 1.2.1.76), acetyl-CoA reductase, butyryl-CoA reductase, propionyl-CoA reductase (EC 1.2.1.3) and others shown in the table below.

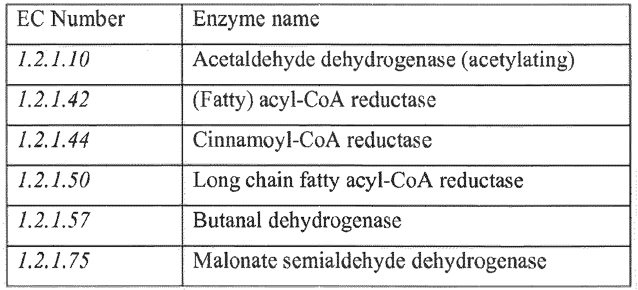

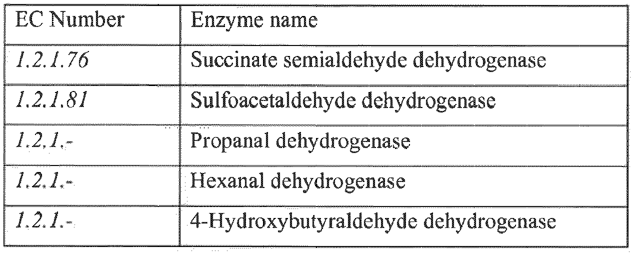

Exemplary fatty acyl-CoA reductases enzymes are encoded by *acr1* of *Acinetobacter calcoaceticus* (Reiser, Journal of Bacteriology 179:2969-2975 (1997)) and *Acinetobacter sp. M-1* (Ishige et al., Appl. Environ. Microbiol. 68:1192-1195 (2002)). Enzymes with succinyl-CoA reductase activity are encoded by *sucD* of *Clostridium kluyveri* (Sohling, J. Bacteriol. 178:871-880 (1996)) and *sucD* of *P. gingivalis* (Takahashi, J. Bacteriol 182:4704-4710 (2000)). Additional succinyl-CoA reductase enzymes participate in the 3-hydroxypropionate/4-hydroxybutyrate cycle of thermophilic archaea including *Metallosphaera sedula* (Berg et al., Science 318:1782-1786 (2007)) and *Thermoproteus neutrophilus* (Ramos-Vera et al., J Bacteriol., 191:4286-4297 (2009)). The *M. sedula* enzyme, encoded by *Msed_0709,* is strictly NADPH-dependent and also has malonyl-CoA reductase activity. The *T. neutrophilus* enzyme is active with both NADPH and NADH. The enzyme acylating acetaldehyde dehydrogenase in *Pseudomonas sp,* encoded by *bphG,* is yet another as it has been demonstrated to oxidize and acylate acetaldehyde, propionaldehyde, butyraldehyde, isobutyraldehyde and formaldehyde (Powlowski, J. Bacteriol. 175:377-385 (1993)). In addition to reducing acetyl-CoA to ethanol, the enzyme encoded by *adhE* in *Leuconostoc mesenteroides* has been shown to oxidize the branched chain compound isobutyraldehyde to isobutyryl-CoA (Kazahaya, J. Gen. Appl. Microbiol. 18:43-55 (1972); and Koo et al., Biotechnol Lett. 27:505-510 (2005)). Butyraldehyde dehydrogenase catalyzes a similar reaction, conversion of butyryl-CoA to butyraldehyde, in solventogenic organisms such as *Clostridium saccharoperbutylacetonicum* (Kosaka et al., Biosci Biotechnol Biochem., 71:58-68 (2007)). Exemplary propionyl-CoA reductase enzymes include *pduP* of *Salmonella typhimurium LT2* (Leal, Arch. Microbiol. 180:353-361 (2003)) and *eutE* from E. *coli* (Skraly, WO Patent No. 2004/024876). The propionyl-CoA reductase of *Salmonella typhimurium LT2,* which naturally converts propionyl-CoA to propionaldehyde, also catalyzes the reduction of 5-hydroxyvalcryl-CoA to 5-hydroxypentanal (WO 2010/068953A2).

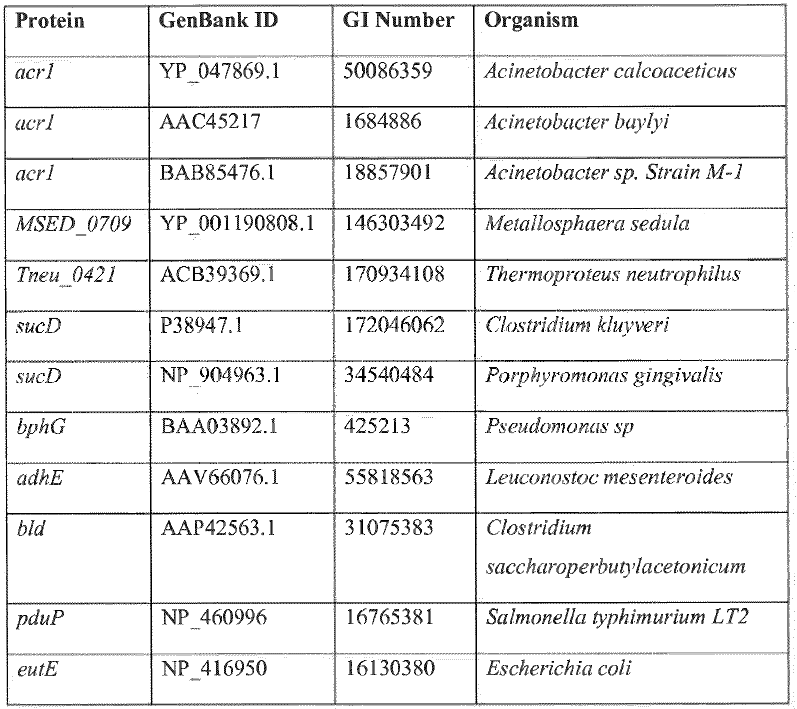

An additional enzyme that converts an acyl-CoA to its corresponding aldehyde is malonyl-CoA reductase which transforms malonyl-CoA to malonic semialdehyde. Malonyl-CoA reductase is a key enzyme in autotrophic carbon fixation via the 3-hydroxypropionate cycle in thermoacidophilic archaeal bacteria (Berg, Science 318:1782-1786 (2007); and Thauer, Science 318:1732-1733 (2007)). The enzyme utilizes NADPH as a cofactor and has been characterized in *Metallosphaera* and *Sulfolobus sp.* (Alber et al., J. Bacteriol. 188:8551-8559 (2006); and Hugler, J. Bacteriol. 184:2404-2410 (2002)). The enzyme is encoded by *Msed_0709* in *Metallosphaera sedula* (Alber et al., J. Bacteriol. 188:8551-8559 (2006); and Berg, Science 318:1782-1786 (2007)). A gene encoding a malonyl-CoA reductase from *Sulfolobus tokodaii* was cloned and heterologously expressed in *E. coli* (Alber et al., J. Bacteriol 188:8551-8559 (2006). This enzyme has also been shown to catalyze the conversion of methylmalonyl-CoA to its corresponding aldehyde (WO2007141208 (2007)). Although the aldehyde dehydrogenase functionality of these enzymes is similar to the bifunctional dehydrogenase from *Chloroflexus aurantiacus,* there is little sequence similarity. Both malonyl-CoA reductase enzyme candidates have high sequence similarity to aspartate-semialdehyde dehydrogenase, an enzyme catalyzing the reduction and concurrent dephosphorylation of aspartyl-4-phosphate to aspartate semialdehyde. Additional gene candidates can be found by sequence homology to proteins in other organisms including *Sulfolobus solfataricus* and *Sulfolobus acidocaldarius* and have been listed below. Yet another candidate for CoA-acylating aldehyde dehydrogenase is the *ald* gene from *Clostridium beijerinckii* (Toth, Appl. Environ. Microbiol. 65:4973-4980 (1999). This enzyme has been reported to reduce acetyl-CoA and butyryl-CoA to their corresponding aldehydes. This gene is very similar to *eutE* that encodes acetaldehyde dehydrogenase of *Salmonella typhimurium* and *E. coli* (Toth, Appl. Environ. Microbiol. 65:4973-4980 (1999).

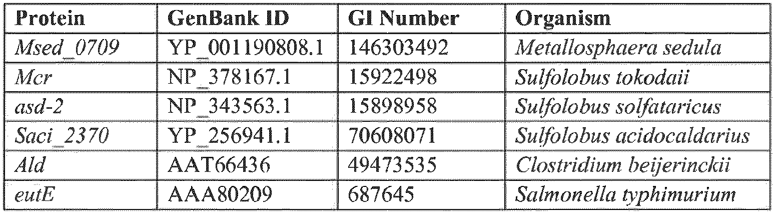

4-Hydroxybutyryl-CoA reductase catalyzes the reduction of 4-hydroxybutyryl-CoA to its corresponding aldehyde. Several acyl-CoA dehydrogenases are capable of catalyzing this activity. The succinate semialdehyde dehydrogenases (SucD) of *Clostridium kluyveri* and *P. gingivalis* were shown in ref. (WO/2008/115840) to convert 4-hydroxybutyryl-CoA to 4-hydroxybutanal as part of a pathway to produce 1,4-butanediol. Many butyraldehyde dehydrogenases are also active on 4-hydroxybutyraldehyde, including *bld* of *Clostridium saccharoperbutylacetonicum* and *bphG of Pseudomonas sp* (Powlowski et al., J. Bacterial. 175:377-385 (1993)). Yet another candidate is the *ald* gene from *Clostridium beijerinckii* (Toth, Appl. Environ. Microbiol. 65:4973-4980 (1999). This gene is very similar to *eutE* that encodes acetaldehyde dehydrogenase of *Salmonella typhimurium* and *E. coli* (Toth, Appl. Environ. Microbiol. 65:4973-4980 (1999). These and additional proteins with 4-hydroxybutyryl-CoA reductase activity are identified below.

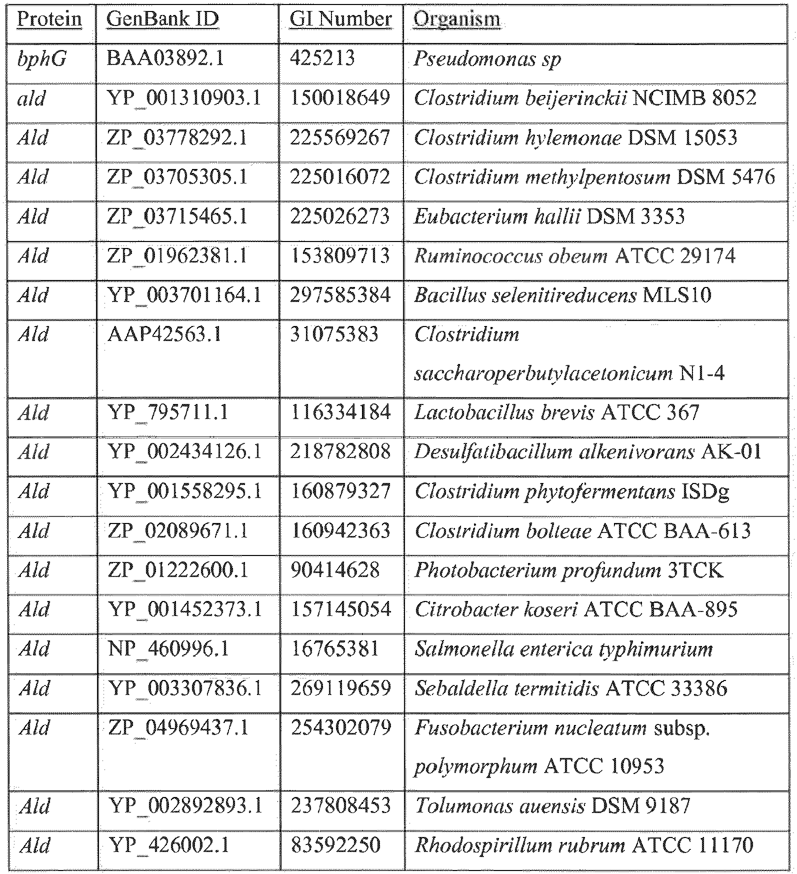

### F) 3-Hydroxybutyryl-CoA Hydrolase, Transferase or Synthetase.

An EC 3.1.2.a CoA hydrolase, EC 2.8.3.a CoA transferase, and/or an EC 6.2.1.a CoA synthetase provide suitable enzyme activity. See below and herein.

### G) 3-Hydroxybutyrate Reductase.

An EC 1.2.1.e Oxidoreductase (acid to aldehyde) provides suitable activity. See below and herein.

### H) 3-Hydroxybutyraldehyde Reductase.

An EC 1.1.1.a Oxidoreductase (oxo to alcohol) provides suitable activity. See herein.

### I) Chemical dehydration or alternatively see corresponding enzymatic pathway in Figure 6.

### J) 3-Hydroxybutyryl-CoA Dehydratase.

An EC 4.2.1. Hydro-lyase provides suitable enzyme activity, and are described below and herein. The enoyl-CoA hydratase of *Pseudomonas putida,* encoded by *ech,* catalyzes the conversion of 3-hydroxybutyryl-CoA to crotonyl-CoA (Roberts et al., Arch.Microbiol 117:99-108 (1978)). This transformation is also catalyzed by the *crt* gene product of *Clostridium acetobutylicum,* the *crt1* gene product of *C*. *kluyveri,* and other clostridial organisms Atsumi et al., Metab Eng 10:305-311 (2008); Boynton et al., J Bacteriol. 178:3015-3024 (1996); Hillmer et al., FEBS Lett. 21:351-354 (1972)). Additional enoyl-CoA hydratase candidates are *phaA* and *phaB,* of *P. putida,* and *paaA* and *paaB* from *P. fluorescens* (Olivera et al., Proc.Natl.Acad.Sci U.S.A 95:6419-6424 (1998)). The gene product of *pimF* in *Rhodopseudomonas palustris* is predicted to encode an enoyl-CoA hydratase that participates in pimeloyl-CoA degradation (Harrison et al., Microbiology 151:727-736 (2005)). Lastly, a number of *Escherichia coli* genes have been shown to demonstrate enoyl-CoA hydratase functionality including *maoC* (Park et al., J Bacteriol. 185:5391-5397 (2003)), *paaF* (Ismail et al., Eur.J Biochem. 270:3047-3054 (2003); Park et al., Appl.Biochem.Biotechnol 113-116:335-346 (2004); Park et al., Biotechnol Bioeng 86:681-686 (2004)) and *paaG* (Ismail et al., Eur.J Biochem. 270:3047-3054 (2003); Park and Lee, Appl.Biochem.Biotechnol 113-116:335-346 (2004); Park and Yup, Biotechnol Bioeng 86:681-686 (2004)).

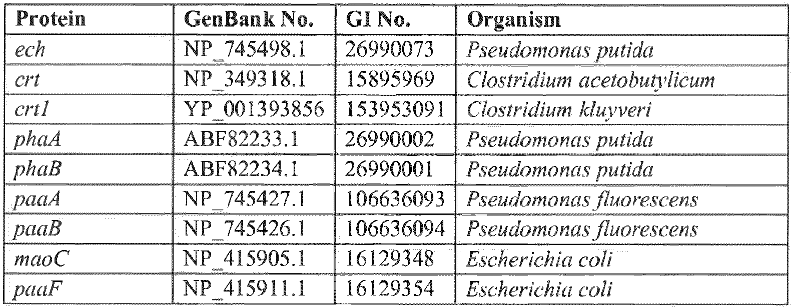

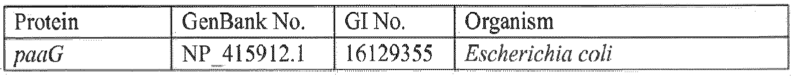

### K) Crotonyl-CoA Reductase (aldehyde forming).

An EC 1.2.1.b Oxidoreductase (acyl-CoA to aldehyde) provides suitable enzyme activity. Acyl-CoA reductases in the 1.2.1 family reduce an acyl-CoA to its corresponding aldehyde. Several acyl-CoA reductase enzymes have been described in the open literature and represent suitable candidates for this step. These are described above and herein.

### L) Crotonyl-CoA Hydrolase, Transferase or Synthetase.

An EC 3.1.2.a CoA hydrolase, EC 2.8.3.a CoA transferase, and/or an EC 6.2.1.a CoA synthetase provide suitable enzyme activity, and are described herein and in the following sections.

### EC 3.1.2.a CoA Hydrolase.

Enzymes in the 3.1.2 family hydrolyze acyl-CoA molecules to their corresponding acids. Several such enzymes have been described in the literature and represent suitable candidates for these steps.

For example, the enzyme encoded by *acot12* from *Rattus norvegicus* brain (Robinson et al., Biochem.Biophys.Res.Commun. 71:959-965 (1976)) can react with butyryl-CoA, hexanoyl-CoA and malonyl-CoA. The human dicarboxylic acid thioesterase, encoded by *acot8,* exhibits activity on glutaryl-CoA, adipyl-CoA, suberyl-CoA, sebacyl-CoA, and dodecanedioyl-CoA (Westin et al.,J.Biol.Chem. 280:38125-38132 (2005)). The closest *E. coli* homolog to this enzyme, *tesB,* can also hydrolyze a range of CoA thiolesters (Naggert et al., J Biol Chem 266:11044-11050 (1991)). A similar enzyme has also been characterized in the rat liver (Deana R., Biochem Int 26:767-773 (1992)). Additional enzymes with hydrolase activity in *E. coli* include *ybgC, paaI,* and *ybdB* (Kuznetsova, et al., FEMS Microbiol Rev, 2005, 29(2):263-279; Song et al., J Biol Chem, 2006, 281(16):11028-38). Though its sequence has not been reported, the enzyme from the mitochondrion of the pea leaf has a broad substrate specificity, with demonstrated activity on acetyl-CoA, propionyl-CoA, butyryl-CoA, palmitoyl-CoA, oleoyl-CoA, succinyl-CoA, and crotonyl-CoA (Zeiher et al., Plant.Physiol. 94:20-27 (1990)) The acetyl-CoA hydrolase, *ACH1,* from *S*. *cerevisiae* represents another candidate hydrolase (Buu et al., J.Biol.Chem. 278:17203-17209 (2003)) .

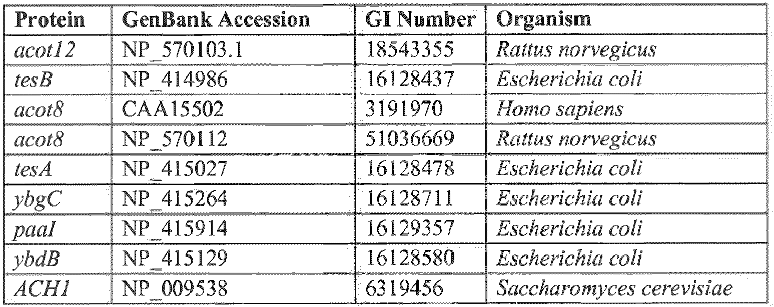

Additional hydrolase enzymes include 3-hydroxyisobutyryl-CoA hydrolase which has been described to efficiently catalyze the conversion of 3-hydroxyisobutyryl-CoA to 3-hydroxyisobutyrate during valine degradation (Shimomura et al., J Biol Chem. 269:14248-14253 (1994)). Genes encoding this enzyme include *hibch* of *Rattus norvegicus* (Shimomura et al., Methods Enzymol. 324:229-240 (2000)) and *Homo sapiens* (Shimomura et al., *supra).* Similar gene candidates can also be identified by sequence homology, including *hibch* of *Saccharomyces cerevisiae* and *BC_2292* of *Bacillus cereus.*

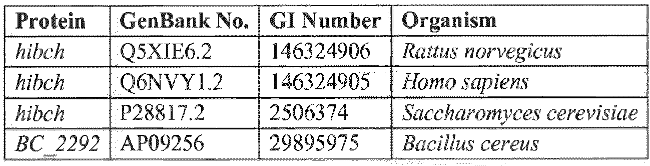

### EC 2.8.3.a CoA transferase.

Enzymes in the 2.8.3 family catalyze the reversible transfer of a CoA moiety from one molecule to another. Several CoA transferase enzymes have been described in the open literature and represent suitable candidates for these steps. These are described below.

Many transferases have broad specificity and thus can utilize CoA acceptors as diverse as acetate, succinate, propionate, butyrate, 2-mcthylacctoacctatc, 3-ketohexanoate, 3-ketopentanoate, valerate, crotonate, 3-mercaptopropionate, propionate, vinylacetate, butyrate, among others. For example, an enzyme from *Roseburia* sp. A2-183 was shown to have butyryl-CoA:acetate:CoA transferase and propionyl-CoA:acctatc:CoA transferase activity (Charricr et al., Microbiology 152, 179-185 (2006)). Close homologs can be found in, for example, *Roseburia intestinalis* LI-82, *Roseburia inulinivorans* DSM 16841, *Eubacterium rectale* ATCC 33656. Another enzyme with propionyl-CoA transferase activity can be found in *Clostridium propionicum* (Selmer et al., Eur J Biochem 269, 372-380 (2002)). This enzyme can use acetate, (R)-lactate, (S)-lactate, acrylate, and butyrate as the CoA acceptor (Selmer et al., Eur J Biochem 269, 372-380 (2002); Schweiger and Buckcl, FEBS Letters, 171(1) 79-84 (1984)). Close homologs can be found in, for example, *Clostridium novyi* NT, *Clostridium beijerinckii* NCIMB 8052, and *Clostridium botulinum* C str. Eklund. *YgfH* encodes a propionyl CoA:succinate CoA transferase in *E. coli* (Haller et al., Biochemistry, 39(16) 4622-4629). Close homologs can be found in, for example, *Citrobacter youngae* ATCC 29220, *Salmonella enterica* subsp. *arizonae serovar,* and *Yersinia intermedia* ATCC 29909.

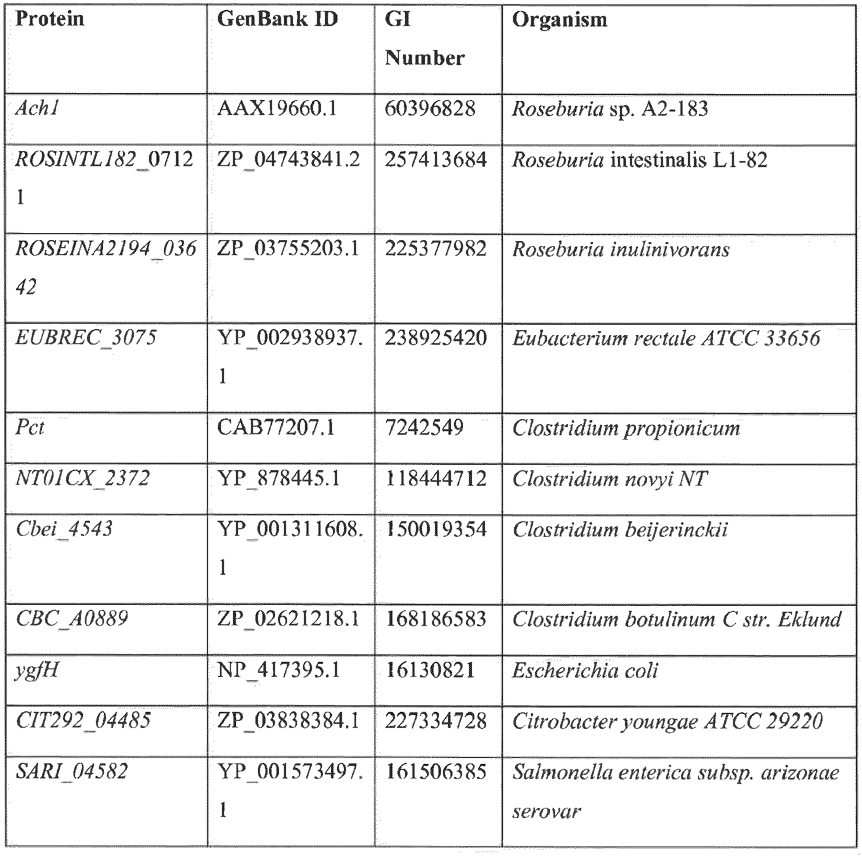

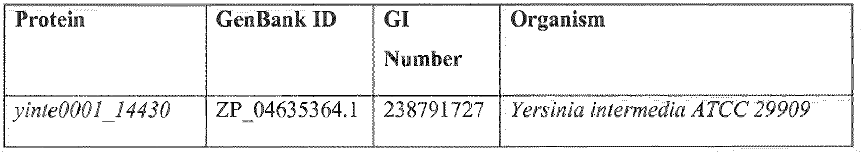

An additional candidate enzyme is the two-unit enzyme encoded by *pcaI* and *pcaJ* in *Pseudomonas,* which has been shown to have 3-oxoadipyl-CoA/succinate transferase activity (Kaschabek et al., *supra).* Similar enzymes based on homology exist in *Acinetobacter* sp. ADP1 (Kowalchuk et al., Gene 146:23-30 (1994)) and *Streptomyces coelicolor.* Additional exemplary succinyl-CoA:3:oxoacid-CoA transferases are present in *Helicobacter pylori* (Corthesy-Theulaz et al., J.Biol.Chem. 272:25659-25667 (1997)) and *Bacillus subtilis* (Stols ct al., Protein.Expr.Purif. 53:396-403 (2007)). These proteins are identified below.

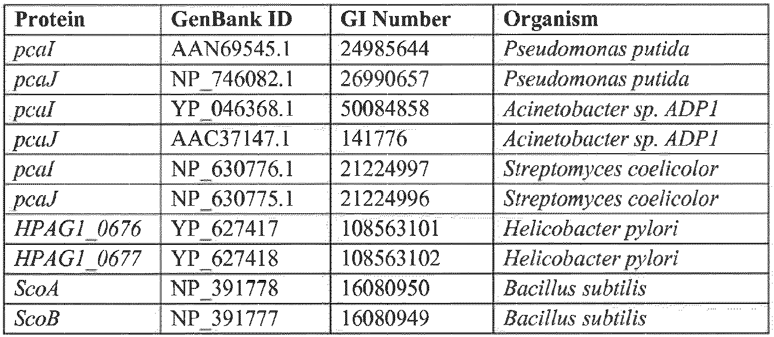

A CoA transferase that can utilize acetate as the CoA acceptor is acetoacetyl-CoA transferase, encoded by the *E. coli atoA* (alpha subunit) and *atoD* (beta subunit) genes (Vanderwinkel et al., Biochem.Biophys.Res Commun. 33:902-908 (1968); Korolev et al., Acta Crystallogr.D Biol Crystallogr. 58:2116-2121 (2002)). This enzyme has also been shown to transfer the CoA moiety to acetate from a variety of branched and linear acyl-CoA substrates, including isobutyrate (Matthies et al., Appl Environ Microbiol 58:1435-1439 (1992)), valerate (Vanderwinkel et al., *supra)* and butanoate (Vanderwinkel et al., *supra*). Similar enzymes exist in *Corynebacterium glutamicum* ATCC 13032 (Duncan et al., Appl Environ Microbiol 68:5186-5190 (2002)), *Clostridium acetobutylicum* (Cary et al., Appl Environ Microbiol 56:1576-1583 (1990)), and *Clostridium saccharoperbutylacetonicum* (Kosaka et al., Biosci.Biotechnol Biochem. 71:58-68 (2007)). These proteins are identified below.

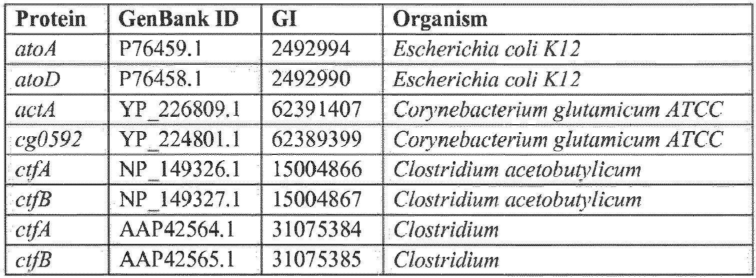

Additional exemplary transferase candidates are catalyzed by the gene products of *cat1, cat2,* and *cat3* of *Clostridium kluyveri* which have been shown to exhibit succinyl-CoA, 4-hydroxybutyryl-CoA, and butyryl-CoA transferase activity, respectively (Seedorf et al., *supra*; Sohling et al., Eur.J Biochem. 212:121-127 (1993); Sohling et al., J Bacteriol. 178:871-880 (1996)). Similar CoA transferase activities are also present in *Trichomonas vaginalis* (van Grinsven et al., J.Biol.Chem. 283:1411-1418 (2008)) and *Trypanosoma brucei* (Riviere et al., J.Biol.Chem. 279:45337-45346 (2004)). These proteins are identified below.

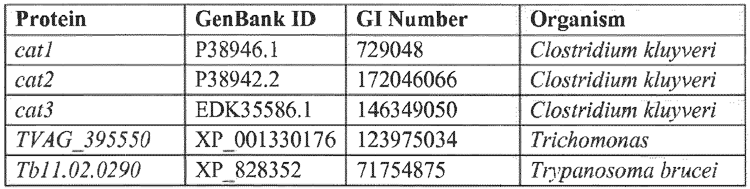

The glutaconatc-CoA-transferase (EC 2.8.3.12) enzyme from anaerobic bacterium *Acidaminococcus fermentans* reacts with diacid glutaconyl-CoA and 3-butenoyl-CoA (Mack et al., FEBS Lett. 405:209-212 (1997)). The genes encoding this enzyme are *gctA* and *gctB.* This enzyme has reduced but detectable activity with other CoA derivatives including glutaryl-CoA, 2-hydroxyglutaryl-CoA, adipyl-CoA and acrylyl-CoA (Buckel et al., Eur.J.Biochem. 118:315-321 (1981)). The enzyme has been cloned and expressed in *E. coli* (Mack et al., Eur.J.Biochem. 226:41-51 (1994)). These proteins are identified below.

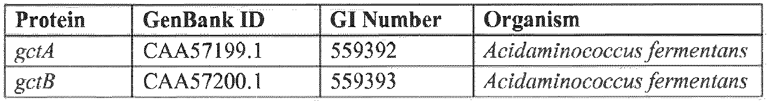

### EC 6.2.1.a CoA synthase (Acid-thiol ligase).

The conversion of acyl-CoA substrates to their acid products can be catalyzed by a CoA acid-thiol ligase or CoA synthetase in the 6.2.1 family of enzymes, several of which are reversible.. Several enzymes catalyzing CoA acid-thiol ligase or CoA synthetase activities have been described in the literature and represent suitable candidates for these steps.

For example, ADP-forming acetyl-CoA synthetase (ACD, EC 6.2.1.13) is an enzyme that couples the conversion of acyl-CoA esters to their corresponding acids with the concomitant synthesis of ATP. ACD 1 from *Archaeoglobus fulgidus,* encoded by AF1211, was shown to operate on a variety of linear and branched-chain substrates including isobutyrate, isopentanoate, and fumarate (Musfeldt et al., J Bacteriol. 184:636-644 (2002)). A second reversible ACD in *Archaeoglobus fulgidus,* encoded by AF1983, was also shown to have a broad substrate range with high activity on cyclic compounds phenylacetate and indoleacetate (Musfeldt and Schonheit, J Bacteriol. 184:636-644 (2002)). The enzyme from *Haloarcula marismortui* (annotated as a succinyl-CoA synthetase) accepts propionate, butyrate, and branched-chain acids (isovalerate and isobutyrate) as substrates, and was shown to operate in the forward and reverse directions (Brasen et al., Arch Microbiol 182:277-287 (2004)). The ACD encoded by *PAE3250* from hyperthermophilic crenarchaeon *Pyrobaculum aerophilum* showed the broadest substrate range of all characterized ACDs, reacting with acetyl-CoA, isobutyryl-CoA (preferred substrate) and phenylacetyl-CoA (Brasen et al, *supra*). Directed evolution or engineering can be used to modify this enzyme to operate at the physiological temperature of the host organism. The enzymes from *A. fulgidus, H. marismortui* and *P. aerophilum* have all been cloned, functionally expressed, and characterized in *E. coli* (Brasen and Schonheit, *supra*; Musfeldt and Schonheit, J Bacteriol. 184:636-644 (2002)). An additional candidate is succinyl-CoA synthetase, encoded by *sucCD* of *E. coli* and *LSC1* and *LSC2* genes of *Saccharomyces cerevisiae.* These enzymes catalyze the formation of succinyl-CoA from succinate with the concomitant consumption of one ATP in a reaction which is reversible *in vivo* (Buck et al., Biochemistry 24:6245-6252 (1985)). The acyl CoA ligase from *Pseudomonas putida* has been demonstrated to work on several aliphatic substrates including acetic, propionic, butyric, valeric, hexanoic, heptanoic, and octanoic acids and on aromatic compounds such as phenylacetic and phenoxyacetic acids (Fernandez-Valverde et al., Appl.Environ.Microbiol. 59:1149-1154 (1993)). A related enzyme, malonyl CoA synthetase (6.3.4.9) from *Rhizobium leguminosarum* could convert several diacids, namely, ethyl-, propyl-, allyl-, isopropyl-, dimethyl-, cyclopropyl-, cyclopropylmethylene-, cyclobutyl-, and benzyl-malonate into their corresponding monothioesters (Pohl et al., J.Am.Chem.Soc. 123:5822-5823 (2001)).

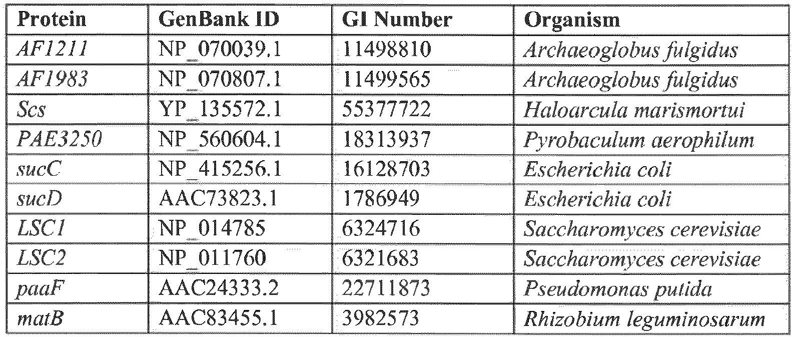

Another candidate enzyme for these steps is 6-carboxyhexanoate-CoA ligase, also known as pimeloyl-CoA ligase (EC 6.2.1.14), which naturally activates pimelate to pimcloyl-CoA during biotin biosynthesis in gram-positive bacteria. The enzyme from *Pseudomonas mendocina,* cloned into *E. coli,* was shown to accept the alternate substrates hexanedioate and nonanedioate (Binieda et al., Biochem.J 340 ( Pt 3):793-801 (1999)). Other candidates are found in *Bacillus subtilis* (Bower et al., J Bacteriol. 178:4122-4130 (1996)) and *Lysinibacillus sphaericus* (formerly *Bacillus sphaericus)* (Ploux et al., Biochem.J 287 ( Pt 3):685-690 (1992)).

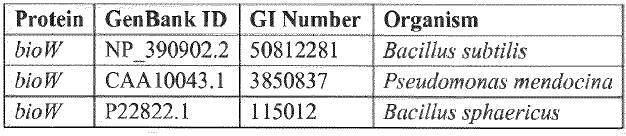

Additional CoA-ligases include the rat dicarboxylate-CoA ligase for which the sequence is yet uncharacterized (Vamecq et al., Biochem.J 230:683-693 (1985)), either of the two characterized phcnylacctatc-CoA ligases from *P. chrysogenum* (Lamas-Macciras et al., Biochem.J 395:147-155 (2006); Wang et al., 360:453-458 (2007)), the phenylacetate-CoA ligase from *Pseudomonas putida* (Martinez-Blanco et al., J Biol Chem 265:7084-7090 (1990)) and the 6-carboxyhexanoate-CoA ligase from *Bacillus subtilis* (Bower et al. J Bacteriol 178(14):4122-4130 (1996)). Acctoacctyl-CoA synthetases from *Mus musculus* (Hasegawa et al., Biochim Biophys Acta 1779:414-419 (2008)) and *Homo sapiens* (Ohgami et al., Biochem.Pharmacol. 65:989-994 (2003)) naturally catalyze the ATP-dependent conversion of acetoacetate into acetoacetyl-CoA.

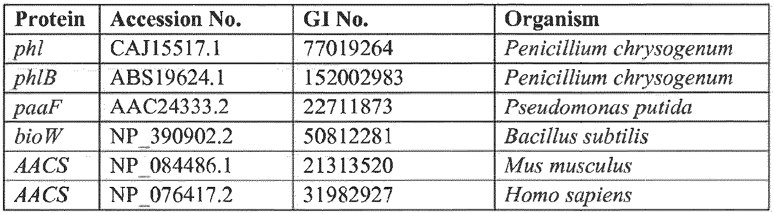

Like enzymes in other classes, certain enzymes in the EC class 6.2.1 have been determined to have broad substrate specificity. The acyl CoA ligase from *Pseudomonas putida* has been demonstrated to work on several aliphatic substrates including acetic, propionic, butyric, valeric, hexanoic, heptanoic, and octanoic acids and on aromatic compounds such as phenylacetic and phenoxyacetic acids (Fernandez-Valverde et al., Applied and Environmental Microbiology 59:1149-1154 (1993)). A related enzyme, malonyl CoA synthetase (6.3.4.9) from *Rhizobium trifolii* could convert several diacids, namely, ethyl-, propyl-, allyl-, isopropyl-, dimethyl-, cyclopropyl-, cyclopropylmethylene-, cyclobutyl-, and benzyl-malonate into their corresponding monothioesters (Pohl et al., J.Am.Chem.Soc. 123:5822-5823 (2001)).

### M) Crotonate Reductase.

A suitable enzyme activity is an 1.2.1.e Oxidoreductase (acid to aldehyde), which include the following.

The conversion of an acid to an aldehyde is thermodynamically unfavorable and typically requires energy-rich cofactors and multiple enzymatic steps. Direct conversion of the acid to aldehyde by a single enzyme is catalyzed by an acid reductase enzyme in the 1.2.1 family. Exemplary acid reductase enzymes include carboxylic acid reductase, alpha-aminoadipate reductase and retinoic acid reductase. Carboxylic acid reductase (CAR), found in *Nocardia iowensis,* catalyzes the magnesium, ATP and NADPH-dependent reduction of carboxylic acids to their corresponding aldehydes (Venkitasubramanian et al., J Biol.Chem. 282:478-485 (2007)). The natural substrate of this enzyme is benzoate and the enzyme exhibits broad acceptance of aromatic substrates including p-toluatc (Venkitasubramanian et al., Biocatalysis in Pharmaceutical and Biotechnology Industries. CRC press (2006)). The enzyme from *Nocardia iowensis,* encoded by *car,* was cloned and functionally expressed in *E*. *coli* (Venkitasubramanian et al., J Biol.Chem. 282:478-485 (2007)). CAR requires post-translational activation by a phosphopantetheine transferase (PPTase) that converts the inactive apo-enzyme to the active holo-enzyme (Hansen et al., Appl.Environ.Microbiol 75:2765-2774 (2009)). Expression of the *npt* gene, encoding a specific PPTase, product improved activity of the enzyme. An additional enzyme candidate found in *Streptomyces griseus* is encoded by the *griC* and *griD* genes. This enzyme is believed to convert 3-amino-4-hydroxybenzoic acid to 3-amino-4-hydroxybenzaldehyde as deletion of either *griC* or *griD* led to accumulation of extracellular 3-acetylamino-4-hydroxybenzoic acid, a shunt product of 3-amino-4-hydroxybenzoic acid metabolism (Suzuki, ct al., J. Antibiot. 60(6):380-387 (2007)). Co-expression of *griC* and *griD* with SGR_665, an enzyme similar in sequence to the *Nocardia iowensis npt,* can be beneficial.

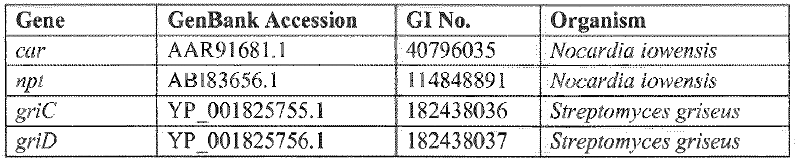

Additional *car* and *npt* genes can be identified based on sequence homology.

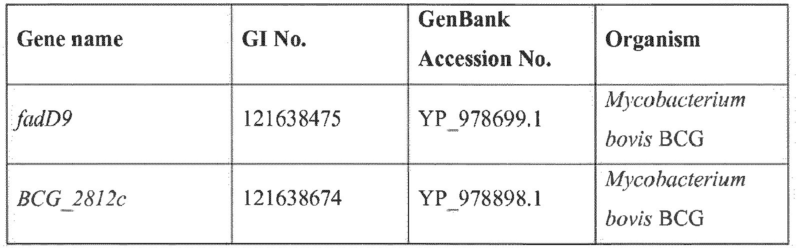

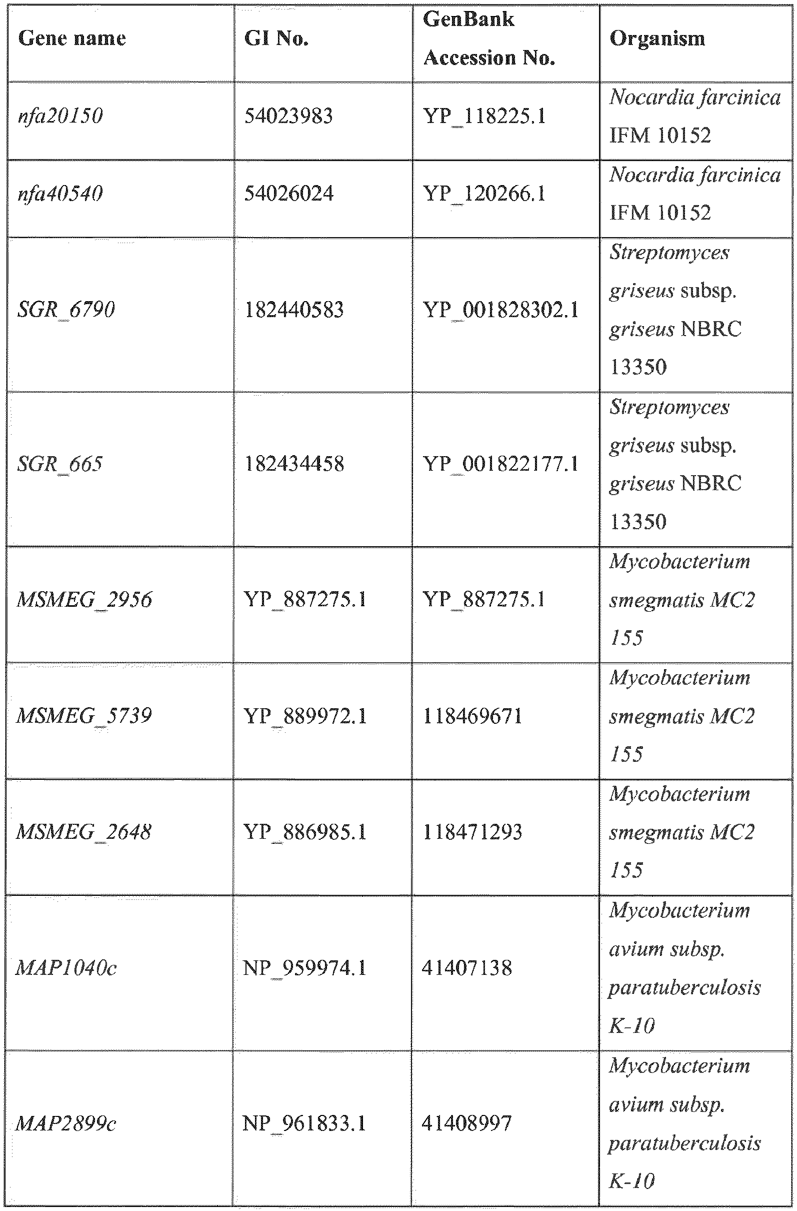

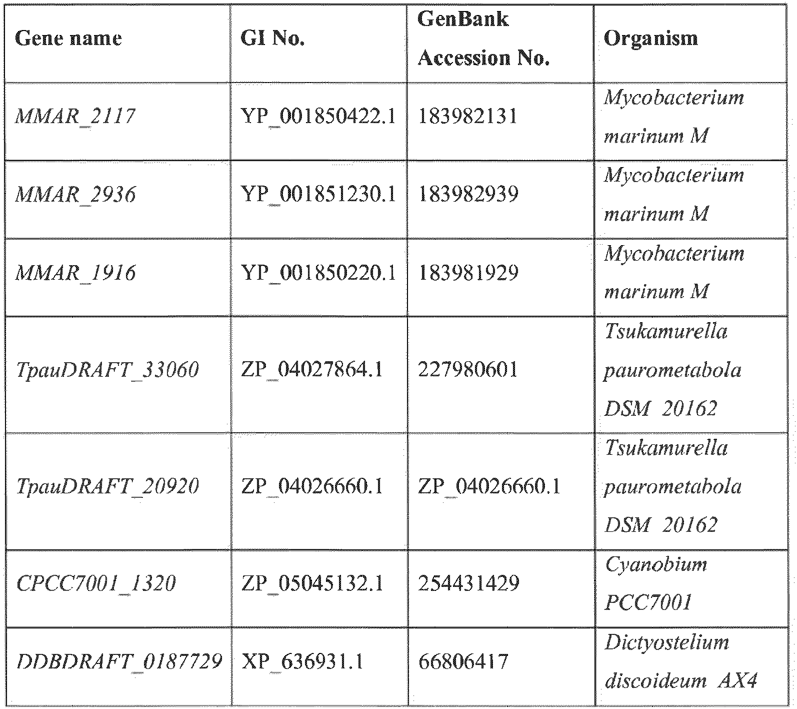

An enzyme with similar characteristics, alpha-aminoadipate reductase (AAR, EC 1.2.1.31), participates in lysine biosynthesis pathways in some fungal species. This enzyme naturally reduces alpha-aminoadipate to alpha-aminoadipate semialdehyde. The carboxyl group is first activated through the ATP-dependent formation of an adenylate that is then reduced by NAD(P)H to yield the aldehyde and AMP. Like CAR, this enzyme utilizes magnesium and requires activation by a PPTase. Enzyme candidates for AAR and its corresponding PPTase are found in *Saccharomyces cerevisiae* (Morris et al., Gene 98:141-145 (1991)), *Candida albicans* (Guo et al., Mol.Genet.Genomics 269:271-279 (2003)), and *Schizosaccharomyces pombe* (Ford et al., Curr.Genet. 28:131-137 (1995)). The AAR from *S. pombe* exhibited significant activity when expressed in *E. coli* (Guo et al., Yeast 21:1279-1288 (2004)). The AAR from *Penicillium chrysogenum* accepts S-carboxymethyl-L-cysteine as an alternate substrate, but did not react with adipate, L-glutamate or diaminopimelate (Hijarrubia et al., J Biol.Chem. 278:8250-8256 (2003)). The gene encoding the *P. chrysogenum* PPTase has not been identified to date and no high-confidence hits were identified by sequence comparison homology searching.

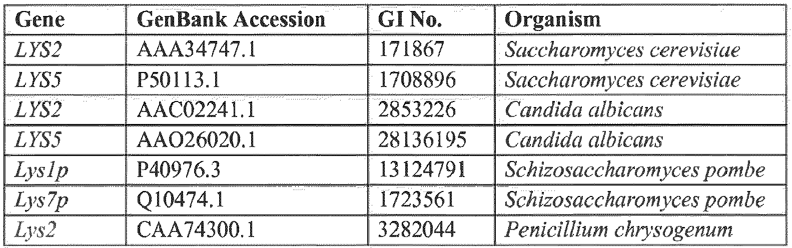

### N) Crotonaldehyde Reductase.

A suitable enzyme activity is provided by an EC 1.1.1.a Oxidoreductase (oxo to alcohol). EC 1.1.1.a Oxidoreductase (oxo to alcohol) includes the following:
The reduction of glutarate semialdehyde to 5-hydroxyvalerate by glutarate semialdehyde reductase entails reduction of an aldehyde to its corresponding alcohol. Enzymes with glutarate semialdehyde reductase activity include the ATEG_00539 gene product of *Aspergillus terreus* and 4-hydroxybutyrate dehydrogenase of *Arcibidopsis thaliana,* encoded by *4hbd* (WO 2010/068953A2). The *A. thaliana* enzyme was cloned and characterized in yeast (Breitkreuz et al., J.Biol.Chem. 278:41552-41556 (2003)).

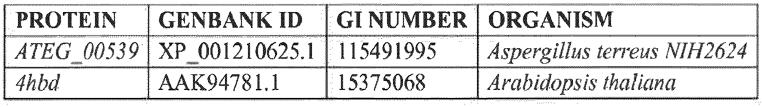

Additional genes encoding enzymes that catalyze the reduction of an aldehyde to alcohol (i.e., alcohol dehydrogenase or equivalently aldehyde reductase) include *alrA* encoding a medium-chain alcohol dehydrogenase for C2-C14 (Tani et al., Appl.Environ.Microbiol. 66:5231-5235 (2000)), *yqhD* and *fucO* from *E*. *coli* (Sulzenbacher et al., 342:489-502 (2004)), and *bdh* I and *bdh* II from *C*. *acetobutylicum* which converts butyraldehyde into butanol (Walter et al., 174:7149-7158 (1992)). YqhD catalyzes the reduction of a wide range of aldehydes using NADPH as the cofactor, with a preference for chain lengths longer than C(3) (Sulzenbacher et al., 342:489-502 (2004);Perez et al., J Biol.Chem. 283:7346-7353 (2008)). The *adhA* gene product from *Zymomonas mobilisE* has been demonstrated to have activity on a number of aldehydes including formaldehyde, acetaldehyde, propionaldehyde, butyraldehyde, and acrolein (Kinoshita et al., Appl Microbiol Biotechnol 22:249-254 (1985)). Additional aldehyde reductase candidates are encoded by *bdh* in *C*. *saccharoperbutylacetonicum* and *Cbei_1722, Cbei_2181* and *Cbei_2421* in *C*. *Beijerinckii.* Additional aldehyde reductase gene candidates in *Saccharomyces cerevisiae* include the aldehyde reductases GRE3, ALD2-6 and HFD1, glyoxylate reductases GOR1 and YPL113C and glycerol dehydrogenase GCYl (WO 2011/022651A1; Atsumi et al., Nature 451:86-89 (2008)). The enzyme candidates described previously for catalyzing the reduction of methylglyoxal to acetol or lactaldehyde are also suitable lactaldehyde reductase enzyme candidates.

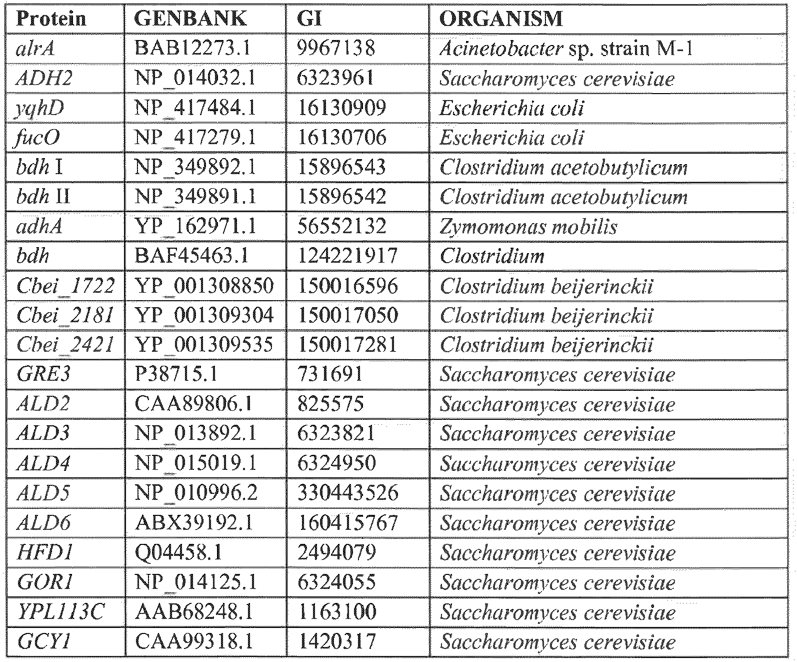

Enzymes exhibiting 4-hydroxybutyrate dehydrogenase activity (EC 1.1.1.61) also fall into this category. Such enzymes have been characterized in *Ralstonia eutropha* (Bravo et al., J Forens Sci, 49:379-387 (2004)) and *Clostridium kluyveri* (Wolff et al., Protein Expr.Purif. 6:206-212 (1995)). Yet another gene is the alcohol dehydrogenase *adhI* from *Geobacillus thermoglucosidasius* (Jeon et al., J Biotechnol 135:127-133 (2008)).

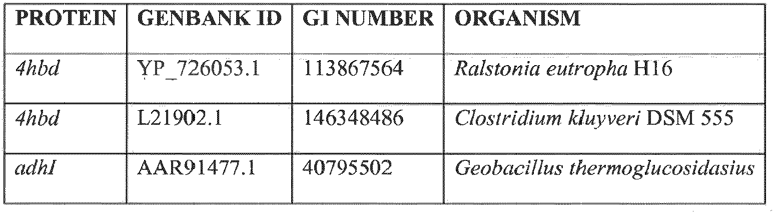

Another exemplary aldehyde reductase is methylmalonate semialdehyde reductase, also known as 3-hydroxyisobutyrate dehydrogenase (EC 1.1.1.31). This enzyme participates in valine, leucine and isoleucine degradation and has been identified in bacteria, eukaryotes, and mammals. The enzyme encoded by *P84067* from *Thermus thermophilus* HB8 has been structurally characterized (Lokanath et al., J Mol Biol, 352:905-17 (2005)). The reversibility of the human 3-hydroxyisobutyrate dehydrogenase was demonstrated using isotopically-labeled substrate (Manning et al., Biochem J, 231:481-4 (1985)). Additional genes encoding this enzyme include *3hidh* in *Homo sapiens* (Hawes et al., Methods Enzymol, 324:218-228 (2000)) and *Oryctolagus cuniculus* (Hawes et al., *supra*; Chowdhury et al., Biosci.Biotechnol Biochem. 60:2043-2047 (1996)), *mmsB* in *Pseudomonas aeruginosa* and *Pseudomonas putida,* and *dhat* in *Pseudomonas putida* (Aberhart et al., J Chem. Soc. [Perkin 1] 6:1404-1406 (1979); Chowdhury et al., Biosci.Biotechnol Biochem. 60:2043-2047 (1996); Chowdhury et al., Biosci.Biotechnol Biochem. 67:438-441 (2003)). Several 3-hydroxyisobutyrate dehydrogenase enzymes have been characterized in the reductive direction, including *mmsB* from *Pseudomonas aeruginosa* (Gokarn et al., US Patent 739676, (2008)) and *mmsB* from *Pseudomonas putida.*

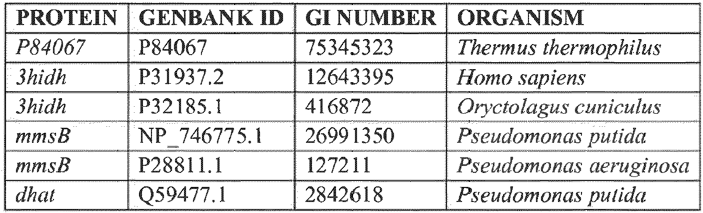

There exist several exemplary alcohol dehydrogenases that convert a ketone to a hydroxyl functional group. Two such enzymes from *E. coli* are encoded by malate dehydrogenase (*mdh*) and lactate dehydrogenase (*ldhA*)*.* In addition, lactate dehydrogenase from *Ralstonia eutropha* has been shown to demonstrate high activities on 2-kctoacids of various chain lengths including lactate, 2-oxobutyrate, 2-oxopentanoate and 2-oxoglutarate (Steinbuchel et al., Eur.J.Biochem. 130:329-334 (1983)). Conversion of alpha-ketoadipate into alpha-hydroxyadipate can be catalyzed by 2-ketoadipate reductase, an enzyme reported to be found in rat and in human placenta (Suda et al., Arch.Biochem.Biophys. 176:610-620 (1976); Suda et al., Biochem.Biophys.Res.Commun. 77:586-591 (1977)). An additional oxidoreductase is the mitochondrial 3-hydroxybutyrate dehydrogenase (*bdh*) from the human heart which has been cloned and characterized (Marks et al., J.Biol.Chem. 267:15459-15463 (1992)). Alcohol dehydrogenase enzymes of*C*. *beijerinckii* (Ismaiel et al., J.Bacteriol. 175:5097-5105 (1993)) and *T. brockii* (Lamed et al., Biochem.J. 195:183-190 (1981); Peretz et al., Biochemistry. 28:6549-6555 (1989)) convert acetone to isopropanol. Methyl ethyl ketone reductase catalyzes the reduction of MEK to 2-butanol. Exemplary MEK reductase enzymes can be found in *Rhodococcus ruber* (Kosjek et al., Biotechnol Bioeng. 86:55-62 (2004)) and *Pyrococcus furiosus* (van der Oost et al., Eur.J.Biochem. 268:3062-3068 (2001)).

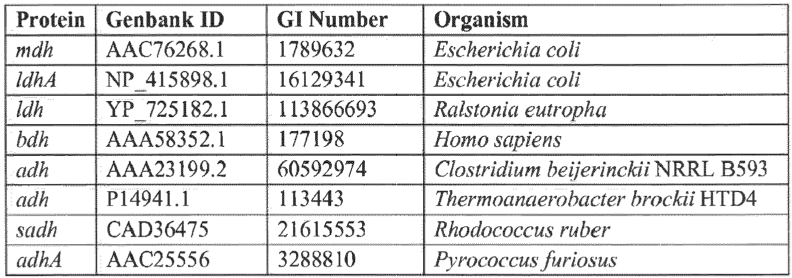

A number of organisms encode genes that catalyze the reduction of 3-oxobutanol to 1,3-butanediol, including those belonging to the genus *Bacillus, Brevibacterium, Candida,* and *Klebsiella* among others, as described by Matsuyama et al. J Mol Cat B Enz, 11:513-521 (2001). One of these enzymes, SADH from *Candida parapsilosis,* was cloned and characterized in *E. coli.* A mutated *Rhodococcus* phenylacetaldehyde reductase (Sar268) and a *Leifonia* alcohol dehydrogenase have also been shown to catalyze this transformation at high yields (Itoh et al., Appl.Microbiol Biotechnol. 75:1249-1256 (2007)).

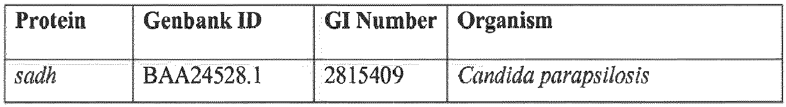

### O) Crotyl Alcohol Kinase.

Crotyl alcohol kinase enzymes catalyze the transfer of a phosphate group to the hydroxyl group of crotyl alcohol. The enzymes described below naturally possess such activity or can be engineered to exhibit this activity. Kinases that catalyze transfer of a phosphate group to an alcohol group are members of the EC 2.7.1 enzyme class. The table below lists several useful kinase enzymes in the EC 2.7.1 enzyme class.

| Enzyme Commission Number | Enzyme Name | Enzyme Commission Number | Enzyme Name | Enzyme Commission Number | Enzyme Name |
|---|---|---|---|---|---|
| 2.7.1.1 | hexokinase | 2.7.1.48 | uridine kinase | 2.7.1.94 | acylglycerol kinase |
| 2.7.1.2 | glucokinase | 2.7.1.49 | hydroxymethylpyrimidine kinase | 2.7.1.95 | kanamycin kinase |
| 2.7.1.3 | kctohcxokinasc | 2.7.1.50 | hydroxycthylthiazolc kinase | 2.7.1.100 | S-methyl-5-thioribose kinase |
| 2.7.1.4 | fructokinasc | 2.7.1.51 | L-fuculokinasc | 2.7.1.101 | tagatose kinase |
| 2.7.1.5 | rhamnulokinase | 2.7.1.52 | fucokinase | 2.7.1.102 | hamamelose kinase |
| 2.7.1.6 | galactokinase | 2.7.1.53 | L-xylulokinase | 2.7.1.103 | viomycin kinase |
| 2.7.1.7 | mannokinase | 2.7.1.54 | D-arabinokinase | 2.7.1.105 | 6-phosphofructo-2-kinase |
| 2.7.1.8 | glucosamine kinase | 2.7.1.55 | allose kinase | 2.7.1.106 | glucose-1,6-bisphosphate synthase |
| 2.7.1.10 | phospho glucokinase | 2.7.1.56 | 1-phosphofructokinase | 2.7.1.107 | diacylglycerol kinase |
| 2.7.1.11 | 6-phosphofructokinase | 2.7.1.58 | 2-dehydro-3-deoxygalactonokinase | 2.7.1.108 | dolichol kinase |
| 2.7.1.12 | gluconokinase | 2.7.1.59 | N-acetylglucosamine kinase | 2.7.1.113 | deoxyguanosine kinase |
| 2.7.1.13 | dehydrogluconokinase | 2.7.1.60 | N-acylmannosamine kinase | 2.7.1.114 | AMP-thymidine kinase |
| 2.7.1.14 | sedoheptulokinase | 2.7.1.61 | acyl-phosphate-hexose phosphotransferase | 2.7.1.118 | ADP-thymidine kinase |
| 2.7.1.15 | ribokinase | 2.7.1.62 | phosphoramidate-hexose phosphotransferase | 2.7.1.119 | hygromycin-B 7"-O-kinase |

| Enzyme Commission Number | Enzyme Name | Enzyme Commission Number | Enzyme Name | Enzyme Commission Number | Enzyme Name |
|---|---|---|---|---|---|
| 2.7.1.16 | ribulokinase | 2.7.1.63 | polyphosphate-glucose phosphotransferase | 2.7.1.121 | phosphoenolpyruvate-glycerone phosphotransferase |
| 2.7.1.17 | xylulokinasc | 2.7.1.64 | inositol 3-kinasc | 2.7.1.122 | xylitol kinase |
| 2.7.1.18 | phosphoribokinase | 2.7.1.65 | scyllo-inosamine 4-kinase | 2.7.1.127 | inositol-trisphosphate 3-kinase |
| 2.7.1.19 | phosphoribulokinase | 2.7.1.66 | undecaprenol kinase | 2.7.1.130 | tetraacyldisaccharide 4'-kinase |
| 2.7.1.20 | adenosine kinase | 2.7.1.67 | 1-phosphatidylinositol 4-kinase | 2.7.1.134 | inositol-tetrakisphosphate 1-kinase |
| 2.7.1.21 | thymidine kinase | 2.7.1.68 | 1-phosphatidylinositol-4-phosphate 5-kinase | 2.7.1.136 | macrolide 2'-kinase |
| 2.7.1.22 | ribosylnicotinamide kinase | 2.7.1.69 | protein-Np-phosphohistidine-sugar phosphotransferase | 2.7.1.137 | phosphatidylinositol 3-kinase |
| 2.7.1.23 | NAD+ kinase | 2.7.1.70 | identical to EC 2.7.1.37. | 2.7.1.138 | ceramide kinase |
| 2.7.1.24 | dephospho-CoA kinase | 2.7.1.71 | shikimate kinase | 2.7.1.140 | inositol-tetrakisphosphate 5-kinase |
| 2.7.1.25 | adcnylyl-sulfatc kinase | 2.7.1.72 | streptomycin 6-kinase | 2.7.1.142 | glycerol-3-phosphate-glucose phosphotransferase |

| Enzyme Commission Number | Enzyme Name | Enzyme Commission Number | Enzyme Name | Enzyme Commission Number | Enzyme Name |
|---|---|---|---|---|---|
| 2.7.1.26 | riboflavin kinase | 2.7.1.73 | inosine kinase | 2.7.1.143 | diphosphate-purine nucleoside kinase |
| 2.7.1.27 | erythritol kinase | 2.7.1.74 | deoxycytidine kinase | 2.7.1.144 | tagatose-6-phosphate kinase |
| 2.7.1.28 | triokinase | 2.7.1.76 | deoxyadcnosinc kinase | 2.7.1.145 | deoxynucleoside kinase |
| 2.7.1.29 | glycerone kinase | 2.7.1.77 | nucleoside phosphotransferase | 2.7.1.146 | ADP-dependent phosphofructokinase |
| 2.7.1.30 | glycerol kinase | 2.7.1.78 | polynucleotide 5'-hydroxyl-kinase | 2.7.1.147 | ADP-dependent glucokinase |
| 2.7.1.31 | glycerate kinase | 2.7.1.79 | diphosphate-glycerol phosphotransferase | 2.7.1.148 | 4-(cytidine 5'-diphospho)-2-C-methyl-D-erythritol kinase |
| 2.7.1.32 | choline kinase | 2.7.1.80 | diphosphate-serine phosphotransferase | 2.7.1.149 | 1-phosphatidylinositol-5-phosphate 4-kinase |
| 2.7.1.33 | pantothenate kinase | 2.7.1.81 | hydroxylysine kinase | 2.7.1.150 | 1-phosphatidylinositol-3-phosphate 5-kinase |
| 2.7.1.34 | pantetheine kinase | 2.7.1.82 | ethanolamine kinase | 2.7.1.151 | inositol-polyphosphate multikinase |
| 2.7.1.35 | pyridoxal kinase | 2.7.1.83 | pseudouridinc kinase | 2.7.1.153 | phosphatidylinositol-4,5-bisphosphate 3-kinasc |

| Enzyme Commission Number | Enzyme Name | Enzyme Commission Number | Enzyme Name | Enzyme Commission Number | Enzyme Name |
|---|---|---|---|---|---|
| 2.7.1.36 | mevalonate kinase | 2.7.1.84 | alkylglyccronc kinase | 2.7.1.154 | phosphatidylinositol-4-phosphate 3-kinase |
| 2.7.1.39 | homoserine kinase | 2.7.1.85 | β-glucoside kinase | 2.7.1.156 | adenosylcobinamide kinase |
| 2.7.1.40 | pyruvate kinase | 2.7.1.86 | NADH kinase | 2.7.1.157 | N-acetylgalactosamine kinase |
| 2.7.1.41 | glucose-1-phosphate phosphodismutase | 2.7.1.87 | streptomycin 3"-kinase | 2.7.1.158 | inositol-pentakisphosphate 2-kinase |
| 2.7.1.42 | riboflavin phosphotransferase | 2.7.1.88 | dihydrostreptomycin-6-phosphate 3'a-kinase | 2.7.1.159 | inositol-1,3,4-trisphosphate 5/6-kinase |
| 2.7.1.43 | glucuronokinase | 2.7.1.89 | thiamine kinase | 2.7.1.160 | 2'-phosphotransferase |
| 2.7.1.44 | galacturonokinase | 2.7.1.90 | diphosphate-fructose-6-phosphate 1-phosphotransferase | 2.7.1.161 | CTP-dependent riboflavin kinase |
| 2.7.1.45 | 2-dehydro-3-deoxygluconokinase | 2.7.1.91 | sphinganine kinase | 2.7.1.162 | N-acetylhexosamine 1-kinase |
| 2.7.1.46 | L-arabinokinase | 2.7.1.92 | 5-dehydro-2-deoxygluconokinase | 2.7.1.163 | hygromycin B 4-O-kinase |
| 2.7.1.47 | D-ribulokinase | 2.7.1.93 | alkylglycerol kinase | 2.7.1.164 | O-phosphoseryl-tRNASec kinase |

Mevalonate kinase (EC 2.7.1.36) phosphorylates the terminal hydroxyl group of mevalonate. Gene candidates for this step include erg12 from S. *cerevisiae, mvk* from *Methanocaldococcus jannaschi, MVK* from *Homo sapeins,* and *mvk* from *Arabidopsis thaliana col.* Additional mevalonate kinase candidates include the feedback-resistant mevalonate kinase from the archcon *Methanosarcina mazei* (Primak et al, AEM, in press (2011)) and the Mvk protein from *Streptococcus pneumoniae* (Andreassi et al, Protein Sci, 16:983-9 (2007)). Mvk proteins from *S. cerevisiae, S. pneumoniae* and *M*. *mazei* were heterologously expressed and characterized in *E. coli* (Primak et al, *supra*)*.* The *S*. *pneumoniae* mevalonate kinase was active on several alternate substrates including cylopropylmevalonate, vinylmevalonate and ethynylmevalonate (Kudoh et al, Bioorg Med Chem 18:1124-34 (2010)), and a subsequent study determined that the ligand binding site is selective for compact, electron-rich C(3)-substituents (Lefurgy et al, J Biol Chem 285:20654-63 (2010)).

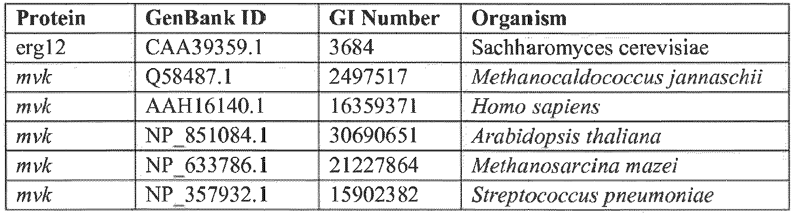

Glycerol kinase also phosphorylates the terminal hydroxyl group in glycerol to form glycerol-3-phosphate. This reaction occurs in several species, including *Escherichia coli, Saccharomyces cerevisiae,* and *Thermotoga maritima.* The *E. coli* glycerol kinase has been shown to accept alternate substrates such as dihydroxyacetone and glyceraldehyde (Hayashi et al., J Biol.Chem. 242:1030-1035 (1967)). T, maritime has two glycerol kinases (Nelson et al., Nature 399:323-329 (1999)). Glycerol kinases have been shown to have a wide range of substrate specificity. Crans and Whiteside studied glycerol kinases from four different organisms (*Escherichia coli, S. cerevisiae, Bacillus stearothermophilus,* and *Candida mycoderma*) (Crans et al., J.Am.Chem.Soc. 107:7008-7018 (2010); Nelson et al., *supra,* (1999)). They studied 66 different analogs of glycerol and concluded that the enzyme could accept a range of substituents in place of one terminal hydroxyl group and that the hydrogen atom at C2 could be replaced by a methyl group. Interestingly, the kinetic constants of the enzyme from all four organisms were very similar.

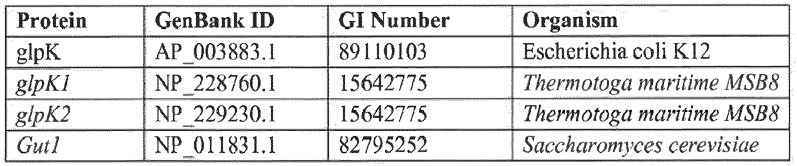

Homoserine kinase is another possible candidate. This enzyme is also present in a number of organisms including *E. coli, Streptomyces sp,* and *S*. *cerevisiae.* Homoserine kinase from *E. coli* has been shown to have activity on numerous substrates, including, L-2-amino,1,4- butanediol, aspartate semialdehyde, and 2-amino-5-hydroxyvalerate (Huo et al., Biochemistry 35:16180-16185 (1996); Huo et al., Arch.Biochem.Biophys. 330:373-379 (1996)). This enzyme can act on substrates where the carboxyl group at the alpha position has been replaced by an ester or by a hydroxymethyl group. The gene candidates are:

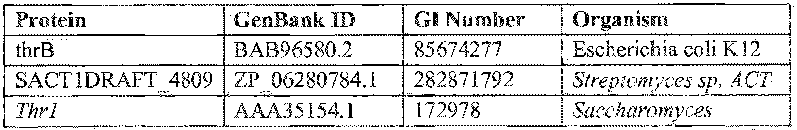

### P) 2-Butenyl-4-phosphate Kinase.

2-Butenyl-4-phosphate kinase enzymes catalyze the transfer of a phosphate group to the phosphate group of 2-butenyl-4-phosphate. The enzymes described below naturally possess such activity or can be engineered to exhibit this activity. Kinases that catalyze transfer of a phosphate group to another phosphate group are members of the EC 2.7.4 enzyme class. The table below lists several useful kinase enzymes in the EC 2.7.4 enzyme class.

| **Enzyme Commission Number** | **Enzyme Name** |
|---|---|
| 2.7.4.1 | polyphosphate kinase |
| 2.7.4.2 | phosphomevalonate kinase |
| 2.7.4.3 | adenylate kinase |

| **Enzyme Commission Number** | **Enzyme Name** |
|---|---|
| 2.7.4.4 | nucleoside-phosphate kinase |
| 2.7.4.6 | nucleoside-diphosphate kinase |
| 2.7.4.7 | phosphomethylpyrimidine kinase |
| 2.7.4.8 | guanylate kinase |
| 2.7.4.9 | dTMP kinase |
| 2.7.4.10 | nucleoside-triphosphate-adenylate kinase |
| 2.7.4.11 | (deoxy)adenylate kinase |
| 2.7.4.12 | T2-induced deoxynucleotide kinase |
| 2.7.4.13 | (deoxy)nucleoside-phosphate kinase |
| 2.7.4.14 | cytidylate kinase |
| 2.7.4.15 | thiamine-diphosphate kinase |
| 2.7.4.16 | thiamine-phosphate kinase |
| 2.7.4.17 | 3-phosphoglyceroyl-phosphate-polyphosphate phosphotransferase |
| 2.7.4.18 | farnesyl-diphosphate kinase |
| 2.7.4.19 | 5-methyldeoxycytidine-5'-phosphate kinase |
| 2.7.4.20 | dolichyl-diphosphate-polyphosphate phosphotransferase |
| 2.7.4.21 | inositol-hexakisphosphate kinase |
| 2.7.4.22 | UMP kinase |
| 2.7.4.23 | ribose 1,5-bisphosphate phosphokinase |
| 2.7.4.24 | diphosphoinositol-pentakisphosphate kinase |
| 2.7.4.- | Farnesyl monophosphate kinase |

| **Enzyme Commission Number** | **Enzyme Name** |
|---|---|
| 2.7.4.- | Geranyl-geranyl monophosphate kinase |
| 2.7.4.- | Phytyl-phosphate kinase |

Phosphomevalonate kinase enzymes are of particular interest. Phosphomevalonate kinase (EC 2.7.4.2) catalyzes the analogous transformation to 2-butenyl-4-phosphate kinase. This enzyme is encoded by *erg8* in *Saccharomyces cerevisiae* (Tsay et al., Mol.Cell Biol. 11:620-631 (1991)) and *mvaK2* in *Streptococcus pneumoniae. Staphylococcus aureus* and *Enterococcus faecalis* (Doun et al., Protein Sci. 14:1134-1139 (2005); Wilding et al., J Bacteriol. 182:4319-4327 (2000)). The *Streptococcus pneumoniae* and *Enterococcus faecalis* enzymes were cloned and characterized in *E. coli* (Pilloff et al., J Biol.Chem. 278:4510-4515 (2003); Doun et al., Protein Sci. 14:1134-1139 (2005)). The *S*. *pneumoniae* phosphomevalonate kinase was active on several alternate substrates including cylopropylmevalonate phosphate, vinylmevalonate phosphate and ethynylmevalonate phosphate (Kudoh et al, Bioorg Med Chem 18:1124-34 (2010)).

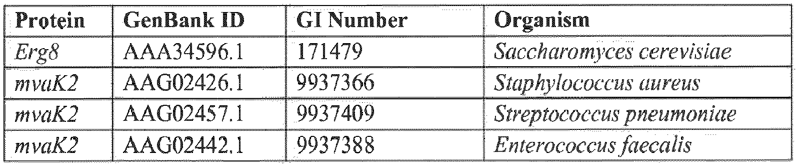

Farnesyl monophosphate kinase enzymes catalyze the CTP dependent phosphorylation of farnesyl monophosphate to farnesyl diphosphate. Similarly, geranylgeranyl phosphate kinase catalyzes CTP dependent phosphorylation. Enzymes with these activities were identified in the microsomal fraction of cultured *Nicotiana tabacum* (Thai et al, PNAS 96:13080-5 (1999)). However, the associated genes have not been identified to date.

### Q) Butadiene Synthase.

Butadiene synthase catalyzes the conversion of 2-butenyl-4-diphosphate to 1,3-butadicnc. The enzymes described below naturally possess such activity or can be engineered to exhibit this activity. Carbon-oxygen lyases that operate on phosphates are found in the EC 4.2.3 enzyme class. The table below lists several useful enzymes in EC class 4.2.3.

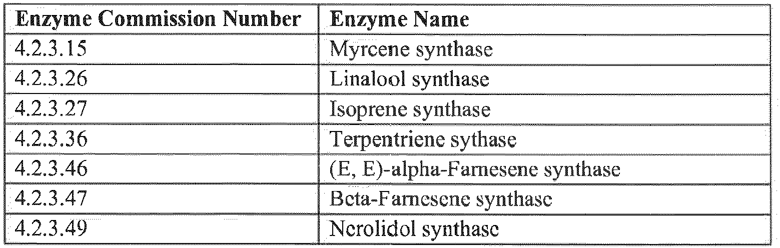

Particularly useful enzymes include isoprene synthase, myrcene synthase and farnesene synthase. Enzyme candidates are described below.

Isoprene synthase naturally catalyzes the conversion of dimethylallyl diphosphate to isoprene, but can also catalyze the synthesis of 1,3-butadiene from 2-butenyl-4-diphosphate. Isoprene synthases can be found in several organisms including *Populus alba* (Sasaki et al., FEBS Letters, 2005, 579 (11), 2514-2518), *Pueraria montana* (Lindberg et al., Metabolic Eng, 12(1):70-79 (2010); Sharkey et al., Plant Physiol., 137(2):700-712 (2005)), and *Populus tremula* x *Populus alba,* also called *Populus canescens* (Miller et al., Planta, 2001, 213 (3), 483-487). The crystal structure of the *Populus canescens* isoprene synthase was determined (Koksal et al, J Mol Biol 402:363-373 (2010)). Additional isoprene synthase enzymes are described in (Chotani et al., WO/2010/031079, Systems Using Cell Culture for Production of Isoprene; Cervin et al., US Patent Application 20100003716, Isoprene Synthase Variants for Improved Microbial Production of Isoprene).

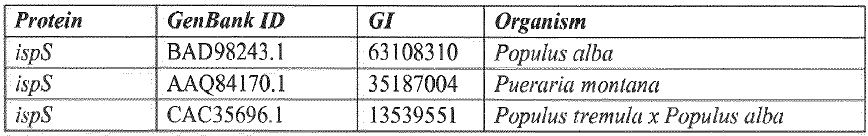

Myrcene synthase enzymes catalyze the dephosphorylation of geranyl diphosphate to beta-myrcene (EC 4.2.3.15). Exemplary myrcene synthases are encoded by MST2 of *Solanum lycopersicum* (van Schic et al, Plant Mol Biol 64:D473-79 (2007)), TPS-Myr of *Picea abies* (Martin et al, Plant Physiol 135:1908-27 (2004)) g-myr of *Abies grandis* (Bohlmann et al, J Biol Chem 272:21784-92 (1997)) and TPS10 of *Arabidopsis thaliana* (Bohlmann et al, Arch Biochem Biophys 375:261-9 (2000)). These enzymes were heterologously expressed in *E*. *coli.*

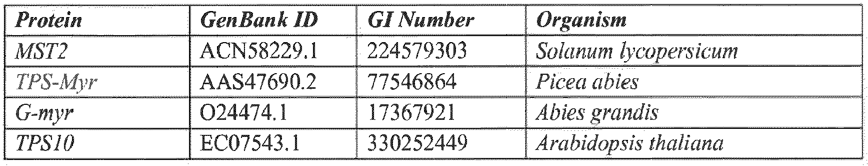

Farnesyl diphosphate is converted to alpha-farnesene and beta-farnesene by alpha-farnesene synthase and beta-farnesene synthase, respectively. Exemplary alpha-farnesene synthase enzymes include *TPS03* and *TPS02* of Arabidopsis thaliana (Faldt et al, Planta 216:745-51 (2003); Huang et al, Plant Physiol 153:1293-310 (2010)), *afs* of *Cucumis sativus* (Mercke et al, Plant Physiol 135:2012-14 (2004), *eafar* of *Malus x domestica* (Green et al, Phytochem 68: 176-88 (2007)) and TPS-Far of Picea abies (Martin, *supra*). An exemplary beta-farnesene synthase enzyme is encoded by *TPS1* of *Zea mays* (Schnee et al, Plant Physiol 130:2049-60 (2002)).

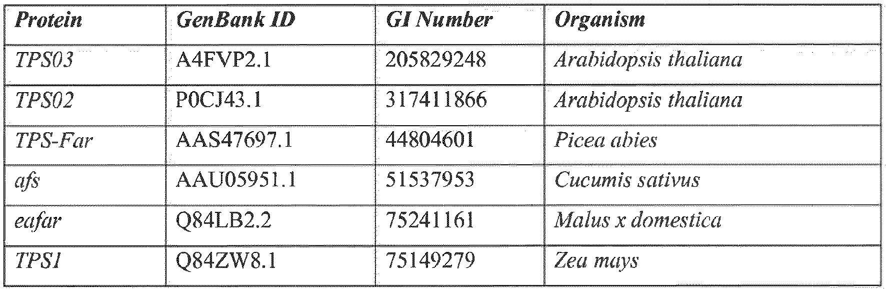

### R) Crotyl Alcohol Diphosphokinase.

Crotyl alcohol diphosphokinase enzymes catalyze the transfer of a diphosphate group to the hydroxyl group of crotyl alcohol. The enzymes described below naturally possess such activity or can be engineered to exhibit this activity. Kinases that catalyze transfer of a diphosphate group are members of the EC 2.7.6 enzyme class. The table below lists several useful kinase enzymes in the EC 2.7.6 enzyme class.

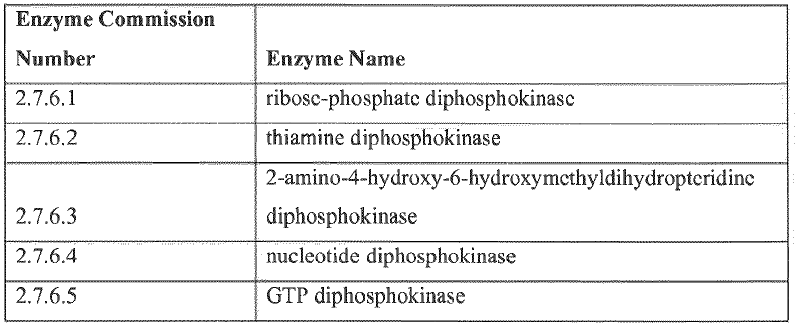

Of particular interest are ribose-phosphate diphosphokinase enzymes which have been identified in *Escherichia coli* (Hove-Jenson et al., J Biol Chem, 1986, 261(15);6765-71) and *Mycoplasma pneumoniae* M129 (McElwain et al, International Journal of Systematic Bacteriology, 1988, 38:417-423) as well as thiamine diphosphokinase enzymes. Exemplary thiamine diphosphokinase enzymes are found in *Arabidopsis thaliana* (Ajjawi, Plant Mol Biol, 2007, 65(1-2);151-62).

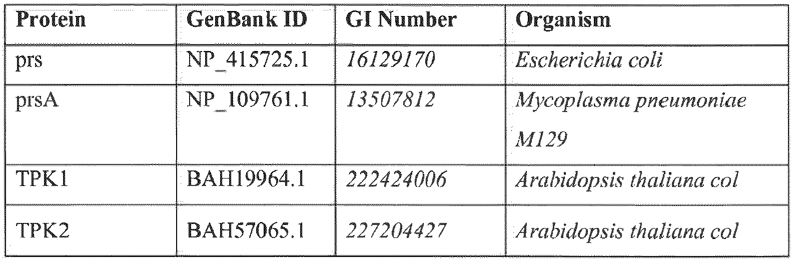

### S) Chemical Dehydration or Crotyl Alcohol Dehydratase.

Converting crotyl alcohol to butadiene using a crotyl alcohol dehydratase can include combining the activities of the enzymatic isomerization of crotyl alcohol to 3-buten-2-ol then dehydration of 3-buten-2-ol to butadiene. An exemplary bifunctional enzyme with isomerase and dehydratase activities is the linalool dehydratase/isomerase of *Castellaniella defragrans.* This enzyme catalyzes the isomerization of geraniol to linalool and the dehydration of linalool to myrcene, reactants similar in structure to crotyl alcohol, 3-buten-2-ol and butadiene (Brodkorb et al, J Biol Chem 285:30436-42 (2010)). Enzyme accession numbers and homologs are listed in the table below.

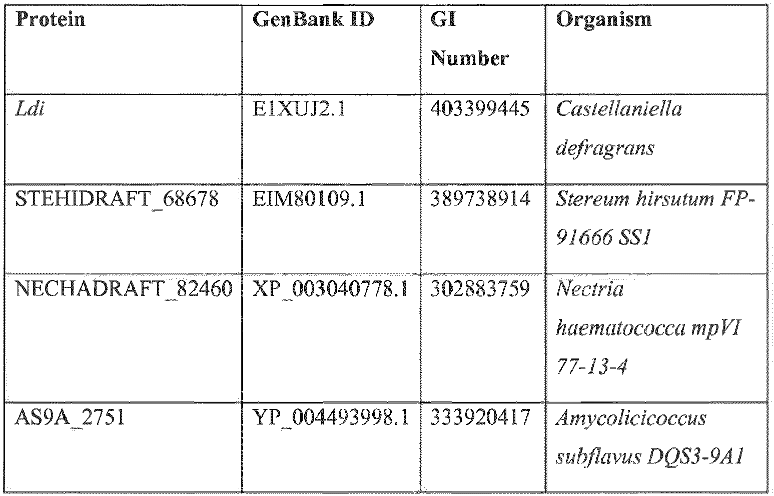

Alternatively, a fusion protein or protein conjugate can be generated using well know methods in the art to generate a bi-functional (dual-functional) enzyme having both the isomerase and dehydratase activities. The fusion protein or protein conjugate can include at least the active domains of the enzymes (or respective genes) of the isomerase and dehydratase reactions. For the first step, the conversion of crotyl alcohol to 3-buten-2-ol, enzymatic conversion can be catalyzed by a crotyl alcohol isomerase (classified as EC 5.4.4). A similar isomerization, the conversion of 2-methyl-3-buten-2-ol to 3-methyl-2-buten-1-ol, is catalyzed by cell extracts of Pseudomonas putida MB-1 (Malone et al, AEM 65 (6): 2622-30 (1999)). The extract may be used in vitro, or the protein or gene(s) associated with the isomerase activity can be isolated and used, even though they have not been identified to date. Alternatively, either or both steps can be done by chemical conversion, or by enzymatic conversion (*in vivo* or *in vitro*)*,* or any combination. Enzymes having the desired activity for the conversion of 3-buten-2-ol to butadiene are provided elsewhere herein.

### T) Butadiene Synthase (monophosphate).

Butadiene synthase (monophosphate) catalyzes the conversion of 2-butenyl-4-phosphate to 1,3-butadiene. Butadiene synthase enzymes are of the EC 4.2.3 enzyme class as described herein that possess such activity or can be engineered to exhibit this activity. Diphosphate lyase enzymes catalyze the conversion of alkyl diphosphates to alkenes. Carbon-oxygen lyases that operate on phosphates are found in the EC 4.2.3 enzyme class. The table below lists several useful enzymes in EC class 4.2.3. Exemplary enzyme candidates are also phosphate lyases.

| **Enzyme Commission No.** | **Enzyme Name** |
|---|---|
| 4.2.3.5 | Chorismate synthase |
| 4.2.3.15 | Myrcene synthase |
| 4.2.3.27 | Isoprene synthase |
| 4.2.3.36 | Terpentriene sythase |
| 4.2.3.46 | (E, E)-alpha-Farnesene synthase |
| 4.2.3.47 | Beta-Farnesene synthase |

Phosphate lyase enzymes catalyze the conversion of alkyl phosphates to alkenes. Carbon-oxygen lyases that operate on phosphates are found in the EC 4.2.3 enzyme class. The table below lists several relevant enzymes in EC class 4.2.3.

| **Enzyme Commission Number** | **Enzyme Name** |
|---|---|
| 4.2.3.5 | Chorismate synthase |
| 4.2.3.15 | Myrcene synthase |
| 4.2.3.26 | Linalool synthase |
| 4.2.3.27 | Isoprene synthase |
| 4.2.3.36 | Terpentriene sythase |
| 4.2.3.46 | (E, E)-alpha-Farnesene synthase |
| 4.2.3.47 | Beta-Farnesene synthase |
| 4.2.3.49 | Nerolidol synthase |
| 4.2.3.- | Methylbutenol synthase |

Isoprene synthase enzymes catalyzes the conversion of dimethylallyl diphosphate to isoprene. Isoprene synthases can be found in several organisms including *Populus alba* (Sasaki et al., FEBS Letters, 2005, 579 (11), 2514-2518), *Pueraria montana* (Lindberg et al., Metabolic Eng, 12(1):70-79 (2010); Sharkey et al., Plant Physiol., 137(2):700-712 (2005)), and *Populus tremula* x *Populus alba,* also called *Populus canescens* (Miller et al., Planta, 2001, 213 (3), 483-487). The crystal structure of the *Populus canescens* isoprene synthase was determined (Koksal et al, J Mol Biol 402:363-373 (2010)). Additional isoprene synthase enzymes are described in (Chotani et al., WO/2010/031079, Systems Using Cell Culture for Production of Isoprene; Cervin et al., US Patent Application 20100003716, Isoprene Synthase Variants for Improved Microbial Production of Isoprene). Another isoprene synthase-like enzyme from *Pinus sabiniana,* methylbutenol synthase, catalyzes the formation of 2-methyl-3-buten-2-ol (Grey et al, J Biol Chem 286: 20582-90 (2011)).

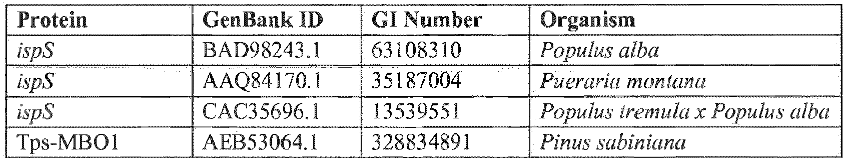

Chorismate synthase (EC 4.2.3.5) participates in the shikimate pathway, catalyzing the dephosphorylation of 5-enolpyruvylshikimate-3-phosphate to chorismate. The enzyme requires reduced flavin mononucleotide (FMN) as a cofactor, although the net reaction of the enzyme does not involve a redox change. In contrast to the enzyme found in plants and bacteria, the chorismate synthase in fungi is also able to reduce FMN at the expense of NADPH (Macheroux et al., Planta 207:325-334 (1999)). Representative monofunctional enzymes are encoded by *aroC* of *E. coli* (White et al., Biochem. J. 251:313-322 (1988)) and *Streptococcus pneumoniae* (Maclean and Ali, Structure 1 1:1499-1511 (2003)). Bifunctional fungal enzymes are found in *Neurospora crassa* (Kitzing et al., J. Biol. Chem. 276:42658-42666 (2001)) and *Saccharomyces cerevisiae* (Jones et al., Mol. Microbiol. 5:2143-2152 (1991)).

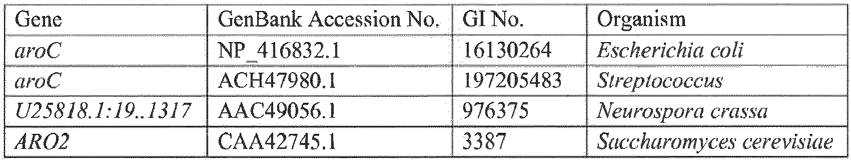

Myrcene synthase enzymes catalyze the dephosphorylation of geranyl diphosphate to beta-myrcene (EC 4.2.3.15). Exemplary myrcene synthases are encoded by MST2 of *Solanum lycopersicum* (van Schie et al, Plant Mol Biol 64:D473-79 (2007)), TPS-Myr of *Picea abies* (Martin et al, Plant Physiol 135:1908-27 (2004)) g-myr of *Abies grandis* (Bohlmann et al, J Biol Chem 272:21784-92 (1997)) and TPS10 of *Arabidopsis thaliana* (Bohlmann et al, Arch Biochem Biophys 375:261-9 (2000)). These enzymes were heterologously expressed in *E. coli.*

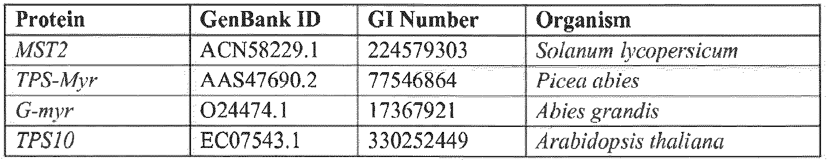

Farnesyl diphosphate is converted to alpha-farnesene and beta-farnesene by alpha-farnesene synthase and beta-farnesene synthase, respectively. Exemplary alpha-farnesene synthase enzymes include *TPS03* and *TPS02* of Arabidopsis thaliana (Faldt et al, Planta 216:745-51 (2003); Huang et al, Plant Physiol 153:1293-3 10 (2010)), *afs* of *Cucumis sativus* (Mercke et al, Plant Physiol 135:2012-14 (2004), *eafar* of *Malus x domestica* (Green et al, Phytochem 68:176-88 (2007)) and TPS-Far of Picea abies (Martin, *supra*). An exemplary beta-farnesene synthase enzyme is encoded by *TPS1* of *Zea mays* (Schnee et al, Plant Physiol 130:2049-60 (2002)).

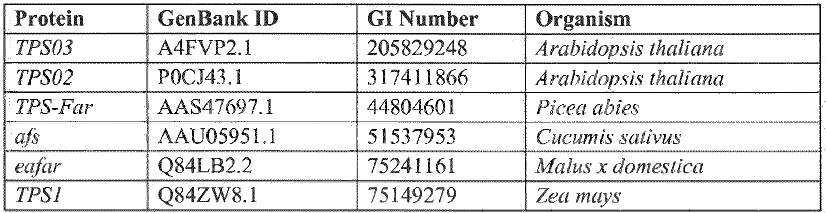

### U) Crotonyl-CoA reductase (alcohol forming) and V) 3-Hydroxybutyryl-CoA reductase (alcohol forming).

The direct conversion of crotonyl-CoA and 3-hydroxybutyryl-CoA substrates to their corresponding alcohols is catalyzed by bifuncitonal enzymes with acyl-CoA reductase (aldehyde forming) activity and aldehyde reductase or alcohol dehydrogenase activities. Exemplary bifunctional oxidoreductases that convert an acyl-CoA to alcohol are described elsewhere herein. **Figure 6** shows pathways for converting 1,3-butanediol to 3-buten-2-ol and/or butadiene. Enzymes in Figure 6 are A. 1,3-butanediol kinase, B. 3-hydroxybutyrylphosphate kinase, C. 3-hydroxybutyryldiphosphate lyase, D. 1,3-butanediol diphosphokinase, E. 1,3-butanediol dehydratase, F. 3-hydroxybutyrylphosphate lyase, G. 3-buten-2-ol dehydratase or chemical reaction. **A. 1,3-Butanediol Kinase.** Phosphorylation of 1,3-butanediol to 3-hydroxybutyrylphosphate is catalyzed by an alcohol kinase enzyme. Alcohol kinase enzymes catalyze the transfer of a phosphate group to a hydroxyl group. Kinases that catalyze transfer of a phosphate group to an alcohol group arc members of the EC 2.7.1 enzyme class. The table below lists several useful kinase enzymes in the EC 2.7.1 enzyme class.

| Enzyme Commission Number | Enzyme Name | Enzyme Commission Number | Enzyme Name | Enzyme Commission Number | Enzyme Name |
|---|---|---|---|---|---|
| 2.7.1.1 | hexokinase | 2.7.1.48 | uridine kinase | 2.7.1.94 | acylglycerol kinase |
| 2.7.1.2 | glucokinase | 2.7.1.49 | hydroxymethylpyrimidine kinase | 2.7.1.95 | kanamycin kinase |
| 2.7.1.3 | ketohexokinase | 2.7.1.50 | hydroxyethylthiazole kinase | 2.7.1.100 | S-methyl-5-thioribose kinase |
| 2.7.1.4 | fructokinase | 2.7.1.51 | L-fuculokinase | 2.7.1.101 | tagatose kinase |
| 2.7.1.5 | rhamnulokinase | 2.7.1.52 | fucokinase | 2.7.1.102 | hamamelose kinase |
| 2.7.1.6 | galactokinase | 2.7.1.53 | L-xylulokinase | 2.7.1.103 | viomycin kinase |
| 2.7.1.7 | mannokinase | 2.7.1.54 | D-arabinokinase | 2.7.1.105 | 6-phosphofructo-2-kinase |
| 2.7.1.8 | glucosamine kinase | 2.7.1.55 | allose kinase | 2.7.1.106 | glucose-1,6-bisphosphate synthase |
| 2.7.1.10 | phosphoglucokinase | 2.7.1.56 | 1 -phosphofructokinase | 2.7.1.107 | diacylglycerol kinase |
| 2.7.1.11 | 6-phosphofructokinase | 2.7.1.58 | 2-dehydro-3-deoxygalactonokinase | 2.7.1.108 | dolichol kinase |
| 2.7.1.12 | gluconokinase | 2.7.1.59 | N-acetylglucosamine kinase | 2.7.1.113 | deoxyguanosine kinase |
| 2.7.1.13 | dehydrogluconokinase | 2.7.1.60 | N-acylmannosamine kinase | 2.7.1.114 | AMP-thymidine kinase |
| 2.7.1.14 | sedoheptulokinase | 2.7.1.61 | acyl-phosphate-hexose phosphotransferase | 2.7.1.118 | ADP-thymidine kinase |
| 2.7.1.15 | ribokinase | 2.7.1.62 | phosphoramidate-hexose phosphotransferase | 2.7.1.119 | hygromycin-B 7"-O-kinase |

| Enzyme Commission Number | Enzyme Name | Enzyme Commission Number | Enzyme Name | Enzyme Commission Number | Enzyme Name |
|---|---|---|---|---|---|
| 2.7.1.16 | ribulokinase | 2.7.1.63 | polyphosphate-glucose phosphotransferase | 2.7.1.121 | phosphoenolpyruvate-glycerone phosphotransferase |
| 2.7.1.17 | xylulokinasc | 2.7.1.64 | inositol 3-kinasc | 2.7.1.122 | xylitol kinase |
| 2.7.1.18 | phosphoribokinase | 2.7.1.65 | scyllo-inosamine 4-kinase | 2.7.1.127 | inositol-trisphosphate 3-kinase |
| 2.7.1.19 | phosphoribulokinase | 2.7.1.66 | undecaprenol kinase | 2.7.1.130 | tetraacyldisaccharide 4'-kinase |
| 2.7.1.20 | adenosine kinase | 2.7.1.67 | 1-phosphatidylinositol 4-kinase | 2.7.1.134 | inositol-tetrakisphosphate 1-kinase |
| 2.7.1.21 | thymidine kinase | 2.7.1.68 | 1-phosphatidylinositol-4-phosphate 5-kinase | 2.7.1.136 | macrolide 2'-kinase |
| 2.7.1.22 | ribosylnicotinamide kinase | 2.7.1.69 | protein-Np-phosphohistidine-sugar phosphotransferase | 2.7.1.137 | phosphatidylinositol 3-kinase |
| 2.7.1.23 | NAD+ kinase | 2.7.1.70 | identical to EC 2.7.1.37. | 2.7.1.138 | ceramide kinase |
| 2.7.1.24 | dephospho-CoA kinase | 2.7.1.71 | shikimate kinase | 2.7.1.140 | inositol-tetrakisphosphate 5-kinase |
| 2.7.1.25 | adenylyl-sulfate kinase | 2.7.1.72 | streptomycin 6-kinase | 2.7.1.142 | glycerol-3 -phosphate-glucose phosphotransferase |

| Enzyme Commission Number | Enzyme Name | Enzyme Commission Number | Enzyme Name | Enzyme Commission Number | Enzyme Name |
|---|---|---|---|---|---|
| 2.7.1.26 | riboflavin kinase | 2.7.1.73 | inosine kinase | 2.7.1.143 | diphosphate-purine nucleoside kinase |
| 2.7.1.27 | erythritol kinase | 2.7.1.74 | deoxycytidine kinase | 2.7.1.144 | tagatose-6-phosphate kinase |
| 2.7.1.28 | triokinase | 2.7.1.76 | deoxyadcnosinc kinase | 2.7.1.145 | deoxynucleoside kinase |
| 2.7.1.29 | glycerone kinase | 2.7.1.77 | nucleoside phosphotransferase | 2.7.1.146 | ADP-dependent phosphofructokinase |
| 2.7.1.30 | glycerol kinase | 2.7.1.78 | polynucleotide 5'-hydroxyl-kinase | 2.7.1.147 | ADP-dependent glucokinase |
| 2.7.1.31 | glycerate kinase | 2.7.1.79 | diphosphate-glycerol phosphotransferase | 2.7.1.148 | 4-(cytidine 5'-diphospho)-2-C-methyl-D-erythritol kinase |
| 2.7.1.32 | choline kinase | 2.7.1.80 | diphosphate-serine phosphotransferase | 2.7.1.149 | 1-phosphatidylinositol-5-phosphate 4-kinase |
| 2.7.1.33 | pantothenate kinase | 2.7.1.81 | hydroxylysine kinase | 2.7.1.150 | 1-phosphatidylinositol-3-phosphate 5-kinase |
| 2.7.1.34 | pantetheine kinase | 2.7.1.82 | ethanolamine kinase | 2.7.1.151 | inositol-polyphosphate multikinase |
| 2.7.1.35 | pyridoxal kinase | 2.7.1.83 | pseudouridine kinase | 2.7.1.153 | phosphatidylinositol-4,5-bisphosphate 3-kinase |

| Enzyme Commission Number | Enzyme Name | Enzyme Commission Number | Enzyme Name | Enzyme Commission Number | Enzyme Name |
|---|---|---|---|---|---|
| 2.7.1.36 | mevalonate kinase | 2.7.1.84 | alkylglycerone kinase | 2.7.1.154 | phosphatidylinositol-4-phosphate 3-kinase |
| 2.7.1.39 | homoserine kinase | 2.7.1.85 | β-glucoside kinase | 2.7.1.156 | adenosylcobinamide kinase |
| 2.7.1.40 | pyruvate kinase | 2.7.1.86 | NADH kinase | 2.7.1.157 | N-acetylgalactosamine kinase |
| 2.7.1.41 | glucose-1-phosphate phosphodismutase | 2.7.1.87 | streptomycin 3"-kinase | 2.7.1.158 | inositol-pentakisphosphate 2-kinase |
| 2.7.1.42 | riboflavin phosphotransferase | 2.7.1.88 | dihydrostreptomycin-6-phosphate 3'a-kinase | 2.7.1.159 | inositol-1,3,4-trisphosphate 5/6-kinase |
| 2.7.1.43 | glucuronokinase | 2.7.1.89 | thiamine kinase | 2.7.1.160 | 2'-phosphotransferase |
| 2.7.1.44 | galacturonokinase | 2.7.1.90 | diphosphate-fructose-6-phosphate 1-phosphotransferase | 2.7.1.161 | CTP-dependent riboflavin kinase |
| 2.7.1.45 | 2-dehydro-3-deoxygluconokinase | 2.7.1.91 | sphinganine kinase | 2.7.1.162 | N-acetylhexosamine 1-kinase |
| 2.7.1.46 | L-arabinokinase | 2.7.1.92 | 5-dehydro-2-deoxygluconokinase | 2.7.1.163 | hygromycin B 4-O-kinase |
| 2.7.1.47 | D-ribulokinase | 2.7.1.93 | alkylglycerol kinase | 2.7.1.164 | O-phosphoseryl-tRNASec kinase |

Mevalonate kinase (EC 2.7.1.36) phosphorylates the terminal hydroxyl group of mevalonate. Gene candidates for this step include erg12 from *S*. *cerevisiae, mvk* from *Methanocaldococcus jannaschi, MVK* from *Homo sapeins,* and *mvk* from *Arabidopsis thaliana col.* Additional mevalonate kinase candidates include the feedback-resistant mevalonate kinase from the archcon *Methanosarcina mazei* (Primak et al, AEM, in press (2011)) and the Mvk protein from *Streptococcus pneumoniae* (Andreassi et al, Protein Sci, 16:983-9 (2007)). Mvk proteins from *S*. *cerevisiae, S. pneumoniae* and *M*. *mazei* were heterologously expressed and characterized in *E. coli* (Primak et al, *supra*). The *S*. *pneumoniae* mevalonate kinase was active on several alternate substrates including cylopropylmevalonate, vinylmevalonate and ethynylmevalonate (Kudoh et al, Bioorg Med Chem 18: 1124-34 (2010)), and a subsequent study determined that the ligand binding site is selective for compact, electron-rich C(3)-substituents (Lefurgy et al, J Biol Chem 285:20654-63 (2010)).

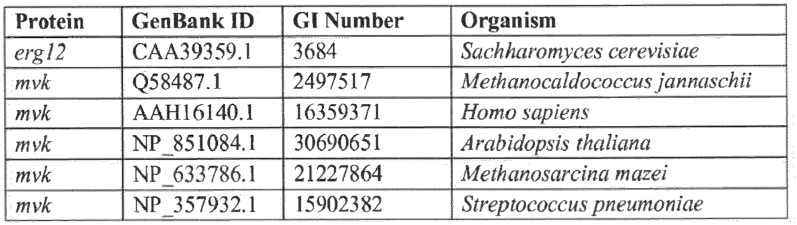

Glycerol kinase also phosphorylates the terminal hydroxyl group in glycerol to form glycerol-3-phosphate. This reaction occurs in several species, including *Escherichia coli, Saccharomyces cerevisiae,* and *Thermotoga maritima.* The *E. coli* glycerol kinase has been shown to accept alternate substrates such as dihydroxyacetone and glyceraldehyde (Hayashi et al., J Biol.Chem. 242:1030-1035 (1967)). *T. maritime* has two glycerol kinases (Nelson et al., Nature 399:323-329 (1999)). Glycerol kinases have been shown to have a wide range of substrate specificity. Crans and Whiteside studied glycerol kinases from four different organisms (*Escherichia coli, S. cerevisiae, Bacillus stearothermophilus,* and *Candida mycoderma*) (Crans et al., J.Am.Chem.Soc. 107:7008-7018 (2010); Nelson et al., *supra,* (1999)). They studied 66 different analogs of glycerol and concluded that the enzyme could accept a range of substituents in place of one terminal hydroxyl group and that the hydrogen atom at C2 could be replaced by a methyl group. Interestingly, the kinetic constants of the enzyme from all four organisms were very similar.

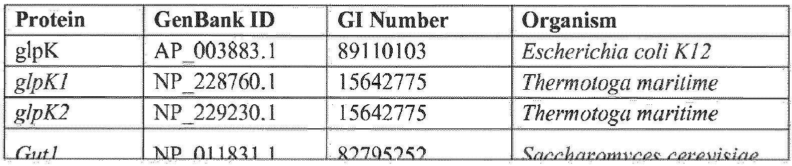

Homoserine kinase is another similar enzyme candidate. This enzyme is also present in a number of organisms including *E. coli, Streptomyces sp,* and *S*. *cerevisiae.* Homoserine kinase from *E. coli* has been shown to have activity on numerous substrates, including, L-2-amino,1,4- butanediol, aspartate semialdehyde, and 2-amino-5-hydroxyvalerate (Huo et al., Biochemistry 35:16180-16185 (1996); Huo et al., Arch.Biochem.Biophys. 330:373-379 (1996)). This enzyme can act on substrates where the carboxyl group at the alpha position has been replaced by an ester or by a hydroxymethyl group. The gene candidates are:

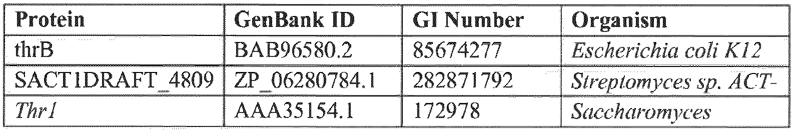

### B. 3-Hydroxybutyrylphosphate Kinase.

Alkyl phosphate kinase enzymes catalyze the transfer of a phosphate group to the phosphate group of an alkyl phosphate. The enzymes described below naturally possess such activity or can be engineered to exhibit this activity. Kinases that catalyze transfer of a phosphate group to another phosphate group are members of the EC 2.7.4 enzyme class. The table below lists several useful kinase enzymes in the EC 2.7.4 enzyme class.

| **Enzyme Commission No.** | **Enzyme Name** |
|---|---|
| 2.7.4.1 | polyphosphate kinase |
| 2.7.4.2 | phosphomevalonate kinase |
| 2.7.4.3 | adenylate kinase |
| 2.7.4.4 | nucleoside-phosphate kinase |

| **Enzyme Commission No.** | **Enzyme Name** |
|---|---|
| 2.7.4.6 | nucleoside-diphosphate kinase |
| 2.7.4.7 | phosphomethylpyrimidine kinase |
| 2.7.4.8 | guanylate kinase |
| 2.7.4.9 | dTMP kinase |
| 2.7.4.10 | nucleoside-triphosphate-adenylate kinase |
| 2.7.4.11 | (deoxy)adenylate kinase |
| 2.7.4.12 | T2-induced deoxynucleotide kinase |
| 2.7.4.13 | (deoxy)nucleoside-phosphate kinase |
| 2.7.4.14 | cytidylate kinase |
| 2.7.4.15 | thiamine-diphosphate kinase |
| 2.7.4.16 | thiamine-phosphate kinase |
| 2.7.4.17 | 3-phosphoglyceroyl-phosphate-polyphosphate phosphotransferase |
| 2.7.4.18 | farnesyl-diphosphate kinase |
| 2.7.4.19 | 5-methyldeoxycytidine-5'-phosphate kinase |
| 2.7.4.20 | dolichyl-diphosphate-polyphosphate phosphotransferase |
| 2.7.4.21 | inositol-hexakisphosphate kinase |
| 2.7.4.22 | UMP kinase |
| 2.7.4.23 | ribose 1,5-bisphosphate phosphokinase |
| 2.7.4.24 | diphosphoinositol-pentakisphosphate kinase |
| 2.7.4.- | Farnesyl monophosphate kinase |
| 2.7.4.- | Geranyl-geranyl monophosphate kinase |

| **Enzyme Commission No.** | **Enzyme Name** |
|---|---|
| 2.7.4.- | Phytyl-phosphate kinase |

Phosphomevalonate kinase enzymes are of particular interest. Phosphomevalonate kinase (EC 2.7.4.2) catalyzes the phosphorylation of phosphomevalonate. This enzyme is encoded by *erg8* in *Saccharomyces cerevisiae* (Tsay et al., Mol.Cell Biol. 11:620-631 (1991)) and *mvaK2* in *Streptococcus pneumoniae, Staphylococcus aureus* and *Enterococcus faecalis* (Doun et al., Protein Sci. 14:1134-1139 (2005); Wilding et al., J Bacteriol. 182:4319-4327 (2000)). The *Streptococcus pneumoniae* and *Enterococcus faecalis* enzymes were cloned and characterized in *E. coli* (Pilloff et al., J Biol.Chem. 278:4510-4515 (2003); Doun et al., Protein Sci. 14:1134-1139 (2005)). The S. *pneumoniae* phosphomevalonate kinase was active on several alternate substrates including cylopropylmevalonate phosphate, vinylmevalonate phosphate and ethynylmevalonate phosphate (Kudoh et al, Bioorg Med Chem 18:1124-34 (2010)).

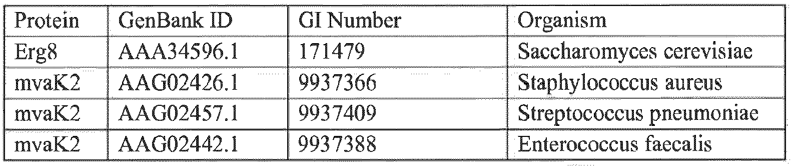

Farnesyl monophosphate kinase enzymes catalyze the CTP dependent phosphorylation of farnesyl monophosphate to farnesyl diphosphate. Similarly, geranylgeranyl phosphate kinase catalyzes CTP dependent phosphorylation. Enzymes with these activities were identified in the microsomal fraction of cultured *Nicotiana tabacum* (Thai et al, PNAS 96:13080-5 (1999)). However, the associated genes have not been identified to date.

### C. 3-Hydroxybutyryldiphosphate Lyase.

Diphosphate lyase enzymes catalyze the conversion of alkyl diphosphates to alkenes. Carbon-oxygen lyases that operate on phosphates are found in the EC 4.2.3 enzyme class. The table below lists several useful enzymes in EC class 4.2.3. described herein. Exemplary enzyme candidates also include phosphate lyases described herein.

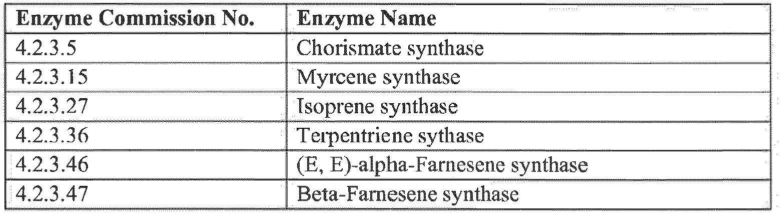

### D. 1,3-Butanediol dehydratase.

Exemplary dehydratase enzymes suitable for dehydrating 1,3-butanediol to 3-buten-2-ol include oleate hydratases, acyclic 1,2-hydratascs and linalool dehydratase. Exemplary enzyme candidates are described above.

### E. 1,3-Butanediol Diphosphokinase.

Diphosphokinase enzymes catalyze the transfer of a diphosphate group to an alcohol group. The enzymes described below naturally possess such activity. Kinases that catalyze transfer of a diphosphate group are members of the EC 2.7.6 enzyme class. The table below lists several useful kinase enzymes in the EC 2.7.6 enzyme class.

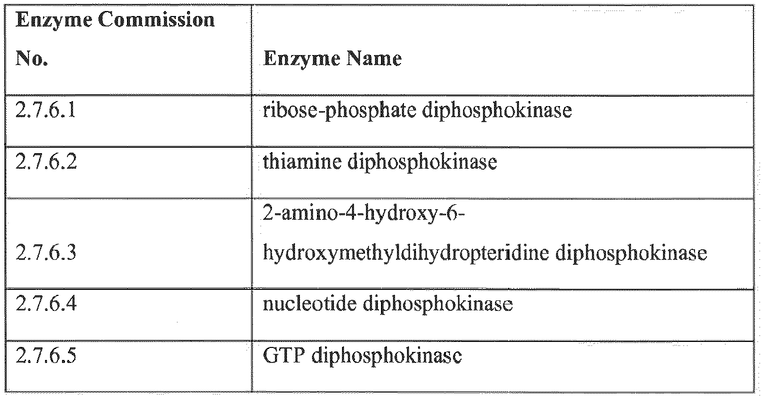

Of particular interest are ribose-phosphate diphosphokinase enzymes, which have been identified in *Escherichia coli* (Hove-Jenson et al., J Biol Chem, 1986, 261(15);6765-71) and *Mycoplasma pneumoniae* M129 (McElwain et al, International Journal of Systematic Bacteriology, 1988, 38:417-423) as well as thiamine diphosphokinase enzymes. Exemplary thiamine diphosphokinase enzymes are found in *Arabidopsis thaliana* (Ajjawi, Plant Mol Biol, 2007, 65(1-2);151-62).

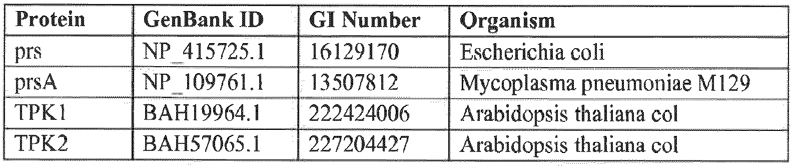

### F. 3-Hydroxybutyrylphosphate Lyase.

Phosphate lyase enzymes catalyze the conversion of alkyl phosphates to alkenes. Carbon-oxygen lyases that operate on phosphates are found in the EC 4.2.3 enzyme class. The table below lists several relevant enzymes in EC class 4.2.3.

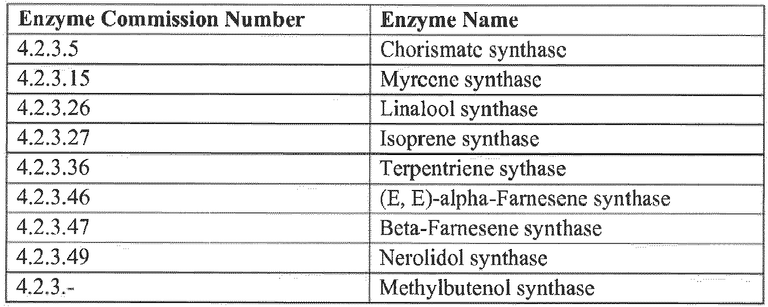

Isoprene synthase enzymes catalyzes the conversion of dimethylallyl diphosphate to isoprene.. Isoprene synthases can be found in several organisms including *Populus alba* (Sasaki et al., FEBS Letters, 2005, 579 (11), 2514-2518), *Pueraria montana* (Lindberg et al., Metabolic Eng, 12(1):70-79 (2010); Sharkey et al., Plant Physiol., 137(2):700-712 (2005)), and *Populus tremula* x *Populus alba,* also called *Populus canescens* (Miller et al., Planta, 2001, 213 (3), 483-487). The crystal structure of the *Populus canescens* isoprene synthase was determined (Koksal et al, J Mol Biol 402:363-373 (2010)). Additional isoprene synthase enzymes are described in (Chotani et al., WO/2010/031079, Systems Using Cell Culture for Production of Isoprene; Cervin et al., US Patent Application 20100003716, Isoprene Synthase Variants for Improved Microbial Production of Isoprene). Another isoprene synthase-like enzyme from *Pinus sabiniana,* methylbutenol synthase, catalyzes the formation of 2-methyl-3-buten-2-ol (Grey et al, J Biol Chem 286: 20582-90 (2011)).

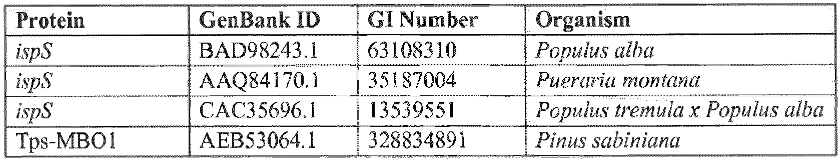

Chorismate synthase (EC 4.2.3.5) participates in the shikimate pathway, catalyzing the dephosphorylation of 5-enolpyruvylshikimate-3-phosphate to chorismate. The enzyme requires reduced flavin mononucleotide (FMN) as a cofactor, although the net reaction of the enzyme does not involve a redox change. In contrast to the enzyme found in plants and bacteria, the chorismate synthase in fungi is also able to reduce FMN at the expense of NADPH (Macheroux et al., Planta 207:325-334 (1999)). Representative monofunctional enzymes are encoded by *aroC* of *E. coli* (White et al., Biochem. J. 251:313-322 (1988)) and *Streptococcus pneumoniae* (Maclean and Ali, Structure 11:1499-1511 (2003)). Bifunctional fungal enzymes are found in *Neurospora crassa* (Kitzing et al., J. Biol. Chem. 276:42658-42666 (2001)) and *Saccharomyces cerevisiae* (Jones et al., Mol. Microbiol. 5:2143-2152 (1991)).

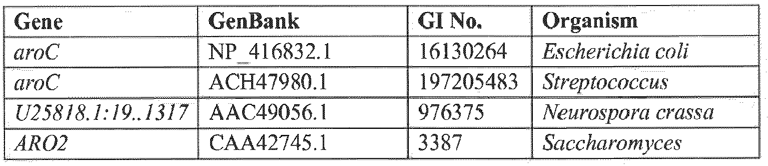

Myrcene synthase enzymes catalyze the dephosphorylation of geranyl diphosphate to beta-myrcene (EC 4.2.3.15). Exemplary myrcene synthases are encoded by MST2 of *Solanum lycopersicum* (van Schie et al, Plant Mol Biol 64:D473-79 (2007)), TPS-Myr of *Picea abies* (Martin et al, Plant Physiol 135:1908-27 (2004)) g-myr of *Abies grandis* (Bohlmann et al, J Biol Chem 272:21784-92 (1997)) and TPS10 of *Arabidopsis thaliana* (Bohlmann et al, Arch Biochem Biophys 375:261-9 (2000)). These enzymes were heterologously expressed in*E*. *coli.*

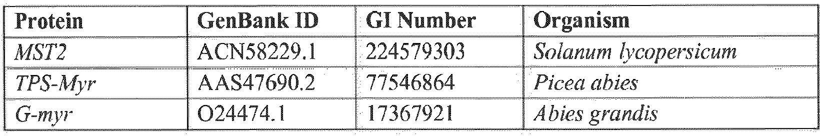

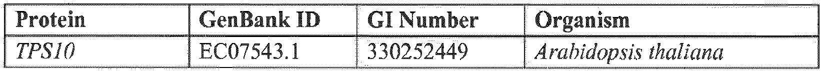

Farnesyl diphosphate is converted to alpha-farnesene and beta-farnesene by alpha-farnesene synthase and beta-farnesene synthase, respectively. Exemplary alpha-farnesene synthase enzymes include *TPS03* and *TPS02* of Arabidopsis thaliana (Faldt et al, Planta 216:745-51 (2003); Huang et al, Plant Physiol 153:1293-310 (2010)), *afs* of *Cucumis sativus* (Mercke et al, Plant Physiol 135:2012-14 (2004), *eafar* of *Malus x domestica* (Green et al, Phytochem 68:176-88 (2007)) and TPS-Far of Picea abies (Martin, *supra).* An exemplary beta-farnesene synthase enzyme is encoded by *TPS1* of *Zea mays* (Schnee et al, Plant Physiol 130:2049-60 (2002)).

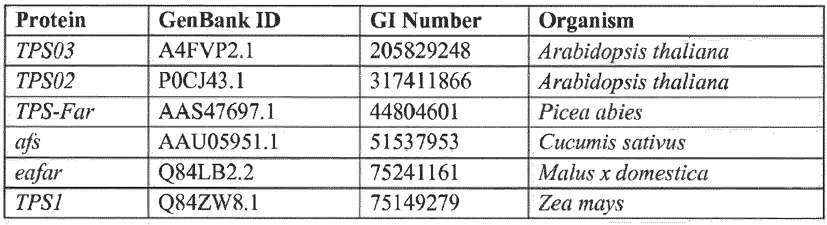

**G. G. 3-Buten-2-ol Dehydratase.** Dehydration of 3-buten-2-ol to butadiene is catalyzed by a 3-buten-2-ol dehydratase enzyme or by chemical dehydration. Exemplary dehydratase enzymes suitable for dehydrating 3-buten-2-ol include oleate hydratase, acyclic 1,2-hydratase and linalool dehydratase enzymes. Exemplary enzymes are described above.

### EXAMPLE XI

### 1,4-Butanediol Synthesis Enzymes

This Example provides genes that can be used for conversion of succinyl-CoA to 1,4-butanediol as depicted in the pathways of Figure 7.

Fig. 7. depicts A) a succinyl-CoA transferase or a succinyl-CoA synthetase, B) a succinyl-CoA reductase (aldehyde forming), C) a 4-HB dehydrogenase, D) a 4-HB kinase, E) a phosphotrans-4-hydroxybutyrylase, F) a 4-hydroxybutyryl-CoA reductase (aldehyde forming), G) a 1,4-butanediol dehydrogenase, H) a succinate reductase, I) a succinyl-CoA reductase (alcohol forming), J) a 4-hydroxybutyryl-CoA transferase or 4-hydroxybutyryl-CoA synthetase, K) a 4-HB reductase, L) a 4-hydroxybutyryl-phosphate reductase, and M) a 4-hydroxybutyryl-CoA reductase (alcohol forming).

### A) Succinyl-CoA Transferase (designated as EB1) or Succinyl-CoA Synthetase (designated as EB2A).

The conversion of succinate to succinyl-CoA is catalyzed by EB1 or EB2A (synthetase or ligase). Exemplary EB1 and EB2A enzymes are described above.

### B) Succinyl-CoA Reductase (aldehyde forming).

Enzymes with succinyl-CoA reductase activity are encoded by *sucD* of *Clostridium kluyveri* (Sohling, J. Bacteriol. 178:871-880 (1996)) and *sucD* of *Porphyromonas gingivalis* (Takahashi, J. Bacteriol 182:4704-4710 (2000)). Additional succinyl-CoA reductase enzymes participate in the 3-hydroxypropionate/4-HB cycle of thermophilic archaea such as *Metallosphaera sedula* (Berg et al., Science 318:1782-1786 (2007)) and *Thermoproteus neutrophilus* (Ramos-Vera et al., J Bacteriol, 191:4286-4297 (2009)). These and other exemplary succinyl-CoA reductase enzymes are described above.

### C) 4-HB Dehydrogenase (designated as EB4).

Enzymes exhibiting EB4 activity (EC 1.1.1.61) have been characterized in Ralstonia eutropha (Bravo et al., J. Forensic Sci. 49:379-387 (2004), *Clostridium kluyveri* (Wolff and Kenealy, Protein Expr. Purif. 6:206-212 (1995)) and *Arabidopsis thaliana* (Breitkreuz et al., J. Biol. Chem. 278:41552-41556 (2003)). Other EB4 enzymes are found in *Porphyromonas gingivalis* and *gbd* of an uncultured bacterium. Accession numbers of these genes are listed in the table below.

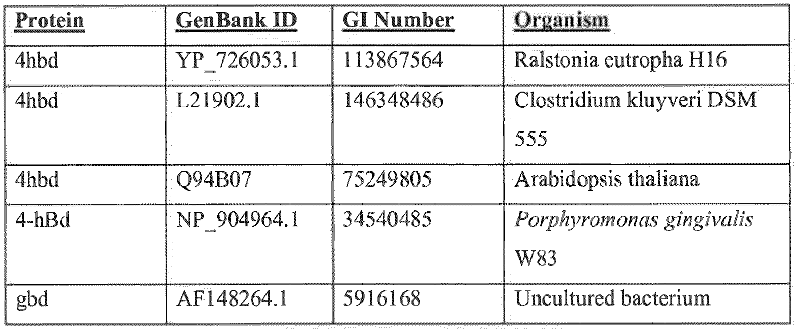

### D) 4-HB Kinase (designated as EB5).

Activation of 4-HB to 4-hydroxybutyryl-phosphate is catalyzed by EB5. Phosphotransferase enzymes in the EC class 2.7.2 transform carboxylic acids to phosphonic acids with concurrent hydrolysis of one ATP. Enzymes suitable for catalyzing this reaction include butyrate kinase, acetate kinase, aspartokinase and gamma-glutamyl kinase. Butyrate kinase carries out the reversible conversion of butyryl-phosphate to butyrate during acidogenesis in *C*. *acetobutylicum* (Cary et al., Appl. Environ. Microbiol. 56:1576-1583 (1990)). This enzyme is encoded by either of the two *buk* gene products (Huang et al., J. Mol. Microbiol. Biotechnol. 2:33-38 (2000)). Other butyrate kinase enzymes are found in *C*. *butyrricum, C. beijerinckii* and *C*. *tetanomorphum* (Twarog and Wolfe, J. Bacteriol. 86:112-117 (1963)). A related enzyme, isobutyrate kinase from *Thermotoga maritime,* has also been expressed in *E. coli* and crystallized (Diao et al., Acta Crystallogr. D. Biol. Crystallogr. 59:1100-1102 (2003); Diao and Hasson, J. Bacteriol. 191:2521-2529 (2009)). Aspartokinase catalyzes the ATP-dependent phosphorylation of aspartate and participates in the synthesis of several amino acids. The aspartokinase III enzyme in *E. coli,* encoded by *lysC,* has a broad substrate range, and the catalytic residues involved in substrate specificity have been elucidated (Keng and Viola, Arch. Biochem. Biophys. 335:73-81 (1996)). Two additional kinases in *E. coli* are also good candidates: acetate kinase and gamma-glutamyl kinase. The *E. coli* acetate kinase, encoded by *ackA* (Skarstedt and Silverstein, J. Biol. Chem. 251:6775-6783 (1976)), phosphorylates propionate in addition to acetate (Hesslinger et al., Mol. Microbiol. 27:477-492 (1998)). The *E*. *coli* gamma-glutamyl kinase, encoded by *proB* (Smith et al., J. Bacteriol. 157:545-551 (1984)), phosphorylates the gamma carbonic acid group of glutamate.

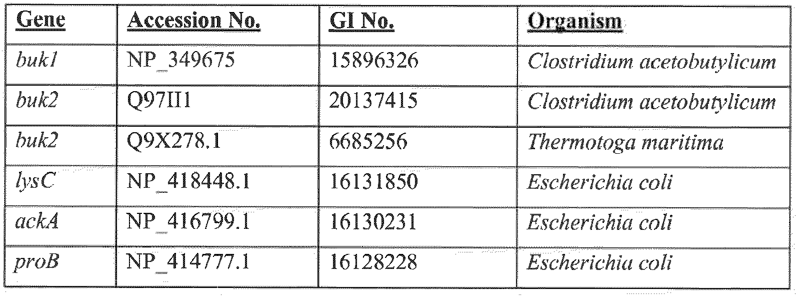

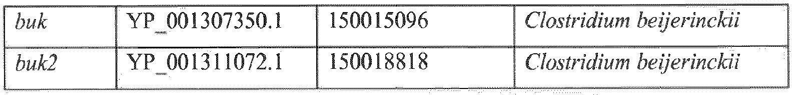

### E) Phosphotrans-4-Hydroxybutyrylase (designated as EB6).

EB6 catalyzes the transfer of the 4-hydroxybutyryl group from phosphate to CoA. Acyltransferases suitable for catalyzing this reaction include phosphotransacetylase and phosphotransbutyrylase. The *pta* gene from *E. coli* encodes an enzyme that can convert acetyl-phosphate into acetyl-CoA (Suzuki, Biochim. Biophys. Acta 191:559-569 (1969)). This enzyme can also utilize propionyl-CoA instead of acetyl-CoA (Hesslinger et al., Mol. Microbiol. 27:477-492 (1998)). Similarly, the *ptb* gene from *C. acetobutylicum* encodes an enzyme that can convert butyryl-CoA into butyryl-phosphate (Walter et al., Gene 134:107-111 (1993)); Huang et al., J Mol. Microbiol. Biotechno.1 2:33-38 (2000). Additional *ptb* genes can be found in *Clostridial* organisms, butyrate-producing bacterium L2-50 (Louis et al., J.Bacteriol. 186:2099-2106 (2004)) and *Bacillus megaterium* (Vazquez et al., Curr. Microbiol. 42:345-349 (2001)).

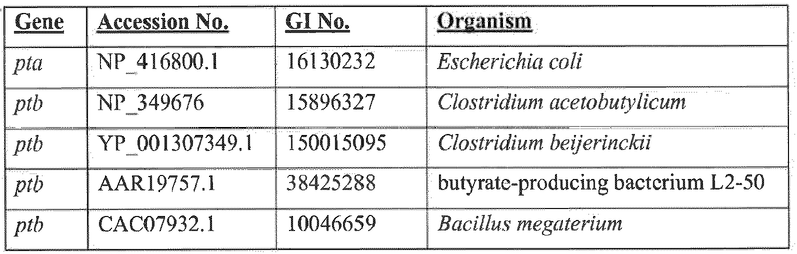

### F) 4-Hydroxybutyryl-CoA Reductase (aldehyde forming).

Enzymes with this activity are described above.

### G) 1,4-Butanediol Dehydrogenase (designated asas EB8).

EB8 catalyzes the reduction of 4-hydroxybutyraldehyde to 1,4-butanediol. Exemplary genes encoding this activity include *alrA* of *Acinetobacter* sp. strain M-1 (Tani et al., Appl.Environ.Microbiol. 66:5231-5235 (2000)), *yqhD* and *fucO* from *E*. *coli* (Sulzenbacher et al., J Mol Biol 342:489-502 (2004)) and *bdh* I and *bdh* II from C. *acetobutylicum* (Walter et al, J. Bacteriol 174:7149-7158 (1992)). Additional EB8 enzymes are encoded by *bdh* in *C*. *saccharoperbutylacetonicum* and *Cbei_1722, Cbei_2181* and *Cbei_2421* in *C*. *beijerinckii.* These and other enzymes with 1,4-butanediol activity are listed in the table below.

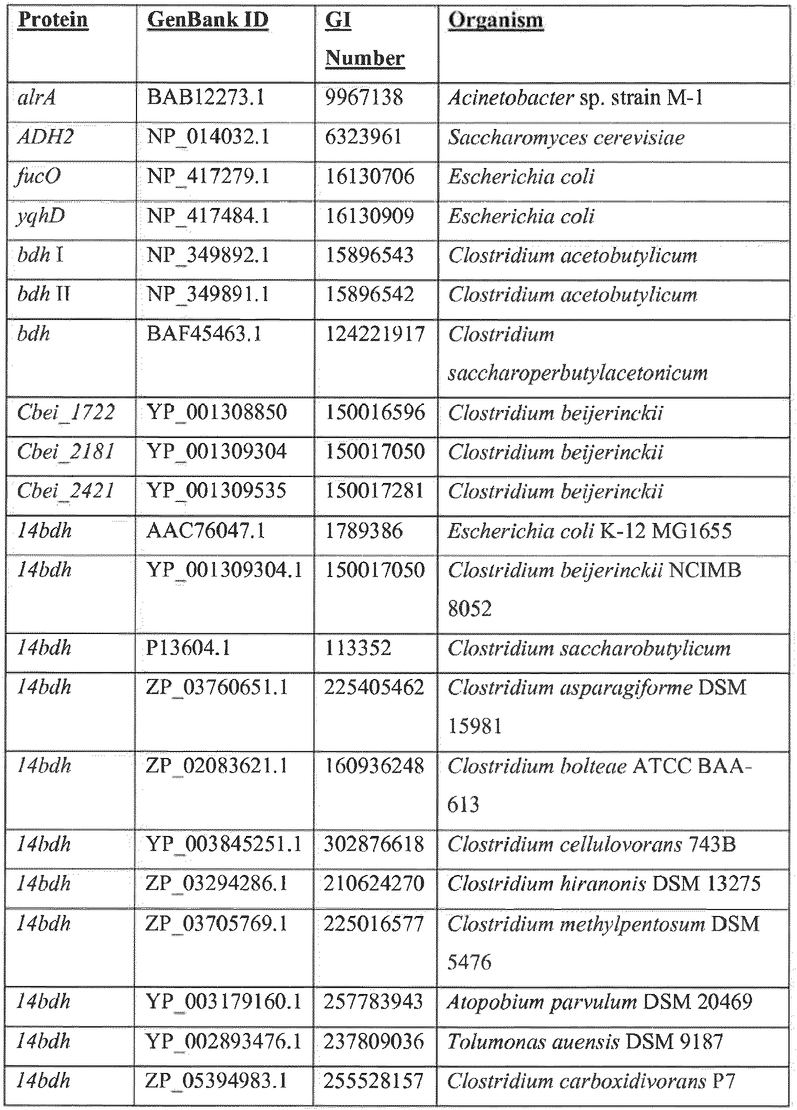

### H) Succinate Reductase.

Direct reduction of succinate to succinate semialdehyde is catalyzed by a carboxylic acid reductase. Exemplary enzymes for catalyzing this transformation are also those described below and herein for **K) 4-Hydroxybutyrate reductase** .

### I) Succinyl-CoA Reductase (alcohol forming) (designated as EB10).

EB10 enzymes are bifunctional oxidoreductases that convert succinyl-CoA to 4-HB. Enzyme candidates described below and herein for **M) 4-hydroxybutyryl-CoA reductase (alcohol forming)** are also suitable for catalyzing the reduction of succinyl-CoA.

### J) 4-Hydroxybutyryl-CoA Transferase or 4-Hydroxybutyryl-CoA Synthetase (designated as EB11).

Conversion of 4-HB to 4-hydroxybutyryl-CoA is catalyzed by a CoA transferase or synthetase. EB11 enzymes include the gene products of *cat1, cat2,* and *cat3* of *Clostridium kluyveri* (Secdorf et al., Proc.Natl.Acad.Sci U.S.A 105:2128-2133 (2008*)*; Sohling et al., J Bacteriol. 178:871-880 (1996)). Similar CoA transferase activities are also present in *Trichomonas vaginalis, Trypanosoma brucei, Clostridium aminobutyricum* and *Porphyromonas gingivalis* (Riviere et al., J.Biol.Chem. 279:45337-45346 (2004); van Grinsvcn et al., J.Biol.Chem. 283:1411-1418 (2008)).

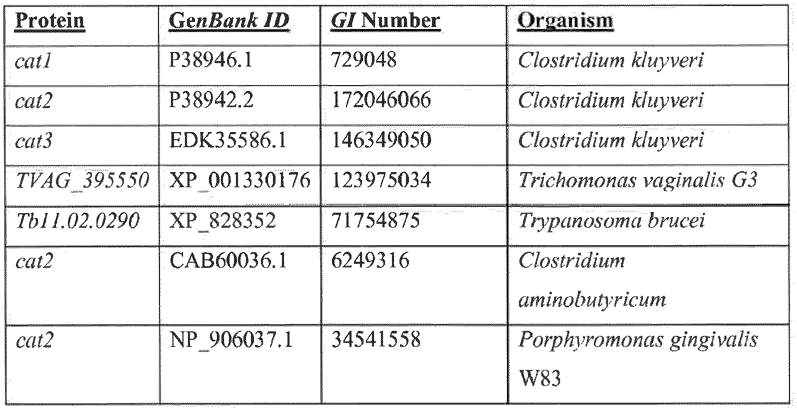

4HB-CoA synthetase catalyzes the ATP-dependent conversion of 4-HB to 4-hydroxybutyryl-CoA. AMP-forming 4-HB-CoA synthetase enzymes are found in organisms that assimilate carbon via the dicarboxylate/hydroxybutyrate cycle or the 3-hydroxypropionate/4-HB cycle. Enzymes with this activity have been characterized in *Thermoproteus neutrophilus* and *Metallosphaera sedula* (Ramos-Vera et al, J Bacteriol 192:5329-40 (2010); Berg et al, Science 318:1782-6 (2007)). Others can be inferred by sequence homology. ADP forming CoA synthetases, such EB2A, are also suitable candidates.

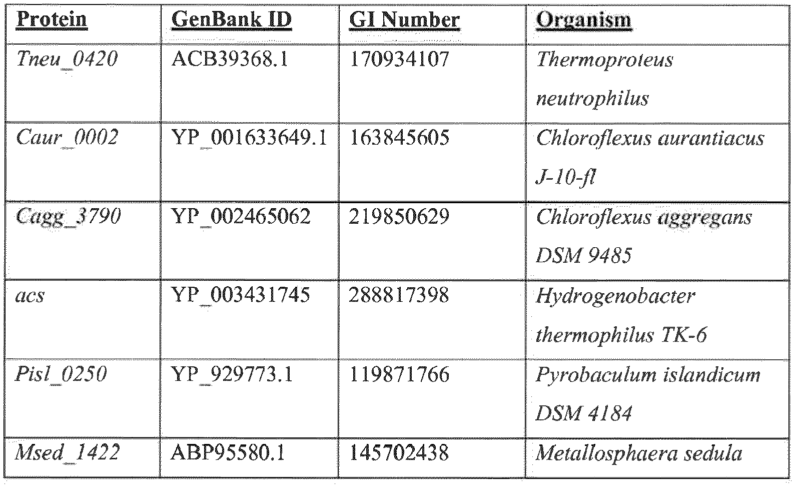

### K) 4-HB Reductase.

Reduction of 4-HB to 4-hydroxybutanal is catalyzed by a carboxylic acid reductase (CAR) such as the Car enzyme found in *Nocardia iowensis.* This enzyme and other carboxylic acid reductases are described above (see EC 1.2.1.e).

### L) 4-Hydroxybutyryl-phosphate Reductase (designated asas EB14).

EB14 catalyzes the reduction of 4-hydroxybutyrylphosphate to 4-hydroxybutyraldehyde. An enzyme catalyzing this transformation has not been identified to date. However, similar enzymes include phosphate reductases in the EC class 1.2.1. Exemplary phosphonate reductase enzymes include G3P dehydrogenase (EC 1.2.1.12), aspartate-semialdehyde dehydrogenase (EC 1.2.1.11) acetylglutamylphosphate reductase (EC 1.2.1.38) and glutamate-5-semialdehyde dehydrogenase (EC 1.2.1.-). Aspartate semialdehyde dehydrogenase (ASD, EC 1.2.1.11) catalyzes the NADPH-dependent reduction of 4-aspartyl phosphate to aspartate-4-semialdehyde. ASD participates in amino acid biosynthesis and recently has been studied as an antimicrobial target (Hadfield et al., Biochemistry 40:14475-14483 (2001)). The *E*. *coli* ASD structure has been solved (Hadfield et al., J Mol.Biol. 289:991-1002 (1999)) and the enzyme has been shown to accept the alternate substrate beta-3-methylaspartyl phosphate (Shames et al., J Biol.Chem. 259:15331-15339 (1984)). The *Haemophilus influenzae* enzyme has been the subject of enzyme engineering studies to alter substrate binding affinities at the active site (Blanco et al., Acta Crystallogr.D.Biol.Crystallogr. 60:1388-1395 (2004); Blanco et al., Acta Crystallogr.D.Biol.Crystallogr. 60:1808-1815 (2004)). Other ASD candidates are found in *Mycobacterium tuberculosis* (Shafiani et al., J Appl Microbiol 98:832-838 (2005)), *Methanococcus jannaschii* (Faehnle et al., J Mol.Biol. 353:1055-1068 (2005)), and the infectious microorganisms *Vibrio cholera* and *Heliobacter pylori* (Moore et al., Protein Expr.Purif. 25:189-194 (2002)). A related enzyme candidate is acetylglutamylphosphate reductase (EC 1.2.1.38), an enzyme that naturally reduces acetylglutamylphosphate to acetylglutamate-5-semialdehyde, found in *S. cerevisiae* (Pauwels et al., Eur.JBiochem. 270:1014-1024 (2003)), *B. subtilis* (O'Reilly et al., Microbiology 140 ( Pt 5):1023-1025 (1994)), *E. coli* (Parsot et al., Gene. 68:275-283 (1988)), and other organisms. Additional phosphate reductase enzymes of *E*. *coli* include glyceraldehyde 3-phosphate dehydrogenase (*gapA* (Branlant et al., Eur.J.Biochem. 150:61-66 (1985))) and glutamate-5-semialdehyde dehydrogenase (*proA* (Smith et al., J.Bacteriol. 157:545-551 (1984))). Genes encoding glutamate-5-semialdehyde dehydrogenase enzymes from *Salmonella typhimurium* (Mahan et al., J Bacteriol. 156:1249-1262 (1983)) and *Campylobacter jejuni* (Louie et al., Mol.Gen.Genet. 240:29-35 (1993)) were cloned and expressed in *E. coli.*

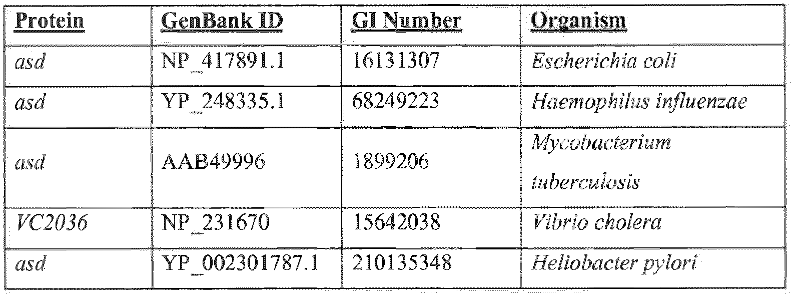

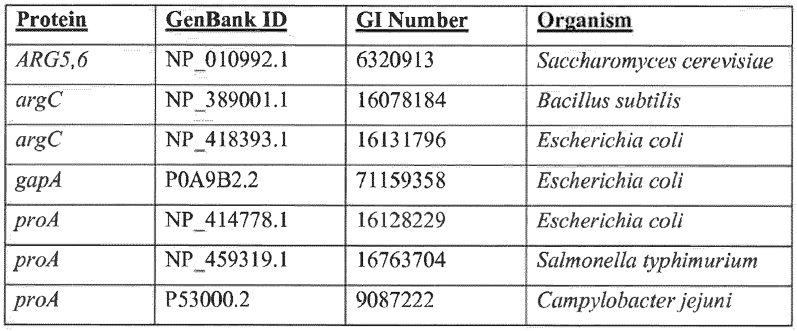

### M) 4-Hydroxybutyryl-CoA Reductase (alcohol forming) (designated as EB15).

EB15 enzymes are bifunctional oxidoreductases that convert an 4-hydroxybutyryl-CoA to 1,4-butanediol. Enzymes with this activity include *adhE* from *E. coli, adhE2* from *C*. *acetobutylicum* (Fontaine et al., J.Bacteriol. 184:821-830 (2002)) and the C. *acetobutylicum* enzymes encoded by *bdh* I and *bdh* II (Walter, et al., J. Bacteriol. 174:7149-7158 (1992)). In addition to reducing acetyl-CoA to ethanol, the enzyme encoded by *adhE* in *Leuconostoc mesenteroides* has been shown to oxide the branched chain compound isobutyraldehyde to isobutyryl-CoA (Kazahaya et al., J.Gen.Appl.Microbiol. 18:43-55 (1972); Koo et al., Biotechnol Lett, 27:505-510 (2005)).

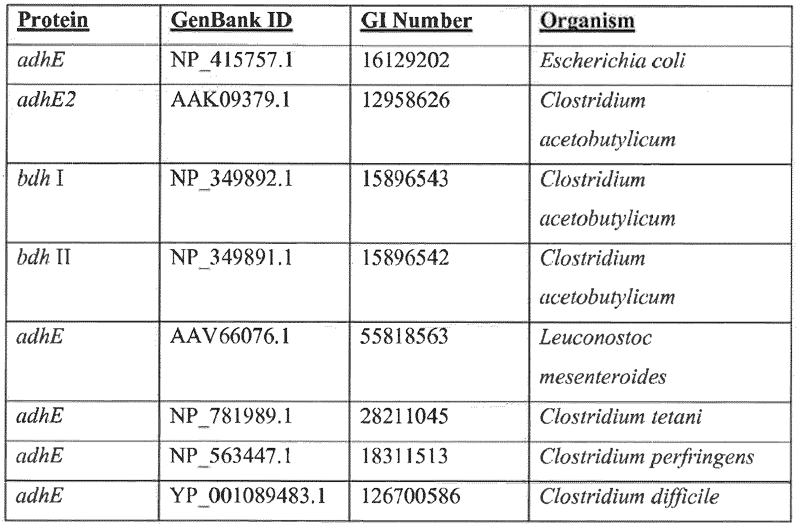

### EXAMPLE XII

### Adipate, 6-Aminocaproate, Caprolactam and Hexamethylenediamine Synthesis Enzymes

This Example provides genes that can be used for conversion of succinyl-CoA and acetyl-CoA to adipate, 6-aminocaproate, caprolactam and hexamethylenediamine as depicted in the pathways of Figure 8.

FIG. 8. depicts enzymes: A) 3-oxoadipyl-CoA thiolase , B) 3-oxoadipyl-CoA reductase, C) 3-hydroxyadipyl-CoA dehydratase, D) 5-carboxy-2-pentenoyl-CoA reductase, E) adipyl-CoA reductase (aldehyde forming), F) 6-aminocaproate transaminase, or 6-aminocaproate dehydrogenase, G) 6-aminocaproyl-CoA/acyl-CoA transferase, or 6-aminocaproyl-CoA synthase , H) amidohydrolase, 1) spontaneous cyclization, J) 6-aminocaproyl-CoA reductase (aldehyde forming), K) HMDA transaminase or HMDA dehydrogenase, L) Adipyl-CoA hydrolase, adipyl-CoA ligase, adipyl-CoA transferase, or phosphotransadipylase/adipate kinase. Transformations depicted in FIG. 8 fall into at least 10 general categories of transformations shown in the Table below. The first three digits of each label correspond to the first three Enzyme Commission number digits which denote the general type of transformation independent of substrate specificity. Below is described a number of biochemically characterized candidate genes in each category. Specifically listed are exemplary genes that can be applied to catalyze the appropriate transformations in FIG. 8 when cloned and expressed.

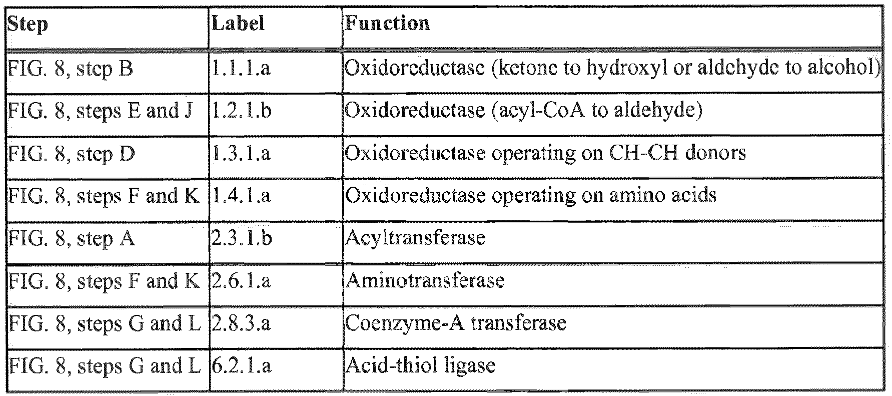

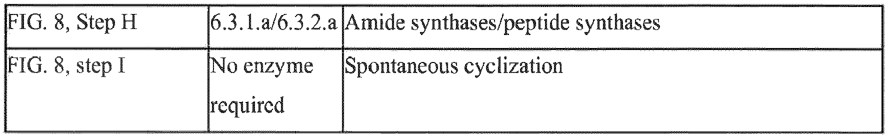

### FIG. 8, Step A - 3-Oxoadipyl-CoA Thiolase.

### EC 2.3. 1.b Acyl transferase.

The first step in the pathway combines acetyl-CoA and succinyl-CoA to form 3-oxoadipyl-CoA. Step A can involve a 3-oxoadipyl-CoA thiolase, or equivalently, succinyl CoA:acetyl CoA acyl transferase (β-ketothiolase). The gene products encoded by *pccaF* in *Pseudomonas* strain B13 (Kaschabek et al., J.Bacteriol. 184:207-215 (2002)), *phaD* in *Pseudomonas putida U* (Olivera et al., Proc. Natl. Acad. Sci. USA 95:6419-6424 (1998)), *paaE* in *Pseudomonas fluorescens ST* (Di Gennaro et al., Arch. Microbiol. 188:117-125 (2007)), and *paaJ* from *E. coli* (Nogales et al., Microbiol. 153:357-365 (2007)) catalyze the conversion of 3-oxoadipyl-CoA into succinyl-CoA and acetyl-CoA during the degradation of aromatic compounds such as phenylacetate or styrene. Since beta-ketothiolase enzymes catalyze reversible transformations, these enzymes can be employed for the synthesis of 3-oxoadipyl-CoA. For example, the ketothiolase *phaA* from *R. eutropha* combines two molecules of acetyl-CoA to form acetoacetyl-CoA (Sato et al., J Biosci Bioeng 103:38-44 (2007)). Similarly, a β-keto thiolase (*bktB*) has been reported to catalyze the condensation of acetyl-CoA and propionyl-CoA to form β-kctovalcryl-CoA (Slater et al., J.Bacteriol. 180:1979-1987 (1998)) in *R*. *eutropha.* The protein sequences for the above-mentioned gene products are well known in the art and can be accessed in the public databases such as GenBank using the following accession numbers.

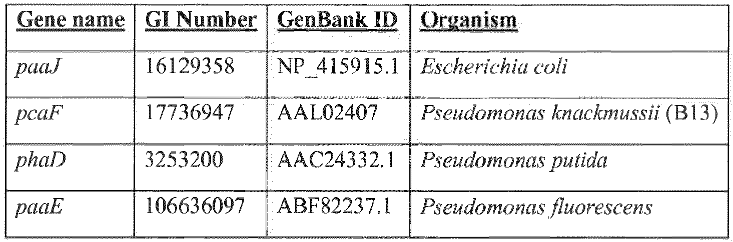

These exemplary sequences can be used to identify homologue proteins in GenBank or other databases through sequence similarity searches (for example, BLASTp). The resulting homologue proteins and their corresponding gene sequences provide additional exogenous DNA sequences for transformation into *E*. *coli* or other suitable host microorganisms to generate production hosts.
For example, orthologs of *paaJ* from *Escherichia coli* K12 can be found using the following GenBank accession numbers:

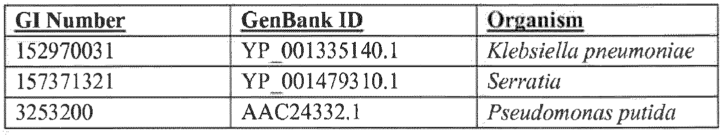

Example orthologs of *pcaF* from *Pseudomonas knackmussii* can be found using the following GenBank accession numbers:

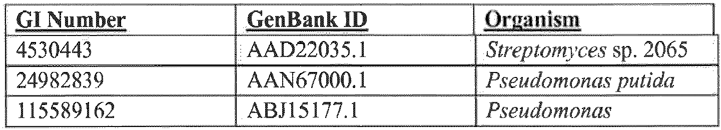

Additional native candidate genes for the ketothiolase step include *atoB,* which can catalyze the reversible condensation of 2 acetyl-CoA molecules (Sato et al., J. Biosci. Bioengineer. 103:38-44 (2007)), and its homolog *yqeF.* Non-native gene candidates include *phaA* (Sato et al., *supra,* 2007) and *bktB* (Slater et al., J. Bacteriol. 180:1979-1987 (1998)) from *R. eutropha,* and the two ketothiolases, *thiA* and *thiB,* from *Clostridium acetobutylicum* (Winzer et al., J. Mol. Microbiol. Biotechnol. 2:531-541 (2000)). The protein sequences for each of these exemplary gene products can be found using the following GenBank accession numbers:

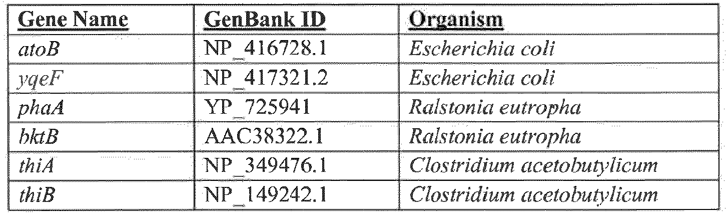

2-Amino-4-oxopentanoate (AKP) thiolase or AKP thiolase (AKPT) enzymes present additional candidates for performing step A. AKPT is a pyridoxal phosphate-dependent enzyme participating in ornithine degradation in *Clostridium sticklandii* (Jeng et al., Biochemistry 13:2898-2903 (1974); Kenklies et al., Microbiology 145:819-826 (1999)). A gene cluster encoding the alpha and beta subunits of AKPT (*or-2* (*ortA*) and *or-3* (*ortB*)) was recently identified and the biochemical properties of the enzyme were characterized (Fonknechten et al., J.Bacteriol. In Press (2009)). The enzyme is capable of operating in both directions and naturally reacts with the D-isomer of alanine. AKPT from *Clostridium sticklandii* has been characterized but its protein sequence has not yet been published. Enzymes with high sequence homology are found in *Clostridium difficile, Alkaliphilus metalliredigenes QYF, Thermoanaerobacter sp. X514,* and *Thermoanaerobacter tengcongensis MB4* (Fonknechten et al., *supra*).

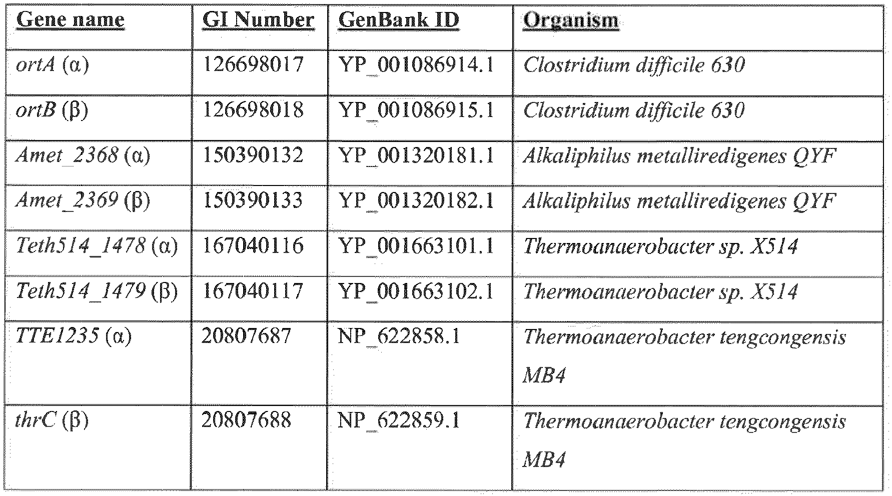

### Step B - 3-Oxoadipyl-CoA Reductase.

### EC 1.1.1.a Oxidoreductases.

Certain transformations depicted in FIG. 8 involve oxidoreductases that convert a ketone functionality to a hydroxyl group. For example, FIG. 8, step B involves the reduction of a 3-oxoacyl-CoA to a 3-hydroxyacyl-CoA. Exemplary enzymes that can convert 3-oxoacyl-CoA molecules, such as 3-oxoadipyl-CoA, into 3-hydroxyacyl-CoA molecules, such as 3-hydroxyadipyl-CoA, include enzymes whose natural physiological roles are in fatty acid beta-oxidation or phenylacetate catabolism. For example, subunits of two fatty acid oxidation complexes in *E. coli,* encoded by *fadB* and *fadJ,* function as 3-hydroxyacyl-CoA dehydrogenases (Binstock et al., Methods Enzymol. 71:403-411 (1981)). Furthermore, the gene products encoded by *phaC* in *Pseudomonas putida U* (Olivera et al., Proc. Natl. Acad. Sci. USA 95:6419-6424 (1998)) and *paaC* in *Pseudomonas fluorescens ST* (Di Gennaro et al., Arch. Microbiol. 188:117-125 (2007)) catalyze the reverse reaction of step B in FIG. 8, that is, the oxidation of 3-hydroxyadipyl-CoA to form 3-oxoadipyl-CoA, during the catabolism of phenylacetate or styrene. Note that the reactions catalyzed by such enzymes are reversible. A similar transformation is also carried out by the gene product of *hbd* in *Clostridium acetobutylicum* (Atsumi et al., Metab. Eng. (epub Sep. 14, 2007); Boynton et al., J. Bacteriol. 178:3015-3024 (1996)). This enzyme converts acetoacetyl-CoA to 3-hydroxybutyryl-CoA. In addition, given the proximity in *E. coli* of *paaH* to other genes in the phenylacetate degradation operon (Nogales et al., Microbiology 153:357-365 (2007)) and the fact that *paaH* mutants cannot grow on phenylacetate (Ismail et al., Eur.JBiochem. 270:3047-3054 (2003)), it is expected that the *E. coli paaH* gene encodes a 3-hydroxyacyl-CoA dehydrogenase.

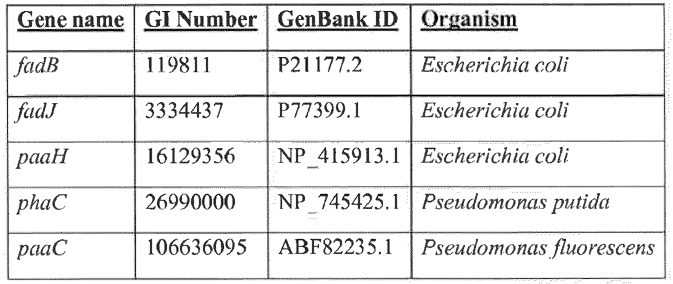

Additional exemplary oxidoreductases capable of converting 3-oxoacyl-CoA molecules to their corresponding 3-hydroxyacyl-CoA molecules include 3-hydroxybutyryl-CoA dehydrogenases. The enzyme from *Clostridium acetobutylicum,* encoded by *hbd,* has been cloned and functionally expressed in *E*. *coli* (Youngleson et al., J. Bacterial. 171:6800-6807 (1989)). Additional gene candidates include *Hbd1* (C-terminal domain) and *Hbd2* (N-terminal domain) in *Clostridium kluyveri* (Hillmer et al., FEBS Lett. 21:351-354 (1972)) and *HSD17B10* in *Bos taurus* (Wakil et al., J. Biol. Chem. 207:631-638 (1954)). Yet other gene candidates demonstrated to reduce acetoacctyl-CoA to 3-hydroxybutyryl-CoA are *phbB* from *Zoogloea ramigera* (Ploux et al., Eur. J. Biochem. 174:177-182 (1988)) and *phαB* from *Rhodobacter sphaeroides* (Alber et al., Mol. Microbiol 61:297-309 (2006)). The former gene candidate is NADPH-dependent, its nucleotide sequence has been determined (Peoples et al., Mol. Microbiol 3:349-357 (1989)) and the gene has been expressed in *E. coli.* Substrate specificity studies on the gene led to the conclusion that it could accept 3-oxopropionyl-CoA as a substrate besides acetoacetyl-CoA (Ploux et al., *supra*).

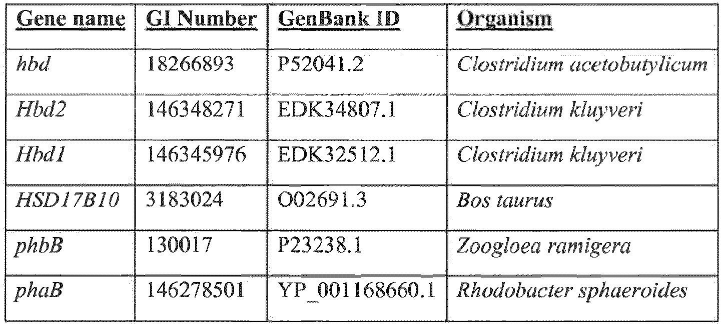

A number of similar enzymes have been found in other species of *Clostridia* and in *Metallosphaera sedula* (Berg et al., Science 318:1782-1786 (2007)).

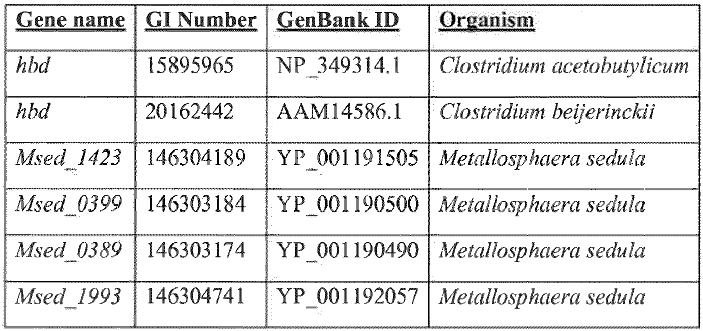

### Step C - 3-Hydroxyadipyl-CoA Dehydratase.

Step C can involve a 3-hydroxyadipyl-CoA dehydratase. The gene product of *crt* from *C*. *acetobutylicum* catalyzes the dehydration of 3-hydroxybutyryl-CoA to crotonyl-CoA (Atsumi et al., Metab. Eng. (epub Sep. 14, 2007); Boynton et al., J. Bacteriol. 178:3015-3024 (1996)). Homologs of this gene are strong candidates for carrying out the third step (step C) in the synthesis pathways exemplified in FIG. 8. In addition, genes known to catalyze the hydroxylation of double bonds in enoyl-CoA compounds represent additional candidates given the reversibility of such enzymatic transformations. For example, the enoyl-CoA hydratases, *phaA* and *phaB,* of *P. putida* are believed to carry out the hydroxylation of double bonds during phenylacetate catabolism (Olivera et al., Proc. Natl. Acad. Sci. USA 95:6419-6424 (1998)) and thus represent additional candidates for incorporation into *E. coli.* The deletion of these genes precludes phenylacetate degradation in *P. putida.* The *paaA* and *paaB* from *P. fluorescens* catalyze analogous transformations (Olivera et al., Proc. Natl. Acad. Sci. USA 95:6419-6424 (1998)). Lastly, a number of *Escherichia coli* genes have been shown to demonstrate enoyl-CoA hydratase functionality including *maoC* (Park and Lee, J. Bacteriol. 185:5391-5397 (2003)), *puαF* (Ismail et al., Eur. J. Biochem. 270:3047-3054 (2003); Park and Lee, Biotechnol. Bioeng. 86:681-686 (2004); Park and Lee, Appl. Biochem. Biotechnol. 113-116:335-346 (2004)), and *paaC* (Ismail et al., *supra,* 2003; Park and Lee, *supra,* 2003; Park and Lee, *supra,* 2004). The protein sequences for each of these exemplary gene products can be found using the following GenBank accession numbers:

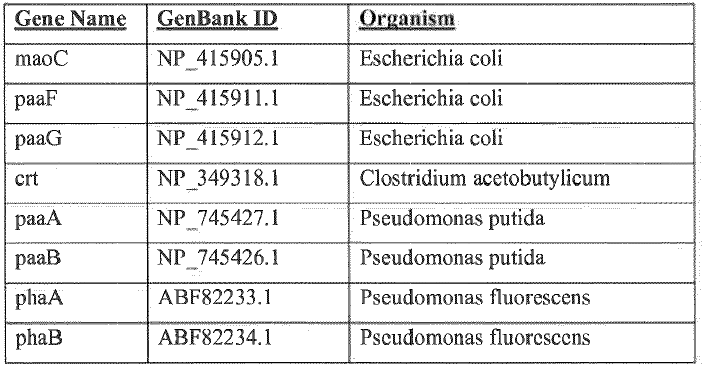

Alternatively, beta-oxidation genes are candidates for the first three steps in adipate synthesis. Candidate genes for the proposed adipate synthesis pathway also include the native fatty acid oxidation genes of *E*. *coli* and their homologs in other organisms. The *E. coli* genes *fndA* and *fadB* encode a multienzyme complex that exhibits ketoacyl-CoA thiolase, 3-hydroxyacyl-CoA dehydrogenase, and enoyl-CoA hydratase activities (Yang et al., Biochem. 30:6788-6795 (1991); Yang ct al., J. Biol. Chem. 265:10424-10429 (1990); Yang et al., J. Biol. Chem. 266:16255 (1991); Nakahigashi and Inokuchi, Nucl. Acids Res. 18: 4937 (1990)). These activities are mechanistically similar to the first three transformations shown in FIG. 8. The *fadI* and *fadJ* genes encode similar functions and are naturally expressed only anaerobically (Campbell et al., Mol. Microbiol. 47:793-805 (2003)). These gene products naturally operate to degrade short, medium, and long chain fatty-acyl-CoA compounds to acetyl-CoA, rather than to convert succinyl-CoA and acetyl-CoA into 5-carboxy-2-pentenoyl-CoA as proposed in FIG. 8. However, it is well known that the ketoacyl-CoA thiolase, 3-hydroxyacyl-CoA dehydrogenase, and enoyl-CoA hydratase enzymes catalyze reversible transformations. Furthermore, directed evolution and related approaches can be applied to tailor the substrate specificities of the native beta-oxidation machinery of *E*. *coli.* Thus these enzymes or homologues thereof can be applied for adipate production. If the native genes operate to degrade adipate or its precursors *in vivo,* the appropriate genetic modifications are made to attenuate or eliminate these functions. However, it may not be necessary since a method for producing poly[(R)-3-hydroxybutyrate] in *E. coli* that involves activating *fadB,* by knocking out a negative regulator, *fadR,* and co-expressing a non-native ketothiolase, *phaA* from *Ralstonia eutropha,* has been described (Sato et al., J. Biosci. Bioeng. 103:38-44 (2007)). This work clearly demonstrated that a - oxidation enzyme, in particular the gene product of *fadB* which encodes both 3-hydroxyacyl-CoA dehydrogenase and enoyl-CoA hydratase activities, can function as part of a pathway to produce longer chain molecules from acetyl-CoA precursors. The protein sequences for each of these exemplary gene products can be found using the following GenBank accession numbers:

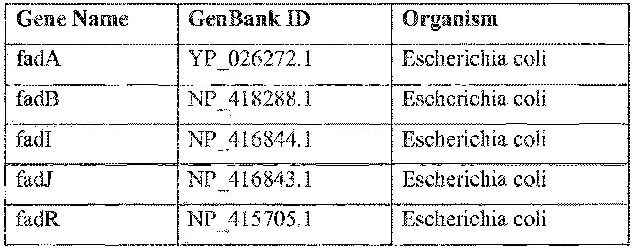

### Step D - 5-Carboxy-2-Pentenoyl-CoA Reductase. EC 1.3.1.a Oxidoreductase operating on CH-CH donors.

Step D involves the conversion of 5-carboxy-2-pentenoyl-CoA to adipyl-CoA by 5-carboxy-2-pentenoyl-CoA reductase. Enoyl-CoA reductase enzymes are suitable enzymes for this transformation.

Whereas the ketothiolase, dehydrogenase, and enoyl-CoA hydratase steps are generally reversible, the enoyl-CoA reductase step is almost always oxidative and irreversible under physiological conditions (Hoffmeister et al., J. Biol. Chem. 280:4329-4338 (2005)). *FadE* catalyzes this likely irreversible transformation in *E*. *coli* (Campbell and Cronan, J. Bacteriol. 184:3759-3764 (2002)). The pathway can involve an enzyme that reduces a 2-enoyl-CoA intermediate, not one such as *FadE* that will only oxidize an acyl-CoA to a 2-enoyl-CoA compound. Furthermore, although it has been suggested that *E. coli* naturally possesses enzymes for enoyl-CoA reduction (Mizugaki et al., J. Biochem. 92:1649-1654 (1982); Nishimaki et al., J. Biochem. 95:1315-1321 (1984)), no *E*. *coli* gene possessing this function has been biochemically characterized.

One exemplary enoyl-CoA reductase is the gene product of *bcd* from *C. acetobutylicum* (Boynton et al., J Bacteriol. 178:3015-3024 (1996); Atsumi et al., Metab. Eng. 2008 10(6):305-311 (2008)(Epub Sep. 14, 2007), which naturally catalyzes the reduction of crotonyl-CoA to butyryl-CoA. Activity of this enzyme can be enhanced by expressing *bcd* in conjunction with expression of the *C. acetobutylicum etfAB* genes, which encode an electron transfer flavoprotein. An additional candidate for the enoyl-CoA reductase step is the mitochondrial enoyl-CoA reductase from *E*. *gracilis* (Hoffmeister et al., J. Biol. Chem. 280:4329-4338 (2005)). A construct derived from this sequence following the removal of its mitochondrial targeting leader sequence was cloned in *E. coli* resulting in an active enzyme (Hoffmeister et al., *supra).* This approach is well known to those skilled in the art of expressing eukaryotic genes, particularly those with leader sequences that may target the gene product to a specific intracellular compartment, in prokaryotic organisms. A close homolog of this gene, TDE0597, from the prokaryote *Treponema denticola* represents a third enoyl-CoA reductase which has been cloned and expressed in *E. coli* (Tucci et al., FEBS Letters 581:1561-1566 (2007)).

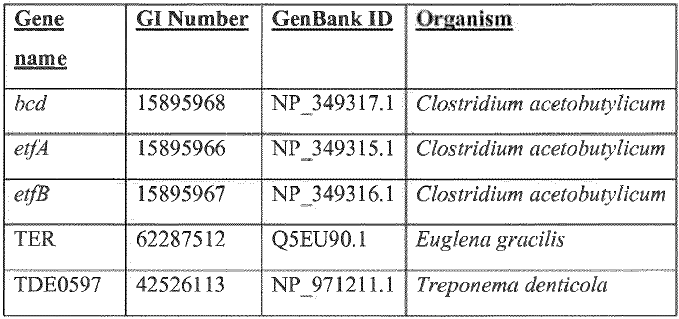

### Step E - Adipyl-CoA Reductase (Aldehyde Forming). EC 1.2.1.b Oxidoreductase (acyl-CoA to aldehyde).

The transformation of adipyl-CoA to adipate semialdehyde in step E can involve an acyl-CoA dehydrogenases capable of reducing an acyl-CoA to its corresponding aldehyde. An EC 1.2.1.b oxidoreductase (acyl-CoA to aldehyde) provides suitable enzyme activity. Exemplary enzymes in this class are described herein and above (for example see description for 3-Hydroxybutyryl-CoA Reductase (aldehyde forming)).

### Step F - 6-Aminocaproate Transaminase or 6-Aminocaproate Dehydrogenase. EC 1.4.1.a Oxidoreductase operating on amino acids.

Step F depicts a reductive amination involving the conversion of adipate semialdehyde to 6-aminocaproate. Most oxidoreductases operating on amino acids catalyze the oxidative deamination of alpha-amino acids with NAD+ or NADP+ as acceptor, though the reactions are typically reversible. Exemplary oxidoreductases operating on amino acids include glutamate dehydrogenase (deaminating), encoded by *gdhA,* leucine dehydrogenase (deaminating), encoded by *ldh,* and aspartate dehydrogenase (deaminating), encoded by *nadX.* The *gdhA* gene product from *Escherichia coli* (McPherson et al., Nucleic.Acids Res. 11:5257-5266 (1983); Korber et al., J.Mol.Biol. 234:1270-1273 (1993)), *gdh* from *Thermotoga maritima* (Kort et al., Extremophiles 1:52-60 (1997); Lebbink et al., J.Mol.Biol. 280:287-296 (1998); Lebbink et al., J.Mol.Biol. 289:357-369 (1999)), and *gdhA1* from *Halobacterium salinarum* (Ingoldsby et al., Gene. 349:237-244 (2005)) catalyze the reversible interconversion of glutamate to 2-oxoglutarate and ammonia, while favoring NADP(H), NAD(H), or both, respectively. The *ldh* gene of *Bacillus cereus* encodes the LeuDH protein that has a wide of range of substrates including leucine, isoleucine, valine, and 2-aminobutanoate (Stoyan et al., J.Biotechnol 54:77-80 (1997); Ansorge et al., Biotechnol Bioeng. 68:557-562 (2000)). The *nadX* gene from *Thermotoga maritime* encoding for the aspartate dehydrogenase is involved in the biosynthesis of NAD (Yang et al., J.Biol.Chem. 278:8804-8808 (2003)).

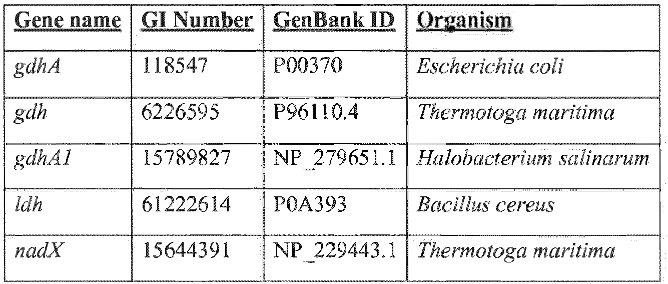

The lysine 6-dehydrogenase (deaminating), encoded by the *lysDH* genes, catalyze the oxidative deamination of the epsilon-amino group of L-lysine to form 2-aminoadipate-6-semialdehyde, which in turn nonenzymatically cyclizes to form Δ¹-piperideine-6-carboxylate (Misono et al., J.Bacteriol. 150:398-401 (1982)). Exemplary enzymes can be found in *Geobacillus stearothermophilus* (Heydari et al., Appl Environ.Microbiol 70:937-942 (2004)), *Agrobacterium tumefaciens* (Hashimoto et al., J Biochem 106:76-80 (1989); Misono et al., *supra*), and *Achromobacter denitrificans* (Ruldeekulthamrong et al., BMB.Rep. 41 :790-795 (2008)). Such enzymes are particularly good candidates for converting adipate semialdehyde to 6-aminocaproate given the structural similarity between adipate semialdehyde and 2-aminoadipate-6-semialdehyde.

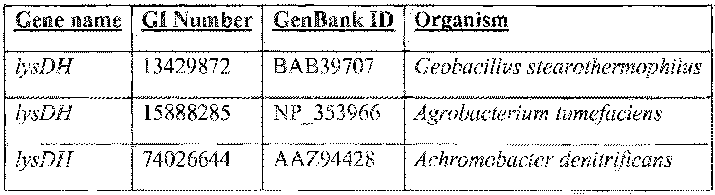

### EC 2.6.1.a Aminotransferase.

Step F of FIG. 8 can also, in certain embodiments, involve the transamination of a 6-aldehyde to an amine. This transformation can be catalyzed by gamma-aminobutyrate transaminase (GABA transaminase). One *E. coli* GABA transaminase is encoded by *gabT* and transfers an amino group from glutamate to the terminal aldehyde of succinyl semialdehyde (Bartsch et al., J. Bacteriol. 172:7035-7042 (1990)). The gene product of *puuE* catalyzes another 4-aminobutyrate transaminase in *E. coli* (Kurihara et al., J.Biol.Chem. 280:4602-4608 (2005)). GABA transaminases in *Mus musculus, Pseudomonas fluorescens,* and *Sus scrofa* have been shown to react with 6-aminocaproic acid (Cooper, Methods Enzymol. 113:80-82 (1985); Scott et al., J. Biol. Chem. 234:932-936 (1959)).

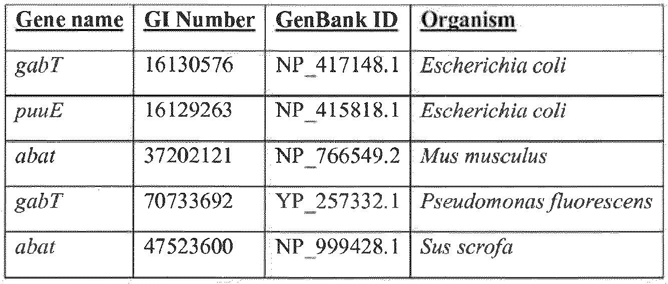

Additional enzyme candidates include putrescine aminotransferases or other diamine aminotransferases. Such enzymes are particularly well suited for carrying out the conversion of 6-aminocaproate semialdehyde to hexamethylenediamine. The *E. coli* putrescine aminotransferase is encoded by the *ygjG* gene and the purified enzyme also was able to transaminatc cadaverine and spermidine (Samsonova et al., BMC Wicrobiol 3:2 (2003)). In addition, activity of this enzyme on 1,7-diaminoheptane and with amino acceptors other than 2-oxoglutarate (e.g., pyruvate, 2-oxobutanoate) has been reported (Samsonova et al., *supra;* Kim, K.H., J Biol Chem 239:783-786 (1964)). A putrescine aminotransferase with higher activity with pyruvate as the amino acceptor than alpha-ketoglutarate is the *spuC* gene of *Pseudomonas aeruginosa* (Lu et al., J Bacteriol 184:3765-3773 (2002)).

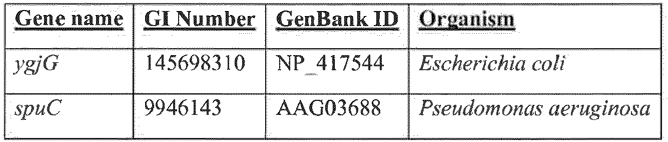

Yet additional candidate enzymes include beta-alanine/alpha-ketoglutarate aminotransferases which produce malonate semialdehyde from beta-alanine (WO08027742). The gene product of SkPYD4 in *Saccharomyces kluyveri* was also shown to preferentially use beta-alanine as the amino group donor (Andersen et al., FEBS.J. 274:1804-1817 (2007)). SkUGA1 encodes a homologue of *Saccharomyces cerevisiae* GABA aminotransferase, UGA1 (Ramos et al., Eur.J.Biochem., 149:401-404 (1985)), whereas SkPYD4 encodes an enzyme involved in both -alanine and GABA transamination (Andersen et al., *supra).* 3-Amino-2-methylpropionate transaminase catalyzes the transformation from methylmalonate semialdehyde to 3-amino-2-methylpropionate. This enzyme has been characterized in *Rattus norvegicus* and *Sus scrofa* and is encoded by *Abat* (Tamaki et al, Methods Enzymol, 324:376-389 (2000)).

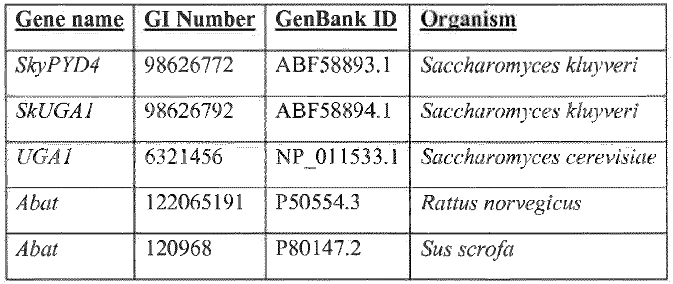

### Step G - 6-Aminocaproyl-CoA/Acyl-CoA Transferase or 6-Aminocaproyl-CoA Synthase.

### 2.8.3.a Coenzyme-A transferase.

CoA transferases catalyze reversible reactions that involve the transfer of a CoA moiety from one molecule to another. For example, step G can be catalyzed by a 6-aminocaproyl-CoA/Acyl CoA transferase. One candidate enzyme for these steps is the two-unit enzyme encoded by *pcaI* and *pcaJ* in *Pseudomonas,* which has been shown to have 3-oxoadipyl-CoA/succinate transferase activity ((Kaschabek and Reineke, J. Bacteriol. 177:320-325 (1995); and Kaschabek. and Reineke, J. Bacteriol. 175:6075-6081 (1993)). Similar enzymes based on homology exist in *Acinetobacter* sp. ADP1 (Kowalchuk et al., Gene 146:23-30 (1994)) and *Streptomyces coelicolor.* Additional exemplary succinyl-CoA:3:oxoacid-CoA transferases are present in *Helicobacter pylori* (Corthesy-Theulaz et al., J.Biol.Chem. 272:25659-25667 (1997)) and *Bacillus subtilis* (Stols et al., Protein. Expr. Purif 53:396-403 (2007)).

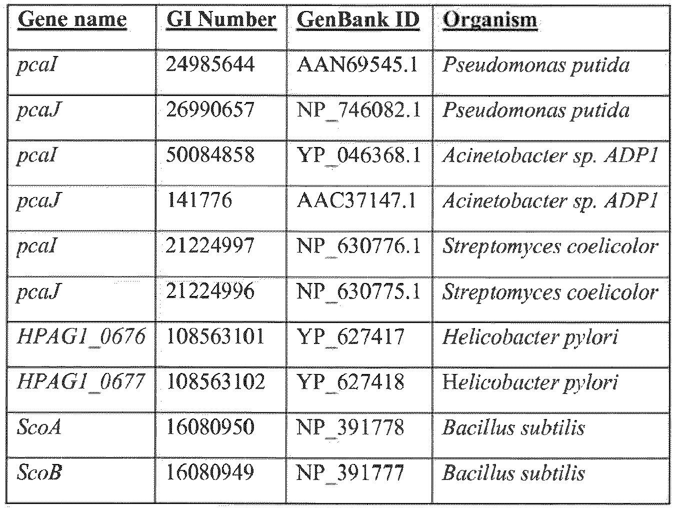

A 3-oxoacyl-CoA transferase that can utilize acetate as the CoA acceptor is acetoacetyl-CoA transferase, encoded by the *E. coli atoA* (alpha subunit) and *atoD* (beta subunit) genes (Vanderwinkel et al., Biochem.Biophys.Res Commun. 33:902-908 (1968); Korolev et al., Acta Cryslallogr.D Biol Crystallogr. 58:2116-2121 (2002)). This enzyme has also been shown to transfer the CoA moiety to acetate from a variety of branched and linear acyl-CoA substrates, including isobutyrate (Matthies et al., Appl Environ Microbiol 58:1435-1439 (1992)), valerate (Vanderwinkel et al., *supra)* and butanoate (Vanderwinkel et al., *supra*). Similar enzymes exist in *Corynebacterium glutamicum* ATCC 13032 (Duncan et al., Appl Environ Microbiol 68:5186-5190 (2002)), *Clostridium acetobutylicum* (Cary et al., Appl Environ Microbiol 56:1576-1583 (1990)), and *Clostridium saccharoperbutylacetonicum* (Kosaka et al., Biosci.Biotechnol Biochem. 71:58-68 (2007)).

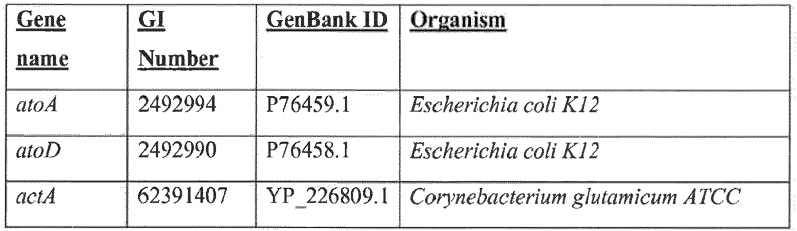

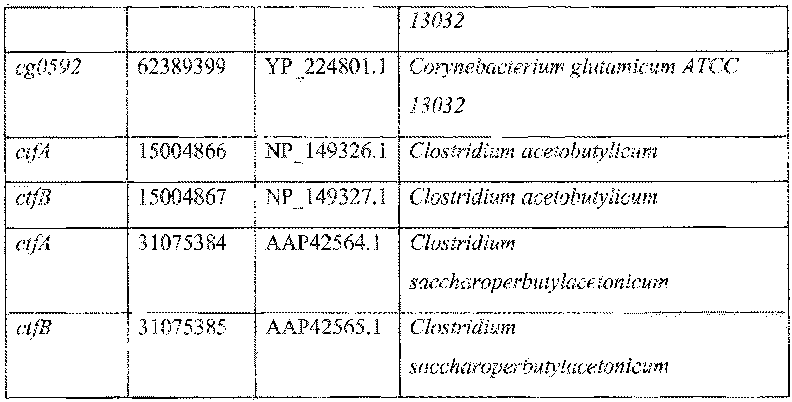

The above enzymes may also exhibit the desired activities on 6-aminocaproate and 6-aminocaproyl-CoA, as in step G. Nevertheless, additional exemplary transferase candidates are catalyzed by the gene products of *cat1, cat2,* and *cat3* of *Clostridium kluyveri* which have been shown to exhibit succinyl-CoA, 4-hydroxybutyryl-CoA, and butyryl-CoA transferase activity, respectively (Seedorf et al., *supra*;Sohling et al., Eur.JBiochem. 212:121-127 (1993);Sohling et al., J Bacterial. 178:871-880 (1996)).

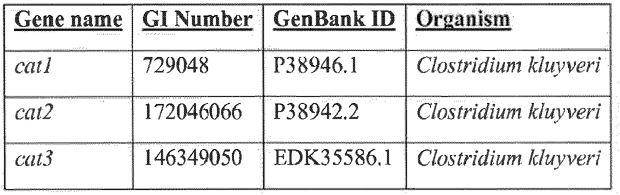

The glutaconate-CoA-transferase (EC 2.8.3.12) enzyme from anaerobic bacterium *Acidaminococcus fermentans* reacts with diacid glutaconyl-CoA and 3-butenoyl-CoA (Mack et al., FEBS Lett. 405:209-212 (1997)). The genes encoding this enzyme are *gctA* and *gctB.* This enzyme has reduced but detectable activity with other CoA derivatives including glutaryl-CoA, 2-hydroxyglutaryl-CoA, adipyl-CoA and acrylyl-CoA (Buckel et al., Eur.J.Biochem. 118:315-321 (1981)). The enzyme has been cloned and expressed in *E. coli* (Mack et al., Eur.J.Biochem. 226:41-51 (1994)).

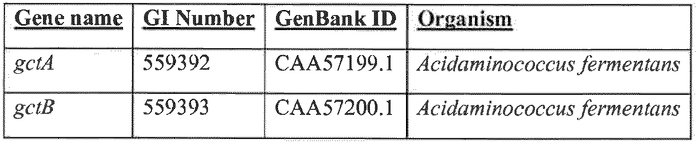

### EC 6.2.1.a Acid-thiol ligase.

Step G can also involve an acid-thiol ligase or synthetase functionality (the terms ligase, synthetase, and synthase are used herein interchangeably and refer to the same enzyme class). Exemplary genes encoding enzymes to carry out these transformations include the *sucCD* genes of *E. coli* which naturally form a succinyl-CoA synthetase complex. This enzyme complex naturally catalyzes the formation of succinyl-CoA from succinate with the contaminant consumption of one ATP, a reaction which is reversible *in vivo* (Buck et al., Biochem. 24:6245-6252 (1985)). Given the structural similarity between succinate and adipate, that is, both are straight chain dicarboxylic acids, it is reasonable to expect some activity of the *sucCD* enzyme on adipyl-CoA.

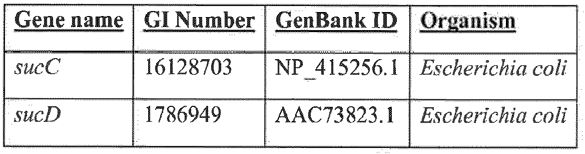

Additional exemplary CoA-ligases include the rat dicarboxylate-CoA ligase for which the sequence is yet uncharacterized (Vamecq et al., Biochemical Journal 230:683-693 (1985)), either of the two characterized phenylacetate-CoA ligases from *P. chrysogenum* (Lamas-Maceiras et al., Biochem. J. 395:147-155 (2005); Wang et al., Biochem Biophy Res Commun 360(2):453-458 (2007)), the phenylacetate-CoA ligase from *Pseudomonas putida* (Martinez-Blanco et al., J. Biol. Chem. 265:7084-7090 (1990)), and the 6-carboxyhexanoate-CoA ligase from *Bacilis subtilis* (Boweret al., J. Bacteriol. 178(14):4122-4130 (1996)). Additional candidate enzymes are acetoacetyl-CoA synthetases from *Mus musculus* (Hasegawa et al., Biochim Biophys Acta 1779:414-419 (2008)) and *Homo sapiens* (Ohgami et al., Biochem Pharmacol 65:989-994 (2003)) which naturally catalyze the ATP-dependent conversion of acetoacetate into acetoacetyl-CoA.

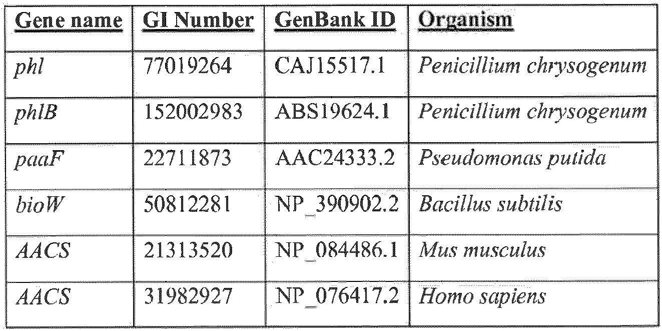

ADP-forming acetyl-CoA synthetase (ACD, EC 6.2.1.13) is another candidate enzyme that couples the conversion of acyl-CoA esters to their corresponding acids with the concurrent synthesis of ATP. Several enzymes with broad substrate specificities have been described in the literature. ACD I from *Archaeoglobusfulgidus,* encoded by AF1211, was shown to operate on a variety of linear and branched-chain substrates including acetyl-CoA, propionyl-CoA, butyryl-CoA, acetate, propionate, butyrate, isobutyryate, isovalerate, succinate, fumarate, phenylacetate, indoleacetate (Musfeldt et al., J Bacteriol 184:636-644 (2002)). The enzyme from *Haloarcula marismortui* (annotated as a succinyl-CoA synthetase) accepts propionate, butyrate, and branched-chain acids (isovalerate and isobutyrate) as substrates, and was shown to operate in the forward and reverse directions (Brasen et al., Arch Microbiol 182:277-287 (2004)). The ACD encoded by PAE3250 from hyperthermophilic crenarchaeon *Pyrobaculum aerophilum* showed the broadest substrate range of all characterized ACDs, reacting with acetyl-CoA, isobutyryl-CoA (preferred substrate) and phenylacetyl-CoA (Brasen et al., *supra*). The enzymes from *A. fulgidus, H. marismortui* and *P. aerophilum* have all been cloned, functionally expressed, and characterized in *E. coli* (Musfeldt et al., *supra;* Brasen et al., *supra*).

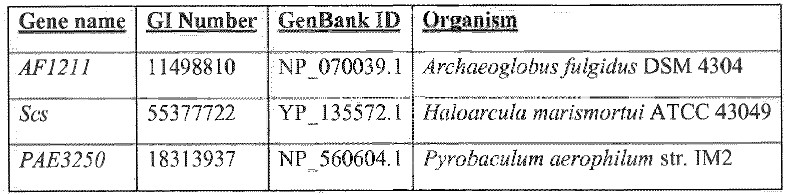

Yet another option is to employ a set of enzymes with net ligase or synthetase activity. For example, phosphotransadipylase and adipate kinase enzymes are catalyzed by the gene products of *buk1, buk2,* and *ptb* from C. *acetobutylicum* (Walter et al., Gene 134:107-111 (1993); Huang et al., J. Mol. Microbiol. Biotechnol. 2:33-38 (2000)). The *ptb* gene encodes an enzyme that can convert butyryl-CoA into butyryl-phosphate, which is then converted to butyrate via either of the *buk* gene products with the concomitant generation of ATP.

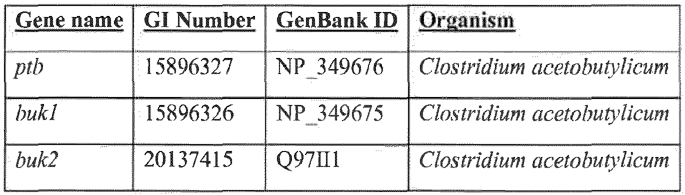

### Step H - Amidohydrolase. EC 6.3.1.a/6.3.2.a Amide synthases/peptide synthases.

The direct conversion of 6-aminocaproate to caprolactam as in step H can involve the formation of an intramolecular peptide bond. Ribosomes, which assemble amino acids into proteins during translation, are nature's most abundant peptide bond-forming catalysts. Nonribosomal peptide synthetases are peptide bond forming catalysts that do not involve messenger mRNA (Schwarzer et al., Nat Prod.Rep. 20:275-287 (2003)). Additional enzymes capable of forming peptide bonds include acyl-CoA synthetase from *Pseudomonas chlororaphis* (Abe et al., J Biol Chem 283:11312-11321 (2008)), gamma-Glutamylputrescine synthetase from *E. coli* (Kurihara et al., J Biol Chem 283:19981-19990 (2008)), and beta-lactam synthetase from *Streptomyces clavuligerus* (Bachmann et al., Proc Natl Acad Sci U SA 95:9082-9086 (1998);Bachmann et al., Biochemistry 39:11187-11193 (2000);Miller et al., Nat Struct.Biol 8:684-689 (2001);Miller et al., Proc Natl Acad Sci U S A 99:14752-14757 (2002);Tahlan et al., Antimicrob.Agents.Chemother. 48:930-939 (2004)).

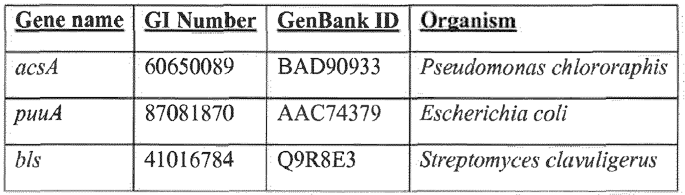

### Step 1 - Spontaneous Cyclization.

The conversion of 6-aminocaproyl-CoA to caprolactam can occur by spontaneous cyclization. Because 6-aminocaproyl-CoA can cyclize spontaneously to caprolactam, it eliminates the need for a dedicated enzyme for this step. A similar spontaneous cyclization is observed with 4-aminobutyryl-CoA which forms pyrrolidinonc (Ohsugi et al., J Biol Chem 256:7642-7651 (1981)).

### Step J - 6-Aminocaproyl-CoA Reductase (Aldehyde Forming).

The transformation of 6-aminocaproyl-CoA to 6-aminocaproate semialdehyde as in step J can involve an acyl-CoA dehydrogenases capable of reducing an acyl-CoA to its corresponding aldehyde. An EC 1.2.1.b oxidoreductase (acyl-CoA to aldehyde) provides suitable enzyme activity. Exemplary enzymes in this class are described herein and above.

### Step K - HMDA Transaminase or HMDA dehydrogenase.

### EC 1.4.1.a Oxidoreductase operating on amino acids.

Step K depicts a reductive animation and entails the conversion of 6-aminocaproate semialdehyde to hexamethylenediamine.

Most oxidoreductases operating on amino acids catalyze the oxidative deamination of alpha-amino acids with NAD+ or NADP+ as acceptor, though the reactions are typically reversible. Exemplary oxidoreductases operating on amino acids include glutamate dehydrogenase (deaminating), encoded by *gdhA,* leucine dehydrogenase (deaminating), encoded by *ldh,* and aspartate dehydrogenase (deaminating), encoded by *nadX.* The *gdhA* gene product from *Escherichia coli* (McPherson et al., Nucleic.Acids Res. 11:5257-5266 (1983); Korber et al., J. Mol. Biol. 234:1270-1273 (1993)), *gdh* from *Thermotoga maritima* (Kort et al., Extremophiles 1:52-60 (1997); Lebbink et al., J.Mol.Biol. 280:287-296 (1998); Lebbink et al., J.Mol.Biol. 289:357-369 (1999)), and *gdhA1* from *Halobacterium salinarum* (Ingoldsby et al., Gene. 349:237-244 (2005)) catalyze the reversible interconversion of glutamate to 2-oxoglutarate and ammonia, while favoring NADP(H), NAD(H), or both, respectively. The *ldh* gene of *Bacillus cereus* encodes the LeuDH protein that has a wide of range of substrates including leucine, isoleucine, valine, and 2-aminobutanoate (Stoyan et al., J.Biotechnol 54:77-80 (1997); Ansorge et al., Biotechnol Bioeng. 68:557-562 (2000)). The *nudX* gene from *Thermotoga maritime* encoding for the aspartate dehydrogenase is involved in the biosynthesis of NAD (Yang et al., J.Biol.Chem. 278:8804-8808 (2003)).

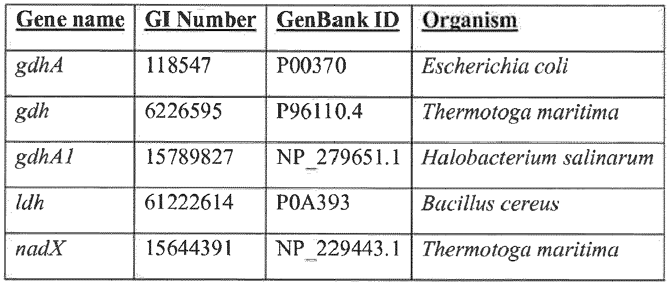

The lysine 6-dehydrogenase (deaminating), encoded by the *lysDH* genes, catalyze the oxidative deamination of the epsilon-amino group of L-lysine to form 2-aminoadipate-6-semialdehyde, which in turn nonenzymatically cyclizes to form Δ¹-piperideine-6-carboxylate (Misono et al., J.Bacteriol. 150:398-401 (1982)). Exemplary enzymes can be found in *Geobacillus stearothermophilus* (Heydari et al., Appl Environ.Microbiol 70:937-942 (2004)), *Agrobacterium tumefaciens* (Hashimoto et al., J Biochem 106:76-80 (1989); Misono et al., *supra),* and *Achromobacter denitrificans* (Ruldeekulthamrong et al., BMB.Rep. 41:790-795 (2008)). Such enzymes arc particularly good candidates for converting adipate semialdehyde to 6-aminocaproate given the structural similarity between adipate semialdehyde and 2-aminoadipate-6-semialdehyde.

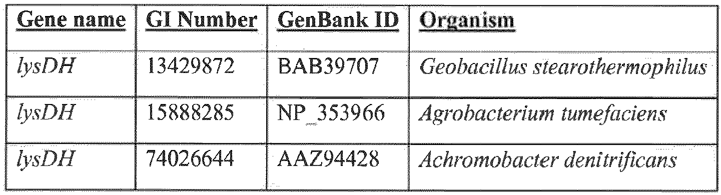

### EC 2.6.1.a Aminotransferase.

Step K, in certain embodiments, can involve the transamination of a 6-aldehyde to an amine. This transformation can be catalyzed by gamma-aminobutyrate transaminase (GABA transaminase). One *E. coli* GABA transaminase is encoded by *gabT* and transfers an amino group from glutamate to the terminal aldehyde of succinyl semialdehyde (Bartsch et al., J. Bacteriol. 172:7035-7042 (1990)). The gene product of *puuE* catalyzes another 4-aminobutyrate transaminase in *E. coli* (Kurihara et al., J.Biol.Chem. 280:4602-4608 (2005)). GABA transaminases in *Mus musculus, Pseudomonas fluorescens,* and *Sus scrofa* have been shown to react with 6-aminocaproic acid (Cooper, Methods Enzymol. 113:80-82 (1985); Scott et al., J. Biol. Chem. 234:932-936 (1959)).

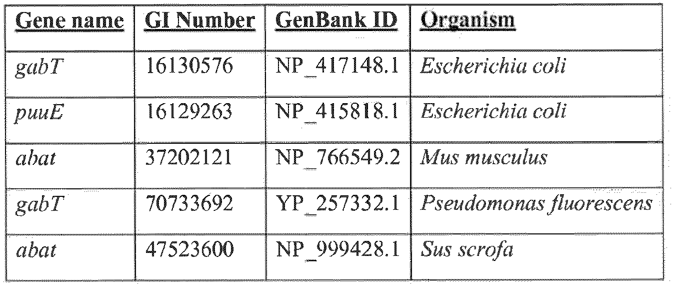

Additional enzyme candidates include putrescine aminotransferases or other diamine aminotransferases. Such enzymes are particularly well suited for carrying out the conversion of 6-aminocaproatc semialdehyde to hcxamcthylcncdiaminc. The *E. coli* putrescine aminotransferase is encoded by the *ygjG* gene and the purified enzyme also was able to transaminate cadaverine and spermidine (Samsonova et al., BNIC Microbiol 3:2 (2003)). In addition, activity of this enzyme on 1,7-diaminoheptane and with amino acceptors other than 2-oxoglutarate (e.g., pyruvate, 2-oxobutanoate) has been reported (Samsonova et al., *supra;* Kim, K.H., J Biol Chem 239:783-786 (1964)). A putrescine aminotransferase with higher activity with pyruvate as the amino acceptor than alpha-ketoglutarate is the *spuC* gene of *Pseudomonas aeruginosa* (Lu et al., J Bacteriol 184:3765-3773 (2002)).

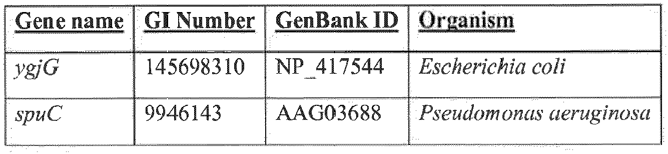

Yet additional candidate enzymes include beta-alanine/alpha-ketoglutarate aminotransferases which produce malonate semialdehyde from beta-alanine (WO08027742). The gene product of SkPYD4 in *Saccharomyces kluyveri* was also shown to preferentially use beta-alanine as the amino group donor (Andersen et al., FEBS.J. 274:1804-1817 (2007)). SkUGAl encodes a homologue of *Saccharomyces cerevisiae* GABA aminotransferase, UGA1 (Ramos et al., Eur.J.Biochem., 149:401-404 (1985)), whereas SkPYD4 encodes an enzyme involved in both -alanine and GABA transamination (Andersen et al., *supra*). 3-Amino-2-methylpropionate transaminase catalyzes the transformation from methylmalonate semialdehyde to 3-amino-2-methylpropionate. This enzyme has been characterized in *Rattus norvegicus* and *Sus scrofa* and is encoded by *Abat* (Tamaki et al, Methods Enzymol, 324:376-389 (2000)).

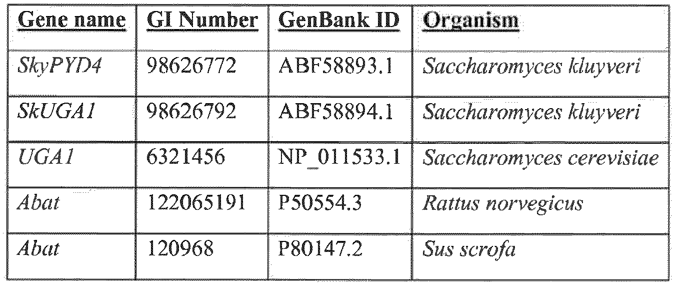

### Step L - Adipyl-CoA Hydrolase, Adipyl-CoA Ligase, Adipyl-CoA Transferase or Phosphotransadipylase/Adipate Kinase.

Step L can involve adipyl-CoA synthetase (also referred to as adipate-CoA ligase), phosphotransadipylase/adipate kinase, adipyl-CoA:acetyl-CoA transferase, or adipyl-CoA hydrolase. From an energetic standpoint, it is desirable for the final step in the adipate synthesis pathway to be catalyzed by an enzyme or enzyme pair that can conserve the ATP equivalent stored in the thioester bond of adipyl-CoA. The product of the *sucC* and *sucD* genes of *E*. *coli,* or homologs thereof, can potentially catalyze the final transformation shown in FIG. 8 should they exhibit activity on adipyl-CoA. The *sucCD* genes naturally form a succinyl-CoA synthetase complex that catalyzes the formation of succinyl-CoA from succinate with the concaminant consumption of one ATP, a reaction which is reversible *in vivo* (Buck et al., Biochem. 24:6245-6252 (1985)). Given the structural similarity between succinate and adipate, that is, both are straight chain dicarboxylic acids, it is reasonable to expect some activity of the *sucCD* enzyme on adipyl-CoA. An enzyme exhibiting adipyl-CoA ligase activity can equivalently carry out the ATP-generating production of adipate from adipyl-CoA, here using AMP and PPi as cofactors, when operating in the opposite physiological. Exemplary CoA-ligases include the rat dicarboxylate-CoA ligase for which the sequence is yet uncharacterized (Vamecq et al., Biochem. J. 230:683-693 (1985)), either of the two characterized phenylacetate-CoA ligases from *P*. *chrysogenum* (Lamas-Maceiras et al., Biochem. J. 395, 147-155 (2005); Wang et al., Biochem. Biophy. Res. Commun. 360:453-458 (2007)), the phenylacetate-CoA ligase from *Pseudomonas putida* (Martinez-Blanco et al., J. Biol. Chem. 265:7084-7090 (1990)), and the 6-carboxyhexanoate-CoA ligase from *Bαcilis subtilis* (Bower et al., J. Bacteriol. 178:4122-4130 (1996)). The protein sequences for each of these exemplary gene products can be found using the following GI numbers and/or GenBank identifiers:

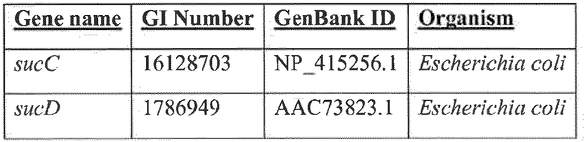

Another option, using phosphotransadipylase/adipate kinase, is catalyzed by the gene products of *buk1, buk2,* and *ptb* from *C. acetobutylicum* (Walter et al., Gene 134:107-111 (1993); Huang et al., J. Mol. Microbiol. Biotechnol. 2:33-38 (2000)), or homologs thereof. The *ptb* gene encodes an enzyme that can convert butyryl-CoA into butyryl-phosphate, which is then converted to butyrate via either of the *buk* gene products with the concomitant generation of ATP. The analogous set of transformations, that is, conversion of adipyl-CoA to adipyl-phosphate followed by conversion of adipyl-phosphate to adipate, can be carried out by the *buk1, buk2, and ptb* gene products. The protein sequences for each of these exemplary gene products can be found using the following GI numbers and/or GenBank identifiers:

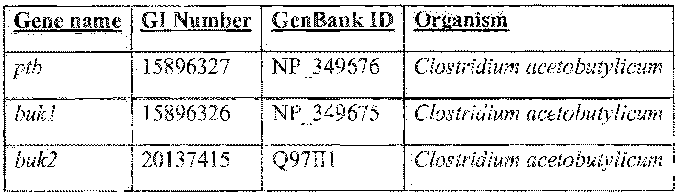

Alternatively, an acetyltransferase capable of transferring the CoA group from adipyl-CoA to acetate can be applied. Similar transformations are catalyzed by the gene products of *cat1, cat2,* and *cat3* of *Clostridium kluyveri* which have been shown to exhibit succinyl-CoA, 4-hydroxybutyryl-CoA, and butyryl-CoA acetyltransferase activity, respectively (Sohling and Gottschalk, J. Bacteriol. 178:871-880 (1996); Seedorf et al., Proc. Natl. Acad. Sci. USA 105:2128-2133 (2008)). The protein sequences for each of these exemplary gene products can be found using the following GI numbers and/or GenBank identifiers:

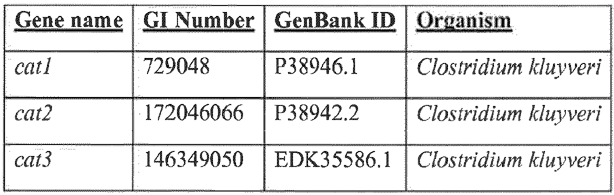

Finally, though not as desirable from an energetic standpoint, the conversion of adipyl-CoA to adipate can also be carried out by an acyl-CoA hydrolase or equivalently a thioesterase. The top *E. coli* gene candidate is *tesB* (Naggert et al., J. Biol. Chem. 266:11044-11050 (1991)), which shows high similarity to the human *acot8,* which is a dicarboxylic acid acetyltransferase with activity on adipyl-CoA (Westin et al., J. Biol. Chem. 280:38125-38132 (2005)). This activity has also been characterized in the rat liver (Deana, Biochem. Int. 26:767-773 (1992)). The protein sequences for each of these exemplary gene products can be found using the following GI numbers and/or GenBank identifiers:

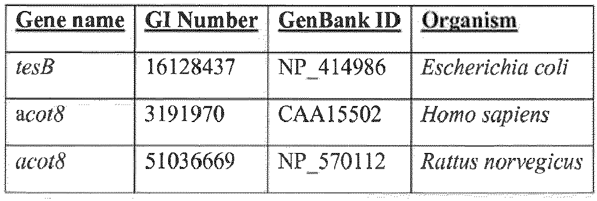

Other native candidate genes include *tesA* (Bonner and Bloch, J. Biol. Chem. 247:3123-3133 (1972)), *ybgC* (Kuznetsova et al., FEMS Microbiol. Rev. 29:263-279 (2005); Zhuang et al., FEBS Lett. 516:161-163 (2002)), *paaI* (Song et al., J. Biol. Chem. 281:11028-11038 (2006)), and *ybdB* (Leduc et al., J. Bacteriol. 189:7112-7126 (2007)). The protein sequences for each of these exemplary gene products can be found using the following GI numbers and/or GenBank identifiers:

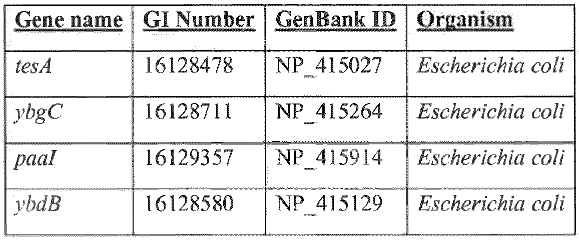

### EC 2.8.3.a Coenzyme-A transferase.

CoA transferases catalyze reversible reactions that involve the transfer of a CoA moiety from one molecule to another. For example, step L can be catalyzed by a adipyl-CoA transferase. One candidate enzyme for this step is the two-unit enzyme encoded by *peaI* and *pcaJ* in *Pseudomonas,* which has been shown to have 3-oxoadipyl-CoA/succinatc transferase activity (Kaschabek and Reineke, J. Bacteriol. 177:320-325 (1995); and Kaschabek. and Reineke, J. Bacteriol. 175:6075-6081 (1993)). Similar enzymes based on homology exist in *Acinetobacter* sp. ADP1 (Kowalchuk et al., Gene 146:23-30 (1994)) and *Streptomyces coelicolor.* Additional exemplary succinyl-CoA:3:oxoacid-CoA transferases are present in *Helicobacter pylori* (Corthesy-Theulaz et al., J.Biol.Chem. 272:25659-25667 (1997)) and *Bacillus subtilis* (Stols et al., Protein.Expr.Purif. 53:396-403 (2007)).

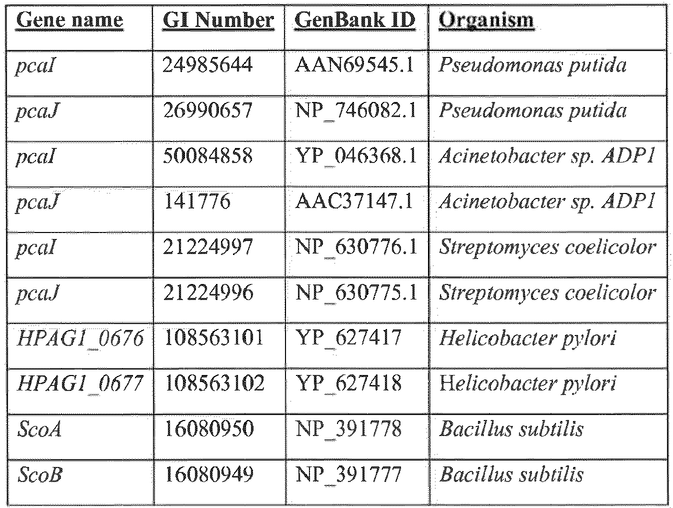

A 3-oxoacyl-CoA transferase that can utilize acetate as the CoA acceptor is acetoacetyl-CoA transferase, encoded by the *E. coli atoA* (alpha subunit) and *atoD* (beta subunit) genes (Vanderwinkel et al., Biochem. Biophys. Res Commun. 33:902-908 (1968); Korolev et al., Acta Crystallogr.D Biol Crystallogr. 58:2116-2121 (2002)). This enzyme has also been shown to transfer the CoA moiety to acetate from a variety of branched and linear acyl-CoA substrates, including isobutyrate (Matthies et al., Appl Environ Microbiol 58:1435-1439 (1992)), valerate (Vanderwinkel et al., Biochem. Biophys. Res. Commun. 33:902-908 (1968)) and butanoate (Vanderwinkel et al., *supra).* Similar enzymes exist in *Corynebacterium glutamicum* ATCC 13032 (Duncan et al., Appl Environ Microbiol 68:5186-5190 (2002)), *Clostridium acetobutylicum* (Cary et al., Appl Environ Microbiol 56:1576-1583 (1990)), and *Clostridium saccharoperbutylacetonicum* (Kosaka et al., Biosci.Biotechnol Biochem. 71:58-68 (2007)).

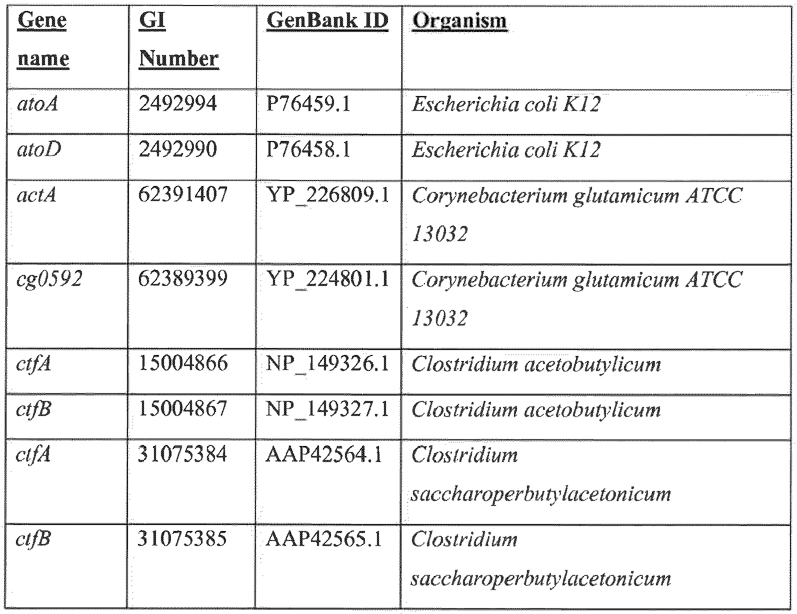

The above enzymes may also exhibit the desired activities on adipyl-CoA and adipate for step L. Nevertheless, additional exemplary transferase candidates are catalyzed by the gene products of *cat1, cat2,* and *cat3* of *Clostridium kluyveri* which have been shown to exhibit succinyl-CoA, 4-hydroxybutyryl-CoA, and butyryl-CoA transferase activity, respectively (Seedorf et al., Proc. Natl. Acad. Sci U. S. A 105:2128-2133 (2008); Sohling et al., Eur.J Biochem. 212:121-127 (1993); Sohling et al., J Bacteriol. 178:871-880 (1996)).

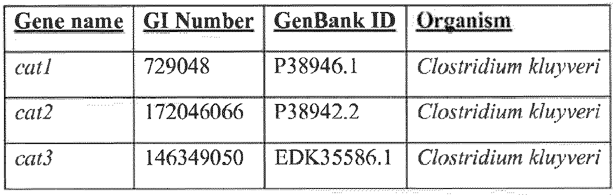

The glutaconate-CoA-transferase (EC 2.8.3.12) enzyme from anaerobic bacterium *Acidaminococcus fermentans* reacts with diacid glutaconyl-CoA and 3-butenoyl-CoA (Mack et al., FEBS Lett. 405:209-212 (1997)). The genes encoding this enzyme are *gctA* and *gctB.* This enzyme has reduced but detectable activity with other CoA derivatives including glutaryl-CoA, 2-hydroxyglutaryl-CoA, adipyl-CoA and acrylyl-CoA (Buckel et al., Eur.J.Biochem. 118:315-321 (1981)). The enzyme has been cloned and expressed in *E. coli* (Mack et al., Eur.J.Biochem. 226:41-51 (1994)).

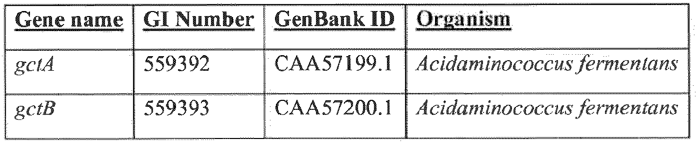

### EXAMPLE XIII

### Methacrylic Acid Synthesis Enzymes

This Example provides genes that can be used for conversion of succinyl-CoA to methacrylic acid as depicted in the pathways of Figure 9.

FIG. 9. depicts 3-Hydroxyisobutyrate and methacrylic acid production are carried out by the following enzymes: A) Methylmalonyl-CoA mutase, B) Methylmalonyl-CoA epimerase, C) Methylmalonyl-CoA reductase (aldehyde forming), D) Methylmalonate semialdehyde reductase, E) 3-hydroxyisobutyrate dehydratase, F) Methylmalonyl-CoA reductase (alcohol forming).

### Step A - Methylmalonyl-CoA mutase (designated as EMA2).

Methylmalonyl-CoA mutase (MCM) (EMA2) (EC 5.4.99.2) is a cobalamin-dependent enzyme that converts succinyl-CoA to methylmalonyl-CoA. In *E. coli,* the reversible adenosylcobalamin-dependent mutase participates in a three-step pathway leading to the conversion of succinate to propionate (Haller et al., Biochemistry 39:4622-4629 (2000)). Overexpression of the EMA2 gene candidate along with the deletion of *YgfG* can be used to prevent the decarboxylation of methylmalonyl-CoA to propionyl-CoA and to maximize the methylmalonyl-CoA available for MAA synthesis. EMA2 is encoded by genes *scpA* in *Escherichia coli* (Bobik and Rasche, Anal. Bioanal. Chem. 375:344-349 (2003); Haller et al., Biochemistry 39:4622-4629 (2000)) and *muta* in *Homo sapiens* (Padovani and Banerjee, Biochemistry 45:9300-9306 (2006)). In several other organisms EMA2 contains alpha and beta subunits and is encoded by two genes. Exemplary gene candidates encoding the two-subunit protein are *Propionibacterium fredenreichii sp. shermani mutA* and *mutB* (Korotkova and Lidstrom, J. Biol. Chem. 279:13652-13658 (2004)), *Methylobacterium extorquens mcmA* and *mcmB* (Korotkova and Lidstrom, *supra,* 2004), and *Ralstonia eutropha sbm1* and *sbm2* (Peplinski et al., Appl. Microbiol. Biotech. 88:1145-59 (2010)). Additional enzyme candidates identified based on high homology to the E. coli spcA gene product are also listed below.

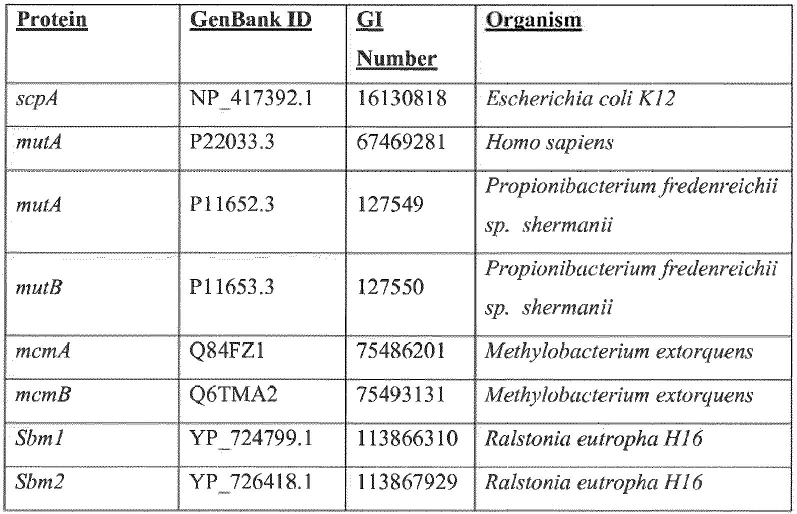

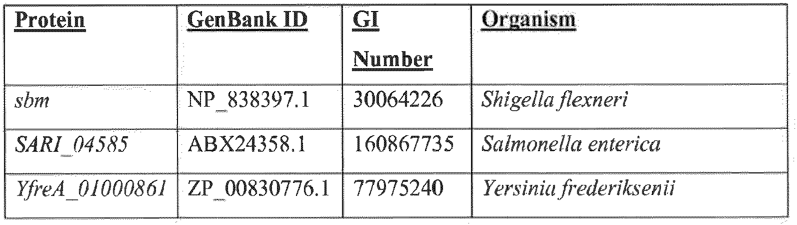

These sequences can be used to identify homologue proteins in GenBank or other databases through sequence similarity searches (for example, BLASTp). The resulting homologue proteins and their corresponding gene sequences provide additional exogenous DNA sequences for transformation into *E. coli* or other suitable host microorganisms to generate production hosts. Additional gene candidates include the following, which were identified based on high homology to the *E. coli spcA* gene product.

There further exists evidence that genes adjacent to the EMA2 catalytic genes contribute to maximum activity. For example, it has been demonstrated that the *meaB* gene from *M. extorquens* forms a complex with EMA2, stimulates *iin vitro* mutase activity, and possibly protects it from irreversible inactivation (Korotkova and Lidstrom, J. Biol. Chem. 279:13652-13658 (2004)). The *M. extorquens meaB* gene product is highly similar to the product of the *E. coli argK* gene (BLASTp: 45% identity, e-value: 4c-67), which is adjacent to *scpA* on the chromosome. No sequence for a *meaB* homolog in *P. freudenreichii* is catalogued in GenBank. However, the *Propionibacterium acnes* KPA171202 gene product, YP_055310.1, is 51% identical to the *M. extorquens meaB* protein and its gene is also adjacent to the EMA2 gene on the chromosome. A similar gene is encoded by H16_B1839 of *Ralstonia eutropha H16.*

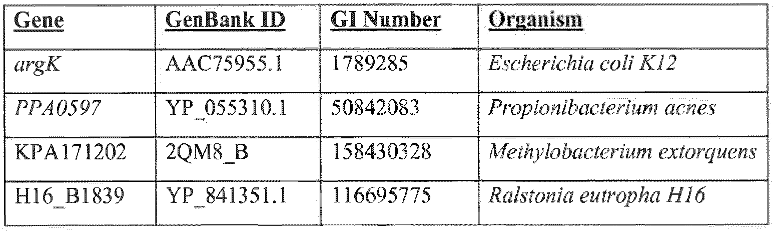

*E. coli* can synthesize adcnosylcobalamin, a necessary cofactor for this reaction, only when supplied with the intermediates cobinamide or cobalamin (Lawrence and Roth. J. Bacteriol. 177:6371-6380 (1995); Lawrence and Roth, Genetics 142:11-24 (1996)). Alternatively, the ability to synthesize cobalamins *de novo* has been conferred upon *E*. *coli* following the expression of heterologous genes (Raux et al., J. Bacteriol. 178:753-767 (1996)).
Alternatively, isobutyryl-CoA mutase (ICM) (EC 5.4.99.13) could catalyze the proposed transformation shown in FIG., step B. ICM is a cobalamin-dependent methylmutase in the EMA2 family that reversibly rearranges the carbon backbone of butyryl-CoA into isobutyryl-CoA (Ratnatilleke et al., J. Biol. Chem. 274:31679-31685 (1999)). A recent study of a novel ICM in *Methylibium petroleiphilum,* along with previous work, provides evidence that changing a single amino acid near the active site alters the substrate specificity of the enzyme (Ratnatilleke et al., J. Biol. Chem. 274:31679-31685 (1999); Rohwerder et al., Appl. Environ. Microbiol. 72:4128-4135. (2006)). This indicates that, if a native enzyme is unable to catalyze or exhibits low activity for the conversion of 4HB-CoA to 3HIB-CoA, the enzyme can be rationally engineered to increase this activity. Exemplary ICM genes encoding homodimeric enzymes include *icmA* in *Streptomyces coelicolor A3* (Alhapel et al., Proc. Natl. Acad. Sci. USA 103:12341-12346 (2006)) and *Mpe_B0541* in *Methylibium petroleiphilum PM1* (Ratnatilleke et al., J. Biol. Chem. 274:31679-31685 (1999); Rohwerder et al., Appl. Environ. Microbiol. 72:4128-4135 (2006)). Genes encoding heterodimeric enzymes include *icm* and *icmB* in *Streptomyces cinnamonensis* (Ratnatilleke et al., J. Biol. Chem. 274:31679-31685 (1999); Vrijbloed et al., J. Bacteriol. 181:5600-5605. (1999); Zerbe-Burkhardt et al., J. Biol. Chem. 273:6508-6517 (1998)). Enzymes encoded by *icmA* and *icmB* genes in *Streptomyces avermitilis MA-4680* show high sequence similarity to known ICMs. These genes/proteins are identified below.

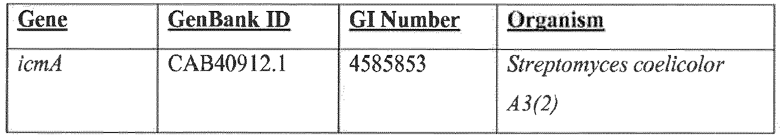

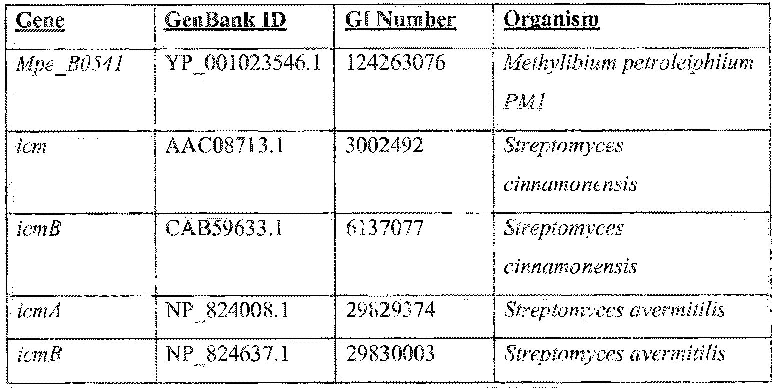

### Step B - Methylmalonyl-CoA epimerase (designated as EMA3).

Methylmalonyl-CoA epimerase (MMCE) (EMA3) is the enzyme that interconverts (R)-methylmalonyl-CoA and (S)-methylmalonyl-CoA. EMA3 is an essential enzyme in the breakdown of odd-numbered fatty acids and of the amino acids valine, isoleucine, and methionine. EMA3 activity is not believed to be encoded in the *E. coli* genome (Boynton et al., J. Bacteriol.178:3015-3024 (1996)), but is present in other organisms such as *Homo sapiens* (*YqjC*) (Fuller and Leadlay,. Biochem. J. 213:643-650 (1983)), *Rattus norvegicus* (*Mcee*) (Bobik and Rasche, J. Biol. Chem. 276:37194-37198 (2001)), *Propionibacterium shermanii* (AF454511) (Fuller, and Leadlay, Biochem. J. 213:643-650 (1983); Haller et al., Biochemistry 39:4622-4629 (2000); McCarihy et al.,. Structure 9:637-646.2001)) and *Caenorhabditis elegans* (*mmce*) (Kuhnl et al., FEBS J. 272:1465-1477 (2005)). An additional gene candidate, AE016877 in *Bacillus cereus,* has high sequence homology to other characterized enzymes. This enzymatic step may or may not be necessary depending upon the stereospecificity of the enzyme or enzymes used for the conversion of methylmalonyl-CoA to 3-HIB. These genes/proteins are described below.

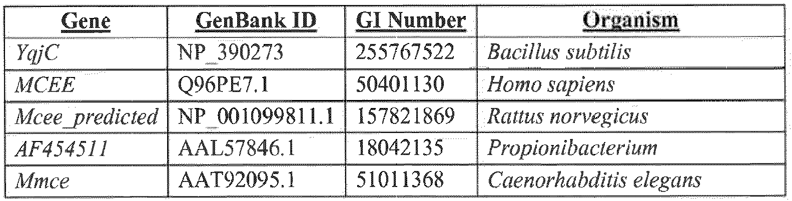

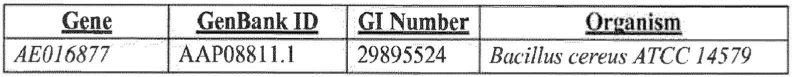

### Step C - Methylmalonyl-CoA reductase (aldehyde forming) (designated as EMA4).

The reduction of methylmalonyl-CoA to its corresponding aldehyde, methylmalonate semialdehyde, is catalyzed by a CoA-dependent aldehyde dehydrogenase (EC 1.2.1.-). Conversion of methylmalonyl-CoA to methylmalonic semialdehyde, is accomplished by a CoA-dependent aldehyde dehydrogenase. An enzyme encoded by a malonyl-CoA reductase gene from *Sulfolobus tokodaii* (Alber et. al., J. Bacteriol. 188(24):8551-8559 (2006)), has been shown to catalyze the conversion of methylmalonyl-CoA to its corresponding aldehyde (WO2007141208). A similar enzyme exists in *Metallosphaera sedula* (Alber et. al., J. Bacteriol. 188(24):8551-8559 (2006)). Several additional CoA dehydrogenases arc capable also of reducing an acyl-CoA to its corresponding aldehyde. The reduction of methylmalonyl-CoA to its corresponding aldehyde, methylmalonate semialdehyde, is catalyzed by a CoA-dependent aldehyde dehydrogenase. Exemplary enzymes include fatty acyl-CoA reductase, succinyl-CoA reductase (EC 1.2.1.76), acetyl-CoA reductase and butyryl-CoA reductase. Exemplary fatty acyl-CoA reductase enzymes are encoded by *acr1* of *Acinetobacter calcoaceticus* (Reiser and Somerville. J. Bacteriol. 179:2969-2975 (1997)), and *Acinetobacter sp. M-1* fatty acyl-CoA reductase (Ishige et al., Appl. Environ. Microbiol. 68:1192-1195 (2002)). Also known is a CoA- and NADP- dependent succinate semialdehyde dehydrogenase (also referred to as succinyl-CoA reductase) encoded by the *sucD* gene in *Clostridium kluyver*i (Sohling and Gottschalk, J. Bacteriol. 178:871-880 (1996); Sohling and Gottschalk, J. Bacteriol. 178:871-880 (1996)) and *sucD* of *P. gingivalis* (Takahashi, J. Bacteriol 182:4704-4710 (2000)). Additional succinyl-CoA reductase enzymes participate in the 3-hydroxypropionate/4-hydroxybutyrate cycle of thermophilic archaea including *Metallosphaera sedula* (Berg et al., Science 318:1782-1786 (2007)) and *Thermoproteus neutrophilus* (Ramos-Vera et al., J Bacteriol, 191:4286-4297 (2009)). The *M. sedula* enzyme, encoded by *Msed_0709,* is strictly NADPH-dependent and also has malonyl-CoA reductase activity. The T. *neutrophilus* enzyme is active with both NADPH and NADH. The enzyme acylating acetaldehyde dehydrogenase in *Pseudomonas sp,* encoded by *bphG,* is also a good candidate as it has been demonstrated to oxidize and acylate acetaldehyde, propionaldehyde, butyraldehyde, formaldehyde and the branched-chain compound isobutyraldehyde (Powlowski et al., J. Bacteriol. 175:377-385 (1993)). In addition to reducing acetyl-CoA to ethanol, the enzyme encoded by *adhE* in *Leuconostoc mesenteroides* has been shown to oxidize the branched chain compound isobutyraldehyde to isobutyryl-CoA (Kazahaya, J. Gen. Appl. Microbiol. 18:43-55 (1972); and Koo et al., Biotechnol Lett. 27:505-510 (2005)). Butyraldehyde dehydrogenase catalyzes a similar reaction, conversion of butyryl-CoA to butyraldehyde, in solventogenic organisms such as *Clostridium saccharoperbutylacetonicum* (Kosaka et al., Biosci Biotechnol Biochem., 71:58-68 (2007)).

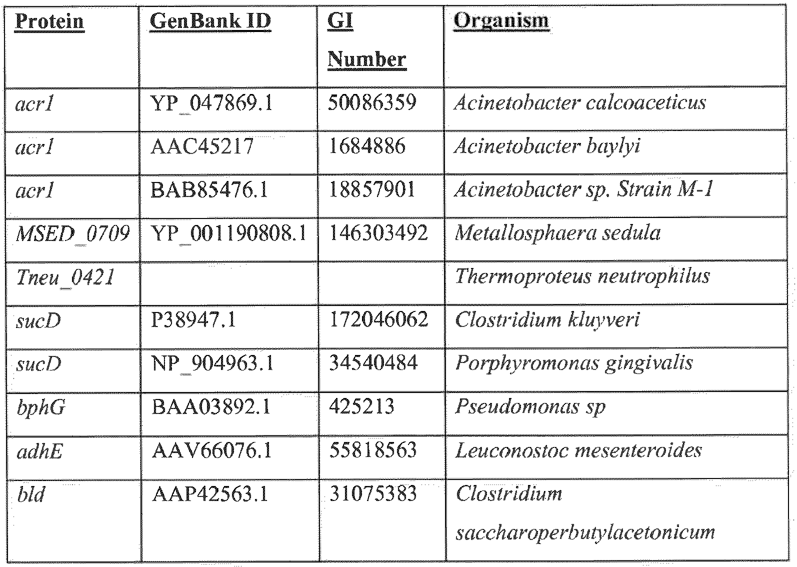

An additional enzyme type that converts an acyl-CoA to its corresponding aldehyde is malonyl-CoA reductase which transforms malonyl-CoA to malonic semialdehyde. Malonyl-CoA reductase is a key enzyme in autotrophic carbon fixation via the 3-hydroxypropionate cycle in thermoacidophilic archaeal bacteria (Berg, Science 318:1782-1786 (2007); and Thauer, Science 318:1732-1733 (2007)). The enzyme utilizes NADPH as a cofactor and has been characterized in *Metallosphaera* and *Sulfolobus sp.* (Alber et al., J. Bacteriol. 188:8551-8559 (2006); and Hugler, J. Bacteriol. 184:2404-2410 (2002)). The enzyme is encoded by *Msed_0709* in *Metallosphaera sedula* (Alber et al., J. Bacteriol. 188:8551 -8559 (2006); and Berg, Science 318:1782-1786 (2007)). A gene encoding a malonyl-CoA reductase from *Sulfolobus tokodaii* was cloned and heterologously expressed in *E*. *coli* (Alber et al., J. Bacteriol 188:8551-8559 (2006). This enzyme has also been shown to catalyze the conversion of methylmalonyl-CoA to its corresponding aldehyde (WO2007141208 (2007)). Although the aldehyde dehydrogenase functionality of these enzymes is similar to the bifunctional dehydrogenase from *Chloroflexus aurantiacus,* there is little sequence similarity. Both malonyl-CoA reductase enzyme candidates have high sequence similarity to aspartatesemialdehyde dehydrogenase, an enzyme catalyzing the reduction and concurrent dephosphorylation of aspartyl-4-phosphate to aspartate semialdehyde. Additional gene candidates can be found by sequence homology to proteins in other organisms including *Sulfolobus solfataricus* and *Sulfolobus acidocaldarius* and have been listed below. Yet another candidate for CoA-acylating aldehyde dehydrogenase is the *ald* gene from *Clostridium beijerinckii* (Toth, Appl. Environ. Microbiol. 65:4973-4980 (1999). This enzyme has been reported to reduce acetyl-CoA and butyryl-CoA to their corresponding aldehydes. This gene is very similar to *eutE* that encodes acetaldehyde dehydrogenase of *Salmonella typhimurium* and *E. coli* (Toth, Appl. Environ. Microbiol. 65:4973-4980 (1999).

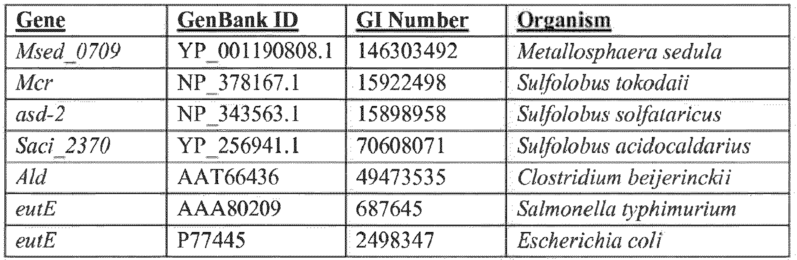

A bifunctional enzyme with acyl-CoA reductase and alcohol dehydrogenase activity can directly convert methylmalonyl-CoA to 3-HIB. Exemplary bifunctional oxidoreductases that convert an acyl-CoA to alcohol include those that transform substrates such as acetyl-CoA to ethanol (for example, *adhE* from *E. coli* (Kessler et al., FEBS.Lett. 281:59-63 (1991))) and butyryl-CoA to butanol (for example, *adhE2* from *C*. *acetobutylicum* (Fontaine et al., J.Bacteriol. 184:821-830 (2002)). The *C*. *acetobutylicum* enzymes encoded by *bdh* I and *bdh* II (Walter, et al., J. Bacteriol. 174:7149-7158 (1992)), reduce acetyl-CoA and butyryl-CoA to ethanol and butanol, respectively. In addition to reducing acetyl-CoA to ethanol, the enzyme encoded by *adhE* in *Leuconostoc mesenteroides* has been shown to oxide the branched chain compound isobutyraldehyde to isobutyryl-CoA (Kazahaya et al., J.Gen.Appl.Microbiol. 18:43-55 (1972); Koo et al., Biotechnol Lett, 27:505-510 (2005)). Another exemplary enzyme can convert malonyl-CoA to 3-HP. An NADPH-dependent enzyme with this activity has characterized in *Chloroflexus aurantiacus* where it participates in the 3-hydroxypropionate cycle (Hugler et al., J Bacteriol, 184:2404-2410 (2002); Strauss et al., Eur JBiochem, 215:633-643 (1993)). This enzyme, with a mass of 300 kDa, is highly substrate-specific and shows little sequence similarity to other known oxidoreductases (Hugler *et al*., *supra).* No enzymes in other organisms have been shown to catalyze this specific reaction; however there is bioinformatic evidence that other organisms may have similar pathways (Klatt et al., Env Microbiol, 9:2067-2078 (2007)). Enzyme candidates in other organisms including *Roseiflexus castenholzii, Erythrobacter sp. NAP1* and marine gamma proteobacterium HTCC2080 can be inferred by sequence similarity.

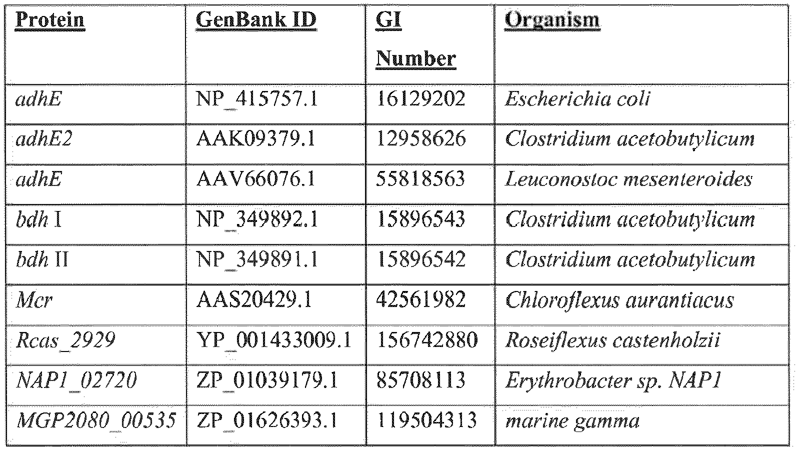

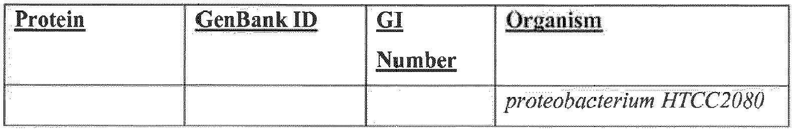

### Step D - Methylmalonate semialdehyde reductase (designated as EMA5).

The reduction of methylmalonate semialdehyde to 3-HIB is catalyzed by EMA5 or 3-HIB dehydrogenase. This enzyme participates in valine, leucine and isoleucine degradation and has been identified in bacteria, eukaryotes, and mammals. The enzyme encoded by *P84067* from *Thermus thermophilus* HB8 has been structurally characterized (Lokanath et al., J. Mol. Biol. 352:905-917 (2005)). The reversibility of the human 3-HIB dehydrogenase was demonstrated using isotopically-labeled substrate (Manning and Pollitt, Biochem. J. 231:481-484 (1985)). Additional genes encoding this enzyme include *3hidh* in *Homo sapiens* (Hawes et al., Methods Enzymol. 324:218-228 (2000)) and *Oryctolagus cuniculus* (Chowdhury et al., Biosci. Biotechnol. Biochem. 60:2043-2047 (1996); Hawes et al., Methods Enzymol. 324:218-228 (2000)), *mmsb* in *Pseudomonas aeruginosa,* and *dhat* in *Pseudomonas putida* (Aberhart and Hsu J Chem.Soc.[Perkin 1] 6:1404-1406 (1979); Chowdhury et al., Biosci. Biotechnol. Biochem. 67:438-441 (2003); Chowdhury et al., Biosci. Biotechnol. Biochem. 60:2043-2047 (1996)). Several 3-HIB dehydrogenase enzymes have been characterized in the reductive direction, including *mmsB* from *Pseudomonas aeruginosa* (Gokarn et al., US Patent 7,393,676 (2008)) and *mmsB* from *Pseudomonas putida.*

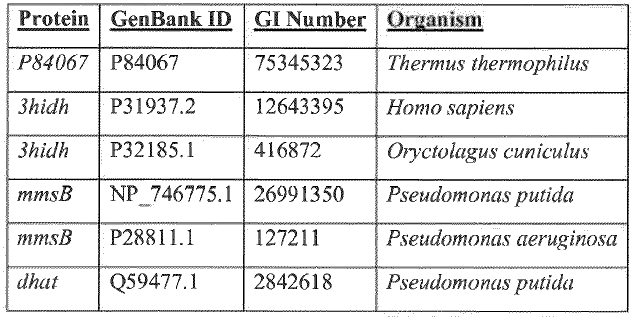

### Step E - 3-HIB dehydratase (designated as EMA6).

The dehydration of 3-HIB to MAA is catalyzed by an enzyme with EMA6 activity (EC 4.2.1.-). The final step involves the dehydration of 3-HIB to MAA The dehydration of 3-HIB to MAA is catalyzed by an enzyme with EMA6 activity. Although no direct evidence for this specific enzymatic transformation has been identified, most dehydratases catalyze the alpha,beta-climination of water, which involves activation of the alpha-hydrogen by an electron-withdrawing carbonyl, carboxylate, or CoA-thiol ester group and removal of the hydroxyl group from the beta-position (Buckel and Barker, J Bacteriol. 117:1248-1260 (1974); Martins et al, Proc. Natl. Acad. Sci. USA 101:15645-15649 (2004)). This is the exact type of transformation proposed for the final step in the methacrylate pathway. In addition, the proposed transformation is highly similar to the 2-(hydroxymethyl)glutarate dehydratase of *Eubacterium barkeri,* which can catalyze the conversion of 2-hydroxymethyl glutarate to 2-methylene glutarate. This enzyme has been studied in the context of nicotinate catabolism and is encoded by *hmd* (Alhapel et al., Proc. Natl. Acad. Sci. USA 103:12341-12346 (2006)). Similar enzymes with high sequence homology are found in *Bacteroides capillosus, Anaerotruncus colihominis,* and *Natranaerobius thermophilius.* Several enzymes are known to catalyze the alpha, beta elimination of hydroxyl groups. Exemplary enzymes include 2-(hydroxymethyl)glutarate dehydratase (EC 4.2.1.-), fumarase (EC 4.2.1.2), 2-keto-4-pentenoate dehydratase (EC 4.2.1.80), citramalate hydrolyase and dimethylmaleate hydratase.

2-(Hydroxymethyl)glutarate dehydratase is a [4Fe-4S]-containing enzyme that dehydrates 2-(hydroxymethyl)glutarate to 2-methylene-glutarate, studied for its role in nicontinate catabolism in *Eubacterium barkeri* (formerly *Clostridium barkeri*) (Alhapel et al., Proc Natl Acad Sci USA 103:12341-12346 (2006)). Similar enzymes with high sequence homology are found in *Bacteroides capillosus, Anaerotruncus colihominis,* and *Natranaerobius thermophilius.* These enzymes are also homologous to the alpha- and beta-subunits of [4Fe-4S]-containing bacterial serine dehydratases, for example, *E. coli* enzymes encoded by *tdcG, sdhB,* and *sdaA*). An enzyme with similar functionality in *E. barkeri* is dimethylmaleate hydratase, a reversible Fe2+-dependent and oxygen-sensitive enzyme in the aconitase family that hydrates dimethylmaeate to form (2R,3S)-2,3-dimethylmalate. This enzyme is encoded by dmdAB (Alhapel et al., Proc Natl Acad Sci USA 103:12341-6 (2006); Kollmann-Koch et al., Hoppe Seylers.Z.Physiol Chem. 365:847-857 (1984)).

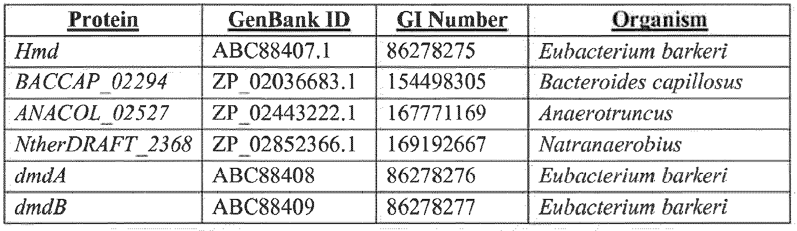

Fumarate hydratase enzymes, which naturally catalyze the reversible hydration of fumarate to malate. Although the ability of fumarate hydratase to react on branched substrates with 3-oxobutanol as a substrate has not been described, a wealth of structural information is available for this enzyme and other researchers have successfully engineered the enzyme to alter activity, inhibition and localization (Weaver, Acta Crystallogr. D Biol. Crystallogr. 61:1395-1401 (2005)). *E. coli* has three fumarases: FumA, FumB, and FumC that are regulated by growth conditions. FumB is oxygen sensitive and only active under anaerobic conditions. FumA is active under microanaerobic conditions, and FumC is the only active enzyme in aerobic growth (Tseng et al., J. Bacteriol. 183:461-467 (2001); Woods et al., Biochem. Biophys. Acta 954:14-26 (1988); Guest et al., J Gen Microbiol 131:2971-2984 (1985)). Exemplary enzyme candidates include those encoded by *fumC* from *Escherichia coli* (Estevez et al., Protein Sci. 11:1552-1557 (2002); Hong and Lee, Biotechnol. Bioprocess Eng. 9:252-255 (2004); Rose and Weaver, Proc. Natl. Acad. Sci. USA 101:3393-3397 (2004)), and enzymes found in *Campylobacter jejuni* (Smith et al., Int. J. Biochem. Cell Biol. 31:961-975 (1999)), *Thermus thermophilus* (Mizobata et al., Arch. Biochem. Biophys. 355:49-55 (1998)), and *Rattus norvegicus* (Kobayashi et al., J. Biochem. 89:1923-1931 (1981)). Similar enzymes with high sequence homology include *fum1* from *Arabidopsis thaliana* and *fumC* from *Corynebacterium glutamicum.* The *MmcBC* fumarase from Pelotomaculum thermopropionicum is another class of fumarase with two subunits (Shimoyama et al., FEMS Microbiol Lett. 270:207-213 (2007)).

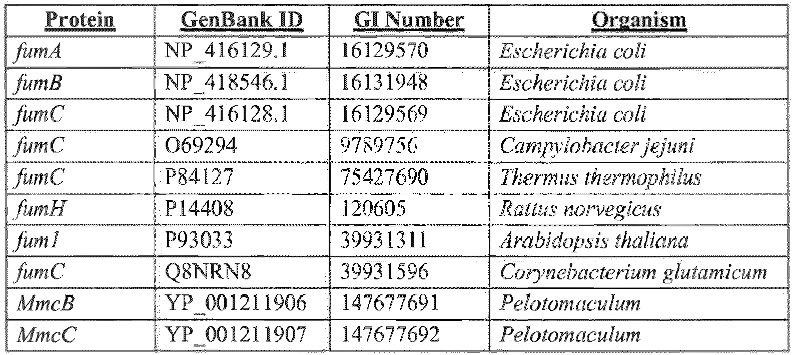

Dehydration of 4-hydroxy-2-oxovalerate to 2-oxopentenoate is catalyzed by 4-hydroxy-2-oxovalerate hydratase (EC 4.2.1.80). This enzyme participates in aromatic degradation pathways and is typically co-transcribed with a gene encoding an enzyme with 4-hydroxy-2-oxovalerate aldolase activity. Exemplary gene products are encoded by *mhpD* of *E*. *coli* (Ferrandez et al., J Bacteriol. 179:2573-2581 (1997); Pollard et al., Eur J Biochem. 251:98-106 (1998)), *todG* and *cmtF* of *Pseudomonas putida* (Lau et al., Gene 146:7-13 (1994); Eaton, J Bcacteriol. 178:1351-1362 (1996)), *cnbE* of *Comamonas* sp. CNB-1 (Ma et al., Appl Environ Microbiol 73:4477-4483 (2007)) and mhpD of *Burkholderia xenovorans* (Wang et al., FEBS J272:966-974 (2005)). A closely related enzyme, 2-oxohepta-4-ene-1,7-dioate hydratase, participates in 4-hydroxyphenylacetic acid degradation, where it converts 2-oxo-hept-4-ene-1,7-dioate (OHED) to 2-oxo-4-hydroxy-hepta-1,7-dioate using magnesium as a cofactor (Burks et al., J.Am.Chem.Soc. 120: (1998)). OHED hydratase enzyme candidates have been identified and characterized in *E. coli C* (Roper et al., Gene 156:47-51 (1995); Izumi et al., J Mol.Biol. 370:899-911 (2007)) and *E*. *coli W* (Pricto et al., J Bacteriol. 178:111-120 (1996)). Sequence comparison reveals homologs in a wide range of bacteria, plants and animals. Enzymes with highly similar sequences are contained in *Klebsiellce pneumonia* (91% identity, eval = 2e-138) and *Salmonella enterica* (91% identity, eval = 4e-138), among others.

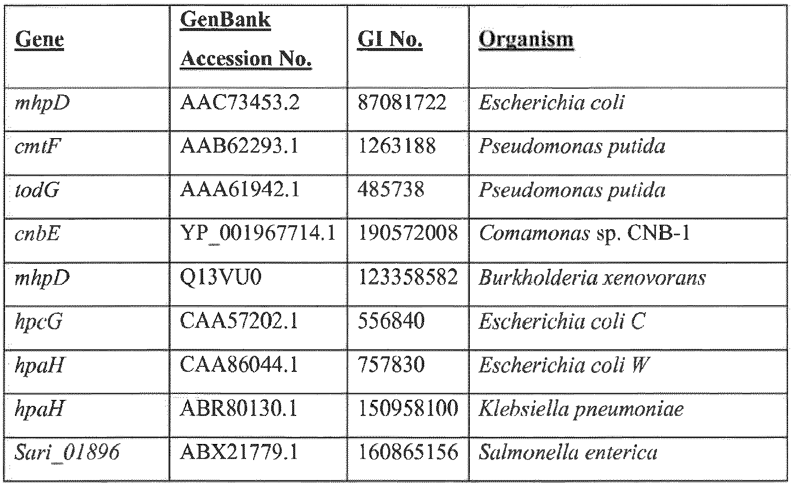

Another enzyme candidate is citramalate hydrolyase (EC 4.2.1.34), an enzyme that naturally dehydrates 2-methylmalate to mesaconate. This enzyme has been studied in *Methanocaldococcus jannaschii* in the context of the pyruvate pathway to 2-oxobutanoate, where it has been shown to have a broad substrate specificity (Drevland et al., J Bacteriol. 189:4391-4400 (2007)). This enzyme activity was also detected in *Clostridium tetanomorphum, Morganella morganii, Citrobacter amalonaticus* where it is thought to participate in glutamate degradation (Kato et al., Arch.Microbiol 168:457-463 (1997)). The *M. jannaschii* protein sequence does not bear significant homology to genes in these organisms.

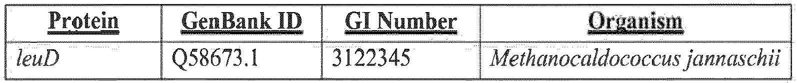

Dimethylmaleate hydratase (EC 4.2.1.85) is a reversible Fe²⁺-dependent and oxygen-sensitive enzyme in the aconitase family that hydrates dimethylmaeate to form (2R,3S)-2,3-dimethylmalate. This enzyme is encoded by *dmdAB* in *Eubacterium barkeri* (Alhapel *et al*., *supra;* Kollmann-Koch et al., Hoppe Seylers.Z.Physiol Chem. 365:847-857 (1984)).

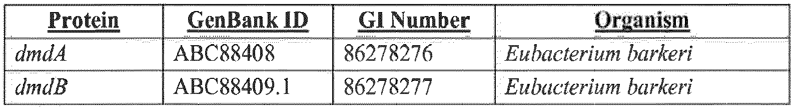

### Step F - Methylmalonyl-CoA reductase (alcohol forming) (designated as EMA7).

Step F can involve a combined Alcohol/Aldehyde dehydrogenase (EC 1.2.1.-). Methylmalonyl-CoA can be reduced to 3-HIB in one step by a multifunctional enzyme with dual acyl-CoA reductase and alcohol dehydrogenase activity. Although the direct conversion of methylmalonyl-CoA to 3-HIB has not been reported, this reaction is similar to the common conversions such as acetyl-CoA to ethanol and butyryl-CoA to butanol, which are catalyzed by CoA-dependent enzymes with both alcohol and aldehyde dehydrogenase activities. Gene candidates include the *E. coli adhE* (Kessler et al., FEBS Lett. 281:59-63 (1991)) and *C*. *acetobutylicum bdh* I and *bdh* II (Walter, et al., J. Bacteriol. 174:7149-7158 (1992)), which can reduce acetyl-CoA and butyryl-CoA to ethanol and butanol, respectively. In addition to reducing acetyl-CoA to ethanol, the enzyme encoded by *adhE* in *Leuconostoc mesenteroides* has been shown to oxide the branched chain compound isobutyraldehyde to isobutyryl-CoA (Kazahaya et al., J. Gen. Appl. Microbiol. 18:43-55 (1972); Koo et al., Biotechnol. Lett. 27:505-510 (2005)). An additional candidate enzyme for converting methylmalonyl-CoA directly to 3-HIB is encoded by a malonyl-CoA reductase from *Chloroflexus aurantiacus* (Hügler, et al., J. Bacteriol. 184(9):2404-2410 (2002).

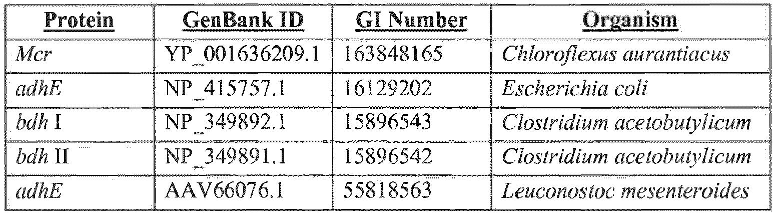

### EXAMPLE XIV

### Methacrylic Acid and 2-Hydroxyisobutyric Synthesis Enzymes

This Example provides genes that can be used for conversion of acetyl-CoA to methacrylic acid and 2-hydroxyisobutyric as depicted in the pathways of Figure 10. Fig. 10. Exemplary pathways enabling production of 2-hydroxyisobutyrate and methacrylic acid from acetyl-CoA. 2-Hydroxyisobutyrate and methacrylic acid production are carried out by the following enzymes: A) acetyl-CoA:acetyl-CoA acyltransferase, B) acetoacetyl-CoA reductase (ketone reducing), C) 3-hydroxybutyrl-CoA mutase, D) 2-hydroxyisobutyryl-CoA dehydratase, E) methacrylyl-CoA synthetase, hydrolase, or transferase, F) 2-hydroxyisobutyryl-CoA synthetase, hydrolase, or transferase.

MAA biosynthesis can proceed from acetyl-CoA in a minimum of five enzymatic steps (see Figure 10). In this pathway, two molecules of acetyl-CoA are combined to form acetoacetyl-CoA, which is then reduced to 3-hydroxybutyryl-CoA. Alternatively, 4-hydroxybutyryl-CoA can be converted to 3-hydroxybutyryl-CoA by way of 4-hydroxybutyryl-CoA dehydratase and crotonase (Martins et al., Proc. Nat. Acad. Sci. USA 101(44) 15645-15649 (2004); Jones and Woods, Microbiol. Rev. 50:484-524 (1986); Berg et al., Science 318(5857) 1782-1786 (2007)). A methylmutase then rearranges the carbon backbone of 3-hydroxybutyryl-CoA to 2-hydroxyisobutyryl-CoA, which is then dehydrated to form methacrylyl-CoA. Alternatively, 2-hydroxyisobutyryl-CoA can be converted to 2-hydroxyisobutyrate, secreted, and recovered as product. The final step converting methacrylyl-CoA to MAA can be performed by a single enzyme shown in the figure or a series of enzymes.

### A) Acetyl-CoA:acetyl-CoA Acyltransferase.

Step A involves acetoacetyl-CoA thiolase (EC 2.3.1.9). The formation of acetoacetyl-CoA from two acetyl-CoA units is catalyzed by acetyl-CoA thiolase. This enzyme is native to *E. coli,* encoded by gene *atoB,* and typically operates in the acetoacetate-degrading direction during fatty acid oxidation (Duncombe and Frerman, Arch. Biochem. Biophys. 176:159-170 (1976); Frerman and Duncombe, Biochim. Biophys. Acta 580:289-297 (1979)). The gene *thlA* from *Clostridium acetobutylicum* was engineered into an isopropanol-producing strain of *E*. *coli* (Hanai et al., Appl. Environ. Microbiol. 73:7814-7818 (2007); Stim-Herndon et al., Gene 154:81-85 (1995)). Additional gene candidates include *thl* from *Clostridium pasteurianum* (Meng and Li. Cloning, Biotechnol. Lett. 28:1227-1232 (2006)) and *ERG10* from S. *cerevisiae* (Hiser et al, J Biol Chem 269:31383-89 (1994)).

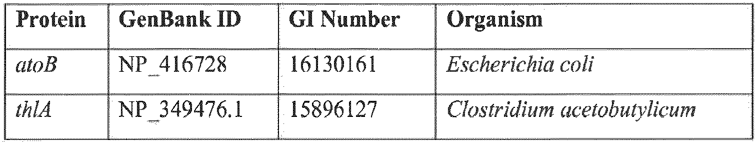

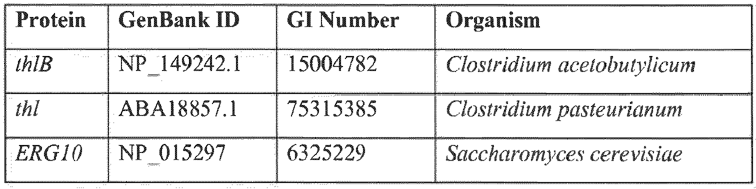

### B) Acetoacetyl-CoA Reductase (ketone reducing).

Step B involves acetoacetyl-CoA reductase (EC#: 1.1.1.35). This second step entails the reduction of acetoacetyl-CoA to 3-hydroxybutyryl-CoA by acetoacetyl-CoA reductase. This enzyme participates in the acetyl-CoA fermentation pathway to butyrate in several species of *Clostridia* and has been studied in detail (Jones and Woods, Microbiol. Rev. 50:484-524 (1986)). The enzyme from *Clostridium acetobulylicum,* encoded by *hbd,* has been cloned and functionally expressed in *E. coli* (Youngleson et al., J. Bacteriol. 171:6800-6807 (1989)). Additionally, subunits of two fatty acid oxidation complexes in *E. coli,* encoded by *fadB* and *fadJ,* function as 3-hydroxyacyl-CoA dehydrogenases (Binstock and Schulz, Methods Enzymol. 71 Pt C:403-411 (1981)). Yet other genes demonstrated to reduce acetoacetyl-CoA to 3-hydroxybutyryl-CoA are *phbB* from *Zoogloea ramigera* (Ploux et al., Eur.J Biochem. 174:177-182 (1988)) and *phaB* from *Rhodobacter sphaeroides* (Alber et al., Mol.Microbiol 61:297-309 (2006)). The former gene is NADPH-dependent, its nucleotide sequence has been determined (Peoples et al., Mol.Microbiol 3:349-357 (1989)) and the gene has been expressed in *E. coli.* Substrate specificity studies on the gene led to the conclusion that it could accept 3-oxopropionyl-CoA as a substrate besides acetoacetyl-CoA (Ploux et al., Eur.JBiochem. 174:177-182 (1988)). Additional gene candidates include *Hbd1* (C-terminal domain) and *Hbd2* (N-terminal domain) in *Clostridium kluyveri* (Hillmer and Gottschalk, Biochim. Biophys. Acta 3334:12-23 (1974)) and *HSD17B10* in *Bos taurus* (Wakil et al.,J. Biol. Chem. 207:631-638 (1954)). The enzyme from *Paracoccus denitrificans* has been functionally expressed and characterized in *E. coli* (Yabutani et al., FEMS Microbiol Lett. 133:85-90 (1995)). A number of similar enzymes have been found in other species of *Clostridia* and in *Metallosphaera sedula* (Berg et al., Science. 318:1782-1786 (2007)). The enzyme from *Candida tropicalis* is a component of the peroxisomal fatty acid beta-oxidation multifunctional enzyme type 2 (MFE-2). The dehydrogenase B domain of this protein is catalytically active on acetoacetyl-CoA. The domain has been functionally expressed in *E. coli,* a crystal structure is available, and the catalytic mechanism is well-understood (Ylianttila et al., Biochem Biophys Res Commun 324:25-30 (2004); Ylianttila et al., J Mol Biol 358:1286-1295 (2006)).

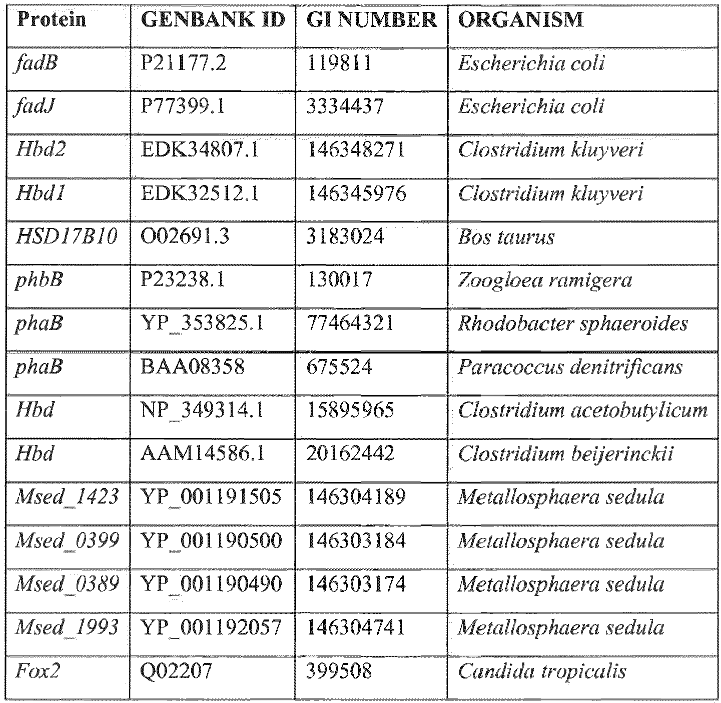

### C) 3-hydroxybutyrl-CoA mutase.

Step C involves 3-hydroxybutyryl-CoA mutase (EC 5.4.99.-). In this step, 3-hydroxybutyryl-CoA is rearranged to form 2-hydroxyisobutyryl-CoA (2-HIBCoA) by 3-hydroxybutyryl-CoA mutase. This enzyme is a novel ICM-like methylmutase recently discovered and characterized in *Methylibium petroleiphilum* (Ratnatilleke et al., J. Biol. Chem. 274:31679-31685 (1999); Rohwerder et al., Appl. Environ. Microbiol. 72:4128-4135 (2006)). This enzyme, encoded by *Mpe_B0541* in *Methylibium petroleiphilum PM1,* has high sequence homology to the large subunit of methylmalonyl-CoA mutase in other organisms including *Rsph17029_3657* in *Rhodobacter sphaeroides* and *Xaut_5021* in *Xanthobacter autotrophicus.* Changes to a single amino acid near the active site alters the substrate specificity of the enzyme (Ratnatilleke et al., *supra,* 1999; Rohwerder et al., *supra,* 2006), so directed engineering of similar enzymes at this site, such as methylmalonyl-CoA mutase or isobutryryl-CoA mutase described previously, can be used to achieve the desired reactivity.

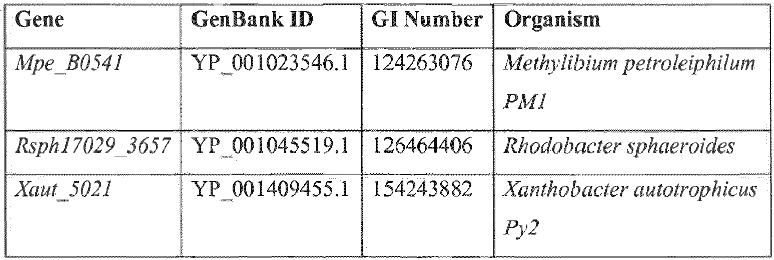

### D) 2-hydroxyisobutyryl-CoA dehydratase.

Step D involves 2-hydroxyisobutyryl-CoA dehydratase. The dehydration of 2-hydroxyacyl-CoA such as 2-hydroxyisobutyryl-CoA can be catalyzed by a special class of oxygen-sensitive enzymes that dehydrate 2-hydroxyacyl-CoA derivatives via a radical-mechanism (Buckel and Golding, Annu. Rev. Microbiol. 60:27-49 (2006); Buckel et al., Curr. Opin. Chem. Biol. 8:462-467 (2004); Buckel et al., Biol. Chem. 386:951-959 (2005); Kim et al., FEBS J. 272:550-561 (2005); Kim et al., FEMS Microbiol. Rev. 28:455-468 (2004); Zhang et al., Microbiology 145 ( Pt 9):2323-2334 (1999)). One example of such an enzyme is the lactyl-CoA dehydratase from *Clostridium propionicum,* which catalyzes the dehydration of lactoyl-CoA to form acryl-CoA (Kuchta and Abeles, J. Biol. Chem. 260:13181-13189 (1985); Hofmeister and Buckel, Eur. J. Biochem. 206:547-552 (1992)). An additional example is 2-hydroxyglutaryl-CoA dehydratase encoded by *hgdABC* from *Acidaminococcus fermentans* (Muëller and Buckel, Eur. J. Biochem. 230:698-704 (1995); Schweiger et al., Eur. J. Biochem. 169:441-448 (1987)). Yet another example is the 2-hydroxyisocaproyl-CoA dehydratase from *Clostridium difficile* catalyzed by *hadBC* and activated by *hadI* (Darley et al., FEBS J. 272:550-61 (2005)). The corresponding sequences for *A. fermentans* and *C*. *difficile* can be found as listed below. The sequence of the complete *C*. *propionicium* lactoyl-CoA dehydratase is not yet listed in publicly available databases. However, the sequence of the beta-subunit corresponds to the GenBank accession number AJ276553 (Selmer et al, Eur J Biochem, 269:372-80 (2002)).

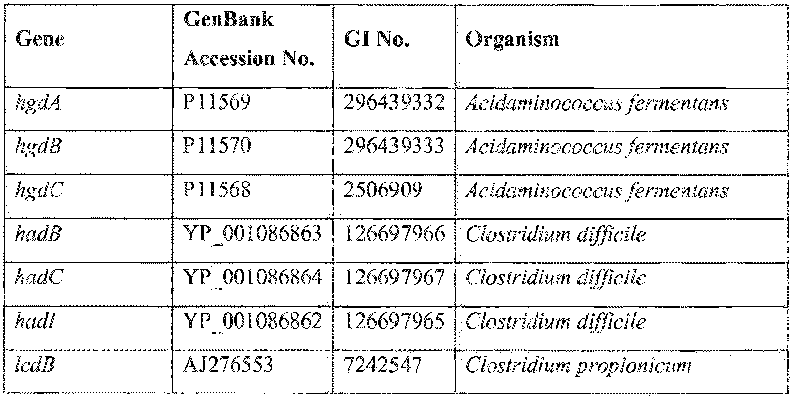

### E) methacrylyl-CoA synthetase, hydrolase, or transferase, and F) 2-hydroxyisobutyryl-CoA synthetase, hydrolase, or transferase.

Steps E and F involve a transferase (EC 2.8.3.-), hydrolase (EC 3.1.2.-), or synthetase (EC 6.2.1.-) with activity on a methacrylic acid or 2-hydroxyisobutyric acid, respectively. Direct conversion of methacrylyl-CoA to MAA or 2-hydroxyisobutyryl-CoA to 2-hydrioxyisobutyrate can be accomplished by a CoA transferase, synthetase or hydrolase. Pathway energetics are most favorable if a CoA transferase or a CoA synthetase is employed to accomplish this transformation. In the transferase family, the enzyme acyl-CoA:acetate-CoA transferase, also known as acetate-CoA transferase, is a suitable candidate to catalyze the desired 2-hydroxyisobutyryl-CoA or methacryl-CoA transferase activity due to its broad substrate specificity that includes branched acyl-CoA substrates (Matthies and Schink, Appl. Environ. Microbiol. 58:1435-1439 (1992); Vanderwinkel et al., Biochem. Biophys. Res. Commun. 33:902-908 (1968)). ADP-forming acetyl-CoA synthetase (ACD) is a promising enzyme in the CoA synthetase family operating on structurally similar branched chain compounds (Brasen and Schonheit, Arch. Microbiol. 182:277-287 (2004); Musfeldt and Schonheit, J. Bacterial. 184:636-644 (2002)). In the CoA-hydrolase family, the enzyme 3-hydroxyisobutyryl-CoA hydrolase has been shown to operate on a variety of branched chain acyl-CoA substrates including 3-hydroxyisobutyryl-CoA, methylmalonyl-CoA, and 3-hydroxy-2-methylbutanoyl-CoA (Hawes et al., Methods Enzymol. 324:218-228 (2000); Hawes et al., J. Biol. Chem. 271:26430-26434 (1996); Shimomura et al., J. Biol. Chem. 269:14248-14253 (1994)). Additional exemplary gene candidates for CoA transferases, synthetases, and hydrolases are discussed elsewhere above.

### EXAMPLE XV

### Attenuation or Disruption of Endogenous Enzymes

This example provides endogenous enzyme targets for attenuation or disruption that can be used for enhancing carbon flux through methanol dehydrogenase and formaldehyde assimilation pathways.

### DHA Kinase

Methylotrophic yeasts typically utilize a cytosolic DHA kinase to catalyze the ATP-dependent activation of DHA to DHAP. DHAP together with G3P is combined to form fructose-1,6-bisphosphate (FBP) by FBP aldolase. FBP is then hydrolyzed to F6P by fructose bisphosphatase. The net conversion of DHA and G3P to F6P by this route is energetically costly (1 ATP) in comparison to the F6P aldolase route, described above and shown in FIG. 1. DHA kinase also competes with F6P aldolase for the DHA substrate. Attenuation of endogenous DHA kinase activity will thus improve the energetics of formaldehyde assimilation pathways, and also increase the intracellular availability of DHA for DHA synthase. DHA kinases of *Saccharomyces cerevisiae,* encoded by DAK1 and DAK2, enable the organism to maintain low intracellular levels of DHA (Molin et al, J Biol Chem 278:1415-23 (2003)). In methylotrophic yeasts DHA kinase is essential for growth on methanol (Luers et al, Yeast 14:759-71 (1998)). The DHA kinase enzymes of *Hansenula polymorpha* and *Pichia pastoris* are encoded by DAK (van der Klei et al, Curr Genet 34:1-11 (1998); Luers et al, *supra*). DAK enzymes in other organisms can be identified by sequence similarity to known enzymes.

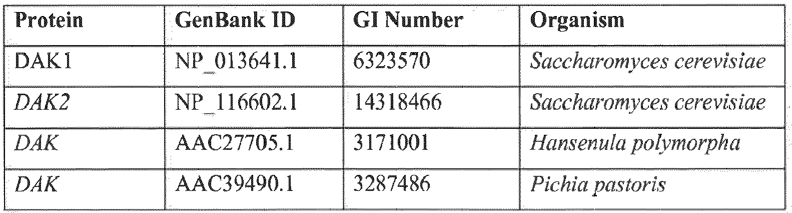

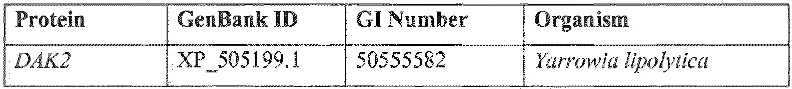

### Methanol Oxidase

Attenuation of redox-inefficient endogenous methanol oxidizing enzymes, combined with increased expression of a cytosolic NADH-dependent methanol dehydrogenase, will enable redox-efficient oxidation of methanol to formaldehyde in the cytosol. Methanol oxidase, also called alcohol oxidase (EC 1.1.3.13), catalyzes the oxygen-dependent oxidation of methanol to formaldehyde and hydrogen peroxide. In eukaryotic organisms, alcohol oxidase is localized in the peroxisome. Exemplary methanol oxidase enzymes are encoded by AOD of *Candida boidinii* (Sakai and Tani, Gene 114:67-73 (1992)); and AOX of *H. polymorpha, P. methanolica* and *P. pastoris* (Ledeboer et al, Nucl Ac Res 13:3063-82 (1985); Koutz et al, Yeast 5:167-77 (1989); Nakagawa et al, Yeast 15:1223-1230 (1999)).

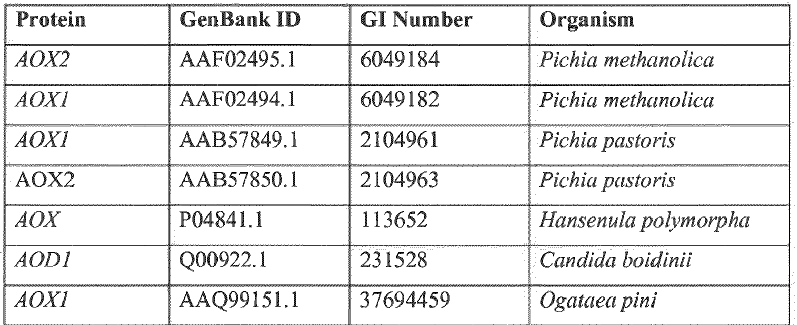

### PQQ-dependent Methanol Dehydrogenase

PQQ-dependent methanol dehydrogenase from *M. extorquens* (*mxaIF*) uses cytochrome as an electron carrier (Nunn et al, Nucl Acid Res 16:7722 (1988)). Methanol dehydrogenase enzymes of methanotrophs such as *Methylococcus capsulatis* function in a complex with methane monooxygenase (MMO) (Myronova et al, Biochem 45:11905-14 (2006)). Note that of accessory proteins, cytochrome CL and PQQ biosynthesis enzymes are needed for active methanol dehydrogenase. Attenuation of one or more of these required accessory proteins, or retargeting the enzyme to a different cellular compartment, would also have the effect of attenuating PQQ-dependent methanol dehydrogenase activity.

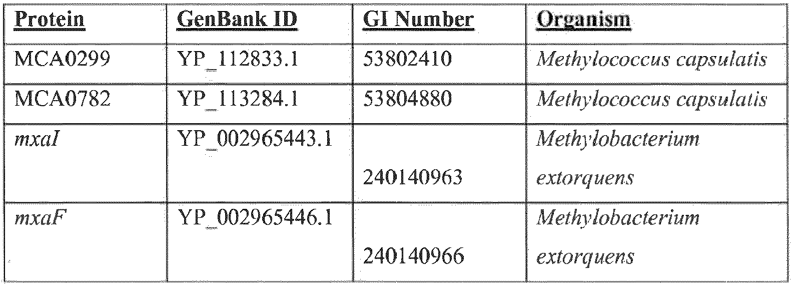

### DHA Synthase and Other Competing Formaldehyde Assimilation and Dissimilation Pathways

Carbon-efficient formaldehyde assimilation can be improved by attenuation of competing formaldehyde assimilation and dissimilation pathways. Exemplary competing assimilation pathways in eukaryotic organisms include the peroxisomal dissimilation of formaldehyde by DHA synthase, and the DHA kinase pathway for converting DHA to F6P, both described herein. Exemplary competing endogenous dissimilation pathways include one or more of the enzymes shown in FIG. 1.

Methylotrophic yeasts normally target selected methanol assimilation and dissimilation enzymes to peroxisomes during growth on methanol, including methanol oxidase, DHA synthase and S-(hydroxymethyl)-glutathione synthase (see review by Yurimoto et al, *supra*)*.* The peroxisomal targeting mechanism comprises an interaction between the peroxisomal targeting sequence and its corresponding peroxisomal receptor (Lametschwandtner et al, J Biol Chem 273:33635-43 (1998)). Peroxisomal methanol pathway enzymes in methylotrophic organisms contain a PTS1 targeting sequence which binds to a peroxisomal receptor, such as Pex5p in *Candida boidinii* (Horiguchi et al, J Bacteriol 183:6372-83 (2001)). Disruption of the PTS1 targeting sequence, the Pex5p receptor and/or genes involved in peroxisomal biogenesis would enable cytosolic expression of DHA synthase, S-(hydroxymethyl)-glutathione synthase or other methanol-inducible peroxisomal enzymes. PTS1 targeting sequences of methylotrophic yeast arc known in the art (Horiguchi et al, *supra).* Identification of peroxisomal targeting sequences of unknown enzymes can be predicted using bioinformatic methods (eg. Neuberger et al, J Mol Biol 328:581-92 (2003))).

### EXAMPLE XVI

### Methanol Assimilation via Methanol Dehydrogenase and the Ribulose Monophosphate pathway

This example shows that co-expression of an active enzymes of the Ribulose Monophosphate (RuMP) pathway can effectively assimilate methanol derived carbon.

An experimental system was designed to test the ability of a MeDH in conjunction with the enzymes H6P synthase (HPS) and 6-phospho-3-hexuloisomerase (PHI) of the RuMP pathway to assimilate methanol carbon into the glycolytic pathway and the TCA cycle. *Escherichia coli* strain ECh-7150 (ΔlacIA, ΔpflB, ΔptsI, ΔPpckA(pckA), ΔPglk(glk), glk::glfB, ΔhycE, ΔfrmR, ΔfrmA, ΔfrmB) was constructed to remove the glutathione-dependent formaldehyde detoxification capability encoded by the FrmA and FrmB enzyme. This strain was then transformed with plasmid pZA23S variants that either contained or lacked gene 2616A encoding a fusion of the HPS and PHI enzymes. These two transformed strains were then each transformed with pZS*13S variants that contained gene 2315L (encoding an active MeDH), or gene 2315 RIP2 (encoding a catalytically inactive MeDH), or no gene insertion. Genes 2315 and 2616 are internal nomenclatures for NAD-dependent methanol dehydrogenase from *Bacillus methanolicus* MGA3 and 2616 is a fused phs-hpi constructs as described in Orita et al. (2007) Appl Microbiol Biotechnol 76:439-45.

The six resulting strains were aerobically cultured in quadruplicate, in 5 ml minimal medium containing 1% arabinose and 0.6 M 13C-methanol as well as 100 ug/ml carbenicillin and 25 µg/ml kanamycin to maintain selection of the plasmids, and 1 mM IPTG to induce expression of the methanol dehydrogenase and HPS-PHI fusion enzymes. After 18 hours incubation at 37°C, the cell density was measured spectrophotometrically at 600 nM wavelength and a clarified sample of each culture medium was submitted for analysis to detect evidence of incorporation of the labeled methanol carbon into TCA-cycle derived metabolites. The label can be further enriched by deleting the gene araD that competes with ribulose-5-phosphate.

¹³C carbon derived from labeled methanol provided in the experiment was found to be significantly enriched in the metabolites pyruvate, lactate, succinate, fumarate, malate, glutamate and citrate, but only in the strain expressing both catalytically active MeDH 2315L and the HPS-PHI fusion 2616A together (data not shown). Moreover, this strain grew significantly better than the strain expressing catalytically active MeDH but lacking expression of the HPS-PHI fusion (data not shown), suggesting that the HPS-PHI enzyme is capable of reducing growth inhibitory levels of formaldehyde that cannot be detoxified by other means in this strain background. These results show that co-expression of an active McDH and the enzymes of the RuMP pathway can effectively assimilate methanol derived carbon and channel it into TCA-cycle derived products.

### EXAMPLE XVII

The following example describes the enzymes and the gene candidates required for production of 2,4-pentadienoate and butadiene as shown in Figure 11.

### Step A, Figure 11: Acetaldehyde Dehydrogenase

The reduction of acetyl-CoA to acetaldehyde can be catalyzed by NAD(P)+-dependent acetaldehyde dehydrogenase (EC 1.2.1.10). Acylating acetaldehyde dehydrogenases of *E. coli* are encoded by *adhE* and *mhpF* (Ferrandez et al, J Bacterial 179:2573-81 (1997)). The *Pseudomonas* sp. CF600 enzyme, encoded by *dmpF,* participates in meta-cleavage pathways and forms a complex with 4-hydroxy-2-oxovalerate aldolase (Shingler et al, J Bacterial 174:711-24 (1992)). BphJ, a nonphosphorylating acylating aldehyde dehydrogenase, catalyzes the conversion of aldehydes to form acyl-coenzyme A in the presence of NAD(+) and coenzyme A (CoA) (Baker et al., Biochemistry, 2012 Jun 5;51(22):4558-67. Epub 2012 May 21). Solventogenic organisms such as *Clostridium acetobutylicum* encode bifunctional enzymes with alcohol dehydrogenase and acetaldehyde dehydrogenase activities. The bifunctional *C. acetobutylicum* enzymes are encoded by *bdh* I and adhE2 (Walter, et al., J. Bacteriol. 174:7149-7158 (1992); Fontaine et al., J.Bacteriol. 184:821-830 (2002)). Yet another candidate for acylating acetaldehyde dehydrogenase is the *ald* gene from *Clostridium beijerinckii* (Toth, Appl. Environ. Microbiol. 65:4973-4980 (1999). This gene is very similar to the *eutE* acetaldehyde dehydrogenase genes of *Salmonella typhimurium* and *E*. *coli* (Toth, Appl. Environ. Microbiol. 65:4973-4980 (1999).

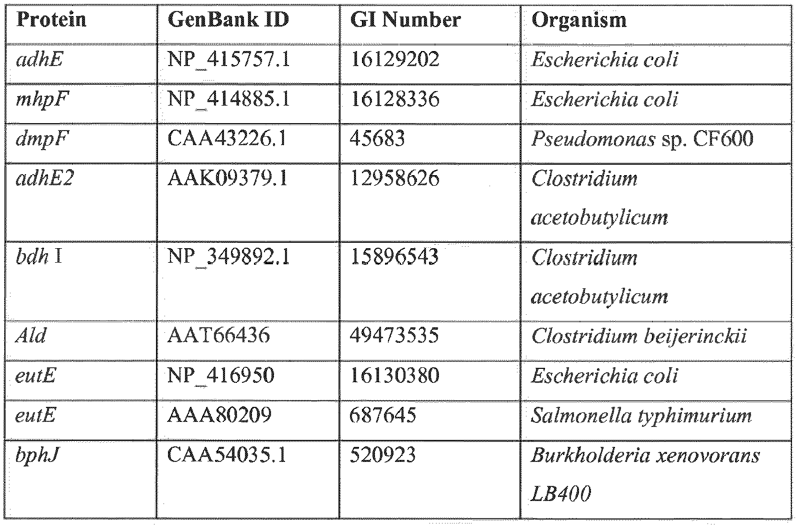

Other acyl-CoA dehydrogenases that reduce an acyl-CoA to its corresponding aldehyde include fatty acyl-CoA reductase (EC 1.2.1.42, 1.2.1.50), succinyl-CoA reductase (EC 1.2.1.76), acetyl-CoA reductase, butyryl-CoA reductase and propionyl-CoA reductase (EC 1.2.1.3). Aldehyde forming acyl-CoA reductase enzymes with demonstrated activity on acyl-CoA, 3-hydroxyacyl-CoA and 3-oxoacyl-CoA substrates are known in the literature. Several acyl-CoA reductase enzymes are active on 3-hydroxyacyl-CoA substrates. For example, some butyryl-CoA reductases from *Clostridial* organisms, are active on 3-hydroxybutyryl-CoA and propionyl-CoA reductase of *L. reuteri* is active on 3-hydroxypropionyl-CoA. An enzyme for converting 3-oxoacyl-CoA substrates to their corresponding aldehydes is malonyl-CoA reductase. Enzymes in this class can be refined using evolution or enzyme engineering methods known in the art to have activity on enoyl-CoA substrates.

Exemplary fatty acyl-CoA reductases enzymes are encoded by *acr1* of *Acinetobacter calcoaceticus* (Reiser, Journal of Bacteriology 179:2969-2975 (1997)) and *Acinetobacter sp. M-1* (Ishige et al., Appl. Environ. Microbiol. 68:1192-1195 (2002)). Two gene products from *Mycobacterium tuberculosis* accept longer chain fatty acyl-CoA substrates of length C16-C18 (Harminder Singh, U. Central Florida (2007)). Yet another fatty acyl-CoA reductase is LuxC of *Photobacterium phosphoreum* (Lee et al, Biochim Biohys Acta 1388:215-22 (1997)). Enzymes with succinyl-CoA reductase activity are encoded by *sucD* of *Clostridium kluyveri* (Sohling, J. Bacteriol. 178:871-880 (1996)) and *sucD* of *P. gingivalis* (Takahashi, J. Bacterial 182:4704-4710 (2000)). Additional succinyl-CoA reductase enzymes participate in the 3-hydroxypropionate/4-hydroxybutyrate cycle of thermophilic archaea including *Metallosphaera sedula* (Berg et al., Science 318:1782-1786 (2007)) and *Thermoproteus neutrophilus* (Ramos-Vera et al., J Bacteriol, 191 :4286-4297 (2009)). The M. *sedula* enzyme, encoded by *Msed_0709,* is strictly NADPH-dependent and also has malonyl-CoA reductase activity. The *T. neutrophilus* enzyme is active with both NADPH and NADH. The enzyme acylating acetaldehyde dehydrogenase in *Pseudomonas sp,* encoded by *bphG,* is yet another as it has been demonstrated to oxidize and acylate acetaldehyde, propionaldehyde, butyraldehyde, isobutyraldehyde and formaldehyde (Powlowski, J. Bacteriol. 175:377-385 (1993)). In addition to reducing acetyl-CoA to ethanol, the enzyme encoded by *adhE* in *Leuconostoc mesenteroides* has been shown to oxidize the branched chain compound isobutyraldehyde to isobutyryl-CoA (Kazahaya, J. Gen. Appl. Microbiol. 18:43-55 (1972); and Koo et al., Biotechnol Lett. 27:505-510 (2005)). Butyraldehyde dehydrogenase catalyzes a similar reaction, conversion of butyryl-CoA to butyraldehyde, in solventogenic organisms such as *Clostridium saccharoperbutylacetonicum* (Kosaka et al., Biosci Biotechnol Biochem., 71:58-68 (2007)). Exemplary propionyl-CoA reductase enzymes include *pduP* of *Salmonella typhimurium LT2* (Leal, Arch. Microbiol. 180:353-361 (2003)) and *eutE* from *E*. *coli* (Skraly, WO Patent No. 2004/024876). The propionyl-CoA reductase of *Salmonella typhimurium LT2,* which naturally converts propionyl-CoA to propionaldehyde, also catalyzes the reduction of 5-hydroxyvaleryl-CoA to 5-hydroxypentanal (WO 2010/068953A2). The propionaldehyde dehydrogenase of *Lactobacillus reuteri*, PduP, has a broad substrate range that includes butyraldehyde, valeraldehyde and 3-hydroxypropionaldehyde (Luo et al, Appl Microbiol Biotech, 89: 697-703 (2011)). Additionally, some acyl-ACP reductase enzymes such as the *orf1594* gene product of *Synechococcus elongatus PCC7942* also exhibit aldehyde-forming acyl-CoA reductase activity (Schirmer et al, Science, 329: 559-62 (2010)).

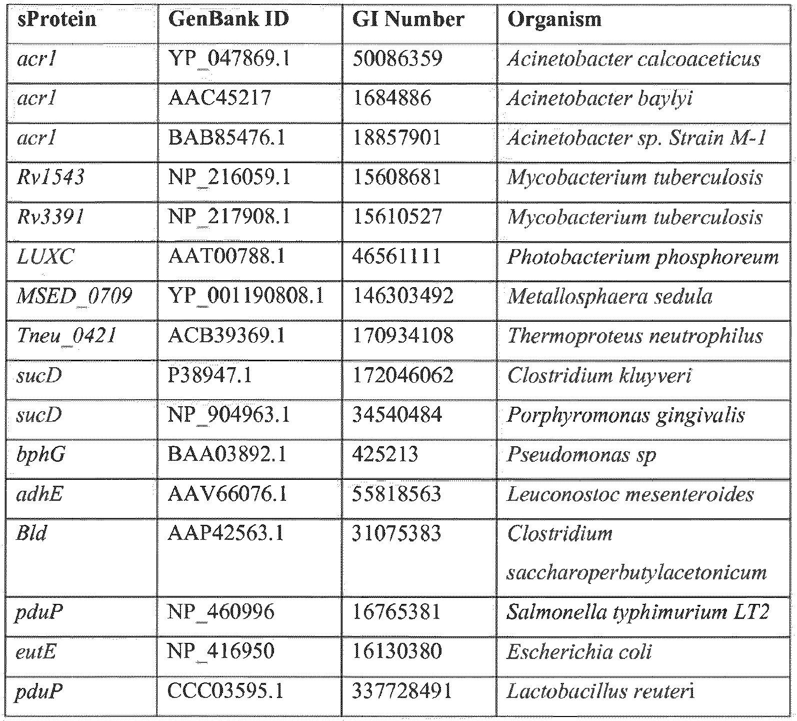

Additionally, some acyl-ACP reductase enzymes such as the orf1594 gene product of *Synechococcus elongatus* PCC7942 also exhibit aldehyde-forming acyl-CoA reductase activity (Schirmer et al, Science, 329: 559-62 (2010)). The S. *elongates* PCC7942 acyl-ACP reductase is coexpressed with an aldehyde decarbonylase in an operon that appears to be conserved in a majority of cyanobacterial organisms. This enzyme, expressed in *E. coli* together with the aldehyde decarbonylase, conferred the ability to produce alkanes. The *P. marinus* AAR was also cloned into *E. coli* and, together with a decarbonylase, demonstrated production of alkanes (see, *e.g.,* US Application 2011/0207203).

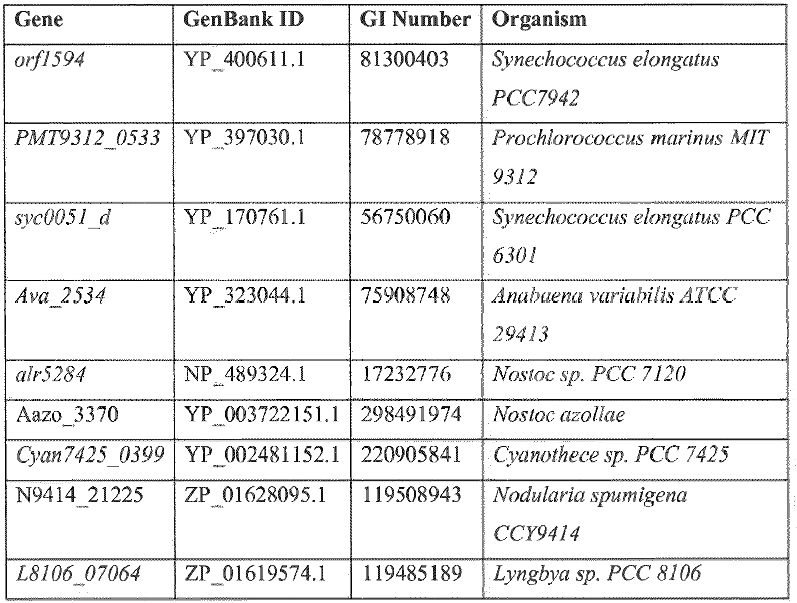

An additional enzyme type that converts an acyl-CoA to its corresponding aldehyde is malonyl-CoA reductase which transforms malonyl-CoA to malonic semialdehyde. Malonyl-CoA reductase is a key enzyme in autotrophic carbon fixation via the 3-hydroxypropionate cycle in thermoacidophilic archaeal bacteria (Berg, Science 318:1782-1786 (2007); and Thauer, Science 318:1732-1733 (2007)). The enzyme utilizes NADPH as a cofactor and has been characterized in *Metallosphaera* and *Sulfolobus sp.* (Alber et al., J. Bacterial. 188:8551-8559 (2006); and Hugler, J. Bacteriol. 184:2404-2410 (2002)). The enzyme is encoded by *Msed_0709* in *Metallosphaera sedula* (Alber et al., J. Bacterial. 188:8551-8559 (2006); and Berg, Science 318:1782-1786 (2007)). A gene encoding a malonyl-CoA reductase from *Sulfolobus tokodaii* was cloned and heterologously expressed in *E*. *coli* (Alber et al., J. Bacterial 188:8551-8559 (2006). This enzyme has also been shown to catalyze the conversion of methylmalonyl-CoA to its corresponding aldehyde (WO2007141208 (2007)). Although the aldehyde dehydrogenase functionality of these enzymes is similar to the bifunctional dehydrogenase from *Chloroflexus aurantiacus,* there is little sequence similarity. Both malonyl-CoA reductase enzyme candidates have high sequence similarity to aspartate-scmialdchydc dehydrogenase, an enzyme catalyzing the reduction and concurrent dephosphorylation of aspartyl-4-phosphate to aspartate semialdehyde. Additional gene candidates can be found by sequence homology to proteins in other organisms including *Sulfolobus solfataricus* and *Sulfolobus acidocaldarius* and have been listed below. Yet another candidate for CoA-acylating aldehyde dehydrogenase is the *aid* gene from *Clostridium beijerinckii* (Toth, Appl. Environ. Microbiol. 65:4973-4980 (1999). This enzyme has been reported to reduce acetyl-CoA and butyryl-CoA to their corresponding aldehydes. This gene is very similar to *eutE* that encodes acetaldehyde dehydrogenase of *Salmonella typhimurium* and *E*. *coli* (Toth, Appl. Environ. Microbiol. 65:4973-4980 (1999).

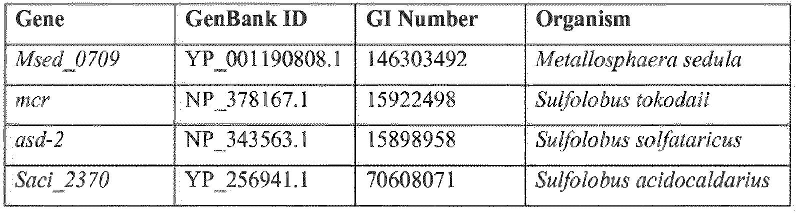

### Step B. Figure 11: 4-hydroxy 2-oxovalerate aldolase

The condensation of pyruvate and acetaldehyde to 4-hydroxy-2-oxovalerate is catalyzed by 4-hydroxy-2-oxovalerate aldolase (EC 4.1.3.39). This enzyme participates in pathways for the degradation of phenols, cresols and catechols. The *E. coli* enzyme, encoded by *mhpE,* is highly specific for acetaldehyde as an acceptor but accepts the alternate substrates 2-ketobutyratc or phenylpyruvate as donors (Pollard et al., Appl Environ Microbiol 64:4093-4094 (1998)). Similar enzymes are encoded by the *cmtG* and *todH* genes of *Pseudomonas putida* (Lau et al., Gene 146:7-13 (1994); Eaton, J Bacteriol. 178:1351-1362 (1996)). In *Pseudomonas* CF600, this enzyme is part of a bifunctional aldolasc-dchydrogcnasc heterodimer encoded by *dmpFG (*Manjasetty et al., Acta Crystallogr.D.Biol Crystallogr. 57:582-585 (2001*)).* The dehydrogenase functionality interconverts acetaldehyde and acetyl-CoA, providing the advantage of reduced cellular concentrations of acetaldehyde, toxic to some cells. It has been shown recently that substrate channeling can occur within this enzyme in the presence of NAD and residues that could play an important role in channeling acetaldehyde into the DmpF site were also identified.

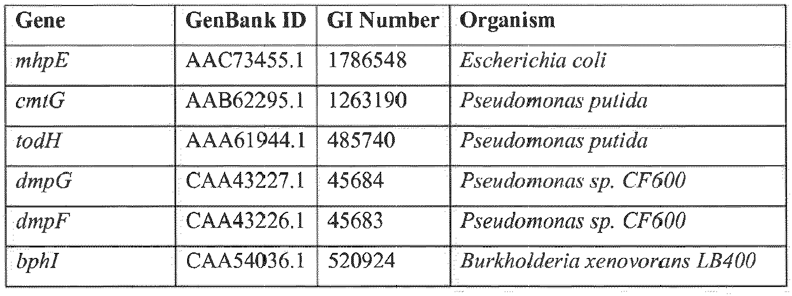

### Step C, Figure 11: 4-hydroxy 2-oxovalerate dehydratase

The dehydration of 4-hydroxy-2-oxovalerate to 2-oxopentenoate is catalyzed by 4-hydroxy-2-oxovalerate hydratase (EC 4.2.1.80). 4-Hydroxy-2-oxovalerate hydratase participates in aromatic degradation pathways and is typically co-transcribed with a gene encoding an enzyme with 4-hydroxy-2-oxovalerate aldolase activity. Exemplary gene products are encoded by *mhpD* of *E. coli* (Ferrandez et al., J Bacterial. 179:2573-2581 (1997); Pollard et al., Eur J Biochem. 251:98-106 (1998)), *todG* and *cmtF* of *Pseudomonas putida* (Lau et al., Gene 146:7-13 (1994); Eaton, J Bacterial. 178:1351-1362 (1996)), *cnbE* of *Comamonas* sp. CNB-1 (Ma et al., Appl Environ Microbiol 73:4417-4483 (2007)) and *mhpD* of *Burkholderia xenovorans* (Wang et al., FEBS J272:966-974 (2005)). A closely related enzyme, 2-oxohepta-4-ene-1,7-dioate hydratase, participates in 4-hydroxyphenylacetic acid degradation, where it converts 2-oxo-hept-4-ene-1,7-dioate (OHED) to 2-oxo-4-hydroxy-hepta-1,7-dioate using magnesium as a cofactor (Burks et al., J.Am.Chem.Soc. 120: (1998)). OHED hydratase enzyme candidates have been identified and characterized in *E. coli* C (Roper et al., Gene 156:47-51 (1995); Izumi et al., J Mol.Biol. 370:899-911 (2007)) and *E. coli W* (Prieto et al., J Bacteriol. 178:111-120 (1996)). Sequence comparison reveals homologs in a wide range of bacteria, plants and animals. Enzymes with highly similar sequences are contained in *Klebsiella pneumonia* (91% identity, eval = 2e-138) and *Salmonella enterica* (91% identity, eval = 4e-138), among others.

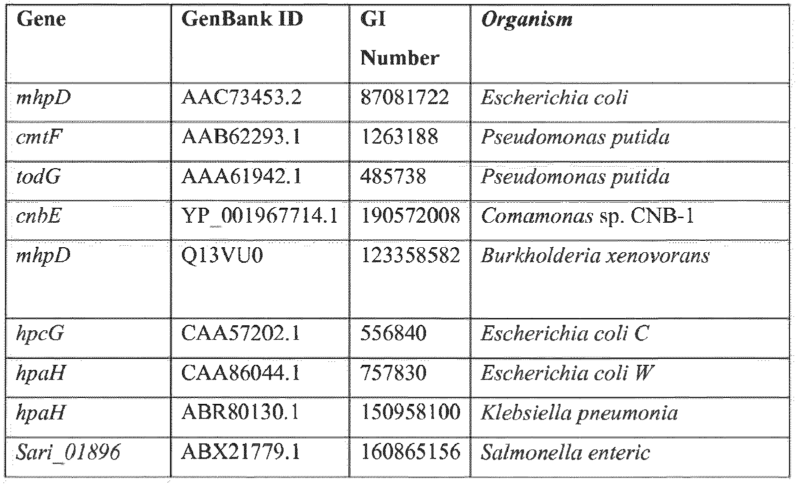

2-(Hydroxymethyl)glutarate dehydratase is a [4Fe-4S]-containing enzyme that dehydrates 2-(hydroxymcthyl)glutaratc to 2-methylene-glutarate, studied for its role in nicontinate catabolism in *Eubacterium barkeri* (formerly *Clostridium barkeri*) (Alhapel et al., Proc Natl Acad Sci 103:12341-6 (2006)). Similar enzymes with high sequence homology are found in *Bacteroides capillosus, Anaerotruncus colihominis,* and *Natranaerobius thermophilius.* These enzymes are homologous to the alpha and beta subunits of [4Fe-4S]-containing bacterial serine dehydratases (e.g., *E. coli* enzymes encoded by *tdcG, sdhB,* and *sdaA).* An enzyme with similar functionality in *E. barkeri* is dimethylmaleate hydratase, a reversible Fe²⁺-dependent and oxygen-sensitive enzyme in the aconitase family that hydrates dimethylmacate to form (2R,3S)-2,3-dimethylmalate. This enzyme is encoded by *dmdAB* (Alhapel et al., Proc Natl Acad Sci USA 103:12341-6 (2006); Kollmann-Koch et al., Hoppe Seylers.Z.Physiol Chem. 365:847-857 (1984)).

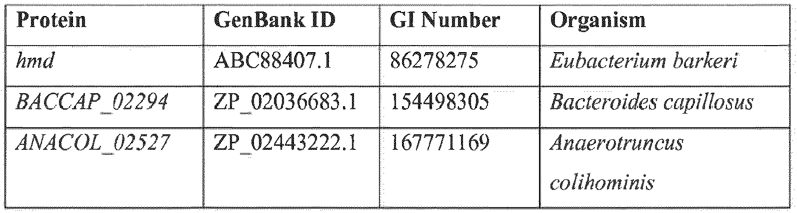

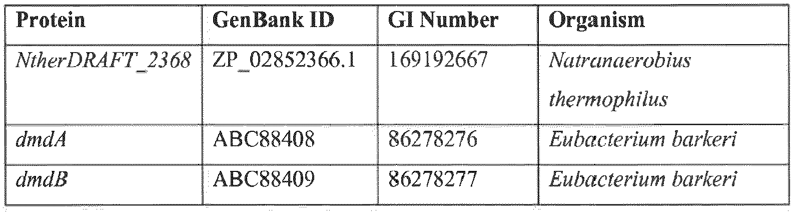

### Step D, Figure 11: 2-oxopentenoate reductase

The reduction of 2-oxopentenoate to 2-hydroxypentenoate is carried out by an alcohol dehydrogenase that reduces a ketone group. Several exemplary alcohol dehydrogenases can catalyze this transformation. Two such enzymes from *E. coli* are encoded by malate dehydrogenase *(mdh)* and lactate dehydrogenase (*ldhA*)*.* In addition, lactate dehydrogenase from *Ralstonia eutropha* has been shown to demonstrate high activities on 2-ketoacids of various chain lengths including lactate, 2-oxobutyrate, 2-oxopentanoate and 2-oxoglutarate (Steinbuchel et al., Eur.J.Biochem. 130:329-334 (1983)). Conversion of alpha-ketoadipate into alpha-hydroxyadipate is catalyzed by 2-ketoadipate reductase, an enzyme found in rat and in human placenta (Suda et al., Arch.Biochem.Biophys. 176:610-620 (1976); Suda et al., Biochem.Biophys.Res.Commun. 77:586-591 (1977)). An additional candidate oxidoreductase is the mitochondrial 3-hydroxybutyrate dehydrogenase (*bdh*) from the human heart which has been cloned and characterized (Marks et al., J.Biol.Chem. 267:15459-15463 (1992)). Alcohol dehydrogenase enzymes of C. *beijerinckii* (Ismaiel et al., J.Bacteriol. 175:5097-5105 (1993)) and *T. brockii* (Lamed et al., Biochem.J. 195:183-190 (1981); Peretz et al., Biochemistry. 28:6549-6555 (1989)) convert acetone to isopropanol. Methyl ethyl ketone reductase catalyzes the reduction of MEK to 2-butanol. Exemplary MEK reductase enzymes can be found in *Rhodococcus ruber* (Kosjek et al., Biotechnol Bioeng. 86:55-62 (2004)) and *Pyrococcus furiosus* (van der et al., Eur.J.Biochem. 268:3062-3068 (2001)).

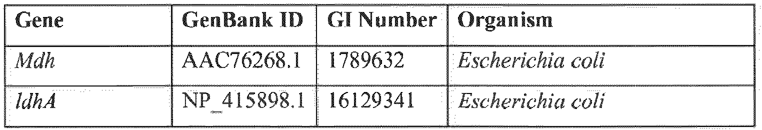

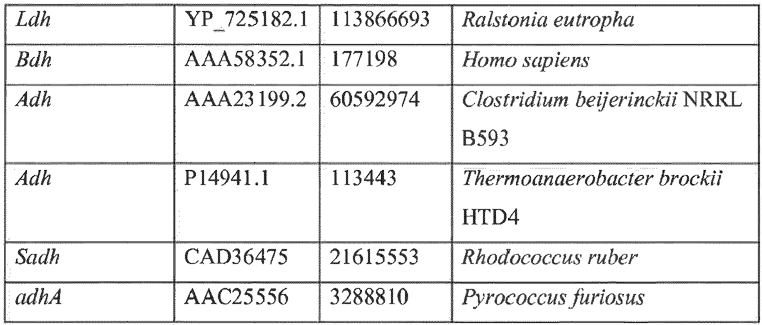

### Step E. Figure 11: 2-hydroxypentenoute dehydratase

Enzyme candidates for the dehydration of 2-hydroxypentenoate (Figure 1, Step E) include fumarase (EC 4.2.1.2), citramalate hydratase (EC 4.2.1.34) and dimethylmaleate hydratase (EC 4.2.1.85). Fumarases naturally catalyze the reversible dehydration of malate to fumarate. Although the ability of fumarase to react with 2-hydroxypentenoate as substrates has not been described in the literature, a wealth of structural information is available for this enzyme and other researchers have successfully engineered the enzyme to alter activity, inhibition and localization (Weaver, Acta Crystallogr D Biol Crystallogr, 61:1395-1401 (2005)). *E. coli* has three fumarases: FumA, FumB, and FumC that are regulated by growth conditions. FumB is oxygen sensitive and only active under anaerobic conditions. FumA is active under microanaerobic conditions, and FumC is the only active enzyme in aerobic growth (Tseng et al., J Bacterial, 183:461-467 (2001); Woods et al., 954:14-26 (1988); Guest et al., J Gen Microbiol 131:2971-2984 (1985)). Additional enzyme candidates are found in *Campylobacter jejuni* (Smith et al., Int.J Biochem.Cell Biol 31:961-975 (1999)), *Thermus thermophilus* (Mizobata et al., Arch.Biochem.Biophys. 355:49-55 (1998)) and *Rattus norvegicus* (Kobayashi et al., J Biochem, 89:1923-1931 (1981)). Similar enzymes with high sequence homology include *fum1* from *Arabidopsis thaliana* and *fumC* from *Corynebacterium glutamicum.* The *mmcBC* fumarase from *Pelotomaculum thermopropionicum* is another class of fumarase with two subunits (Shimoyama et al., FEMS Microbiol Lett, 270:207-213 (2007)). Citramalate hydrolyase naturally dehydrates 2-methylmalate to mesaconate. This enzyme has been studied in *Methanocaldococcus jannaschii* in the context of the pyruvate pathway to 2-oxobutanoate, where it has been shown to have a broad substrate specificity (Drevland et al., J Bacteriol. 189:4391-4400 (2007)). This enzyme activity was also detected in *Clostridium tetanomorphum, Morganella morganii, Citrobacter amalonaticus* where it is thought to participate in glutamate degradation (Kato et al., Arch.Microbiol 168:457-463 (1997)). The *M. jannaschii* protein sequence does not bear significant homology to genes in these organisms. Dimethylmaleate hydratase is a reversible Fe²⁻-dependent and oxygen-sensitive enzyme in the aconitase family that hydrates dimethylmaeate to form (2R,3S)-2,3-dimethylmalate. This enzyme is encoded by *dmdAB* in *Eubacterium barkeri* (Alhapel et al., *supra;* Kollmann-Koch et al., Hoppe Seylers.Z.Physiol Chem. 365:847-857 (1984)).

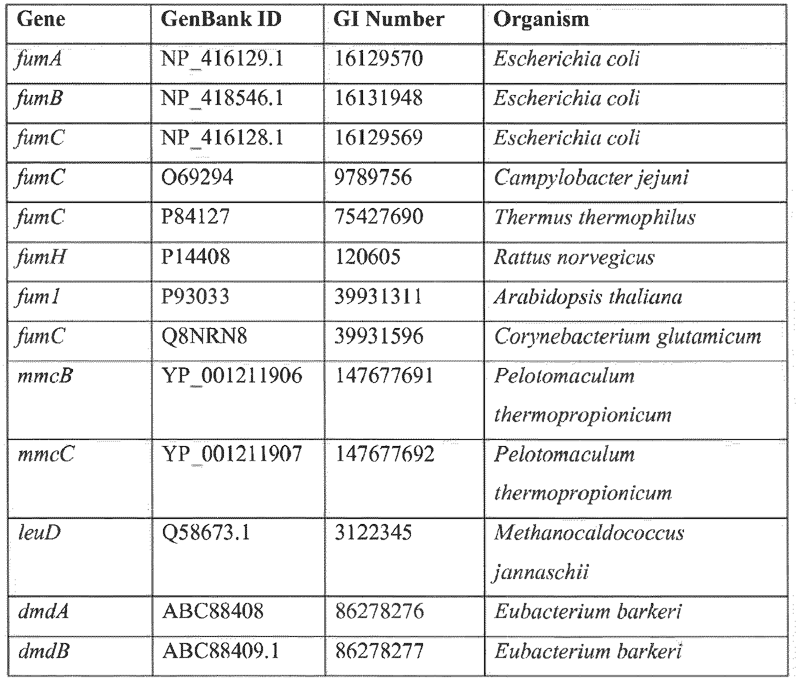

Oleate hydratases catalyze the reversible hydration of non-activated alkenes to their corresponding alcohols. These enzymes represent additional suitable candidates as suggested in WO2011076691. Oleate hydratases from *Elizabethkingia meningoseptica* and *Streptococcus pyogenes* have been characterized (WO 2008/119735). Examples include the following proteins.

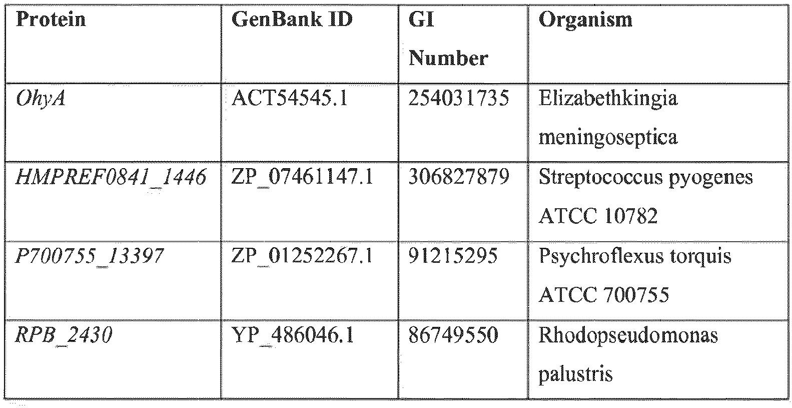

### Step F, Figure 11: 2,4-pentadienoate decarboxylase

The decarboxylation reactions of 2,4-pentadienoate to butadiene (step F of Figure 1) are catalyzed by enoic acid decarboxylase enzymes. Exemplary enzymes are sorbic acid decarboxylase, aconitate decarboxylase, 4-oxalocrotonate decarboxylase and cinnamate decarboxylase. Sorbic acid decarboxylase converts sorbic acid to 1,3-pentadiene. Sorbic acid decarboxylation by *Aspergillus niger* requires three genes: *padA1, ohbA1,* and *sdrA* (Plumridge et al. Fung. Genet. Bio, 47:683-692 (2010). *PadA1* is annotated as a phenylacrylic acid decarboxylase, *ohbA1* is a putative 4-hydroxybenzoic acid decarboxylase, and *sdrA* is a sorbic acid decarboxylase regulator. Additional species have also been shown to decarboxylate sorbic acid including several fungal and yeast species (Kinderlerler and Hatton, Food Addit Contam., 7(5):657-69 (1990); Casas et al., Int J Food Micro., 94(1):93-96 (2004); Pinches and Apps, Int. J. Food Microbiol. 116: 182-185 (2007)). For example, *Aspergillus oryzae* and *Neosartorya fischeri* have been shown to decarboxylate sorbic acid and have close homologs to *padA1, ohbA1,* and *sdrA.*

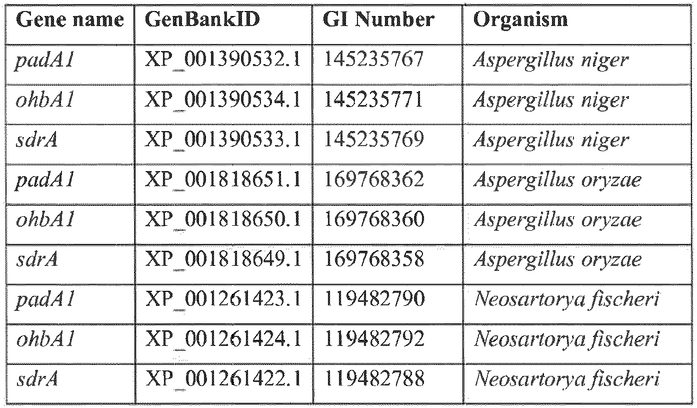

Aconitate decarboxylase (EC 4.1.1.6) catalyzes the final step in itaconate biosynthesis in a strain of *Candida* and also in the filamentous fungus *Aspergillus terreus* (Bonnarme et al. J Bacterial. 177:3573-3578 (1995); Willke and Vorlop, Appl Microbiol. Biotechnol 56:289-295 (2001)). A cis-aconitate decarboxylase (CAD) (EC 4.1.16) has been purified and characterized from *Aspergillus terreus* (Dwiarti et al., J. Biosci. Bioeng. 94(1): 29-33 (2002)). Recently, the gene has been cloned and functionally characterized (Kanamasa et al., Appl. Microbia I Biotechnol 80:223-229 (2008)) and (WO/2009/014437). Several close homologs of CAD are listed below (EP 2017344A1; WO 2009/014437 Al). The gene and protein sequence of CAD were reported previously (EP 2017344 A1; WO 2009/014437 A1), along with several close homologs listed in the table below.

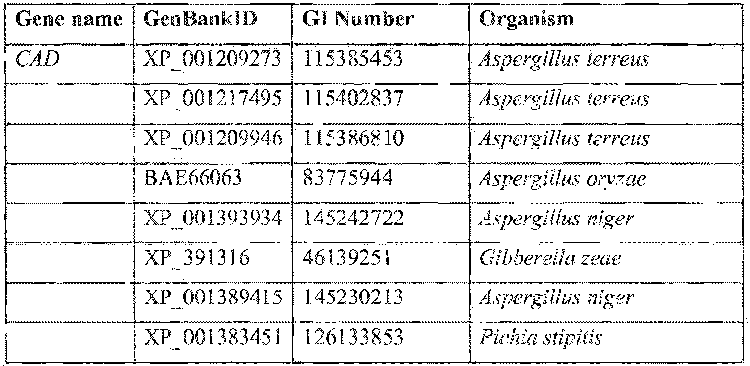

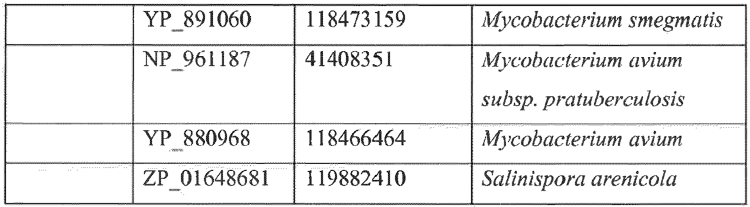

An additional class of decarboxylases has been characterized that catalyze the conversion of cinnamate (phenylacrylate) and substituted cinnamate derivatives to the corresponding styrene derivatives. These enzymes are common in a variety of organisms and specific genes encoding these enzymes that have been cloned and expressed in *E. coli* are: *pad 1* from *Saccharomyces cerevisae* (Clausen et al., Gene 142:107-112 (1994)), *pdc* from *Lactobacillus plantarum* (Barthelmebs et al., Appl Environ Microbiol. 67:1063-1069 (2001); Qi et al., Metab Eng 9:268-276 (2007); Rodriguez et al., .I.Agric.Food Chem. 56:3068-3072 (2008)), *pofK (pad)* from *Klebsiella oxylocα* (Uchiyama et al., Biosci.Biotechnol.Biochem. 72:116-123 (2008); Hashidoko et al., Biosci.Biotech.Biochem. 58:217-218 (1994)), *Pedicoccus pentosaceus* (Barthelmebs et al., Appl Environ Microbiol. 67:1063-1069 (2001)), and *padC* from *Bacillus subtilis* and *Bacillus pumilus* (Shingler et al., J. Bacteriol., 174:711-724 (1992)). A ferulic acid decarboxylase *from Pseudomonas fluorescens* also has been purified and characterized (Huang et al., J.Bacteriol. 176:5912-5918 (1994)). Importantly, this class of enzymes have been shown to be stable and do not require either exogenous or internally bound co-factors, thus making these enzymes ideally suitable for biotransformations (Sariaslani, Annu.Rev.Microbiol. 61:51-69 (2007)).

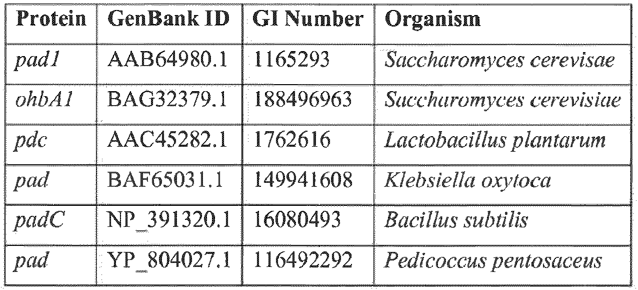

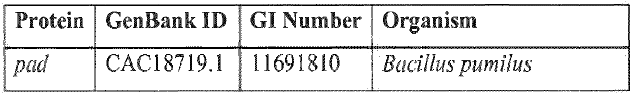

4-Oxalocronate decarboxylase catalyzes the decarboxylation of 4-oxalocrotonate to 2-oxopentanoate. This enzyme has been isolated from numerous organisms and characterized. The decarboxylase typically functions in a complex with vinylpyruvate hydratase. Genes encoding this enzyme include *dmpH* and *dmpE* in *Pseudomonas sp. (strain 600)* (Shingler et al., J. Bacteriol., 174:711-724 (1992)), *xylII* and *xylIII* from *Pseudomonas putida* (Kato et al., Arch.Microbiol 168:457-463 (1997); Stanley et al., Biochemistry 39:3514 (2000); Lian et al., J.Am.Chem.Soc. 116:10403-10411 (1994)) and *Reut_B5691* and *Reut_B5692* from *Ralstonia eutropha JMP134* (Hughes et al., J Bacterial, 158:79-83 (1984)). The genes encoding the enzyme from *Pseudomonas sp. (strain 600)* have been cloned and expressed in *E*. *coli* (Shingler et al., J. Bacteriol. 174:711-724 (1992)). The 4-oxalocrotonate decarboxylase encoded by *xylI* in *Pseudomonas putida* functions in a complex with vinylpyruvate hydratase. A recombinant form of this enzyme devoid of the hydratase activity and retaining wild type decarboxylase activity has been characterized (Stanley et al., Biochem. 39:718-26 (2000)). A similar enzyme is found in *Ralstonia pickettii* (formerly *Pseudomonas pickettii*) (Kukor et al., J Bacterial. 173:4587-94 (1991)).

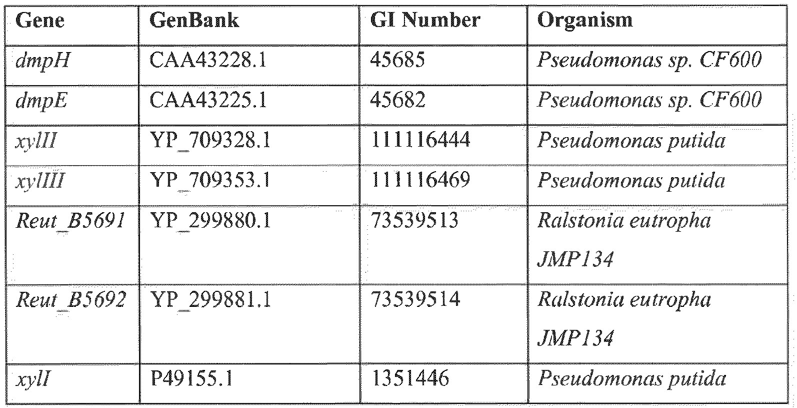

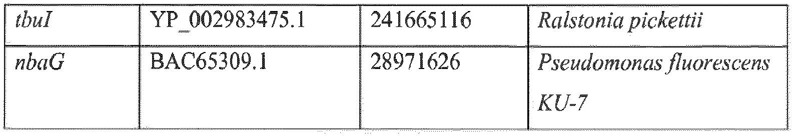

Numerous characterized enzymes decarboxylate amino acids and similar compounds, including aspartate decarboxylase, lysine decarboxylase and ornithine decarboxylase. Aspartate decarboxylase (EC 4.1.1.11) decarboxylates aspartate to form beta-alanine. This enzyme participates in pantothenate biosynthesis and is encoded by gene *panD* in *Escherichia coli* (Dusch et al., Appl.Environ.Microbiol 65:1530-1539 (1999); Ramjee et al., Biochem.J323 ( Pt 3):661-669 (1997); Merkel et al., FEMS Microbiol Lett. 143:247-252 (1996); Schmitzberger et al., EMBO J 22:6193-6204 (2003)). The enzymes from *Mycobacterium tuberculosis* (Chopra et al., Protein Expr.Purif. 25:533-540 (2002)) and *Corynebacterium glutanicum* (Dusch et al., Appl. En viron.Microbiol 65:1530-1539 (1999)) have been expressed and characterized in *E. coli.*

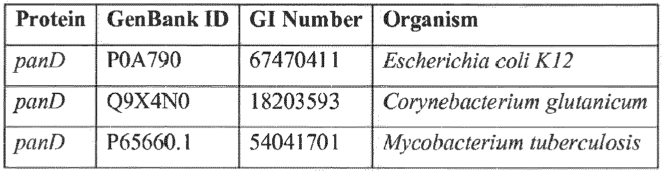

Lysine decarboxylase (EC 4.1.1.18) catalyzes the decarboxylation of lysine to cadaverine. Two isozymes of this enzyme are encoded in the *E. coli* genome by genes *cadA* and *ldcC.* CadA is involved in acid resistance and is subject to positive regulation by the *cadC* gene product (Lemonnier et al., Microbiology 144 ( Pt 3):751-760 (1998)). CadC accepts hydroxylysine and S-aminoethylcysteine as alternate substrates, and 2-aminopimelate and 6-aminocaproate act as competitive inhibitors to this enzyme (Sabo et al., Biochemistry 13:662-670 (1974)). The constitutively expressed *Ide* gene product is less active than CadA (Lemonnier and Lane, Microbiology 144 ( Pt 3):751-760 (1998)). A lysine decarboxylase analogous to CadA was recently identified in *Vibrio parahaemolyticus* (Tanaka et al., J Appl Microbiol 104:1283-1293 (2008)). The lysine decarboxylase from *Selenomonas ruminantium,* encoded by *ldc,* bears sequence similarity to eukaryotic ornithine decarboxylases, and accepts both L-lysine and L-ornithine as substrates (Takatsuka et al., Biosci.Biotechnol Biochem. 63:1843-1846 (1999)). Active site residues were identified and engineered to alter the substrate specificity of the enzyme (Takatsuka et al., JBacteriol. 182:6732-6741 (2000)). Several ornithine decarboxylase enzymes (EC 4.1.1.17) also exhibit activity on lysine and other similar compounds. Such enzymes are found in *Nicotiana glutinosa* (Lee et al., Biochem.J 360:657-665 (2001)), *Lactobacillus sp. 30a* (Guirard et al., JBiol.Chem. 255:5960-5964 (1980)) and *Vibrio vulnificus* (Lee et al., J Biol.Chem. 282:27115-27125 (2007)). The enzymes from *Lactobacillus sp. 30a* (Momany et al., J Mol.Biol. 252:643-655 (1995)) and *V. vulnificus* have been crystallized. The *V. vulnificus* enzyme efficiently catalyzes lysine decarboxylation and the residues involved in substrate specificity have been elucidated (Lee et al., J Biol.Chem. 282:27115-27125 (2007)). A similar enzyme has been characterized in *Trichomonas vaginalis.* (Yarlett et al., Biochem. J 293 ( Pt 2):487-493 (1993)).

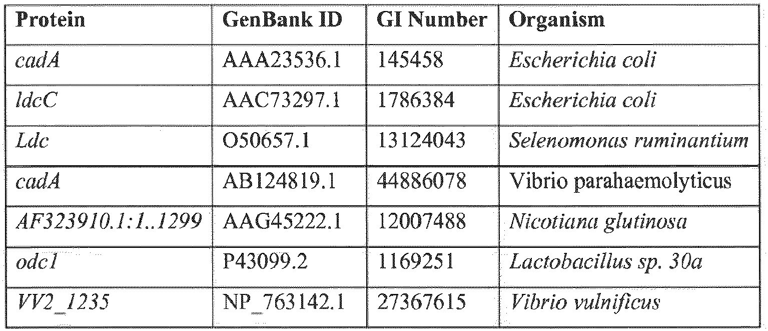

### Steps G and J, Figure 11: 2-oxopentenoate ligase and 2-hydroxypentenoate ligase

ADP and AMP-forming CoA ligases (6.2.1) with broad substrate specificities have been described in the literature. The ADP-forming acetyl-CoA synthetase (ACD, EC 6.2.1.13) from *Archaeoglobus fulgidus,* encoded by AF1211, was shown to operate on a variety of linear and branched-chain substrates including isobutyrate, isopentanoate, and fumarate (Musfeldt et al., J Bacteriol. 184:636-644 (2002)). A second reversible ACD in *Archaeoglobus fulgidus,* encoded by *AF1983,* was also indicated to have a broad substrate range (Musfeldt et al., *supra*)*.* The enzyme from *Haloarcula marismortui,* annotated as a succinyl-CoA synthetase, accepts propionate, butyrate, and branched-chain acids (isovalerate and isobutyrate) as substrates, and was shown to operate in the forward and reverse directions (Brasen et al., Arch.Microbiol 182:277-287 (2004)). The ACD encoded by *PAE3250* from hyperthermophilic crenarchaeon *Pyrobaculum aerophilum* showed the broadest substrate range of all characterized ACDs, reacting with acetyl-CoA, isobutyryl-CoA (preferred substrate) and phenylacetyl-CoA (Brasen and Schonheit, Arch.Microbiol 182:277-287 (2004)). Directed evolution or engineering can be used to modify this enzyme to operate at the physiological temperature of the host organism. The enzymes from *A. fulgidus, H. marismortui* and *P. aerophilum* have all been cloned, functionally expressed, and characterized in *E. coli* (Brasen and Schonheit, Arch.Microbiol 182:277-287 (2004); Musfeldt and Schonheit, J Bacterial. 184:636-644 (2002)). An additional enzyme is encoded by *sucCD* in E. *coli,* which naturally catalyzes the formation of succinyl-CoA from succinate with the concomitant consumption of one ATP, a reaction which is reversible *in vivo* (Buck et al., Biochemistry 24:6245-6252 (1985)). The acyl CoA ligase from *Pseudomonas putida* has been indicated to work on several aliphatic substrates including acetic, propionic, butyric, valeric, hexanoic, heptanoic, and octanoic acids and on aromatic compounds such as phenylacetic and phenoxyacetic acids (Fernandez-Valverde et al., Appl.Environ.Microbiol. 59:1149-1154 (1993)). A related enzyme, malonyl CoA synthetase (6.3.4.9) from *Rhizobium leguminosarum* could convert several diacids, namely, ethyl-, propyl-, allyl-, isopropyl-, dimethyl-, cyclopropyl-, cyclopropylmcthylcnc-, cyclobutyl-, and benzyl-malonate into their corresponding monothioesters (Pohl et al., J.Am.Chem.Soc. 123:5822-5823 (2001)). Recently, a CoA dependent acetyl-CoA ligase was also identified in *Propionibacterium acidipropionici ATCC 4875* (Parizzi et al., BMC Genomics. 2012; 13: 562). This enzyme is distinct from the AMP-dependent acetyl-CoA synthetase and is instead related to the ADP-forming succinyl-CoA synthetase complex (SCSC). Genes releted tto the SCSC (a and β subunits) complex were also found in *Propionibacterium acnes* KPA171202 and *Microlunatus phophovorus* NM-1.

The acylation of acetate to acetyl-CoA is catalyzed by enzymes with acetyl-CoA synthetase activity. Two enzymes that catalyze this reaction are AMP-forming acetyl-CoA synthetase (EC 6.2.1.1) and ADP-forming acetyl-CoA synthetase (EC 6.2.1.13). AMP-forming acetyl-CoA synthetase (ACS) is the predominant enzyme for activation of acetate to acetyl-CoA. Exemplary ACS enzymes are found in *E*. *coli* (Brown et al., J.Gen.Microbiol 102:327-336 (1977)), *Ralstonia eutropha* (Priefert et al., J. Bacteriol 174:6590-6599 (1992)), *Methanothermobacter thermautotrophicus* (Ingram-Smith et al., Archaea. 2:95-107 (2007)), *Salmonella enterica* (Gulick et al., Biochemistry 42:2866-2873 (2003*))* and *Saccharomyces cerevisiae* (Jogl et al., Biochemistry, 43:1425-1431 (2004)).

Methylmalonyl-CoA synthetase from *Rhodopseudomonas palustris* (MatE) converts methylmalonate and malonate to methylmalonyl-CoA and malonyl-CoA, respectively. Structure-based mutagenesis of this enzyme improved CoA synthetase activity with the alternate substrates ethylmalonate and butylmalonate (Crosby et al, AEM, in press (2012)).

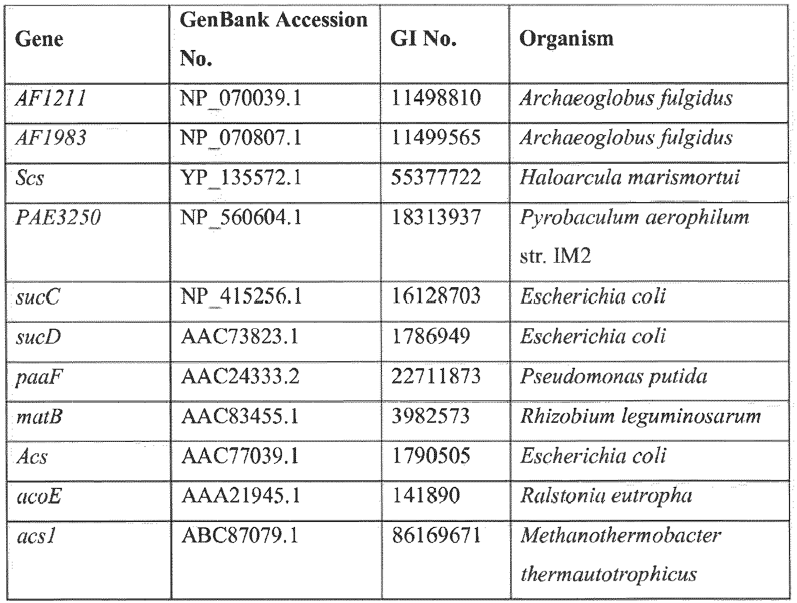

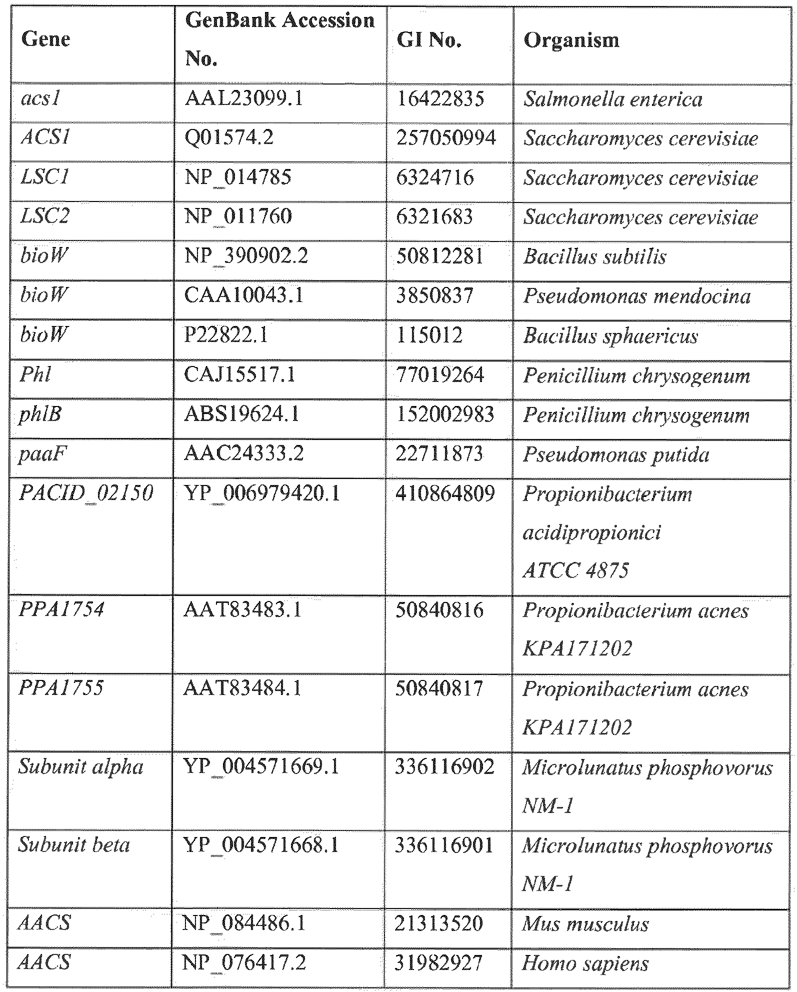

4HB-CoA synthetase catalyzes the ATP-dependent conversion of 4-hydroxybutyrate to 4-hydroxybutyryl-CoA. AMP-forming 4-HB-CoA synthetase enzymes are found in organisms that assimilate carbon via the dicarboxylate/hydroxybutyrate cycle or the 3-hydroxypropionate/4-hydroxybutyrate cycle. Enzymes with this activity have been characterized in *Thermoproteus neutrophilus* and *Metallosphaera sedula* (Ramos-Vera et al, J Bacterial 192:5329-40 (2010); Berg et al, Science 318:1782-6 (2007)). Others can be inferred by sequence homology.

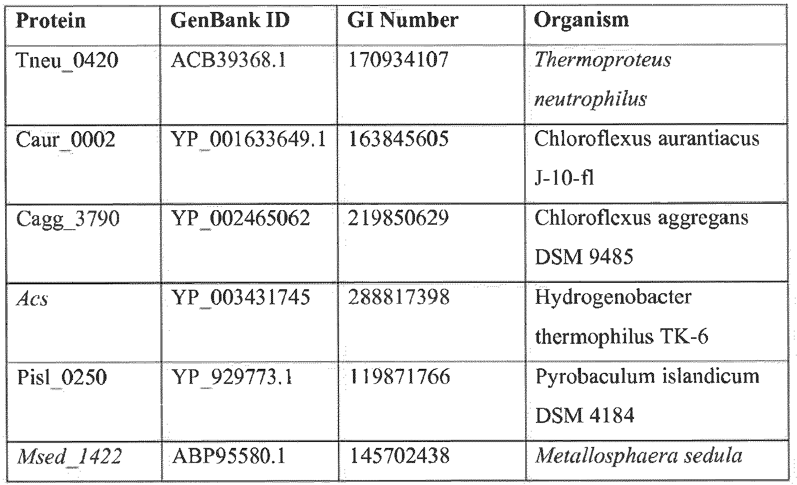

### Step 1, Figure 11: 2-oxopentenoyl-CoA reductase

The reduction of 2-oxopentenoyl CoA to 2-hydroxypentanoyl-CoA can be accomplished by 3-oxoacyl-CoA reductase enzymes (EC 1.1.1.35) that typically convert 3-oxoacyl-CoA molecules into 3-hydroxyacyl-CoA molecules and are often involved in fatty acid beta-oxidation or phenylacetate catabolism. For example, subunits of two fatty acid oxidation complexes in *E. coli,* encoded by *fadB* and *fadJ,* function as 3-hydroxyacyl-CoA dehydrogenases (Binstock et al., Methods Enzymol. 71 Pt C:403-411 (1981)). Given the proximity in *E. coli* of *paaH to* other genes in the phenylacetate degradation operon (Nogales et al., Microbiology, 153:357-365 (2007)) and the fact that *paaH* mutants cannot grow on phenylacetate (Ismail et al., Eur.J Biochem. 270:3047-3054 (2003)), it is expected that the *E. coli paaH* gene also encodes a 3-hydroxyacyl-CoA dehydrogenase. Additional 3-oxoacyl-CoA enzymes include the gene products of *phaC* in *Pseudomonas putida* (Olivera et al., Proc.Natl.Acad.Sci U.S.A 95:6419-6424 (1998)) and *paaC* in *Pseudomonas fluorescens* (Di et al., Arch.Microbiol 188:117-125 (2007)). These enzymes catalyze the reversible oxidation of 3-hydroxyadipyl-CoA to 3-oxoadipyl-CoA during the catabolism of phenylacetate or styrene.

Acetoacetyl-CoA reductase participates in the acetyl-CoA fermentation pathway to butyrate in several species of *Clostridia* and has been studied in detail (Jones et al., Microbiol Rev. 50:484-524 (1986)). The enzyme from *Clostridium acetobutylicum,* encoded by *hbd,* has been cloned and functionally expressed in *E*. *coli* (Youngleson et al., J Bacteriol. 171:6800-6807 (1989)). Yet other genes demonstrated to reduce acetoacetyl-CoA to 3-hydroxybutyryl-CoA are *phbB* from *Zoogloea ramigera* (Ploux et al., Eur.JBiocheni. 174:177-182 (1988)) *andphaB* from *Rhodobacter sphaeroides* (Alber et al., Mol.Microbiol 61:297-309 (2006)). The former gene is NADPH-dependent, its nucleotide sequence has been determined (Peoples et al., Mol.Microbiol 3:349-357 (1989)) and the gene has been expressed in *E. coli.* Substrate specificity studies on the gene led to the conclusion that it could accept 3-oxopropionyl-CoA as a substrate besides acetoacetyl-CoA (Ploux et al., Eur.J Biochem. 1 74:177-182 (1988)). Additional genes include *phaB* in *Paracoccus denitrificans, Hbd1* (C-terminal domain) and *Hbd2* (N-terminal domain) in *Clostridium kluyveri* (Hillmcr and Gottschalk, Biochim. Biophys. Acta 3334:12-23 (1974)) and *HSD17B10* in *Bos taurus* (Wakil et al., JBiol.Chem. 207:631-638 (1954)). The enzyme from *Paracoccus denitrificans* has been functionally expressed and characterized in *E. coli* (Yabutani et al., FEMS Microbiol Lett. 133:85-90 (1995)). A number of similar enzymes have been found in other species of *Clostridia* and in *Metallosphaera sedula* (Berg et al., Science. 318:1782-1786 (2007)). The enzyme from *Candida tropicalis* is a component of the peroxisomal fatty acid beta-oxidation multifunctional enzyme type 2 (MFE-2). The dehydrogenase B domain of this protein is catalytically active on acetoacetyl-CoA. The domain has been functionally expressed in *E. coli,* a crystal structure is available, and the catalytic mechanism is well-understood (Ylianttila et al., Biochem Biophys Res Commun 324:25-30 (2004); Ylianttila et al., J Mol Biol 358:1286-1295 (2006)). 3-Hydroxyacyl-CoA dehydrogenases that accept longer acyl-CoA substrates (eg. EC 1.1.1.35) are typically involved in beta-oxidation. An example is *HSD17B10* in *Bos taurus* (WAKIL et al., JBiol.Chem. 207:631-638 (1954)). phbB from Cupriavidus necatar codes for a 3-hydroxyvalcryl-CoA dehydrogenase activity.

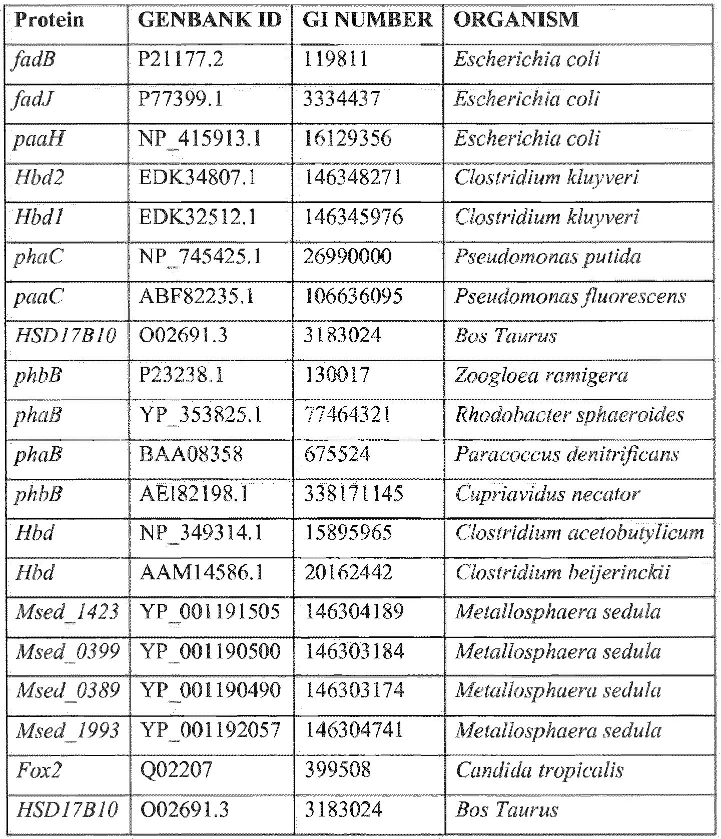

Other exemplary enzymes that can carry this reaction are 2-hydroxyacid dehydrogenases. Such an enzyme. characterized from the halophilic archaeon *Haloferax mediterranei* catalyses a reversible stereospecific reduction of 2-ketocarboxylic acids into the corresponding D-2-hydroxycarboxylic acids. The enzyme is strictly NAD-dependent and prefers substrates with a main chain of 3-4 carbons (pyruvate and 2-oxobutanoate). Activity with 4-methyl-2-oxopentanoate is 10-fold lower. _Two such enzymes from *E. coli* are encoded by malate dehydrogenase *(mdh)* and lactate dehydrogenase *(ldhA).* In addition, lactate dehydrogenase from *Ralstonia eutropha* has been shown to demonstrate high activities on 2-ketoacids of various chain lengths includings lactate, 2-oxobutyrate, 2-oxopentanoate and 2-oxoglutarate (Steinbuchel et al., Eur.J.Biochem. 130:329-334 (1983)). Conversion of alpha-ketoadipate into alpha-hydroxyadipate can be catalyzed by 2-kctoadipatc reductase, an enzyme reported to be found in rat and in human placenta (Suda et al., Arch.Biochem.Biophys. 176:610-620 (1976); Suda et al., Biochem.Biophys.Res.Commun. 77:586-591 (1977)). An additional oxidoreductase is the mitochondrial 3-hydroxybutyrate dehydrogenase *(bdh)* from the human heart which has been cloned and characterized (Marks et al., J.Biol.Chem. 267:15459-15463 (1992)). Alcohol dehydrogenase enzymes of C. *beijerinckii* (Ismaiel et al., J.Bacterial. 175:5097-5105 (1993)) and *T. brockii* (Lamed et al., Biochem.J. 195:183-190 (1981); Peretz et al., Biochemistry. 28:6549-6555 (1989)) convert acetone to isopropanol. Methyl ethyl ketone reductase catalyzes the reduction of MEK to 2-butanol. Exemplary MEK reductase enzymes can be found in *Rhodococcus ruber* (Kosjek et al., Biotechnol Bioeng. 86:55-62 (2004)) and *Pyrococcus furiosus* (van der et al., Eur.J.Biochem. 268:3062-3068 (2001)).

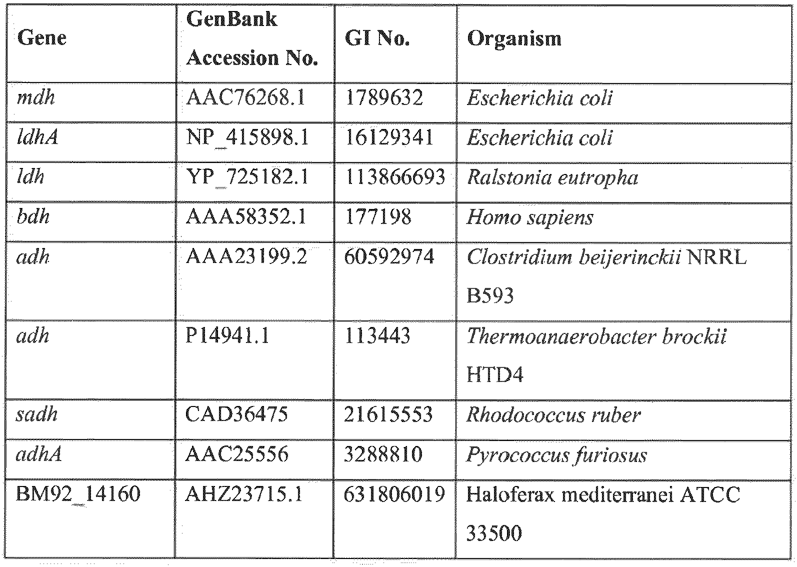

### Step M, Figure 11: 2,4-Pentadienoyl-CoA hydrolase

CoA hydrolysis of 2,4-pentadienoyl CoA can be catalyzed by CoA hydrolases or thioesterases in the EC class 3.1.2. Several CoA hydrolases with broad substrate ranges are suitable enzymes for hydrolyzing these intermediates. For example, the enzyme encoded by *acot12* from *Rattus norvegicus* brain (Robinson et al., Biochem.Biophys.Res.Commun. 71:959-965 (1976)) can react with butyryl-CoA, hexanoyl-CoA and malonyl-CoA. The human dicarboxylic acid thioesterase, encoded by *acot8,* exhibits activity on glutaryl-CoA, adipyl-CoA, suberyl-CoA, scbacyl-CoA, and dodecanedioyl-CoA (Westin et al., J.Biol.Chem. 280:38125-38132 (2005)). The closest *E. coli* homolog to this enzyme, *tesB,* can also hydrolyze a range of CoA thiolesters (Naggert et al., J Biol Chem 266:11044-11050 (1991). A similar enzyme has also been characterized in the rat liver (Deana R., Biochem Int 26:767-773 (1992)). Additional enzymes with hydrolase activity in *E. coli* include *ybgC, paaI,* yciA, and *ybdB* (Kuznetsova, et al., FEMS Microbiol Rev, 2005, 29(2):263-279; Song et al., J Biol Chem, 2006, 281(16):11028-38). Though its sequence has not been reported, the enzyme from the mitochondrion of the pea leaf has a broad substrate specificity, with demonstrated activity on acetyl-CoA, propionyl-CoA, butyryl-CoA, palmitoyl-CoA, oleoyl-CoA, succinyl-CoA, and crotonyl-CoA (Zeiher et al., Plant.Physiol. 94:20-27 (1990)) The acetyl-CoA hydrolase, *ACH1,* from *S*. *cerevisiae* represents another candidate hydrolase (Buu et al., J.Biol.Chem. 278:17203-17209 (2003)) .

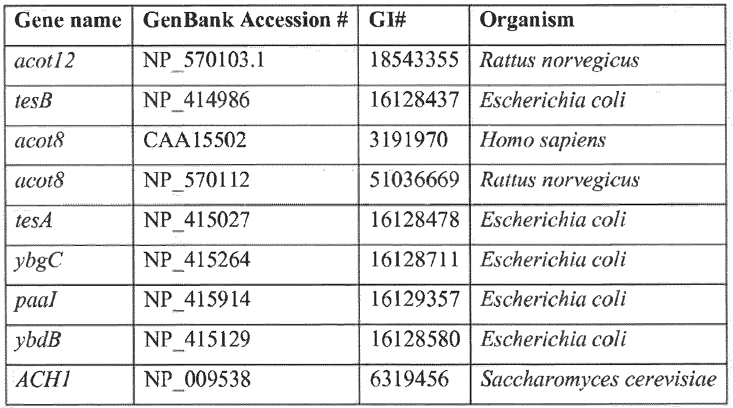

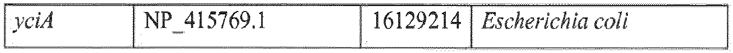

Yet another candidate hydrolase is the glutaconatc CoA-transferasc from *Acidaminococcus fermentans.* This enzyme was transformed by site-directed mutagenesis into an acyl-CoA hydrolase with activity on glutaryl-CoA, acetyl-CoA and 3-butenoyl-CoA (Mack et al., FEBS.Letl. 405:209-212 (1997)).This suggests that the enzymes encoding succinyl-CoA:3-kctoacid-CoA transferases and acetoacetyl-CoA:acetyl-CoA transferases may also serve as candidates for this reaction step but would require certain mutations to change their function.

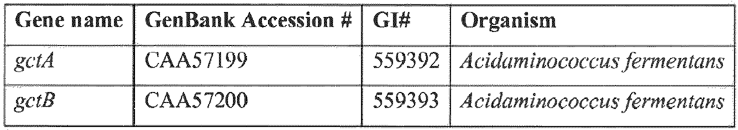

Additional hydrolase enzymes include 3-hydroxyisobutyryl-CoA hydrolase which has been described to efficiently catalyze the conversion of 3-hydroxyisobutyryl-CoA to 3-hydroxyisobutyrate during valine degradation (Shimomura et al., J Biol Chem. 269:14248-14253 (1994)). Genes encoding this enzyme include *hibch* of *Rattus norvegicus* (Shimomura et al., Methods Enzymol. 324:229-240 (2000)) and *Homo sapiens* (Shimomura et al., *supra).* Similar gene candidates can also be identified by sequence homology, including *hibch* of *Saccharomyces cerevisiae* and *BC_2292* of *Bacillus cereus.*

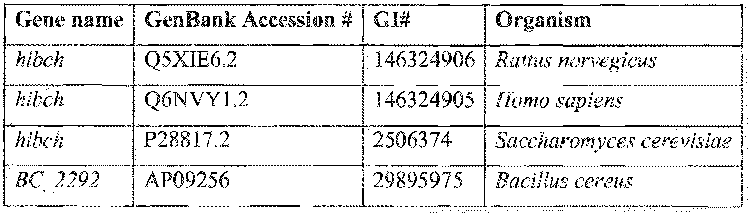

Methylmalonyl-CoA is converted to methylmalonate by methylmalonyl-CoA hydrolase (EC 3.1.2.7).This enzyme, isolated from *Rattus norvegicus* liver, is also active on malonyl-CoA and propionyl-CoA as alternative substrates (Kovachy et al., J. Biol. Chem., 258: 11415-11421 (1983)).

### Steps H, K and N, Figure 11: 2-oxopentenoate: acetyl CoA transferase, 2-hydroxypentenoate: acetyl-CoA CoA transferase, 2,4-Pentadienoyl-CoA: acetyl CoA CoA transferase

Several transformations require a CoA transferase to activate carboxylic acids to their corresponding acyl-CoA derivatives. CoA transferase enzymes have been described in the open literature and represent suitable candidates for these steps. These are described below.

The gene products of *cat1, cat2,* and *cat3* of *Clostridium kluyveri* have been shown to exhibit succinyl-CoA, 4-hydroxybutyryl-CoA, and butyryl-CoA transferase activity, respectively (Seedorf et al., Proc.Natl.Acad.Sci U.S.A 105:2128-2133 (2008); Sohling et al., J Bacterial. 178:871-880 (1996)). Similar CoA transferase activities are also present in *Trichomonas vaginalis, Trypanosoma brucei, Clostridium aminobutyricum* and *Porphyromonas gingivalis* (Riviere et al., J.Biol.Chem. 279:45337-45346 (2004); van Grinsven et al., J.Biol.Chem. 283:1411-1418 (2008)).

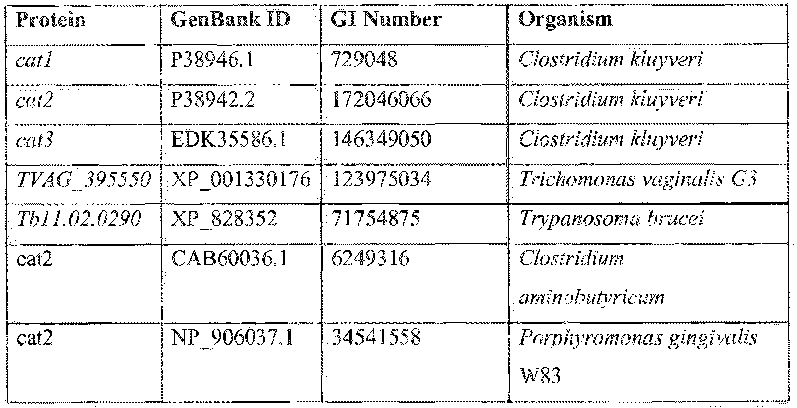

A fatty acyl-CoA transferase that utilizes acetyl-CoA as the CoA donor is acetoacetyl-CoA transferase, encoded by the *E. coli atoA* (alpha subunit) and *atoD* (beta subunit) genes (Korolev et al., Acta Crystallogr.D.Biol.Crystallogr. 58:2116-2121 (2002); Vandcrwinkcl et al., 33:902-908 (1968)). This enzyme has a broad substrate range on substrates of chain length C3-C6 (Sramek et al., Arch Biochem Biophys 171:14-26 (1975)) and has been shown to transfer the CoA moiety to acetate from a variety of branched and linear 3-oxo and acyl-CoA substrates, including isobutyrate (Matthies et al., Appl Environ.Microbial 58: 1435-1439 (1992)), valerate (Vanderwinkel et al., Biochem.Biophys.Res.Commun. 33:902-908 (1968)) and butanoate (Vanderwinkel et al., Biochem.Biophys.Res.Commun. 33:902-908 (1968)). This enzyme is induced at the transcriptional level by acetoacetate, so modification of regulatory control may be necessary for engineering this enzyme into a pathway (Pauli et al., Eur.J Biochem. 29:553-562 (1972)). Similar enzymes exist in *Corynebacterium glutamicum* ATCC 13032 (Duncan et al., Appl Environ Microbiol, 68:5186-5190 (2002)), *Clostridium acetobutylicum* (Cary et al., Appl Environ Microbiol 56:1576-1583 (1990); Wiesenborn et al., Appl Environ Microbiol 55:323-329 (1989)), and *Clostridium succharoperbutylacetonicum* (Kosaka et al., Biosci.Biotechnol Biochem. 71:58-68 (2007)).

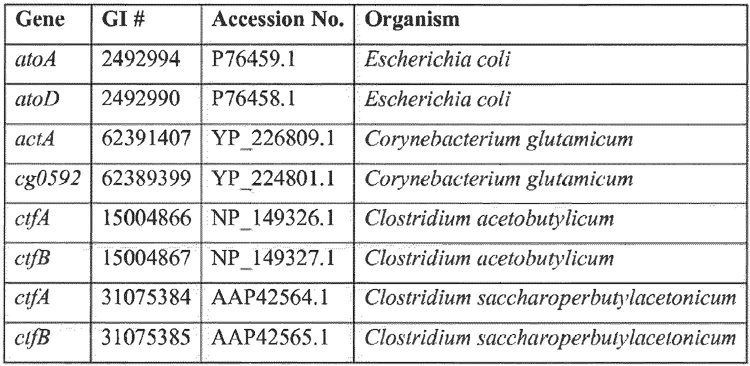

### Step L, Figure 11: 2-hydroxypentenoyl-CoA dehydratase

The dehydration of 2-hydroxypentenoyl-CoA can be catalyzed by a special class of oxygen-sensitive enzymes that dehydrate 2-hydroxyacyl-CoA derivatives by a radical-mechanism (Buckel and Golding, Annu. Rev. Microbiol. 60:27-49 (2006); Buckel et al., Curr. Opin. Chem. Biol. 8:462-467 (2004); Buckel et al., Biol. Chem. 386:951-959 (2005); Kim et al., FEBSJ. 272:550-561 (2005); Kim et al., FEMS Microbiol. Rev. 28:455-468 (2004); Zhang et al., Microbiology 145 ( Pt 9):2323-2334 (1999)). One example of such an enzyme is the lactyl-CoA dehydratase from *Clostridium propionicum,* which catalyzes the dehydration of lactoyl-CoA to form acryloyl-CoA (Kuchta and Abeles, J. Biol. Chem. 260:13181-13189 (1985); Hofmeister and Buckel, Eur. J. Biochem. 206:547-552 (1992)). An additional example is 2-hydroxyglutaryl-CoA dehydratase encoded by *hgdABC* from *Acidaminococcus fermentans* (Muëller and Buckel, Eur. J. Biochem. 230:698-704 (1995); Schweiger et al., Eur. J. Biochem. 169:441-448 (1987)). Purification of the dehydratase from *A. fermentans* yielded two components, A and D. Component A (HgdC) acts as an activator or initiator of dehydration. Component D is the actual dehydratase and is encoded by HgdAB. Variations of this enzyme have been found in *Clostridum symbiosum* and *Fusobacterium nucleatum.* Component A, the activator, from A. fermentans is active with the actual dehydratse (component D) from C. *symbiosum* and is reported to have a specific activity of 60 per second, as compared to 10 per second with the component D from *A. fermentans.* Yet another example is the 2-hydroxyisocaproyl-CoA dehydratase from *Clostridium difficile* catalyzed by *hadBC* and activated by *hadI* (Darley et al., FEBS J. 272:550-61 (2005)). The sequence of the complete *C*. *propionicium* lactoyl-CoA dehydratase is not yet listed in publicly available databases. However, the sequence of the beta-subunit corresponds to the GenBank accession number AJ276553 (Selmer et al, Eur J Biochem, 269:372-80 (2002)). The dehydratase from *Clostridium sporogens* that dehydrates phenyllactyl-CoA to cinnamoyl-CoA is also a potential candidate for this step. This enzyme is composed of three subunits, one of which is a CoA transferase. The first step comprises of a CoA transfer from cinnamoyl-CoA to phenyllactate leading to the formation of phenyllactyl-CoA and cinnamate. The product cinnamate is released. The dehydratase then converts phenyllactyl-CoA into cinnamoyl-CoA. FldA is the CoA transferase and FldBC arc related to the alpha and beta subunits of the dehydratase, component D, from *A. fermentans.*

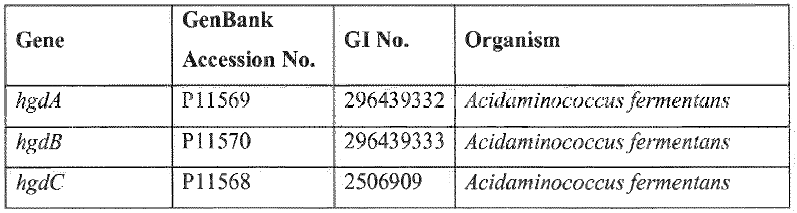

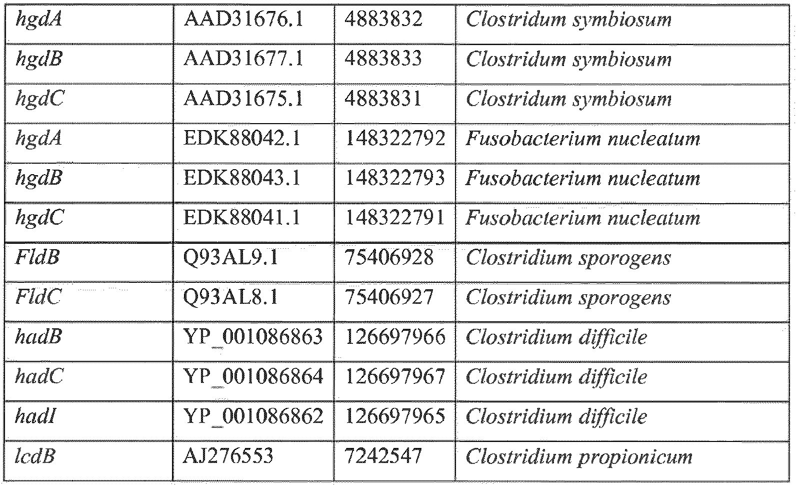

Another dehydratase that can potentially conduct such a biotransformation is the enoyl-CoA hydratase (4.2.1.17) of *Pseudomonas putida,* encoded by *ech* that catalyzes the conversion of 3-hydroxybutyryl-CoA to crotonyl-CoA (Roberts et al., Arch.Microbiol 117:99-108 (1978)). This transformation is also catalyzed by the *crt* gene product of *Clostridium acetobutylicum,* the *crt1* gene product of *C*. *kluyveri,* and other clostridial organisms Atsumi et al., Metab Eng 10:305-311 (2008); Boynton et al., JBacteriol. 178:3015-3024 (1996); Hillmer et al., FEBS Lett. 21:351-354 (1972)). Additional enoyl-CoA hydratase candidates are *phaA* and *phaB,* of *P. putida,* and *paaA* and *paaB* from *P. fluorescens* (Olivera et al., Proc.Natl.Acad.Sci U.S.A 95:6419-6424 (1998)). The gene product of*pimF* in *Rhodopseudomonas palustris* is predicted to encode an enoyl-CoA hydratase that participates in pimeloyl-CoA degradation (Harrison et al., Microbiology 151:727-736 (2005)). Lastly, a number of *Escherichia coli* genes have been shown to demonstrate enoyl-CoA hydratase functionality including *maoC* (Park et al.,J Bacterial. 185:5391-5397 (2003)), *paaF* (Ismail et al., Eur.J Biochem. 270:3047-3054 (2003); Park et al., Appl.Biochem.Biotechnol 113-116:335-346 (2004); Park et al., Biotechnol Bioeng 86:681-686 (2004)) and *paaG* (Ismail et al., Eur.J Biochem. 270:3047-3054 (2003); Park and Lee, Appl.Biochem.Biotechnol 113-116:335-346 (2004); Park and Yup, Biotechnol Bioeng 86:681-686 (2004)).

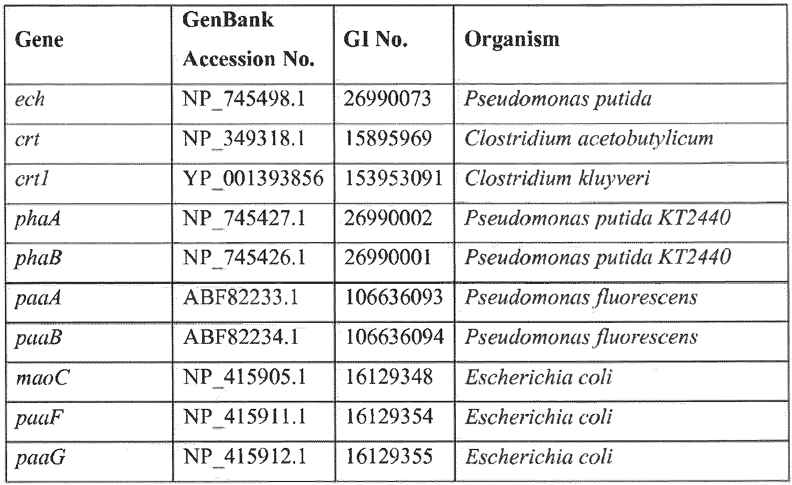

Alternatively, the *E. coli* gene products of *fadA* and *fadB* encode a multicnzymc complex involved in fatty acid oxidation that exhibits enoyl-CoA hydratase activity (Yang et al., Biochemistry 30:6788-6795 (1991); Yang, J Bacterial. 173:7405-7406 (1991); Nakahigashi et al., Nucleic Acids Res. 18:4937 (1990)). Knocking out a negative regulator encoded by *fadR* can be utilized to activate the *fadB* gene product (Sato et al., JBiosci.Bioeng 103:38-44 (2007)). The *fadI* and *fadJ* genes encode similar functions and are naturally expressed under anaerobic conditions (Campbell et al., Mol.Microbiol 47:793-805 (2003)).

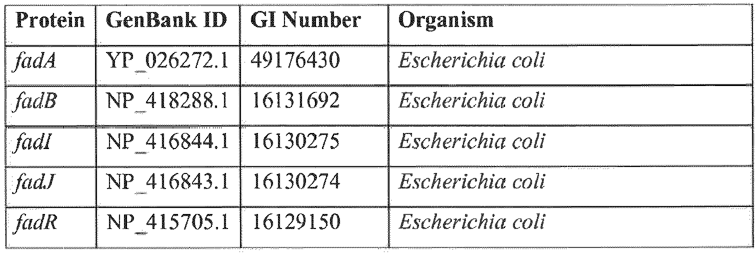

Throughout this application various publications have been referenced. The disclosures of these publications in their entireties, including GenBank and GI number publications, are hereby incorporated by reference in this application in order to more fully describe the state of the art to which this invention pertains. Although the invention has been described with reference to the examples provided above, it should be understood that various modifications can be made without departing from the spirit of the invention.

### The invention is characterized by the following items:

1. A non-natural microbial organism capable of producing acetyl-CoA, or acetyl-CoA and oxaloacetate, the organism comprising:
   (a) a pathway comprising phosphoketolase for producing acetyl-CoA (PK pathway);
   (b) a non-phosphotransferase system (non-PTS) for sugar uptake comprising a modification to increase non-PTS activity; and optionally
   (c) a modification that attenuates or eliminates a PTS activity.
2. The non-natural organism of item 1further comprising one or more modification(s) to the organism's electron transport chain to enhance efficiency of ATP production, to enhance availability of reducing equivalents, or both.
3. A non-natural microbial organism capable of producing acetyl-CoA, or acetyl-CoA and oxaloacetate, the organism comprising:
   (a) a pathway comprising phosphoketolase for producing acetyl-CoA (PK pathway); and
   (b) one or more modification(s) to the organism's electron transport chain to enhance efficiency of ATP production, to enhance availability of reducing equivalents, or both.
4. The non-natural organism of item 3 further comprising (c) a non-phosphotransferase system (non-PTS) for sugar uptake comprising a modification to increase non-PTS activity; (d) a modification that attenuates or eliminates PTS activity, or both (c) and (d).
5. The non-natural organism of any one of the previous items, wherein the PK pathway comprises one, two or three exogenous nucleic acids encoding a PK pathway enzyme expressed in sufficient amount to enhance production of acetyl-CoA.
6. The non-natural organism of any one of the previous items wherein the PK pathway comprises:
   (c1) 1T and 1V; or
   (c2) 1T, 1W, and 1X;
   wherein 1T is a fructose-6-phosphate phosphoketolase, wherein 1V is a phosphotransacetylase, wherein 1W is an acetate kinase, and wherein 1X is an acetyl-CoA transferase, an acetyl-CoA synthetase, or an acetyl-CoA ligase.
7. The non-natural organism of any one of the previous items wherein the PK pathway comprises:
   (c3) 1U and 1V;
   (c4) 1U, 1W, and 1X;
   wherein 1U is a xylulose-5-phosphate phosphoketolase, wherein 1V is a phosphotransacetylase, wherein 1W is an acetate kinase, and wherein 1X is an acetyl-CoA transferase, an acetyl-CoA synthetase, or an acetyl-CoA ligase.
8. The non-natural organism of item 1 or 4 wherein the modification that attenuates or eliminates a PTS activity is attenuation or elimination of a PTS enzyme or protein.
9. The non-natural organism of item 8 wherein the modification decreases expression of the PTS enzyme or protein to attenuate or eliminate PTS activity or the modification decreases activity of the PTS enzyme or protein to attenuate or eliminate PTS activity.
10. The non-natural organism of any one of the previous items having attenuated or eliminated expression of a PTS enzyme or protein, wherein the PTS enzyme or protein is selected from the group consisting of Enzyme I (EI), histidine phosphocarrier protein (HPr), Enzyme II (EII), and transmembrane Enzyme II C (EIIC).
11. The non-natural organism of any one of the above items having attenuated or eliminated expression of PTS activity as caused by alteration of E1.
12. The non-natural organism of any one of the above items having attenuated or eliminated PTS activity as caused by alteration of ptsI in *E. coli* or *E. coli* ptsI homologs.
13. The non-natural organism of any one of items 1, 2, or 4-12, wherein the modification to increase non-PTS activity comprises increased expression or activity of a non-PTS permease, a non-PTS sugar kinase, a facilitator protein, or combinations thereof.
14. The non-natural organism of item 13 wherein the non-PTS permease is glucose permease, and/or the non-PTS sugar kinase is glucokinase.
15. The non-natural organism of item 13 wherein the facilitator protein binds glucose and is encoded by glf.
16. The non-natural organism of items 13 and 14 wherein the glucokinase is encoded by *E. coli* glk or *E. coli* glk homologs, and the permease is encoded by galP in *E. coli* or *E. coli* galP homologs.
17. The non-natural organism of any one of items 13-16 wherein the non-PTS comprises one, two, three or more exogenous nucleic acids encoding the non-PTS enzyme or protein expressed in sufficient amount to increase non-PTS activity.
18. The non-natural organism of any one of the above items, capable of a rate of sugar uptake that is at least at least 10%, 25%, 50%, 75%, 100%, 125% or 150% greater than the rate of sugar uptake of an organism that does not include the modification to increase non-PTS activity.
19. The non-natural organism of any one of the previous items further comprising a modification that attenuates or eliminates activity of pyruvate kinase.
20. The non-natural organism of any one of the previous items further comprising one or modifications to enhance synthesis of oxaloacetate.
21. The non-natural organism of item 20 wherein the one or more modification(s) to enhance synthesis of oxaloacetate comprises increasing the expression or activity of phosphoenolpyruvate (PEP) synthase, pyruvate carboxylase, phosphoenolpyruvate carboxylase, malic enzyme, or combinations thereof.
22. The non-natural organism of item 21 wherein the PEP synthase is encoded by ppsA in *E. coli* or *E. coli* ppsA homologs.
23. The non-natural organism of item 21 wherein the phosphoenolpyruvate carboxylase is encoded by ppc in *E. coli* or its *E. coli* ppc homologs .
24. The non-natural organism of item 21 where the pyruvate carboxylase is encoded by pyc in *Rhizobium etli, Lactococcus lactis,* or *Rhizobium etli* or *Lactococcus lactis* pyc homologs.
25. The non-natural organism of item 21 wherein the malic enzyme is encoded by sfcA or maeB in *E. coli* or *E. coli* sfcA or maeB homologs.
26. The non-natural organism of any one of the items 1-25 further comprising a modification that causes expression or increased expression of one or more of enzymes selected from the group consisting of ribose-5-phosphate isomerase, ribulose-5-phosphate epimerase, transaldolase, and transketolase.
27. The non-natural organism of any one of items 2-26 having one or more modification(s) to the organism's electron transport chain that enhance efficiency of ATP production that comprise (i) attenuation or elimination of an NADH-dependent dehydrogenase that does not translocate protons, or (ii) attenuation or elimination of a first cytochrome oxidase that has a lower efficiency of proton translocation per pair of electrons as compared to a second cytochrome oxidase having a higher efficiency of proton translocation as expressed by the organism, or both, wherein the first and second cytochrome oxidases are native or heterologous.
28. The non-natural organism of item 27, wherein the first and second cytochrome oxidases are native cytochrome oxidases or wherein the first cytochrome oxidase is native and the second cytochrome oxidase is heterologous.
29. The non-natural organism of item 27 or 28 wherein the first cytochrome oxidase is encoded by cydAB, appBC, ygiN, or any combination of these.
30. The non-natural organism of any one of items 27-29, wherein the NADH-dependent dehydrogenase is one or more of Ndh, WrbA, YhdH, YieF, YtfG, Qor and MdaB .
31. The non-natural organism of any one of items 27-30, wherein the one or more modification(s) to the organism's electron transport chain that enhance efficiency of ATP production comprise (i) increasing expression of a native or heterologous NADH dehydrogenase(s), (ii) increasing expression of a native or heterologous cytochrome oxidase(s), by attenuating arcA, or both (i) and (ii).
32. The non-natural organism of any one of items 27-31 having one or more modification(s) that enhance the availability of a reducing equivalent, wherein the reducing equivalent is NADH, NADPH, or both.
33. The non-natural organism of item 32, wherein the modification to enhance availability of reducing equivalents comprises attenuation or deletion of a non (proton)-translocating NADH dehydrogenase
34. The non-natural organism of item 33, wherein the non (proton)-translocating NADH dehydrogenase is selected from the group consisting of *E. coli* Ndh, WrbA, YhdH, YieF, YtfG, Qor, MdaB, and their homologues thereof.
35. The non-natural organism of item 27, wherein the modification to enhance the availability of reducing equivalents comprises (i) increasing the expression or activity of a pyruvate dehydrogenase, (ii) increasing the expression or activity of a pyruvate formate lyase together with a formate dehydrogenase generating NADH, NADPH or both, (iii) attenuation or elimination of a native formate dehydrogenase that does not produce NADH, or any combination of (i) - (iii), in the organism.
36. The non-natural organism of any one of the previous items further comprising a formaldehyde assimilation pathway.
37. The non-natural organism of item 36 comprising a pathway from Xu5P to F6P or from ribulose-5-phosphate (Ru5P) to F6P.
38. The non-natural organism of item 37 wherein the pathway from Xu5P or Ru5P to F6P comprises:
   (d1) 1B and 1C; and/or
   (d2) 1D and 1Z;
   wherein 1B is 3-hexulose-6-phosphate synthase and 1C is 6-phospho-3-hexuloisomerase; (1D) dihydroxyacetone synthase and (1Z) fructose-6-phosphate aldolase.
39. The non-natural organism of any one of the above items further comprising a methanol oxidation pathway.
40. The non-natural organism of item 39 comprising at least one exogenous nucleic acid encoding a methanol oxidation pathway enzyme expressed in a sufficient amount to produce formaldehyde in the presence of methanol, wherein said methanol oxidation pathway comprises a methanol dehydrogenase, and optionally wherein the methanol oxidation pathway enzyme is methanol dehydrogenase.
41. The non-natural organism of any one of the previous items further comprising attenuation of one or more endogenous enzymes selected from DHA kinase, methanol oxidase, PQQ-dependent methanol dehydrogenase, DHA synthase, or any combination thereof.
42. The non-natural organism of any one of the previous items further comprising attenuation of one or more endogenous enzymes of a competing formaldehyde assimilation or dissimilation pathway.
43. The non-natural organism of any one of the previous items, wherein said non-naturally occurring microbial organism is capable of growing in a culture medium with lower specific oxygen supply rate as compared to the culture medium for growth of a microorganism that does not include the phosphoketolase pathway.
44. The non-natural organism of any one of the previous items further comprising a pathway capable of producing succinyl-CoA, malonyl-CoA, acetoacetyl-CoA or any combination thereof, wherein said pathway converts acetyl-CoA to said succinyl-CoA, malonyl-CoA, acetoacetyl-CoA or any combination thereof by one or more enzymes.
45. The non-natural organism of any one of the previous items further comprising a pathway capable of producing a bioderived compound.
46. The non-natural organism of item 45 wherein said bioderived compound is an alcohol, a glycol, an organic acid, an alkene, a diene, an isoprenoid, an organic amine, an organic aldehyde, a vitamin, a nutraceutical or a pharmaceutical.
47. The non-natural organism of item 46, wherein said alcohol is selected from the group consisting of:
   (i) a biofuel alcohol, wherein said biofuel is a primary alcohol, a secondary alcohol, a diol or triol comprising C3 to C10 carbon atoms;
   (ii) n-propanol or isopropanol; and
   (iii) a fatty alcohol, wherein said fatty alcohol comprises C4 to C27 carbon atoms, C8 to C18 carbon atoms, C12 to C18 carbon atoms, or C12 to C14 carbon atoms.
48. The non-natural organism of item 47, wherein said biofuel alcohol is 1-propanol, isopropanol, 1-butanol, isobutanol, 1-pentanol, isopentenol, 2-methyl-1-butanol, 3-methyl-1-butanol, 1-hexanol, 3-methyl-1-pentanol, 1-heptanol, 4-methyl-1-hexanol, and 5-methyl-1-hexanol.
49. The non-natural organism of item 45, wherein said bioderived compound is selected from the group consisting of:
   (i) 1,4-butanediol or an intermediate thereto, wherein said intermediate is optionally 4-hydroxybutanoic acid (4-HB) or gamma-butryolactone;
   (ii) butadiene (1,3-butadiene) or an intermediate thereto, wherein said intermediate is optionally 1,4-butanediol, 1,3-butanediol, 2,3-butanediol, crotyl alcohol, 3-buten-2-ol (methyl vinyl carbinol), isoprene, or 3-buten-1-ol;
   (iii) 1,3-butanediol or an intermediate thereto, wherein said intermediate is optionally 3-hydroxybutyrate (3-HB), 3-hydroxy pent-4-enoate, 2,4-pentadienoate, crotyl alcohol or 3-buten-1-ol;
   (iv) adipate, 6-aminocaproic acid, caprolactam, hexamethylenediamine, levulinic acid or an intermediate thereto, wherein said intermediate is optionally adipyl-CoA or 4-aminobutyryl-CoA;
   (v) methacrylic acid or an ester thereof, 3-hydroxyisobutyrate, 2-hydroxyisobutyrate, or an intermediate thereto, wherein said ester is optionally methyl methacrylate or poly(methyl methacrylate);
   (vi) 1,2-propanediol (propylene glycol), 1,3-propanediol, glycerol, ethylene glycol, diethylene glycol, triethylene glycol, dipropylene glycol, tripropylene glycol, neopentyl glycol, bisphenol A or an intermediate thereto;
   (vii) succinic acid or an intermediate thereto;
   (viii) a fatty alcohol, a fatty aldehyde or a fatty acid comprising C4 to C27 carbon atoms, C8 to C18 carbon atoms, C12 to C18 carbon atoms, or C12 to C14 carbon atoms, wherein said fatty alcohol is optionally dodecanol (C12; lauryl alcohol), tridecyl alcohol (C13; 1-tridecanol, tridecanol, isotridecanol), myristyl alcohol (C14; 1-tetradecanol), pentadecyl alcohol (C15; 1-pentadecanol, pentadecanol), cetyl alcohol (C16; 1-hexadecanol), heptadecyl alcohol (C17; 1-n-heptadecanol, heptadecanol) and stearyl alcohol (C18; 1-octadecanol) or palmitoleyl alcohol (C16 unsaturated; cis-9-hexadecen-1-ol); and
   (ix) an isoprenoid, optionally the isoprenoid is isoprene, or an intermediate thereto.
50. The non-natural organism of item 45 comprising a 1,3-butanediol pathway.
51. The non-natural organism of item 45 comprising a crotyl alcohol pathway.
52. The non-natural organism of item 45 comprising a butadiene pathway.
53. The non-natural organism of item 45 comprising a 1,4-butanediol pathway
54. The non-natural organism of item 45 comprising an adipic acid pathway.
55. The non-natural organism of item 45 comprising a 6-aminocaproic acid pathway.
56. The non-natural organism of item 45 further comprising a caprolactam pathway
57. The non-natural organism of item 45 comprising a hexamethylenediamine pathway.
58. The non-natural organism of item 45 comprising a methacrylic acid pathway.
59. The non-natural organism of item 45 comprising a 2-hydroxyisobutyric acid pathway.
60. The non-natural organism of item 45 comprising an isoprene pathway.
61. The non-natural organism of citem 45 comprising a pathway producing 2,4-pentadienoate.
62. The non-natural organism of item 45 comprising a pathway producing methyl vinyl carbinol.
63. The non-natural organism of item 45 comprising a succinyl-CoA pathway.
64. The non-natural organism of any one of the previous items which is bacteria, fungi, or yeast.
65. The non-natural organism of item 64 that is *Escherichia, Corynebacterium, Bacillus, Pichia* or *Saccharomyces.*
66. A method for producing a bioderived compound, comprising culturing the non-natural organism of any one of items 45-65 under conditions and for a sufficient period of time to produce said bioderived compound.
67. The method of item 66, wherein said method further comprises separating the bioderived compound from other components in the culture.
68. The method of item 67, wherein the separating comprises extraction, continuous liquid-liquid extraction, pervaporation, membrane filtration, membrane separation, reverse osmosis, electrodialysis, distillation, crystallization, centrifugation, extractive filtration, ion exchange chromatography, absorption chromatography, or ultrafiltration.
69. A non-natural microbial organism capable of producing acetyl-CoA, or acetyl-CoA and oxaloacetate, the organism comprising:
   (a) a pathway comprising phosphoketolase for producing acetyl-CoA (PK pathway);
   (b) a non-phosphotransferase system (non-PTS) for sugar uptake comprising a modification to increase non-PTS activity;
   (c) a modification that attenuates or eliminates activity of pyruvate kinase, and optionally
   (d) a modification that attenuates or eliminates a PTS activity.
70. The non-natural organism of item 69 further comprising one or more modification(s) to the organism's electron transport chain to enhance efficiency of ATP production, to enhance availability of reducing equivalents, or both.
71. A non-natural microbial organism capable of producing acetyl-CoA, or acetyl-CoA and oxaloacetate, the organism comprising:
   (a) a pathway comprising phosphoketolase for producing acetyl-CoA (PK pathway);
   (b) one or more modification(s) to the organism's electron transport chain to enhance efficiency of ATP production, to enhance availability of reducing equivalents, or both; and
   (c) a modification that attenuates or eliminates activity of pyruvate kinase
72. The non-natural organism of item 70 further comprising (c) a non-phosphotransferase system (non-PTS) for sugar uptake comprising a modification to increase non-PTS activity; (d) a modification that attenuates or eliminates PTS activity, or both (c) and (d).

## Claims

1. A non-natural microbial organism capable of producing acetyl-CoA, or acetyl-CoA and oxaloacetate, the organism comprising:
(a) a pathway comprising phosphoketolase for producing acetyl-CoA (PK pathway);
(b) a non-phosphotransferase system (non-PTS) for sugar uptake comprising a modification to increase non-PTS activity;
(c) a modification that attenuates or eliminates activity of pyruvate kinase, and optionally
(d) a modification that attenuates or eliminates a PTS activity.

2. The non-natural organism of claim 1, further comprising one or more modification(s) to the organism's electron transport chain to enhance efficiency of ATP production, to enhance availability of reducing equivalents, or both.

3. A non-natural microbial organism capable of producing acetyl-CoA, or acetyl-CoA and oxaloacetate, the organism comprising:
(a) a pathway comprising phosphoketolase for producing acetyl-CoA (PK pathway);
(b) one or more modification(s) to the organism's electron transport chain to enhance efficiency of ATP production, to enhance availability of reducing equivalents, or both; and
(c) a modification that attenuates or eliminates activity of pyruvate kinase.

4. The non-natural organism of claim 1, further comprising (c) a non-phosphotransferase system (non-PTS) for sugar uptake comprising a modification to increase non-PTS activity; (d) a modification that attenuates or eliminates PTS activity, or both (c) and (d).

5. The non-natural organism of any one of the previous claims, wherein the PK pathway comprises one, two or three exogenous nucleic acids encoding a PK pathway enzyme expressed in sufficient amount to enhance production of acetyl-CoA.

6. The non-natural organism of any one of the previous claims, wherein the PK pathway comprises:
(c1) 1T and 1V; or
(c2) 1T, 1W, and 1X;
wherein 1T is a fructose-6-phosphate phosphoketolase, wherein 1V is a phosphotransacetylase, wherein 1W is an acetate kinase, and wherein 1X is an acetyl-CoA transferase, an acetyl-CoA synthetase, or an acetyl-CoA ligase.

7. The non-natural organism of any one of the previous claims, wherein the PK pathway comprises:
(c3) 1U and 1V;
(c4) 1U, 1W, and 1X;
wherein 1U is a xylulose-5-phosphate phosphoketolase, wherein 1V is a phosphotransacetylase, wherein 1W is an acetate kinase, and wherein 1X is an acetyl-CoA transferase, an acetyl-CoA synthetase, or an acetyl-CoA ligase.

8. The non-natural organism of any one of the previous claims, wherein the modification that attenuates or eliminates a PTS activity is attenuated or eliminated expression of a PTS enzyme or protein, wherein the PTS enzyme or protein is selected from the group consisting of Enzyme I (El), histidine phosphocarrier protein (HPr), Enzyme II (EII), and transmembrane Enzyme II C (EIIC).

9. The non-natural organism of any one of the previous claims, wherein the modification to increase non-PTS activity comprises increased expression or activity of a non-PTS permease, a non-PTS sugar kinase, a facilitator protein, or combinations thereof.

10. The non-natural organism of any one of the previous claims, further comprising one or more modifications to enhance synthesis of oxaloacetate, wherein the one or more modification(s) to enhance synthesis of oxaloacetate comprises increasing the expression or activity of phosphoenolpyruvate (PEP) synthase, pyruvate carboxylase, phosphoenolpyruvate carboxylase, malic enzyme, or combinations thereof.

11. The non-natural organism of any one of the previous claims, further comprising
(a) a modification that causes expression or increased expression of one or more of enzymes selected from the group consisting of ribose-5-phosphate isomerase, ribulose-5-phosphate epimerase, transaldolase, and transketolase, or
(b) attenuation of one or more endogenous enzymes selected from DHA kinase, methanol oxidase, PQQ-dependent methanol dehydrogenase, DHA synthase, or any combination thereof; or combinations of (a) - (b).

12. The non-natural organism of any one of the previous claims further comprising
(a) a formaldehyde assimilation pathway,
(b) a methanol oxidation pathway comprising at least one exogenous nucleic acid encoding a methanol oxidation pathway enzyme expressed in a sufficient amount to produce formaldehyde in the presence of methanol, wherein said methanol oxidation pathway comprises a methanol dehydrogenase, and optionally wherein the methanol oxidation pathway enzyme is methanol dehydrogenase.

13. The non-natural organism of any one of the previous claims, further comprising a pathway capable of producing a bioderived compound, wherein said bioderived compound is an alcohol, a glycol, an organic acid, an alkene, a diene, an isoprenoid, an organic amine, an organic aldehyde, a vitamin, a nutraceutical or a pharmaceutical.

14. The non-natural organism of any one of the previous claims, comprising deletion of pyruvate kinase isozyme pykF and deletion of PTS system ptsI.

15. A method for producing a bioderived compound, comprising culturing the non-natural organism of any one of claims 1-14 under conditions and for a sufficient period of time to produce said bioderived compound.
